# EUROPEAN PATENT SPECIFICATION

(11) **EP 3 526 199 B1**
(45) Date of publication and mention of the grant of the patent: **13.04.2022**
(21) Application number: 17783868.7
(22) Date of filing: 13.10.2017
(51) Int. Cl.: C07D 233/30, C07D 233/32, C07D 233/40, C07D 233/54, C07D 233/70, C07D 233/96

(54) **INHIBITORS OF ALPHA-AMINO-BETA-CARBOXYMUCONIC ACID SEMIALDEHYDE DECARBOXYLASE**
INHIBITOREN DER ALPHA-AMINO-BETA-CARBOXYMUCONSÄURE-SEMIALDEHYDDECARBOXYLASE
INHIBITEURS DE LA SEMIALDÉHYDE DÉCARBOXYLASE DE L'ACIDE ALPHA-AMINO-BÊTA-CARBOXYMUCONIQUE

(30) Priority: 14.10.2016 US 201662408258 P
(43) Date of publication of application: 21.08.2019
(73) Proprietor: Tes Pharma S.r.l., 06073 Corciano (IT)
(72) Inventor: PELLICCIARI, Roberto, 06123 Perugia (IT)
(74) Representative: Cooley (UK) LLP
(86) International application number: PCT/EP2017/076252
(87) International publication number: WO 2018/069532

(56) References cited:
- WO-A1-2016/030534
- US-A- 3 637 684
- US-A- 5 736 550
- US-A1- 2004 186 119
- Parthiv K Chaudhari ET AL: "SYNTHESIS AND BIOLOGICAL STUDIES OF TRIHYDRO PYRIDO [2, 3-D] PYRIMIDINES 6 -CARBONITRILE", , 2 October 2011 (2011-10-02), XP055432277, Retrieved from the Internet: URL:http://www.ijlpr.com/admin/php/uploads /18_pdf.pdf [retrieved on 2017-12-06]
- WAEL A. EL-SAYED ET AL: "Synthesis and In Vitro Antitumor Activity of New Substituted Thiopyrimidine Acyclic Nucleosides and Their Thioglycoside Analogs", NUCLEOSIDES, NUCLEOTIDES AND NUCLEIC ACIDS., vol. 28, no. 4, 22 May 2009 (2009-05-22), pages 261-274, XP055432300, US ISSN: 1525-7770, DOI: 10.1080/15257770902946165
- DESHMUKH M B ET AL: "A novel and efficient one step synthesis of 2-amino-5-cyano-6-hydroxy-4-aryl pyrimidines and their anti-bacterial activity", EUROPEAN JOURNAL OF MEDICINAL CHEMISTRY, vol. 44, no. 6, 1 June 2009 (2009-06-01), pages 2651-2654, XP026049367, EDITIONS SCIENTIFIQUE ELSEVIER, PARIS, FR ISSN: 0223-5234, DOI: 10.1016/J.EJMECH.2008.10.018 [retrieved on 2008-10-26]
- DATABASE CA [Online] CHEMICAL ABSTRACTS SERVICE, COLUMBUS, OHIO, US; KHAN, MD. WAHAB ET AL: "An expeditious synthesis of 6-amido-(1H,3H)-pyrimidine-2,4-diones from uracil-6-carboxylic acid", XP002776429, retrieved from STN Database accession no. 2015:1068125
- DATABASE CA [Online] CHEMICAL ABSTRACTS SERVICE, COLUMBUS, OHIO, US; RUDRAPAL, M. ET AL: "Synthesis and evaluation of heterocyclic scaffold-based styrene conjugates as new antibacterial agents", XP002776430, retrieved from STN Database accession no. 2014:1541749
- DATABASE CA [Online] CHEMICAL ABSTRACTS SERVICE, COLUMBUS, OHIO, US; SARG, MARWA TAHA MOSTAFA ET AL: "Efficient utilization of 6-aminouracil to synthesize fused and related heterocyclic compounds and their evaluation as prostate cytotoxic agents with cathepsin B inhibition", XP002776431, retrieved from STN Database accession no. 2014:1245646
- DATABASE CA [Online] CHEMICAL ABSTRACTS SERVICE, COLUMBUS, OHIO, US; LANE, J. ROBERT ET AL: "Structure-based ligand discovery targeting orthosteric and allosteric pockets of dopamine receptors", XP002776432, retrieved from STN Database accession no. 2013:1881652
- DATABASE CA [Online] CHEMICAL ABSTRACTS SERVICE, COLUMBUS, OHIO, US; LACK, NATHAN A. ET AL: "Targeting the Binding Function 3 (BF3) Site of the Human Androgen Receptor through Virtual Screening [Erratum to document cited in CA156:000564]", XP002776433, retrieved from STN Database accession no. 2011:1692287
- DATABASE CA [Online] CHEMICAL ABSTRACTS SERVICE, COLUMBUS, OHIO, US; XU, LIBIN ET AL: "Compatible Injection and Detection Systems for Studying the Kinetics of Excess Electron Transfer", XP002776434, retrieved from STN Database accession no. 2007:319545
- DATABASE CA [Online] CHEMICAL ABSTRACTS SERVICE, COLUMBUS, OHIO, US; GERHAEUSER, CLARISSA ET AL: "Synthesis of lunularic acid derivatives as chemopreventive agents", XP002776435, retrieved from STN Database accession no. 2001:747733
- DATABASE CA [Online] CHEMICAL ABSTRACTS SERVICE, COLUMBUS, OHIO, US; LAPUCHA, ANDRZEJ ET AL: "Mass spectrometry of heterocyclic analogs of stilbenes: (E)-styrylthiouracils. (E)-styryluracils and (E)-styrylthiazolo[3,2-a]pyrimidin-5-ones" , XP002776436, retrieved from STN Database accession no. 1991:491518
- DATABASE CA [Online] CHEMICAL ABSTRACTS SERVICE, COLUMBUS, OHIO, US; PAZDRO, HELENA: "Orotamides", XP002776437, retrieved from STN Database accession no. 1967:18691
- DATABASE CA [Online] CHEMICAL ABSTRACTS SERVICE, COLUMBUS, OHIO, US; KLOSA, JOSEF: "Synthesis of uracilcarboxamides", XP002776438, retrieved from STN Database accession no. 1965:15332
- Xiaowei Wang ET AL: "Design, Synthesis, and Biological Evaluation of 1-[(2-Benzyloxyl/alkoxyl)methyl]-5-halo-6- aryluracils as Potent HIV-1 Non-nucleoside Reverse Transcriptase Inhibitors with an Improved Drug Resistance Profile", JOURNAL OF MEDICINAL CHEMISTRY, vol. 55, no. 5, 17 February 2012 (2012-02-17), pages 2242-2250, XP055689455, US ISSN: 0022-2623, DOI: 10.1021/jm201506e
- R D Youssefyeh ET AL: "Photocyclodehydration of 6-o-Biphenyloxy-l,3-dimethyluracil", Journal of the American Chemical Society, vol. 96, 1 January 1974 (1974-01-01), pages 315-316, XP055689459,
- Kosaku Hirota ET AL: "Pyrimidine Derivatives and Related Compounds. Part 37 Novel Nucleophilic Substitutions of 5-Bromo-6-methyluracils or 5-Bromo-6- bromomethyluracils with Aromatic Amines", J. Chem. Soc. Perkin. Trans. I, 1 January 1981 (1981-01-01), pages 2943-2947, XP055689461,
- GEISMAN ALEXANDER N ET AL: "1,6-Bis[(benzyloxy)methyl]uracil derivatives-Novel antivirals with activity against HIV-1 and influenza H1N1 virus", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 24, no. 11, 5 April 2016 (2016-04-05) , pages 2476-2485, XP029527569, ISSN: 0968-0896, DOI: 10.1016/J.BMC.2016.04.010
- YANPING HE ET AL: "Nonnucleoside HIV-1 Reverse Transcriptase Inhibitors, Part 4[1]. Synthesis and Anti-HIV Activity of N-1-[beta]-Carbonyl-6-naphthyl-methyl Analogues of HEPT", MONATSHEFTE FÜR CHEMIE - CHEMICAL MONTHLY ; AN INTERNATIONAL JOURNAL OF CHEMISTRY, SPRINGER-VERLAG, AU, vol. 136, no. 7, 1 July 2005 (2005-07-01), pages 1233-1245, XP019378814, ISSN: 1434-4475, DOI: 10.1007/S00706-005-0325-8
- W Erian Ayman: "[beta]-Enaminoesters as Building Blocks in Heterocyclic Synthesis. A Novel Synthesis of Fused Azines by Using Blaise Reaction as a Key Step", J. Prakt. Chem., vol. 341, no. 2, 1 January 1999 (1999-01-01), pages 147-151, XP055689464,

## Description

### CROSS REFERENCE TO RELATED APPLICATIONS

This application claims the benefit of U.S. Provisional Application No. 62/408,258, filed October 14, 2016.

### FIELD OF THE DISCLOSURE

The present disclosure relates to compounds capable of modulating the activity of α-amino-β-carboxymuconic acid semialdehyde decarboxylase (ACMSD). The compounds of the disclosure may be used in methods for the prevention and/or the treatment of diseases and disorders associated with defects in NAD⁺ biosynthesis, e.g., metabolic disorders, neurodegenerative diseases, chronic inflammatory diseases, kidney diseases, and diseases associated with ageing.

### BACKGROUND OF THE DISCLOSURE

ACMSD is a critical enzyme for tryptophan metabolism, and regulates NAD⁺ biosynthesis from tryptophan. ACMSD is a zinc-dependent amidohydrolase that participates in picolinic acid (PA), quinolinic acid (QA) and NAD⁺ homeostasis. ACMSD stands at a branch point of the NAD⁺ biosynthetic pathway from tryptophan and determines the final fate of the amino acid, *i*.*e*., transformation into PA, complete oxidation through the citric acid cycle, or conversion into NAD⁺ through QA synthesis.

ACMSD has been purified from liver, kidney, and brain human tissues. There are two isoforms ACMSD1 and ACMSD2 derived from a differential splicing of *ACMSD* gene transcription but only ACMSD 1 is endowed with enzymatic activity. ACMSD 1 directs ACMS (α-amino-ω-carboxymuconic acid semialdehyde) to the acetyl-CoA pathway, and when ACMSD1 is inhibited, ACMS is non-enzymatically converted to quinolinic acid (QA) leading to the formation of NAD⁺ and an increase in the intracellular level of NAD⁺.

Increased levels of NAD⁺ have been shown to protect against neuronal degeneration, improve muscle function and oxidative metabolism in mice, and enhance lifespan in worms. Whilst reduced levels of NAD⁺ have been associated with a range of pathophysiological states including type 2 diabetes (T2D), hyperlipidemia (elevated cholesterol and TAGs), mitochondrial diseases, neutropenia, cancers, and kidney disorders.

The inhibition of ACMSD thus represents a novel approach to increase NAD⁺ levels and modify disease pathophysiologies associated with defects in NAD⁺ biosynthesis.

WO2016030534A1 discloses inhibitors of α-amino-β-carboxymuconic acid semialdehyde decarboxylase.

### SUMMARY OF THE DISCLOSURE

It is an object of embodiments of the disclosure to provide novel series of compounds capable of modulating the activity of α-amino-β-carboxymuconic acid semialdehyde decarboxylase (ACMSD or α-amino-β-carboxymuconate-ε-semialdehyde decarboxylase), which compounds are useful for the prevention and/or the treatment of diseases and disorders associated with defects in NAD⁺ biosynthesis, e.g., metabolic disorders, neurodegenerative diseases, chronic inflammatory diseases, kidney diseases, and diseases associated with ageing.

Compounds of Formula (I) or Formula (II), as defined herein, may be used in the treatment of a disease or disorder in which ACMSD plays a role. The disclosure features compounds for use in methods of treating a disease or disorder associated with ACMSD dysfunction or with abnormalities in NAD⁺ biosynthesis by administering to subjects suffering from or susceptible to developing a disease or disorder associated with ACMSD dysfunction a therapeutically effective amount of one or more compounds that increases intracellular NAD⁺ by ACMSD 1 inhibition, in an amount sufficient to activate sirtuins (SIRTs) and the downstream targets of SIRTs, such as PGC-1α, FoxO1 and/or superoxide dismutase (SOD). The methods of the present disclosure can be used in the treatment of ACMSD dependent diseases by inhibiting ACMSD. Inhibition of ACMSD may provide a novel approach to the prevention and treatment of metabolic disorders, neurodegenerative diseases, chronic inflammatory diseases, kidney diseases, diseases associated with ageing and other ACMSD dependent diseases, or diseases characterized by defective NAD⁺ synthesis.

Accordingly, a first aspect of the present disclosure relates to a compound represented by Formula (I): 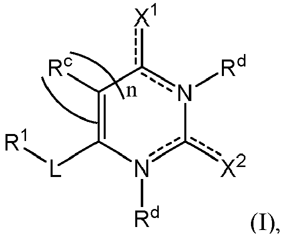 or pharmaceutically acceptable salts or tautomers thereof, wherein L, X¹, X², R¹, R^{c}, R^{d} and n are defined herein below.

Another aspect of the present disclosure relates to pharmaceutical compositions comprising a compound of Formula (I), or a pharmaceutically acceptable salt thereof, and at least one of a pharmaceutically acceptable carrier, diluent, or excipient.

In another aspect, the present disclosure relates to a compound of Formula (II): 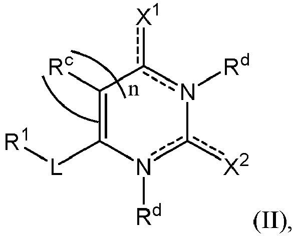 or pharmaceutically acceptable salts or tautomers thereof, wherein L, X¹, X², R¹, R^{c}, R^{d} and n are defined herein below, for use in a method of treating, preventing or reducing the risk of a disease or disorder associated with α-amino-β-carboxymuconate-ε-semialdehyde decarboxylase (ACMSD) dysfunction, the method comprising administering to a subject suffering from or susceptible to developing a disease or disorder associated with ACMSD dysfunction a therapeutically effective amount of the compound.

Another aspect of the present disclosure relates to a compound of Formula (I) or Formula (II), or a pharmaceutically acceptable salt thereof, for use in a method for treating a disease or condition associated with ACMSD dysfunction, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

In another aspect, the present disclosure relates to a compound of Formula (I) or Formula (II), or a pharmaceutically acceptable salt thereof, for use in a method for preventing a disease or condition associated with ACMSD dysfunction, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Another aspect of the present disclosure relates to a compound of Formula (I) or Formula (II), or a pharmaceutically acceptable salt thereof, for use in a method for reducing the risk of a disease or condition associated with ACMSD dysfunction, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

In another aspect, the present disclosure relates to a pharmaceutical composition for use in a method for treating, preventing, or reducing the risk of a disease or condition associated with ACMSD dysfunction, wherein the pharmaceutical composition comprises a compound of Formula (I) or a pharmaceutically acceptable salt thereof and at least one of a pharmaceutically acceptable carrier, diluent, or excipient, and wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Also disclosed (but not included in the claims) is a pharmaceutical composition for use in a method for treating, preventing, reducing the risk of, or ameliorating a disease or condition mediated by ACMSD, wherein the pharmaceutical composition comprises a compound of Formula (II), or a pharmaceutically acceptable salt thereof and at least one of a pharmaceutically acceptable carrier, diluent, or excipient.

Also disclosed (but not included in the claims) is a method for the manufacture of a medicament for treating a disease or condition mediated by ACMSD, wherein the medicament comprises a compound of Formula (I) or Formula (II), or a pharmaceutically acceptable salt thereof.

Also disclosed (but not included in the claims) is a method for the manufacture of a medicament for preventing a disease or condition mediated by ACMSD, wherein the medicament comprises a compound of Formula (I) or Formula (II), or a pharmaceutically acceptable salt thereof.

Also disclosed (but not included in the claims) is a method for the manufacture of a medicament for reducing the risk of a disease or condition mediated by ACMSD, wherein the medicament comprises a compound of Formula (I) or Formula (II), or a pharmaceutically acceptable salt thereof.

Also disclosed (but not included in the claims) is a method for the manufacture of a medicament for ameliorating a disease or condition mediated by ACMSD, wherein the medicament comprises a compound of Formula (I) or Formula (II), or a pharmaceutically acceptable salt thereof.

Also disclosed (but not included in the claims) is a method for the manufacture of a medicament for treating a disease or condition mediated by ACMSD, wherein the medicament comprises a pharmaceutical composition comprising one or more compounds of Formula (I) or Formula (II), or a pharmaceutically acceptable salt thereof, and at least one of a pharmaceutically acceptable carrier, diluent, or excipient.

Also disclosed (but not included in the claims) is a method for the manufacture of a medicament for preventing a disease or condition mediated by ACMSD, wherein the medicament comprises a pharmaceutical composition comprising one or more compounds of Formula (I) or Formula (II), or a pharmaceutically acceptable salt thereof, and at least one of a pharmaceutically acceptable carrier, diluent, or excipient.

Also disclosed (but not included in the claims) is a method for the manufacture of a medicament for reducing the risk of a disease or condition mediated by ACMSD, wherein the medicament comprises a pharmaceutical composition comprising one or more compounds of Formula (I) or Formula (II), or a pharmaceutically acceptable salt thereof, and at least one of a pharmaceutically acceptable carrier, diluent, or excipient.

Also disclosed (but not included in the claims) is a method for the manufacture of a medicament for ameliorating a disease or condition mediated by ACMSD, wherein the medicament comprises a pharmaceutical composition comprising one or more compounds of Formula (I) or Formula (II), or a pharmaceutically acceptable salt thereof, and at least one of a pharmaceutically acceptable carrier, diluent, or excipient.

In another aspect, the present disclosure relates to a compound of Formula (I), or a pharmaceutically acceptable salt thereof, for use in a method of treating, preventing or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Another aspect of the present disclosure relates to a pharmaceutical composition comprising a compound of Formula (I), or a pharmaceutically acceptable salt thereof, and at least one of a pharmaceutically acceptable carrier, diluent, or excipient, for use in a method of treating, preventing or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

In another aspect, the present disclosure relates to a compound of Formula (I), or a pharmaceutically acceptable salt thereof, for use in a method of treating, preventing or reducing the risk of a disorder associated with mitochondrial dysfunction, the method comprising administering to the subject suffering from or susceptible to developing a metabolic disorder a therapeutically effective amount of the compound, wherein said disorder associated with mitochondrial dysfunction is an inherited mitochondrial disease, a common metabolic disorder, a neurodegenerative disease, an aging related disorder, a kidney disorder, or a chronic inflammatory disease.

Another aspect of the present disclosure relates to a pharmaceutical composition comprising a compound of Formula (I), or a pharmaceutically acceptable salt thereof, and at least one of a pharmaceutically acceptable carrier, diluent, or excipient, for use in a method of treating, preventing or reducing the risk of a disorder associated with mitochondrial dysfunction, the method comprising administering to the subject suffering from or susceptible to developing a metabolic disorder a therapeutically effective amount of the compound, wherein said disorder associated with mitochondrial dysfunction is an inherited mitochondrial disease, a common metabolic disorder, a neurodegenerative disease, an aging related disorder, a kidney disorder, or a chronic inflammatory disease.

Also disclosed (but not included in the claims) is a compound of Formula (I) or Formula (II), or a pharmaceutically acceptable salt thereof for use in promoting oxidative metabolism.

Also disclosed (but not included in the claims) is a pharmaceutical composition comprising a compound of Formula (I) or Formula (II), or a pharmaceutically acceptable salt thereof, and at least one of a pharmaceutically acceptable carrier, diluent, or excipient, for use in promoting oxidative metabolism.

In certain aspects, the ACMSD modulating compounds may be administered alone or in combination with other compounds, including other ACMSD modulating compounds, or other therapeutic agents.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar to or equivalent to those described herein can be used in the practice and testing of the disclosure, suitable methods and materials are described below. The references cited herein are not admitted to be prior art to the claimed disclosure. In the case of conflict, the present specification, including definitions, will control. In addition, the materials, methods, and examples are illustrative only and not intended to be limiting.

Other features and advantages of the disclosure will be apparent from the following detailed description and claims.

### DETAILED DESCRIPTION OF THE DISCLOSURE

### Compounds of Formula (I)

The present disclosure relates to compounds of Formula (I): 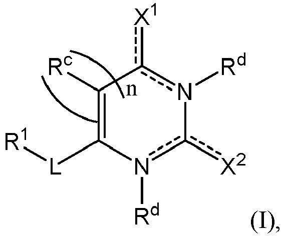 and pharmaceutically acceptable salts or tautomers thereof, wherein:
X¹ is O, S, OR² or SH;
X² is O, S, OR² or SR²;
L is -(CH₂)ₘCH=CH(CH₂)ₚ-, -(CH₂)*ₒ-*, 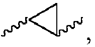 -(CH₂)ₘC=(O)NR³(CH₂)ₚ-,-(CH₂)ₘNR³C=(O)(CH₂)ₚ-, pyridinyl, or thiophenyl;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, Y²(C(R^{f})₂)ᵣCO₂R^{x}, -O(C(R^{f})₂)ᵣC(O)NHR^{g}, - (C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R)₂)heterocycloalkyl, -O(C(R^{f})₂)ᵣ(C₃-C₇)cycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, - (C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- or 6-membered heteroaryl ring optionally substituted with one or more -CO₂H;
R^{c} is-CN;
each R^{d} is independently at each occurrence absent, H, or methyl;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In one embodiment, the compound of Formula (I) is a compound of Formula (Ia), (Ib), (Ic), or (Id): 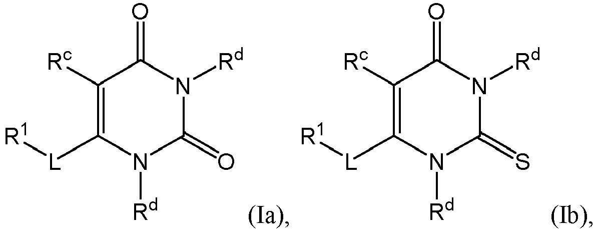 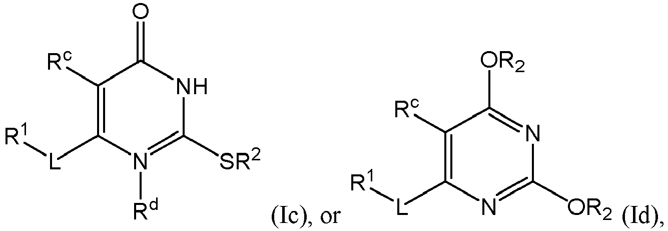 and pharmaceutically acceptable salts or tautomers thereof.

In another embodiment, the compound of Formula (I) is a compound of Formula (Ie), (If), (Ig), or (Ih): 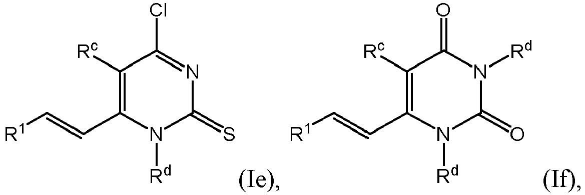 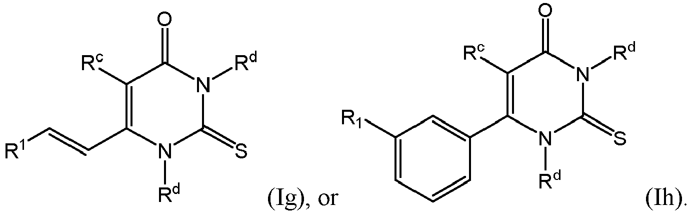 and pharmaceutically acceptable salts or tautomers thereof.

In another embodiment, the compound of Formula (I) is a compound of Formula (Ii) or (Ij): 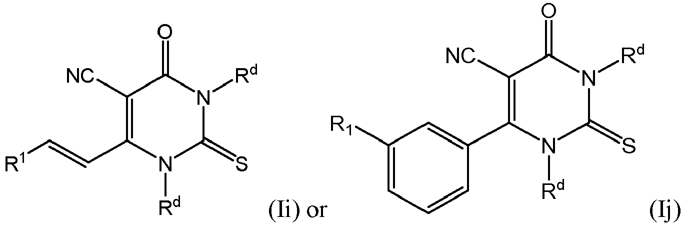 and pharmaceutically acceptable salts or tautomers thereof.

In some embodiments of the Formula (I), (Ia), (Ib), (Ic), (Id),(Ie), (If), (Ig), (Ih), (Ii), and (Ij),
X¹ is O or OR²;
X² is S or OR²;
L is -(CH₂)ₘCH=CH(CH₂)ₚ-, -(CH₂)*ₒ*-, 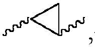 -(CH₂)ₘC=(O)NR³(CH₂)ₚ- or -(CH₂)ₘNR³C=(O)(CH₂)p-;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, or - CH=CHCO₂R^{x}, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- or 6-membered heteroaryl ring optionally substituted with one or more -CO₂H;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of the Formula (I), (Ia), (Ib), (Ic), (Id), (Ie), (If), (Ie), (If), (Ig) (Ih), (Ii), and (Ij),
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, or -(C(R^{f})₂)ᵣC₆-C₁₀ aryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of the Formula (I), (Ia), (Ib), (Ic), (Id), (Ie), (If), (Ie), (If), (Ig) (Ih), (Ii), and (Ij),
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, -O(C(R^{f})₂)ᵣheteroaryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, -(C(R^{f})₂)ᵣC₆-C₁₀ aryl, or -O(C(R^{f})₂)ᵣheteroaryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond

In some embodiments of the Formula (I), (Ia), (Ib), (Ic), (Id), (Ie), (If), (Ie), (If), (Ig) (Ih), (Ii), and (Ij), X¹ is O. In another embodiment, X¹ is S. In yet another embodiment, X¹ is OR². In another embodiment, X¹ is SH. In another embodiment, X¹ is O or S. In yet another embodiment, X¹ is O or OR². In yet another embodiment, X¹ is O or OCH₃.

In some embodiments of the Formula (I), (Ia), (Ib), (Ic), (Id), (Ie), (If), (Ie), (If), (Ig) (Ih), (Ii), and (Ij), X² is O. In another embodiment, X² is S. In yet another embodiment, X² is OR². In another embodiment, X² is SR². In another embodiment, X² is O or S. In yet another embodiment, X² is O, S, or SR². In another embodiment, X² is O, S, or SCH₃. In another embodiment, X² is S or OCH₃.

In some embodiments of the Formula (I), (Ia), (Ib), (Ic), (Id), (Ie), (If), (Ie), (If), (Ig) (Ih), (Ii), and (Ij), L is -(CH₂)ₘCH=CH(CH₂)ₚ-, -(CH₂)*ₒ*-, or 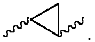 In another embodiment, L is
-(CH₂)ₘC=(O)(CH₂)ₚ-, -(CH₂)ₘC=(O)NR³(CH₂)ₚ-, or
-(CH₂)ₘR³C=(O)(CH₂)ₚ-. In yet another embodiment, L is -(CH₂)ₘCH=CH(CH₂)ₚ- or 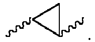 In another embodiment, L is -(CH₂)*ₒ*-. In yet another embodiment, L is - (CH₂)ₘCH=CH(CH₂)ₚ-, -(CH₂)*ₒ*-, 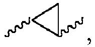 -(CH₂)ₘC=(O)NR³(CH₂)ₚ-, or - (CH₂)ₘNR³C=(O)(CH₂)ₚ-. In another embodiment, L is -CH=CH-, -(CH₂)*ₒ*-, 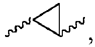 C=(O)NR³-, or -NR³C=(O)-. In another embodiment, L is -(CH₂)ₘCH=CH(CH₂)ₚ-. In another embodiment, L is -(CH₂)ₘCH=CH(CH₂)ₚ-,pyridinyl, or thiophenyl. In another embodiment, L is pyridinyl, or thiophenyl. In yet another embodiment, L is -CH=CH-.

In some embodiments of the Formula (I), (Ia), (Ib), (Ic), (Id),(Ie), (If), (Ig), (Ih), (Ii), and (Ij), Y² is O. In another embodiment, Y² is NH. In yet another embodiment, Y² is S. In another embodiment, Y² is O or NH. In yet another embodiment, Y² is O or S. In another embodiment, Y² is NH or S.

In some embodiments of the Formula (I), (Ia), (Ib), (Ic), (Id), (Ie), (If), (Ie), (If), (Ig) (Ih), (Ii), and (Ij), R¹ is C₆-C₁₀ aryl substituted with R^{a} and R^{b}, and optionally substituted with one to two R^{e}. In another embodiment, R¹ is heteroaryl comprising one or two 5- to 7-membered rings and 1-4 heteroatoms selected from N, O and S, substituted with R^{a} and R^{b}, and optionally substituted with one to two R^{e}. In another embodiment, R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}. In another embodiment, R¹ is C₆-C₁₀ aryl or heteroaryl comprising one 5- to 7-membered rings and 1-4 heteroatoms selected from N, O and S, wherein the aryl and heteroaryl are substituted with R^{a} and R^{b}. In another embodiment, R¹ is C₆-C₁₀ aryl or heteroaryl comprising two 5- to 7-membered rings and 1-4 heteroatoms selected from N, O and S, wherein the aryl and heteroaryl are substituted with R^{a} and R^{b}.

In another embodiment, R¹ is C₆-C₁₀ aryl substituted with R^{a} and R^{b}. In yet another embodiment, R¹ is heteroaryl comprising one or two 5- to 7-membered rings and 1-4 heteroatoms selected from N, O and S, substituted with R^{a} and R^{b}. In yet another embodiment, R¹ is heteroaryl comprising one 5- to 7-membered rings and 1-4 heteroatoms selected from N, O and S, substituted with R^{a} and R^{b}. In yet another embodiment, R¹ is heteroaryl comprising two 5- to 7-membered rings and 1-4 heteroatoms selected from N, O and S, substituted with R^{a} and R^{b}. In another embodiment, R¹ is phenyl, pyridinyl, or thiophenyl, wherein each is substituted with R^{a} and R^{b}, and optionally substituted with one to two R^{e}. In another embodiment, R¹ is phenyl or pyridinyl, wherein each is substituted with R^{a} and R^{b}, and optionally substituted with one to two R^{e}. In another embodiment, R¹ is phenyl, pyridinyl, or thiophenyl, wherein each is substituted with R^{a} and R^{b}. In yet another embodiment, R¹ is phenyl or pyridinyl, wherein each is substituted with R^{a} and R^{b}. In yet another embodiment, R¹ is phenyl substituted with R^{a} and R^{b}.

In some embodiments of the Formula (I), (Ia), (Ib), (Ic), (Id),(Ie), (If), (Ig), (Ih), (Ii), and (Ij), R² is H. In another embodiment, R² is C₁-C₄ alkyl. In yet another embodiment, R² is H or C₁-C₂ alkyl. In another embodiment, R² is H, methyl, or ethyl. In another embodiment, R² is H or methyl.

In some embodiments of the Formula (I), (Ia), (Ib), (Ic), (Id), (Ie), (If), (Ie), (If), (Ig) (Ih), (Ii), and (Ij), R³ is H. In another embodiment, R³ is C₁-C₄ alkyl. In yet another embodiment, R³ is H or C₁-C₂ alkyl. In another embodiment, R³ is H, methyl, or ethyl. In another embodiment, R³ is H or methyl.

In some embodiments of the Formula (I), (Ia), (Ib), (Ic), (Id), (Ie), (If), (Ie), (If), (Ig) (Ih), (Ii), and (Ij), R^{a} is H, -(C(R^{f})₂)ᵣCO₂R^{x},-Y²(C(R^{f})₂)ᵣCO₂R^{x}, -O(C(R^{f})₂)ᵣC(O)NHR^{g}, - (C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)heterocycloalkyl, -(C(R^{f})₂)ᵣS(O)₂OH, -(C(R^{f})₂)P(O)₂OH, -O(C(R^{f})₂)ᵣOH, -OR^{y}, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(W)₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{a} is H, -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -OH, -O-CH₂CH₂OH, -OCH₃, -OCH₂CH₃, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

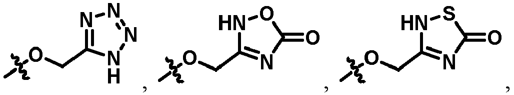

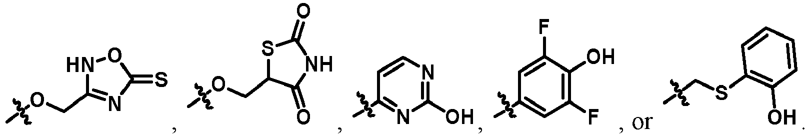

In some embodiments of the Formula (I), (Ia), (Ib), (Ic), (Id),(Ie), (If), (Ig), (Ih), (Ii), and (Ij), R^{a} is H or -OR^{y}, or R^{a} and R^{b} when on adjacent atoms together with the atoms to which they are attached form a 5- to 6-membered heteroaryl ring optionally substituted with one or more -CO₂H. In another embodiment, R^{a} is H or -OR^{y}. In another embodiment, R^{a} is H, -OCH₃, -OCH₂CH₃, or -OH. In another embodiment, R^{a} is H, -OCH₃, or -OCH₂CH₃. In another embodiment, R^{a} is H, -OCH₃, or -OCH₂CH₃.

In some embodiments of the Formula (I), (Ia), (Ib), (Ic), (Id),(Ie), (If), (Ig), (Ih), (Ii), and (Ij), R^{a} is -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(W)₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{a} is - (C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{a} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -OH, -O-CH₂CH₂OH, -OCH₃, -OCH₂CH₃, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃, 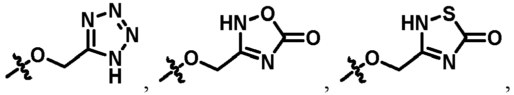 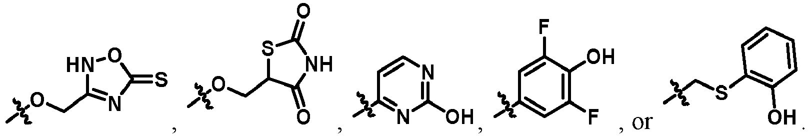 In another embodiment, R^{a} is -(CH₂)CO₂H, -O(CH₂)CO₂H, -O(CH₂CH₂)CO₂H, -S(CH₂)CO₂H, -NH(CH₂)CO₂H, O(CH₂)C(O)NH₂, -O(CH₂)C(O)NS(O)₂N(CH₃)₂, -O(CH₂)C(O)NHOH, -O(CH₂)C(O)NS(O)₂N(CH₃)₂,-CH=CHCO₂H, -O(CH₂CH₂)P(O)₂OH, -O(CH₂CH₂)S(O)₂OH, -O-CH₂CH₂OH,

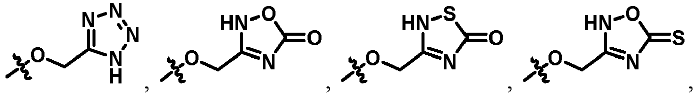

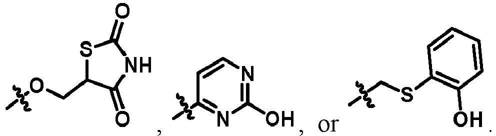

In another embodiment, R^{a} is -CO₂H, -O(CH₂)CO₂H, -C(CH₂)₂CO₂H, -CH(CH₂)CO₂H, -O(CH₂)CO₂CH₂CH₃, -OH, -OCH₃, 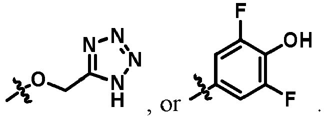 In another embodiment, R^{a} is -CO₂H, -(CH₂)CO₂H, -O(CH₂)CO₂H, -C(CH₂)₂CO₂H, -CH(CH₂)CO₂H, -O(CH₂)CO₂CH₂CH₃, -OH, -OCH₃, or 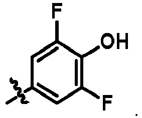 In another embodiment, R^{a} is -(CH₂)CO₂H, -O(CH₂)CO₂H, -C(CH₂)₂CO₂H, -CH(CH₂)CO₂H, OCH₃, or

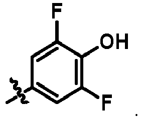

In some embodiments of the Formula (I), (Ia), (Ib), (Ic), (Id),(Ie), (If), (Ig), (Ih), (Ii), and (Ij), R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, or -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, or - CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

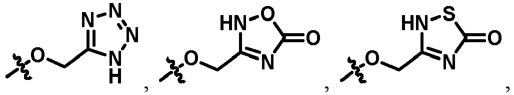

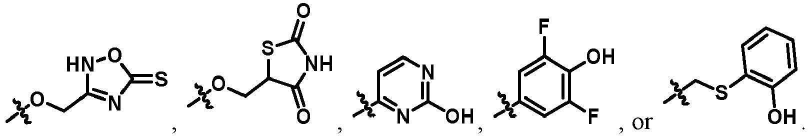

In some embodiments of the Formula (I), (Ia), (Ib), (Ic), (Id),(Ie), (If), (Ig), (Ih), (Ii), and (Ij), R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{b} is - (C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃, 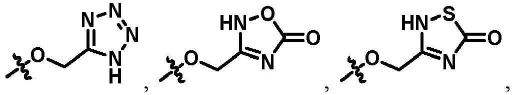 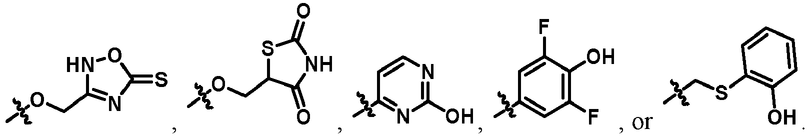 In another embodiment, R^{b} is -(CH₂)CO₂H, -O(CH₂)CO₂H, -O(CH₂CH₂)CO₂H, -S(CH₂)CO₂H, -NH(CH₂)CO₂H, O(CH₂)C(O)NH₂, -O(CH₂)C(O)NS(O)₂N(CH₃)₂, -O(CH₂)C(O)NHOH, - O(CH₂)C(O)NS(O)₂N(CH₃)₂,-CH=CHCO₂H, -O(CH₂CH₂)P(O)₂OH, -O(CH₂CH₂)S(O)₂OH, - CH₂CH₂OH,

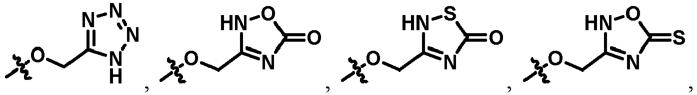

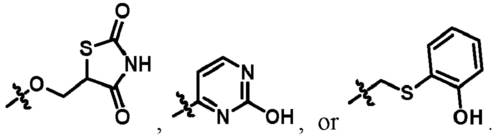

In another embodiment, R^{b} is -CO₂H, -O(CH₂)CO₂H, -C(CH₂)₂CO₂H, -CH(CH₂)CO₂H, -O(CH₂)CO₂CH₂CH₃, 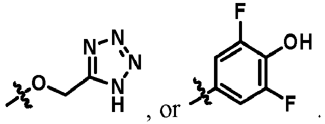 In another embodiment, R^{b} is -CO₂H, -(CH₂)CO₂H, -O(CH₂)CO₂H, -C(CH₂)₂CO₂H, -CH(CH₂)CO₂H, -O(CH₂)CO₂CH₂CH₃, or 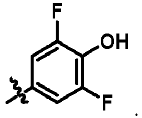 In another embodiment, R^{b} is - (CH₂)CO₂H, -O(CH₂)CO₂H, -C(CH₂)₂CO₂H, -CH(CH₂)CO₂H, or

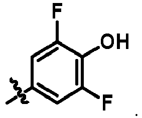

In some embodiments of the Formula (I), (Ia), (Ib), (Ic), (Id), (Ie), (If), (Ie), (If), (Ig) (Ih), (Ii), and (Ij), R^{a} and R^{b} when on adjacent atoms together with the atoms to which they are attached form a C₆-C₁₀ aryl ring optionally substituted with one or more -CO₂H. In another embodiment, R^{a} and R^{b} when on adjacent atoms together with the atoms to which they are attached form a 5- to 6-membered heteroaryl ring optionally substituted with one or more -CO₂H. In another embodiment, R^{a} and R^{b} when on adjacent atoms together with the atoms to which they are attached form a 5- or 6-membered heteroaryl ring optionally substituted with one to two -CO₂H. In another embodiment, R^{a} and R^{b} when on adjacent atoms together with the atoms to which they are attached form a 5-membered heteroaryl ring optionally substituted with one or more -CO₂H. In another embodiment, R^{a} and R^{b} when on adjacent atoms together with the atoms to which they are attached form a furan ring optionally substituted with one or more -CO₂H.

In the Formulas (I), (Ia), (Ib), (Ic), (Id), (Ie), (If), (Ie), (If), (Ig) (Ih), (Ii), and (Ij), R^{c} is -CN.

In some embodiments of the Formula (I), (Ia), (Ib), (Ic), (Id), (Ie), (If), (Ie), (If), (Ig) (Ih), (Ii), and (Ij), each R^{d} is independently at each occurrence absent or H. In another embodiment, each R^{d} is independently at each occurrence H.

In some embodiments of the Formula (I), (Ia), (Ib), (Ic), (Id), (Ie), (If), (Ie), (If), (Ig) (Ih), (Ii), and (Ij), each R^{e} is independently at each occurrence C₁-C₃ alkyl, C₂-C₃ alkenyl, or C₂-C₃ alkynyl, halogen, C₁-C₃ haloalkyl, -NHR^{z}, -OH, or -CN. In another embodiment, each R^{e} is independently at each occurrence C₁-C₆ alkyl, C₂-C₆ alkenyl, or C₂-C₆ alkynyl. In another embodiment, each R^{e} is independently at each occurrence halogen, C₁-C₆ haloalkyl, - NHR^{z}, -OH, or -CN. In another embodiment, each R^{e} is independently at each occurrence C₁-C₆ alkyl, C₂-C₆ alkenyl, or C₂-C₆ alkynyl halogen, or C₁-C₆ haloalkyl. In another embodiment, each R^{e} is independently at each occurrence halogen, -NHR^{z}, -OH, or -CN. In another embodiment, each R^{e} is independently at each occurrence halogen or -OH.

In some embodiments of the Formula (I), (Ia), (Ib), (Ic), (Id), (Ie), (If), (Ie), (If), (Ig) (Ih), (Ii), and (Ij), each R^{f} is independently H or C₁-C₃ alkyl. In another embodiment, each R^{f} is C₁-C₄ alkyl. In yet another embodiment, each R^{f} is H or C₁-C₂ alkyl. In another embodiment, each R^{f} is H, methyl, or ethyl. In yet another embodiment, each R^{f} is H or methyl. In another embodiment, each R^{f} is independently each H.

In some embodiments of the Formula (I), (Ia), (Ib), (Ic), (Id), (Ie), (If), (Ie), (If), (Ig) (Ih), (Ii), and (Ij), R^{g} is C₁-C₃ alkyl or OH. In another embodiment, R^{g} is -S(O)₂(C₁-C₃ alkyl), or S(O)₂N(C₁-C₃ alkyl)₂. In another embodiment, R^{g} is H or C₁-C₃ alkyl. In another embodiment, R^{g} is OH, -S(O)₂(C₁-C₃ alkyl), or -S(O)₂N(C₁-C₃ alkyl)₂. In another embodiment, R^{g} is H, C₁-C₃ alkyl, OH, -S(O)₂(C₁-C₃ alkyl), or -S(O)₂N(C₁-C₃ alkyl)₂. In another embodiment, R^{g} is H, C₁-C₂ alkyl, OH, -S(O)₂(C₁-C₂ alkyl), or -S(O)₂N(C₁-C₂ alkyl)₂. In another embodiment, R^{g} is H, methyl, OH, -S(O)₂CH₃, or -S(O)₂N(CH₃)₂.

In some embodiments of the Formula (I), (Ia), (Ib), (Ic), (Id), (Ie), (If), (Ie), (If), (Ig) (Ih), (Ii), and (Ij), R^{x} is H or C₁-C₃ alkyl. In another embodiment, R^{x} is C₁-C₄ alkyl. In yet another embodiment, R^{x} is H or C₁-C₂ alkyl. In another embodiment, R^{x} is H, methyl, or ethyl. In yet another embodiment, R^{x} is H or methyl. In another embodiment, R^{x} is H.

In some embodiments of the Formula (I), (Ia), (Ib), (Ic), (Id), (Ie), (If), (Ie), (If), (Ig) (Ih), (Ii), and (Ij), m is 0. In another embodiment, m is 1. In yet another embodiment, m is 2. In another embodiment, m is 0 or 1. In yet another embodiment, m is 1 or 2.

In some embodiments of the Formula (I), (Ia), (Ib), (Ic), (Id), (Ie), (If), (Ie), (If), (Ig) (Ih), (Ii), and (Ij), p is 0. In another embodiment, p is 1. In yet another embodiment, p is 2. In another embodiment, p is 0 or 1. In yet another embodiment, p is 1 or 2.

In some embodiments of the Formula (I), (Ia), (Ib), (Ic), (Id), (Ie), (If), (Ie), (If), (Ig) (Ih), (Ii), and (Ij), r is 0. In another embodiment, r is 1. In yet another embodiment, r is 2. In another embodiment, r is 0 or 1. In yet another embodiment, r is 1 or 2.

In the Formulas (I), (Ia), (Ib), (Ic), (Id), (Ie), (If), (Ie), (If), (Ig) (Ih), (Ii), and (Ij), n is 1.

In some embodiments of the Formula (I), (Ia), (Ib), (Ic), (Id), (Ie), (If), (Ie), (If), (Ig) (Ih), (Ii), and (Ij), o is 1. In another embodiment, o is 2. In another embodiment, o is 3. In another embodiment, o is 4. In another embodiment, o is 1, 2, or 3. In another embodiment, o is 1 or 2. In another embodiment, o is 2 or 3. In another embodiment, o is 3 or 4.

In some embodiments of the Formula (I), (Ia), (Ib), (Ic), (Id), (Ie), (If), (Ie), (If), (Ig) (Ih), (Ii), and (Ij), the dotted line is a double bond. In other embodiments, the dotted line is a absent. In some embodiments, 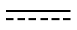 is a double bond. In some embodiments, 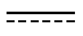 is a single bond.

In some embodiments of the Formula (I), (Ia), (Ib), (Ic), (Id), (Ie), (If), (Ie), (If), (Ig) (Ih), (Ii), and (Ij), R¹ is C₆-C₁₀ aryl. In another embodiment, R¹ is phenyl.

In some embodiments of the Formula (I), (Ia), (Ib), (Ic), (Id), (Ie), (If), (Ie), (If), (Ig) (Ih), (Ii), and (Ij), R¹ is heteroaryl. In another embodiment, R¹ is pyridinyl or thiophenyl. In yet another embodiment, R¹ is thiophenyl. In another embodiment, R¹ is pyridinyl.

In some embodiments of the Formula (I), (Ia), (Ib), (Ic), (Id), (Ie), (If), (Ie), (If), (Ig) (Ih), (Ii), and (Ij), R^{a} is H and R^{b} is -(C(R^{f})₂)ᵣCO₂R^{x}, -O(C(R^{f})₂)ᵣCO₂R^{x}, or -(C(R^{f})₂)ᵣC₆-C₁₀ aryl. In another embodiment, R^{a} is H and R^{b} is -CO₂H, -CH₂CO₂H, -OCH₂CO₂R^{x}, -OCH(CH₃)CO₂R^{x},-OC(CH₃)₂CO₂R, or

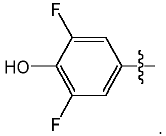

In some embodiments of the Formula (I), (Ia), (Ib), (Ic), (Id), (Ie), (If), (Ie), (If), (Ig) (Ih), (Ii), and (Ij), R^{a} is OR^{y} and R^{b} is -O(C(R^{f})₂)ᵣCO₂R^{x}, or -O(C(R^{f})₂)ᵣheteroaryl. In another embodiment, R^{a} is H and R^{b} is -(C(R^{f})₂)ᵣCO₂R^{x}, -Y²(C(R^{f})₂)ᵣCO₂R^{x}, -(C(R^{f})₂)ᵣS-C₆-C₁₀ aryl, -(C(R^{f})₂)ᵣheteroaryl, -(C(R^{f})₂)ᵣP(O)₂OH, -(C(R^{f})₂)ᵣS(O)₂OH, or-CH=CHCO₂R^{x}, wherein the aryl and heteroaryl are substituted with one to three substituents selected from halogen and OH; or R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H.

In some embodiments of the Formula (I), (Ia), (Ib), (Ic), (Id), (Ie), (If), (Ie), (If), (Ig) (Ih), (Ii), and (Ij), R^{a} is H, -CH₃, -OCH₃,-OH, -OCH₂CH₃, -OCH₂CH₂CH₃, -OCH₂CH(CH₃)₂, -O-cyclopropyl, -O-pyrrolidinyl, or -OCH₂cyclopropyl, or R^{a} and R^{b} when on adjacent atoms together with the atoms to which they are attached form a 5- to 6-membered heteroaryl ring optionally substituted with one or more -CO₂H; and R^{b} is -CH₃, -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, - O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

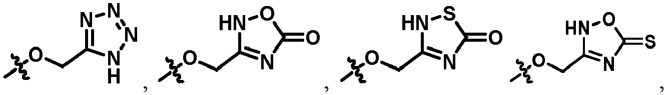

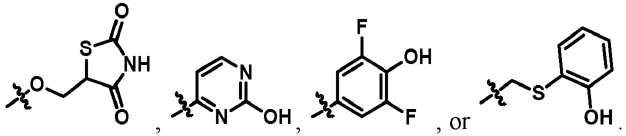

In some embodiments of the Formula (I), (Ia), (Ib), (Ic), (Id), (Ie), (If), (Ie), (If), (Ig) (Ih), (Ii), and (Ij), R^{a} is H, -OCH₃, -OH, or -OCH₂CH₃, or R^{a} and R^{b} when on adjacent atoms together with the atoms to which they are attached form a 5- to 6-membered heteroaryl ring optionally substituted with one or more -CO₂H; and R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -- O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

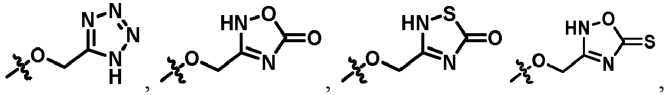

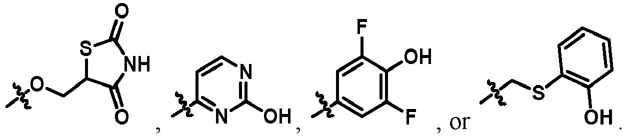

In some embodiments of the Formula (I), (Ia), (Ib), (Ic), (Id), (Ie), (If), (Ie), (If), (Ig) (Ih), (Ii), and (Ij), R^{a} is H, -OCH₃, or -OCH₂CH₃; and R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, - O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

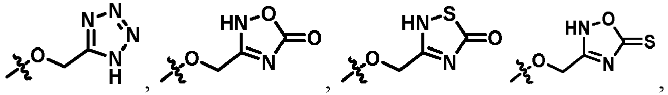

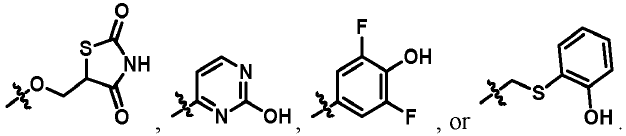

In some embodiments of the Formula (I), (Ia), (Ib), (Ic), (Id), (Ie), (If), (Ie), (If), (Ig) (Ih), (Ii), and (Ij), R^{a} is H or -OCH₃; and R^{b} is -CO₂H, -(CH₂)CO₂H, -O(CH₂)CO₂H, -S(CH₂)CO₂H, -NH(CH₂)CO₂H, -O(CH₂CH₂)CO2H, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

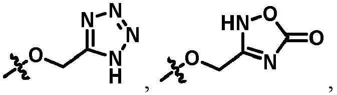

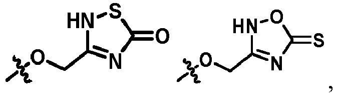

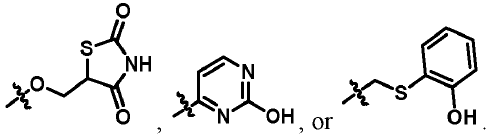

In some embodiments of the Formula (I), (Ia), (Ib), (Ic), (Id), (Ie), (If), (Ie), (If), (Ig) (Ih), (Ii), and (Ij), R^{a} is H, -OCH₃, or -OCH₂CH₃; and R^{b} is -CO₂H, -(CH₂)CO₂H, -O(CH₂)CO₂H, -C(CH₂)₂CO₂H, -CH(CH₂)CO₂H,-OH, or

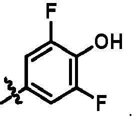

In some embodiments of the Formula (I), (Ia), (Ib), (Ic), (Id), (Ie), (If), (Ie), (If), (Ig) (Ih), (Ii), and (Ij), R^{a} is H, -OH, -OCH₃, or -OCH₂CH₃; and R^{b} is -CO₂H, -(CH₂)CO₂H,-O(CH₂)CO₂H, -C(CH₂)₂CO₂H, -CH(CH₂)CO₂H, -O(CH₂)CO₂CH₂CH₃,

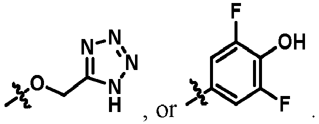

In some embodiments of the Formula (I), (Ia), (Ib), (Ic), (Id), (Ie), (If), (Ie), (If), (Ig) (Ih), (Ii), and (Ij), R^{a} is H or -OCH₃; and R^{b} is -O(CH₂)CO₂H, -C(CH₂)₂CO₂H, -CH(CH₂)CO₂H, or

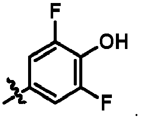

In some embodiments of the Formula (I), (Ia), (Ib), (Ic), (Id), (Ie), (If), (Ie), (If), (Ig) (Ih), (Ii), and (Ij), one of R^{a} or R^{b} is a carboxylic acid or a carboxylic acid bioisostere.

In some embodiments of the Formula (I), (Ia), (Ib), (Ic), (Id), (Ie), (If), (Ie), (If), (Ig) (Ih), (Ii), and (Ij), R^{a} is -CO₂H, -(CH₂)CO₂H, or -OCH₂CO₂H. In other embodiments, R^{a} is -CO₂CH₃, -CO₂CH2CH₃, -CO₂CH₂CH₂CH₃, -CO₂CH(CH₃)₂, -(CH₂)CO₂CH₃, -(CH₂)CO₂CH₂CH₃, -(CH₂)CO₂CH₂CH₂CH₃, or -(CH₂)CO₂CH(CH₃)₂.

In some embodiments of the Formula (I), (Ia), (Ib), (Ic), (Id), (Ie), (If), (Ie), (If), (Ig) (Ih), (Ii), and (Ij), R^{a} is -P(O)(OH)OH, -(CH₂)P(O)(OH)OH, -P(O)(OH)OCH₃, -P(O)(OH)OCH₂CH₃, -P(O)(OH)OCH₂CH₂CH₃, -P(O)(OH)OCH(CH₃)₂, -(CH₂) P(O)(OH)OCH₃, -(CH₂)P(O)(OH)OCH₂CH₃, -(CH₂)P(O)(OH)OCH₂CH₂CH₃, or -(CH₂)P(O)(OH)OCH(CH₃)₂.

In some embodiments of the Formula (I), (Ia), (Ib), (Ic), (Id), (Ie), (If), (Ie), (If), (Ig) (Ih), (Ii), and (Ij), R^{a} is -S(O)₂OH, -(CH₂)S(O)₂OH, -C(O)NHCN, or -(CH₂)C(O)NHCN.

In some embodiments of the Formula (I), (Ia), (Ib), (Ic), (Id), (Ie), (If), (Ie), (If), (Ig) (Ih), (Ii), and (Ij), R^{a} is -C(O)NHS(O)₂CH₃, -C(O)NHS(O)₂CH₂CH₃, -C(O)NHS(O)₂CH₂CH₂CH₃, -C(O)NHS(O)₂CH(CH₃)₂, -(CH₂)C(O)NHS(O)₂CH₃,-(CH₂)C(O)NHS(O)₂CH₂CH₃,-(CH₂)C(O)NHS(O)₂CH₂CH₂CH₃, or -(CH₂)C(O)NHS(O)₂CH(CH₃)₂.

In some embodiments of the Formula (I), (Ia), (Ib), (Ic), (Id), (Ie), (If), (Ie), (If), (Ig) (Ih), (Ii), and (Ij), R^{a} is 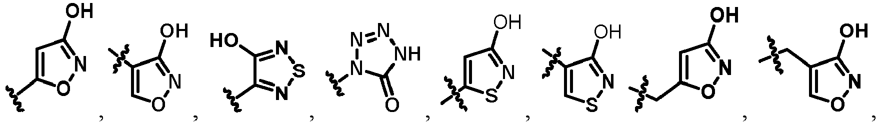 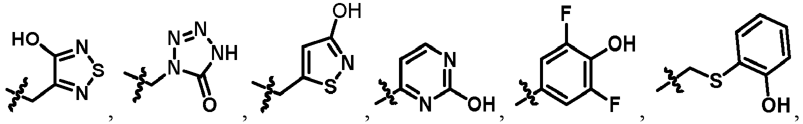 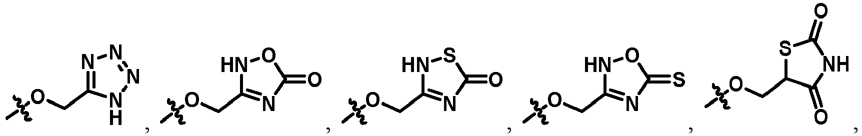 or

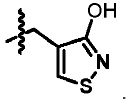

In some embodiments of the Formula (I), (Ia), (Ib), (Ic), (Id), (Ie), (If), (Ie), (If), (Ig) (Ih), (Ii), and (Ij), R^{a} is

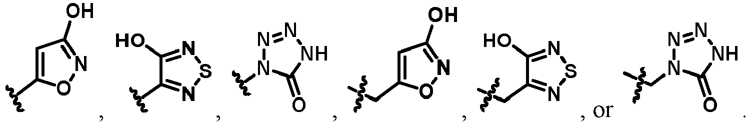

In some embodiments of the Formula (I), (Ia), (Ib), (Ic), (Id), (Ie), (If), (Ie), (If), (Ig) (Ih), (Ii), and (Ij), R^{a} is

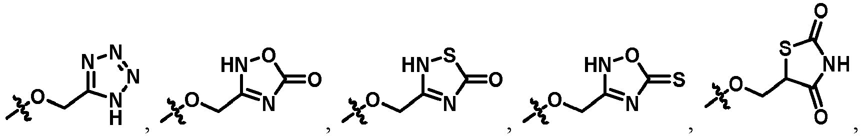

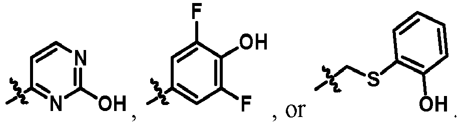

In some embodiments of the Formula (I), (Ia), (Ib), (Ic), (Id), (Ie), (If), (Ie), (If), (Ig) (Ih), (Ii), and (Ij), R^{b} is -CO₂H, -(CH₂)CO₂H, or -OCH₂CO₂H. In other embodiments, R^{b} is -CO₂CH₃, -CO₂CH₂CH₃, -CO₂CH₂CH₂CH₃, -CO₂CH(CH₃)₂, -(CH₂)CO₂CH₃, -(CH₂)CO₂CH₂CH₃, -(CH₂)CO₂CH₂CH₂CH₃, or -(CH₂)CO₂CH(CH₃)₂.

In some embodiments of the Formula (I), (Ia), (Ib), (Ic), (Id), (Ie), (If), (Ie), (If), (Ig) (Ih), (Ii), and (Ij), R^{b} is -P(O)(OH)OH, -(CH₂)P(O)(OH)OH, -P(O)(OH)OCH₃, -P(O)(OH)OCH₂CH₃,-P(O)(OH)OCH₂CH₂CH₃,-P(O)(OH)OCH(CH₃)₂, -(CH₂) P(O)(OH)OCH₃, -(CH₂)P(O)(OH)OCH₂CH₃, -(CH₂)P(O)(OH)OCH₂CH₂CH₃, or -(CH₂)P(O)(OH)OCH(CH₃)₂.

In some embodiments of the Formula (I), (Ia), (Ib), (Ic), (Id), (Ie), (If), (Ie), (If), (Ig) (Ih), (Ii), and (Ij), R^{b} is -S(O)₂OH,-(CH₂)S(O)₂OH, -C(O)NHCN, or -(CH₂)C(O)NHCN.

In some embodiments of the Formula (I), (Ia), (Ib), (Ic), (Id), (Ie), (If), (Ie), (If), (Ig) (Ih), (Ii), and (Ij), R^{b} is -C(O)NHS(O)₂CH₃, -C(0)NHS(O)₂CH₂CH₃, -C(O)NHS(O)₂CH₂CH₂CH₃, -C(O)NHS(O)₂CH(CH₃)₂, -(CH₂)C(O)NHS(O)₂CH₃, -(CH₂)C(O)NHS(O)₂CH₂CH₃, -(CH₂)C(O)NHS(O)₂CH₂CH₂CH₃, or -(CH₂)C(O)NHS(O)₂CH(CH₃)₂.

In some embodiments of the Formula (I), (Ia), (Ib), (Ic), (Id), (Ie), (If), (Ie), (If), (Ig) (Ih), (Ii), and (Ij), R^{b} is 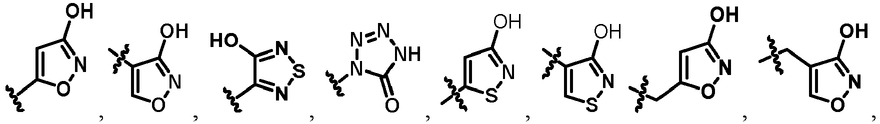 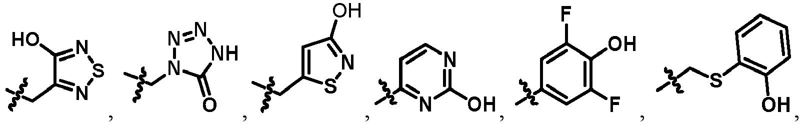 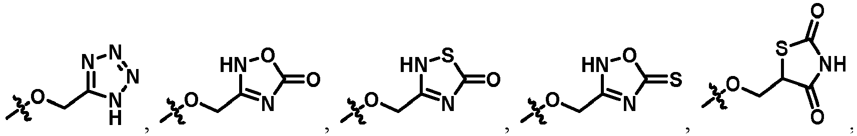 or

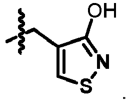

In some embodiments of the Formula (I), (Ia), (Ib), (Ic), (Id), (Ie), (If), (Ie), (If), (Ig) (Ih), (Ii), and (Ij), R^{b} is

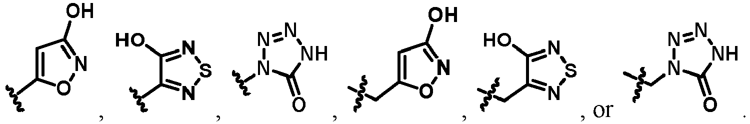

In some embodiments of the Formula (I), (Ia), (Ib), (Ic), (Id), (Ie), (If), (Ie), (If), (Ig) (Ih), (Ii), and (Ij), R^{b} is

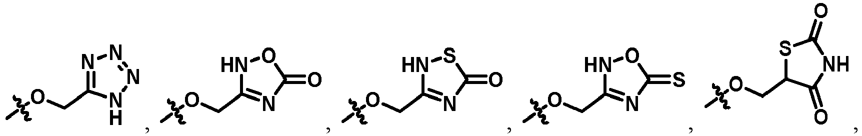

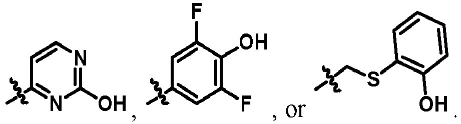

In some embodiments, the compound of Formula (I) is a compound selected from:

| **Cmpd No.** | **Structure** | **Chemical Name** |
|---|---|---|
| **I-2** | 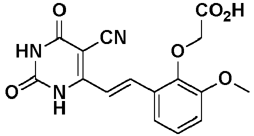 | (E)-2-(2-(2-(5-cyano-2, 6-dioxo-1,2,3,6-tetrahydropyrimidin-4-yl)vinyl)-6-methoxyphenoxy)acetic acid; |
| **I-3** | 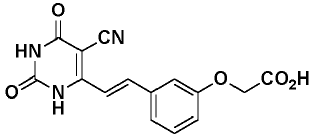 | (E)-2-(3-(2-(5-cyano-2,6-dioxo-1,2,3,6-tetrahydropyrimidin-4-yl)vinyl)phenoxy)acetic acid; |
| **I-4** | 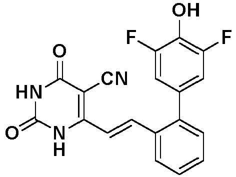 | (E)-6-(2-(3',5'-difluoro-4'-hydroxy-[1,1'-biphenyl]-2-yl)vinyl)-2,4-dioxo-1,2,3,4-tetrahydropyrimidine-5-carbonitrile; |
| **I-5** | 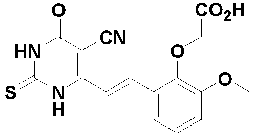 | (E)-2-(2-(2-(5 -cyano-6-oxo-2-thioxo-1,2,3,6-tetrahydropyrimidin-4-yl)vinyl)-6-methoxyphenoxy)acetic acid; |
| **I-6** | 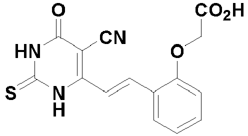 | (E)-2-(2-(2-(5-cyano-6-oxo-2-thioxo-1,2,3,6-tetrahydropyrimidin-4-yl)vinyl)phenoxy)acetic acid; |
| **I-7** | 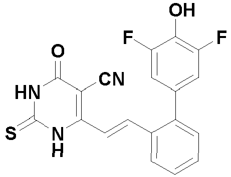 | (E)-6-(2-(3',5'-difluoro-4'-hydroxy-[1,1'-biphenyl]-2-yl)vinyl)-4-oxo-2-thioxo-1,2,3,4-tetrahydropyrimidine-5-carbonitrile; |
| **I-8** | 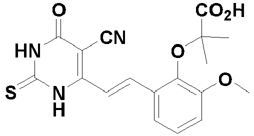 | (E)-2-(2-(2-(5-cyano-6-oxo-2-thioxo-1,2,3,6-tetrahydropyrimidin-4-yl)vinyl)-6-methoxyphenoxy)-2-methylpropanoic acid; |
| **I-9** | 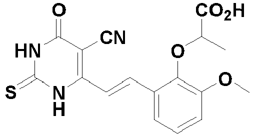 | (E)-2-(2-(2-(5-cyano-6-oxo-2-thioxo-1,2,3,6-tetrahydropyrimidin-4-yl)vinyl)-6-methoxyphenoxy)propanoic acid; |
| | | |
| **I-11** | 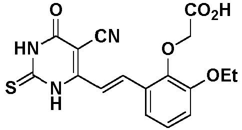 | (E)-2-(2-(2-(5-cyano-6-oxo-2-thioxo-1,2,3,6-tetrahydropyrimidin-4-yl)vinyl)-6-ethoxyphenoxy)acetic acid; |
| **I-12** | 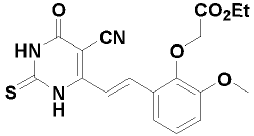 | ethyl (E)-2-(2-(2-(5-cyano-6-oxo-2-thioxo-1,2,3,6-tetrahydropyiimidin-4-yl)vinyl)-6-methoxyphenoxy)acetate; |
| | | |
| **I-14** | 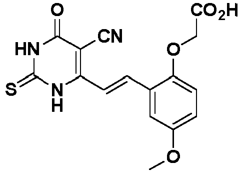 | (E)-2-(2-(2-(5-cyano-6-oxo-2-thioxo-1,2,3,6-tetrahydropyrimidin-4-yl)vinyl)-4-methoxyphenoxy)acetic acid; |
| **I-15** | 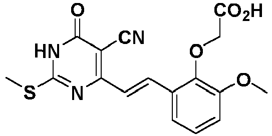 | (E)-2-(2-(2-(5-cyano-2-(methylthio)-6-oxo-1,6-dihydropyrimidin-4-yl)vinyl)-6-methoxyphenoxy)acetic acid; |
| **I-18** | 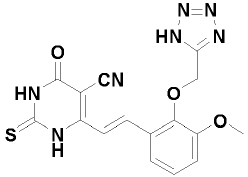 | (E)-6-(2-((1H-tetrazol-5-yl)methoxy)-3-methoxystyryl)-4-oxo-2-thioxo-1,2,3,4-tetrahydropyrimidine-5-carbonitrile; and |
| **I-19** | 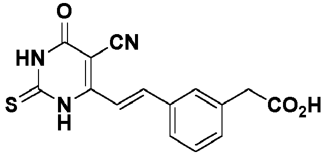 | (E)-2-(3-(2-(5-cyano-6-oxo-2-thioxo-1,2,3,6-tetrahydropyrimidin-4-yl)vinyl)phenyl)acetic acid; |

In other embodiments, the compound of Formula (I) is a compound selected from:

| **Cmpd No.** | **Structure** | **Chemical Name** |
|---|---|---|
| **I-20** | 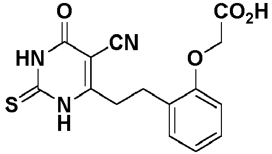 | 2-(2-(2-(5-cyano-6-oxo-2-thioxo-1,2,3,6-tetrahydropyrimidin-4-yl)ethyl)phenoxy)acetic acid; |
| **I-23** | 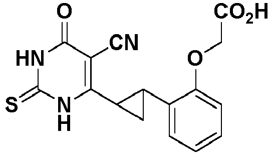 | 2-(2-(2-(5-cyano-6-oxo-2-thioxo-1,2,3,6-tetrahydropyrimidin-4-yl)cyclopropyl)phenoxy)acetic acid; |
| **I-25** | 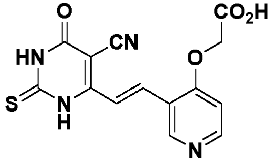 | (E)-2-((3 -(2-(5 -cyano-6-oxo-2-thioxo-1,2,3, 6-tetrahydropyrimidin-4-yl)vinyl)pyridin-4-yl)oxy)acetic acid; |
| **I-26** | 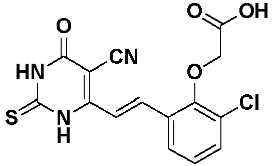 | (E)-2-(2-chloro-6-(2-(5-cyano-6-oxo-2-thioxo-1,2,3,6-tetrahydropyrimidin-4-yl)vinyl)phenoxy)acetic acid; |
| **I-27** | 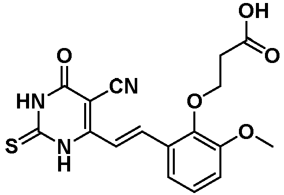 | (E)-3-(2-(2-(5-cyano-6-oxo-2-thioxo-1,2,3,6-tetrahydropyrimidin-4-yl)vinyl)-6-methoxyphenoxy)propanoic acid; |
| **I-28** | 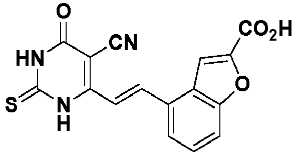 | (E)-4-(2-(5 -cyano-6-oxo-2-thioxo-1,2,3,6-tetrahydropyrimidin-4-yl)vinyl)benzofuran-2-carboxylic acid; |
| **I-30** | 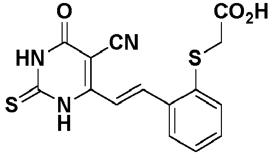 | (E)-2-((2-(2-(5-cyano-6-oxo-2-thioxo-1,2,3,6-tetrahydropyrimidin-4-yl)vinyl)phenyl)thio)acetic acid; |
| **I-31** | 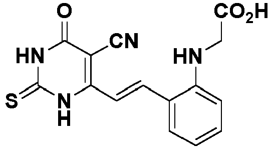 | (E)-(2-(2-(5-cyano-6-oxo-2-thioxo-1,2,3,6-tetrahydropyrimidin-4-yl)vinyl)phenyl)glycine; |
| **I-32** | 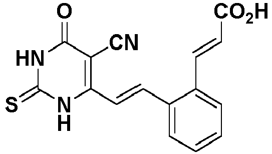 | (E)-3-(2-((E)-2-(5-cyano-6-oxo-2-thioxo-1,2,3,6-tetrahydropyrimidin-4-yl)vinyl)phenyl)acrylic acid; |
| **I-35** | 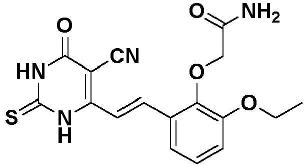 | (E)-2-(2-(2-(5-cyano-6-oxo-2-thioxo-1,2,3,6-tetrahydropyrimidin-4-yl)vinyl)-6-ethoxyphenoxy)acetamide; |
| **I-37** | 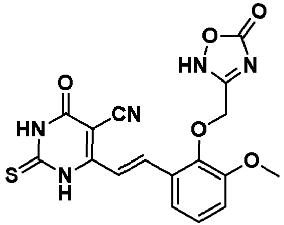 | (E)-6-(3 -methoxy-2-((5-oxo-2, 5-dihydro-1,2,4-oxadiazol-3-yl)methoxy)styryl)-4-oxo-2-thioxo-1,2,3,4-tetrahydropyrimidine-5-carbonitrile; |
| **I-38** | 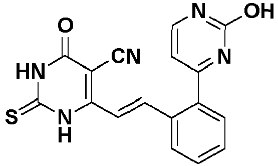 | (E)-6-(2-(2-hydroxypyrimidin -4-yl)styrl)-4-oxo-2-thioxo-1,2,3,4-tetrahydropyrimidine-5-carbonitrile; |
| **I-39** | 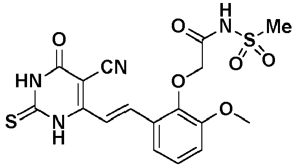 | (E)-2-(2-(2-(5-cyano-6-oxo-2-thioxo-1,2,3,6-tetrahydropyrimidin-4-yl)vinyl)-6-methoxyphenoxy)-N-(methylsulfonyl)acetamide; |
| **I-40** | 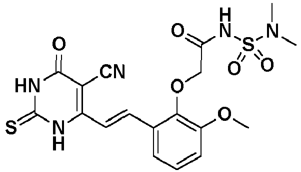 | (E)-2-(2-(2-(5-cyano-6-oxo-2-thioxo-1,2,3,6-tetrahydropyrimidin-4-yl)vinyl)-6-methoxyphenoxy)-N-(N,N-dimethylsulfamoyl)acetamide; |
| **I-41** | 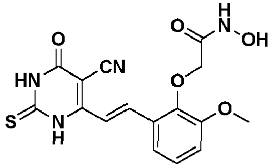 | (E)-2-(2-(2-(5-cyano-6-oxo-2-thioxo-1,2,3,6-tetrahydropyrimidin-4-yl)vinyl)-6-methoxyphenoxy)-N-hydroxy acetamide; |
| **I-42** | 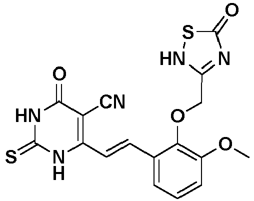 | (E)-6-(3-methoxy-2-((5-oxo-2, 5-dihydro-1,2,4-thiadiazol-3-yl)methoxy)styryl)-4-oxo-2-thioxo-1,2,3,4-tetrahydropyrimidine-5-carbonitrile; |
| **I-43** | 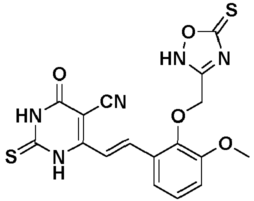 | (E)-6-(3-methoxy-2-((5-thioxo-2,5-dihydro-1,2,4-oxadiazol-3-yl)methoxy)styryl)-4-oxo-2-thioxo-1,2,3,4-tetrahydropyrimidine-5-carbonitrile; |
| **I-44** | 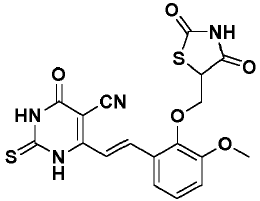 | (E)-6-(2-((2,4-dioxothiazolidin-5-yl)methoxy)-3-methoxystyryl)-4-oxo-2-thioxo-1,2,3,4-tetrahydropyrimidine-5-carbonitrile; |
| **I-45** | 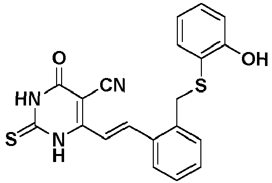 | (E)-6-(2-(((2-hydroxyphenyl)thio)methyl)styryl)-4-oxo-2-thioxo-1,2,3,4-tetrahydropyrimidine-5-carbonitrile; |
| **I-46** | 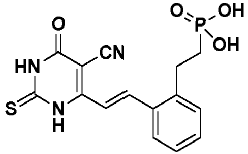 | (E)-(2-(2-(5-cyano-6-oxo-2-thioxo-1,2,3,6-tetrahydropyrimidin-4-yl)vinyl)phenethyl)phosphonic acid; |
| **I-47** | 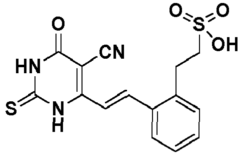 | (E)-2-(2-(2-(5-cyano-6-oxo-2-thioxo-1,2,3,6-tetrahydropyrimidin-4-yl)vinyl)phenyl)ethane-1-sulfonic acid; and |
| **I-48** | 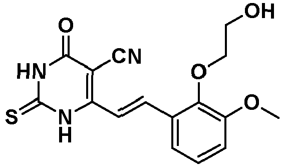 | (E)-6-(2-(2-hydroxyethoxy)-3-methoxystyryl)-4-oxo-2-thioxo-1,2,3,4-tetrahydropyrimidine-5-carbonitrile |
| **I-49** | 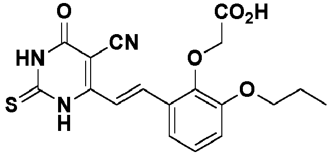 | (E)-2-(2-(2-(5-cyano-6-oxo-2-thioxo-1,2,3,6-tetrahydropyrimidin-4-yl)vinyl)-6-propoxyphenoxy)acetic acid |
| **I-50** | 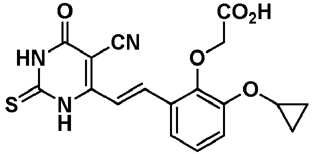 | (E)-2-(2-(2-(5-cyano-6-oxo-2-thioxo-1,2,3,6-tetrahydropyrimidin-4-yl)vinyl)-6-cyclopropoxyphenoxy)acetic acid |
| **I-51** | 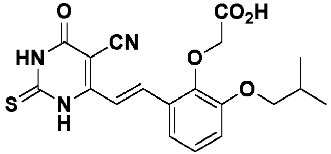 | (E)-2-(2-(2-(5-cyano-6-oxo-2-thioxo-1,2,3,6-tetrahydropyrimidin-4-yl)vinyl)-6-isobutoxyphenoxy)acetic acid |
| **I-52** | 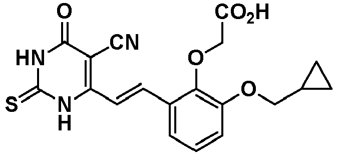 | (E)-2-(2-(2-(5-cyano-6-oxo-2-thioxo-1,2,3,6-tetrahydropyrimidin-4-yl)vinyl)-6-(cyclopropylmethoxy)phenoxy)acetic acid |
| **I-53** | 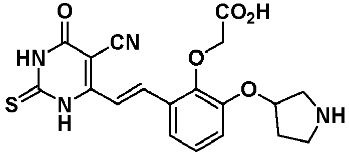 | (E)-2-(2-(2-(5-cyano-6-oxo-2-thioxo-1,2,3,6-tetrahydropyrimidin-4-yl)vinyl)-6-(pyrrolidin-3-yloxy)phenoxy)acetic acid |
| **I-59** | 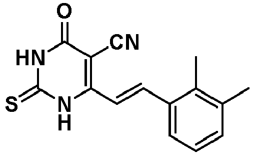 | (E)-6-(2,3-dimethylstyryl)-4-oxo-2-thioxo-1,2,3,4-tetrahydropyrimidine-5-carbonitrile |
| **.I-60** | 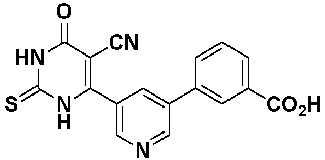 | 3-(5-(5-cyano-6-oxo-2-thioxo-1,2,3,6-tetrahydropyrimidin-4-yl)pyridin-3-yl)benzoic acid |
| **I-61** | 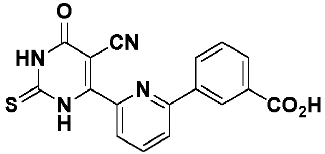 | 3-(6-(5-cyano-6-oxo-2-thioxo-1,2,3,6-tetrahydropyrimidin-4-yl)pyridin-2-yl)benzoic acid |
| **I-62** | 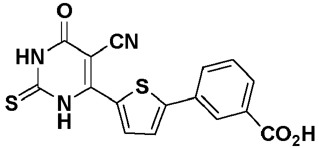 | 3-(5-(5-cyano-6-oxo-2-thioxo-1,2,3,6-tetrahydropyrimidin-4-yl)thiophen-2-yl)benzoic acid |

Also disclosed herein, but not claimed, are the following compounds:

| | | |
|---|---|---|
| **I-10** | 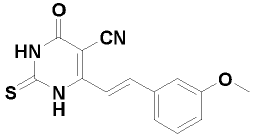 | (E)-6-(3 -methoxystyryl)-4-oxo-2-thioxo-1,2,3,4-tetrahydropyrimidine-5-carbonitrile; |
| **I-13** | 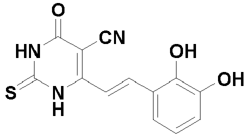 | (E)-6-(2,3-dihydroxystyryl)-4-oxo-2-thioxo-1,2,3,4-tetrahydropyrimidine-5-carbonitrile; |
| **I-16** | 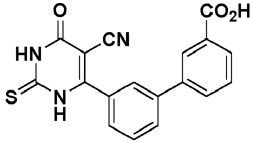 | 3'-(5-cyano-6-oxo-2-thioxo-1,2,3,6-tetrahydropyrimidin-4-yl)-[1,1'-biphenyl]-3-carboxylic acid; |
| **I-21** | 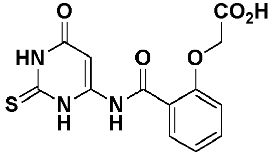 | 2-(2-((6-oxo-2-thioxo-1,2,3,6-tetrahydropyrimi din -4-yl)carbamoyl)phenoxy)acetic acid; |
| **I-22** | 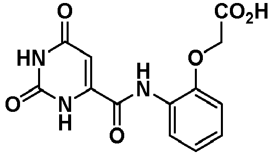 | 2-(2-(2,6-dioxo-1,2,3,6-tetrahydropyrimi dine-4-carboxamido)phenoxy)acetic acid; |
| **I-24** | 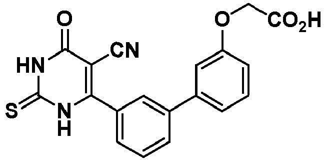 | 2-((3'-(5-cyano-6-oxo-2-thioxo-1,2,3,6-tetrahydropyrimidin-4-yl)-[1,1'-biphenyl]-3-yl)oxy)acetic acid; |
| **I-33** | 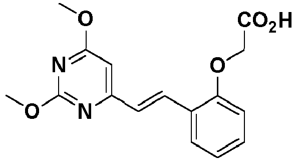 | (E)-2-(2-(2-(2,6-dimethoxypyrimidin-4-yl)vinyl)phenoxy)acetic acid; |
| **I-34** | 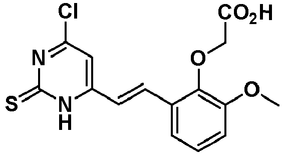 | (E)-2-(2-(2-(6-chloro-2-thioxo-2,3-dihydropyrimidin-4-yl)vinyl)-6-methoxyphenoxy)acetic acid; |
| **I-54** | 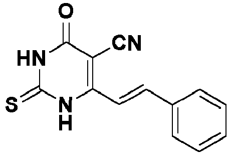 | (E)-4-oxo-6-styryl-2-thioxo-1,2,3,4-tetrahydropyrimidine-5-carbonitrile |
| **I-55** | 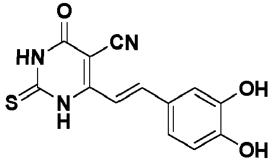 | (E)-6-(3,4-dihydroxystyryl)-4-oxo-2-thioxo-1,2,3,4-tetrahydropyrimidine-5-carbonitrile |
| **I-56** | 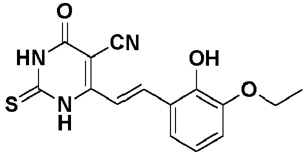 | (E)-6-(3-ethoxy-2-hydroxystyryl)-4-oxo-2-thioxo-1,2,3,4-tetrahydropyrimidine-5-carbonitrile |
| **I-57** | 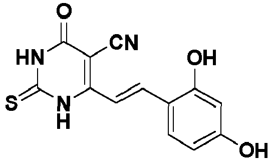 | (E)-6-(2,4-dihydroxystyryl)-4-oxo-2-thioxo-1,2,3,4-tetrahydropyrimidine-5-carbonitrile |
| **I-58** | 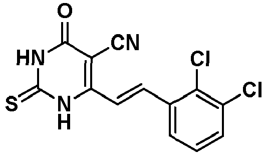 | (E)-6-(2,3-dichlorostyryl)-4-oxo-2-thioxo-1,2,3,4-tetrahydropyrimidine-5-carbonitrile |
| **I-63** | 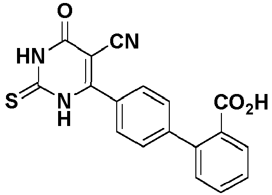 | 4'-(5-cyano-6-oxo-2-thioxo-1,2,3,6-tetrahydropyrimidin-4-yl)-[1,1'-biphenyl]-2-carboxylic acid |
| **I-64** | 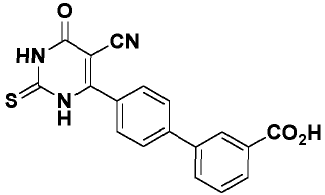 | 4'-(5-cyano-6-oxo-2-thioxo-1,2,3,6-tetrahydropyrimidin-4-yl)-[1,1'-biphenyl]-3-carboxylic acid |
| **I-65** | 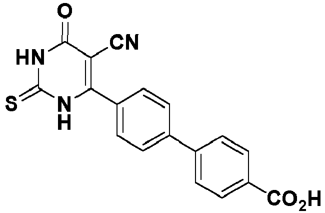 | 4'-(5-cyano-6-oxo-2-thioxo-1,2,3,6-tetrahydropyrimidin-4-yl)-[1,1'-biphenyl]-4-carboxylic acid |
| **I-66** | 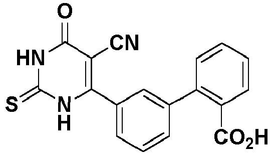 | 3'-(5-cyano-6-oxo-2-thioxo-1,2,3,6-tetrahydropyrimidin-4-yl)-[1,1'-biphenyl]-2-carboxylic acid |
| **I-67** | 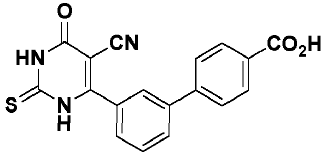 | 3'-(5-cyano-6-oxo-2-thioxo-1,2,3,6-tetrahydropyrimidin-4-yl)-[1,1'-biphenyl]-4-carboxylic acid |
| **I-68** | 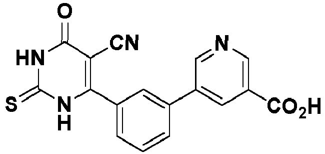 | 5-(3-(5-cyano-6-oxo-2-thioxo-1,2,3,6-tetrahydropyrimidin-4-yl)phenyl)nicotinic acid |
| **I-69** | 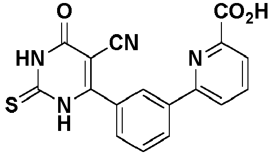 | 6-(3-(5-cyano-6-oxo-2-thioxo-1,2,3,6-tetrahydropyrimidin-4-yl)phenyl)picolinic acid |
| **I-70** | 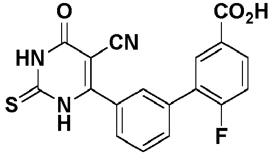 | 3'-(5-cyano-6-oxo-2-thioxo-1,2,3,6-tetrahydropyrimidin-4-yl)-6-fluoro-[1,1'-biphenyl]-3-carboxylic acid |
| **I-71** | 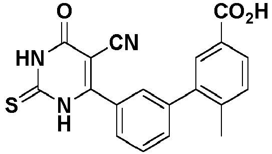 | 3'-(5-cyano-6-oxo-2-thioxo-1,2,3,6-tetrahydropyrimidin-4-yl)-6-methyl-[1,1'-biphenyl]-3-carboxylic acid |
| **I-72** | 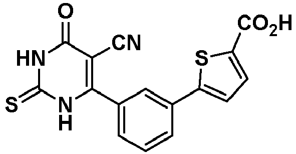 | 5-(3-(5-cyano-6-oxo-2-thioxo-1,2,3,6-tetrahydropyrimidin-4-yl)phenyl)thiophene-2-carboxylic acid |
| **I-73** | 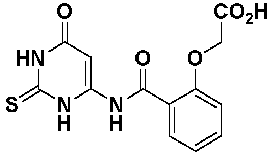 | 2-(2-((6-oxo-2-thioxo-1,2,3,6-tetrahydropyrimi din -4-yl)carbamoyl)phenoxy)acetic acid; |
| **I-74** | 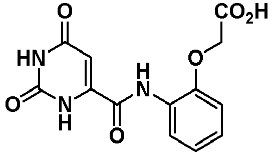 | 2-(2-(2,6-dioxo-1,2,3,6-tetrahydropyrimi dine-4-carboxamido)phenoxy)acetic acid; |
| **I-75** | 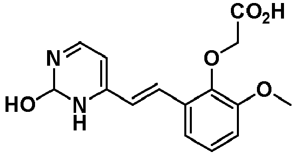 | (E)-2-(2-(2-(2-hydroxy-2,3-dihydropyrimidin-4-yl)vinyl)-6-methoxyphenoxy)acetic acid; |

In another aspect, the present disclosure relates to a compound for use in a method of treating, preventing, or reducing the risk of a disease or disorder associated with α-amino-β-carboxymuconate-ε-semialdehyde decarboxylase (ACMSD) dysfunction comprising administering to the subject suffering from or susceptible to developing a disease or disorder associated with ACMSD dysfunction a therapeutically effective amount of the compound, wherein the compound is represented by Formula (II): 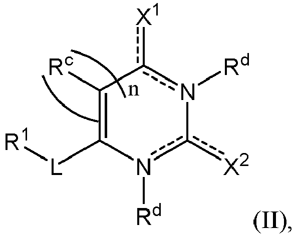 or a pharmaceutically acceptable salt or tautomer thereof, wherein:
X¹ is H, O, S, OR² or SH;
X² is O, S, OR² or SR²;
L is -(CH₂)ₘCH=CH(CH₂)p-, -(CH₂)*ₒ*-, 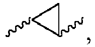 -(CH₂)ₘC=(O)NR³(CH₂)ₚ-,-(CH₂)ₘNR³C=(O)(CH₂)ₚ-,pyridinyl, or thiophenyl;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, - (C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -O(C(R^{f})₂)ᵣ(C₃-C₇)cycloalkyl, -(C(Rf)₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{X}, -Y²(C(R^{f})₂)ᵣCO₂R^{x}, -O(C(R^{f})₂)ᵣC(O)NHR^{g}, - (C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl,-(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H;
R^{c} is-CN or -NO₂;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II),
X¹ is H, O, S, OR² or SH;
X² is O, S, OR² or SR²;
L is -(CH₂)ₘCH=CH(CH₂)ₚ-, -(CH₂)*ₒ-,* 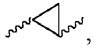 -(CH₂)ₘC=(O)NR³(CH₂)ₚ-, or -(CH₂)ₘNR³C=(O)(CH₂)ₚ-;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl,-(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, - O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{X}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, - O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H;
R^{c} is -CN or -NO₂;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In one embodiment, the compound of Formula (II) is a compound of Formula (IIa), (IIb), (IIc), or (IId): 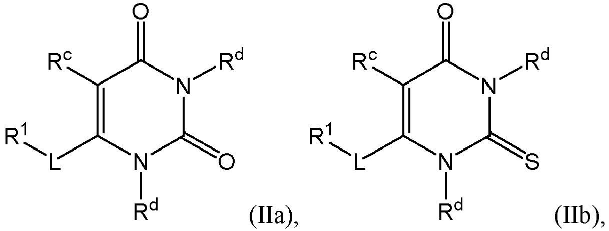 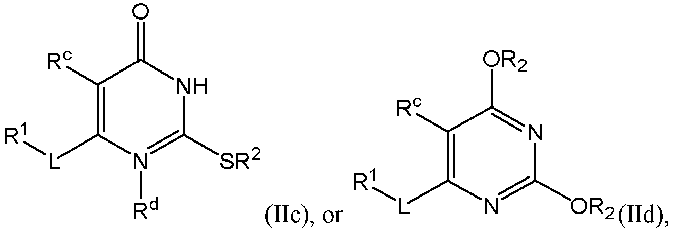 and pharmaceutically acceptable salts or tautomers thereof.

In another embodiment, the compound of Formula (II) is a compound of Formula (IIf), or (IIg): 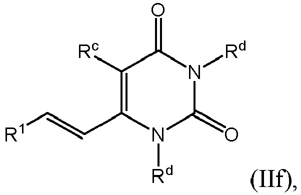 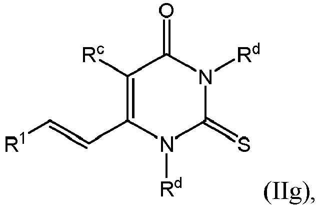 or. and pharmaceutically acceptable salts or tautomers thereof.

In another embodiment, the compound of Formula (II) is a compound of Formula (IIi) : 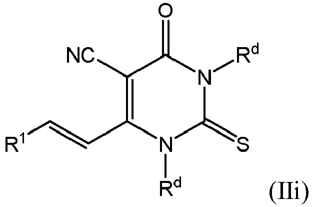 and pharmaceutically acceptable salts or tautomers thereof.

In some embodiments of Formula (II), (IIa), (IIb), (IIc), (IId), (IIf), (IIg), and (IIi)),
X¹ is H, O or OR²;
X² is S, O, or OR²;
L is -(CH₂)ₘCH=CH(CH₂)ₚ-, -(CH₂)*ₒ-*, 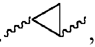 -(CH₂)ₘC=(O)NR³(CH₂)ₚ-, or -(CH₂)ₘNR³C=(O)(CH₂)ₚ-;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, or -CH=CHCO₂R^{x}, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H;
R^{c} is-CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II), (IIa), (IIb), (IIc), (IId), (IIf), (IIg), and (IIi),
X¹ is O or OR²;
X² is S or OR²;
L is -(CH₂)ₘCH=CH(CH₂)ₚ-, -(CH₂)*ₒ-,* 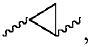 -(CH₂)ₘC=(O)NR³(CH₂)ₚ-, or -(CH₂)ₘNR³C=(O)(CH₂)ₚ-;
Y² is O, NH or S;
R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{X}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, or -CH=CHCO₂R^{x}, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II), (IIa), (IIb), (IIc), (IId), (IIf), (IIg), and (IIi),
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, or -(C(R^{f})₂)ᵣC₆-C₁₀ aryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II), (IIa), (IIb), (IIc), (IId), (IIf), (IIg), and (IIi),
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, -O(C(R^{f})₂)ᵣheteroaryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, -(C(R^{f})₂)ᵣC₆-C₁₀ aryl, or -O(C(R^{f})₂)ᵣheteroaryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II), (IIa), (IIb), (IIc), (IId), (IIf), (IIg), and (IIi),
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, -O(C(R^{f})₂)ᵣheteroaryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, - -(C(R^{f})₂)ᵣC₆-C₁₀ aryl, or -O(C(R^{f})₂)ᵣheteroaryl, wherein the aryl is substituted with one to three substituents selected from halogen and OH;
R^{c} is -CN or NO₂;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

In some embodiments of Formula (II), (IIa), (IIb), (IIc), (IId), (IIf), (IIg), and(IIi), X¹ is O. In another embodiment, X¹ is S. In yet another embodiment, X¹ is OR². In another embodiment, X¹ is SH. In another embodiment, X¹ is H. In another embodiment, X¹ is O or S. In yet another embodiment, X¹ is O or OR₂. In another embodiment, X¹ is H, O,or OCH₃. In yet another embodiment, X¹ is O or OCH₃.

In some embodiments of Formula (II), (IIa), (IIb), (IIc), (IId), (IIf), (IIg), and (IIi), X² is O. In another embodiment, X² is S. In yet another embodiment, X² is OR². In another embodiment, X² is SR². In another embodiment, X² is O or S. In yet another embodiment, X² is O, S, or SR². In another embodiment, X² is O, S, or SCH₃. In another embodiment, X² is S or OCH₃.

In some embodiments of Formula (II), (IIa), (IIb), (IIc), (IId), (IIf), (IIg), and (IIi), L is -(CH₂)ₘCH=CH(CH₂)ₚ-, -(CH₂)*ₒ-,* or 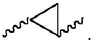 In another embodiment, L is -(CH₂)ₘC=(O)NR³(CH₂)ₚ-, or -(CH₂)ₘNR³C=(O)(CH₂)ₚ-. In yet another embodiment, L is -(CH₂)ₘCH=CH(CH₂)ₚ-, or 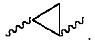 In another embodiment, L is -(CH₂)*ₒ*-. In yet another embodiment, L is - (CH₂)ₘCH=CH(CH₂)p-, -(CH₂)*ₒ*-, 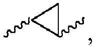 -(CH₂)ₘC=(O)NR³(CH₂)ₚ-, or - (CH₂)ₘNR³C=(O)(CH₂)ₚ-. In another embodiment, L is -CH=CH-, -(CH₂)*ₒ*-, 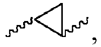 C=(O)NR³-, or -NR³C=(O)-. In another embodiment, L is -(CH₂)ₘCH=CH(CH₂)ₚ-. In another embodiment, L is -(CH₂)ₘCH=CH(CH₂)ₚ-,pyridinyl, or thiophenyl. In another embodiment, L is pyridinyl, or thiophenyl. In yet another embodiment, L is -CH=CH-.

In some embodiments of Formula (II), (IIa), (IIb), (IIc), (IId), (IIf), (IIg), and (IIi), Y² is O. In another embodiment, Y² is NH. In yet another embodiment, Y² is S. In another embodiment, Y² is O or NH. In yet another embodiment, Y² is O or S. In another embodiment, Y² is NH or S.

In some embodiments of Formula (II), (IIa), (IIb), (IIc), (IId), (IIf), (IIg), and (IIi), R¹ is C₆-C₁₀ aryl substituted with R^{a} and R^{b}, and optionally substituted with one to two R^{e}. In another embodiment, R¹ is heteroaryl, wherein the heteroaryl comprises one or two 5- to 7-membered rings and 1-4 heteroatoms selected from N, O and S, and substituted with R^{a} and R^{b}, and optionally substituted with one to two R^{e}. In another embodiment, R¹ is C₆-C₁₀ aryl or heteroaryl, wherein the heteroaryl comprises one or two 5- to 7-membered rings and 1-4 heteroatoms selected from N, O and S, and wherein the aryl and heteroaryl are substituted with R^{a} and R^{b}. In another embodiment, R¹ is C₆-C₁₀ aryl or heteroaryl comprising one 5- to 7-membered ring and 1-4 heteroatoms selected from N, O and S, wherein the aryl and heteroaryl are substituted with R^{a} and R^{b}. In another embodiment, R¹ is C₆-C₁₀ aryl or heteroaryl comprising two 5- to 7-membered rings and 1-4 heteroatoms selected from N, O and S, wherein the aryl and heteroaryl are substituted with R^{a} and R^{b}.

In another embodiment, R¹ is C₆-C₁₀ aryl substituted with R^{a} and R^{b}. In yet another embodiment, R¹ is heteroaryl comprising one or two 5- to 7-membered rings and 1-4 heteroatoms selected from N, O and S, substituted with R^{a} and R^{b}. In yet another embodiment, R¹ is heteroaryl comprising one 5- to 7-membered ring and 1-4 heteroatoms selected from N, O and S, substituted with R^{a} and R^{b}. In yet another embodiment, R¹ is heteroaryl comprising two 5- to 7-membered rings and 1-4 heteroatoms selected from N, O and S, substituted with R^{a} and R^{b}. In another embodiment, R¹ is phenyl, pyridinyl, or thiophenyl, wherein each is substituted with R^{a} and R^{b}, and optionally substituted with one to two R^{e}. In another embodiment, R¹ is phenyl or pyridinyl, wherein each is substituted with R^{a} and R^{b}, and optionally substituted with one to two R^{e}. In another embodiment, R¹ is phenyl, pyridinyl, or thiophenyl, wherein each is substituted with R^{a} and R^{b}. In yet another embodiment, R¹ is phenyl or pyridinyl, wherein each is substituted with R^{a} and R^{b}. In yet another embodiment, R¹ is phenyl substituted with R^{a} and R^{b}.

In some embodiments of Formula (II), (IIa), (IIb), (IIc), (IId), (IIf), (IIg), and (IIi), R² is H. In another embodiment, R² is C₁-C₄ alkyl. In yet another embodiment, R² is H or C₁-C₂ alkyl. In another embodiment, R² is H, methyl, or ethyl. In another embodiment, R² is H or methyl.

In some embodiments of Formula (II), (IIa), (IIb), (IIc), (IId), (IIf), (IIg), and (IIi), R³ is H. In another embodiment, R³ is C₁-C₄ alkyl. In yet another embodiment, R³ is H or C₁-C₂ alkyl. In another embodiment, R³ is H, methyl, or ethyl. In another embodiment, R³ is H or methyl.

In some embodiments of Formula (II), (IIa), (IIb), (IIc), (IId), (IIf), (IIg), and (IIi), R^{a} is H, -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -OR^{y}, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{a} is H, -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{a} is H, -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -OH, -O-CH₂CH₂OH, -OCH₃, -OCH₂CH₃, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃, 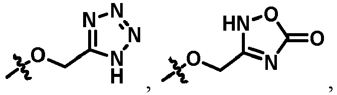 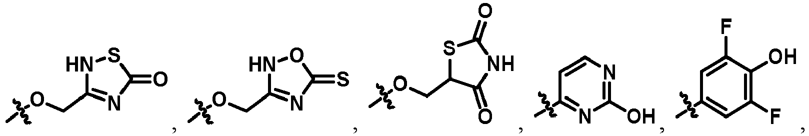 or

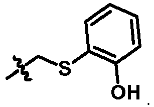

In some embodiments of Formula (II), (IIa), (IIb), (IIc), (IId), (IIf), (IIg), and (IIi), R^{a} is H or -OR^{y}, or R^{a} and R^{b} when on adjacent atoms together with the atoms to which they are attached form a 5- to 6-membered heteroaryl ring optionally substituted with one or more - CO₂H. In another embodiment, R^{a} is H or -OR^{y}. In another embodiment, R^{a} is H, -OCH₃, - OCH₂CH₃, or -OH. In another embodiment, R^{a} is H, -OCH₃, or -OCH₂CH₃.

In some embodiments of Formula (II), (IIa), (IIb), (IIc), (IId), (IIf), (IIg), and (IIi), R^{a} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, O(C(R^{f})₂)ᵣheteroaryl, -O(C(R^{f})₂)ᵣheterocycloalkyl, -(C(R^{f})₂)rS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{a} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{a} is -CO₂H, -(CH₂)CO₂H, -O(CH₂)CO₂H,-S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -OH, -O-CH₂CH₂OH, -OCH₃, -OCH₂CH₃, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃, 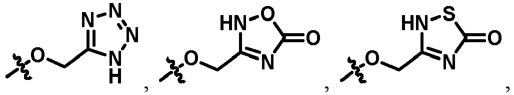 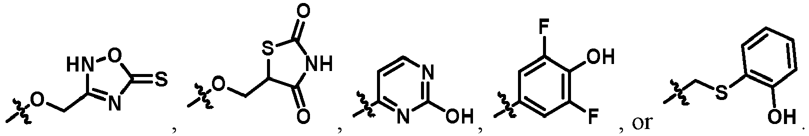 In another embodiment, R^{a} is -(CH₂)CO₂H, -O(CH₂)CO₂H,-O(CH₂CH₂)CO₂H, -S(CH₂)CO₂H, -NH(CH₂)CO₂H, O(CH₂)C(O)NH₂, -O(CH₂)C(O)NS(O)₂N(CH₃)₂, -O(CH₂)C(O)NHOH, -O(CH₂)C(O)NS(O)₂N(CH₃)₂,-CH=CHCO₂H, -O(CH₂CH₂)P(O)₂OH, -O(CH₂CH₂)S(O)₂OH, -O-CH₂CH₂OH,

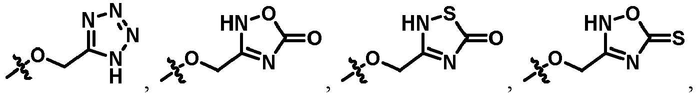

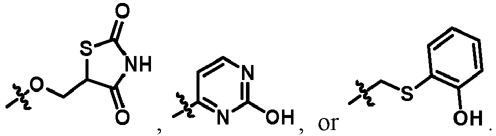

In another embodiment, R^{a} is -CO₂H, -O(CH₂)CO₂H, -C(CH₂)₂CO₂H, -CH(CH₂)CO₂H, -O(CH₂)CO₂CH₂CH₃, -OH, -OCH₃, 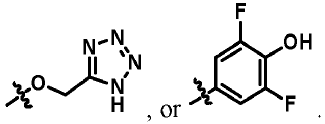 In another embodiment, R^{a} is -CO₂H, -(CH₂)CO₂H, -O(CH₂)CO₂H, -C(CH₂)₂CO₂H, -CH(CH₂)CO₂H, -O(CH₂)CO₂CH₂CH₃, -OH, -OCH₃, or 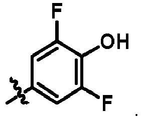 In another embodiment, R^{a} is -(CH₂)CO₂H, -O(CH₂)CO₂H, -C(CH₂)₂CO₂H, -CH(CH₂)CO₂H, OCH₃, or

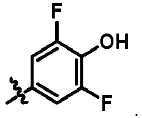

In some embodiments of Formula (II), (IIa), (IIb), (IIc), (IId), (IIf), (IIg), and (IIi), ), R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl,-(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)rS(O)₂OH, -(C(R^{f})₂)ᵣP(O)₂OH, - O(C(R^{f})₂)ᵣOH, or -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{b} is -(C(R^{f})₂)ᵣCO₂R^{x},-Y²(C(R^{f})₂)ᵣCO₂R^{x}, -O(C(R^{f})₂)ᵣC(O)NHR^{g},-(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, or -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, Cl, -O(CH₂)C(O)NH₂, -O(CH₂)C(O)NHOH, -O(CH₂)C(O)NS(O)₂N(CH₃)₂, -O(CH₂)C(O)NS(O)₂CH₃,

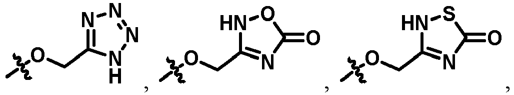

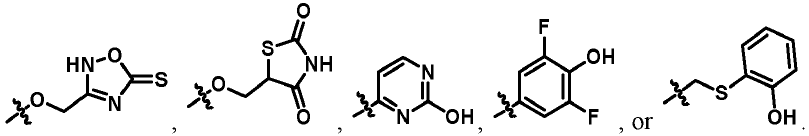

In some embodiments of Formula (II), (IIa), (IIb), (IIc), (IId), (IIf), (IIg), or (IIi), R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, -O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S, or R^{a} and R^{b} when on adjacent atoms together with the atoms to which they are attached form a 5- to 6-membered heteroaryl ring optionally substituted with one or more -CO₂H. In another embodiment, R^{b} is -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, O(C(R^{f})₂)ᵣheteroaryl, -O(C(R^{f})₂)ᵣheterocycloalkyl, -(C(R^{f})₂)ᵣS(O)₂OH, -(C(R^{f})₂)ᵣP(O)₂OH, - O(C(R^{f})₂)ᵣOH, -CH=CHCO₂R^{x}, wherein the aryl and heteroaryl are optionally substituted with one to three substituents each independently selected from halogen and OH, and wherein the heterocycloalkyl is substituted with one to two =O or =S. In another embodiment, R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -OH, -O-CH₂CH₂OH, -OCH₃, -OCH₂CH₃, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃, 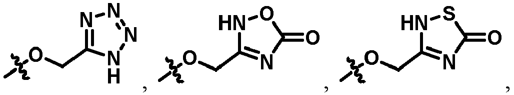 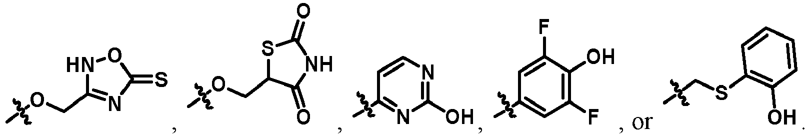 In another embodiment, R^{b} is -(CH₂)CO₂H, -O(CH₂)CO₂H, -O(CH₂CH₂)CO₂H, -S(CH₂)CO₂H, -NH(CH₂)CO₂H, O(CH₂)C(O)NH₂, -O(CH₂)C(O)NS(O)₂N(CH₃)₂, -O(CH₂)C(O)NHOH, -O(CH₂)C(O)NS(O)₂N(CH₃)₂,-CH=CHCO₂H, -O(CH₂CH₂)P(O)₂OH, -O(CH₂CH₂)S(O)₂OH, -O-CH₂CH₂OH,

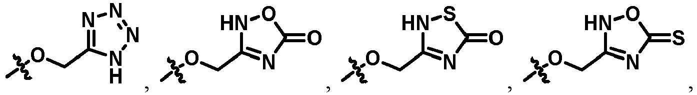

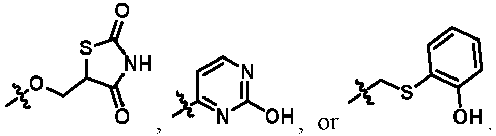

In another embodiment, R^{b} is -CO₂H, -O(CH₂)CO₂H, -C(CH₂)₂CO₂H, -CH(CH₂)CO₂H, -O(CH₂)CO₂CH₂CH₃, 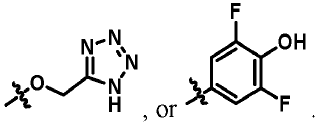 In another embodiment, R^{b} is -CO₂H, -(CH₂)CO₂H, -O(CH₂)CO₂H, -C(CH₂)₂CO₂H, -CH(CH₂)CO₂H, -O(CH₂)CO₂CH₂CH₃, or 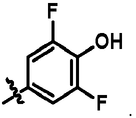 In another embodiment, R^{b} is - (CH₂)CO₂H, -O(CH₂)CO₂H, -C(CH₂)₂CO₂H, -CH(CH₂)CO₂H, OCH₃, or

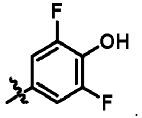

In some embodiments of Formula (II), (IIa), (IIb), (IIc), (IId), (IIf), (IIg), and (IIi), R^{a} and R^{b} when on adjacent atoms together with the atoms to which they are attached form a C₆-C₁₀ aryl ring optionally substituted with one or more -CO₂H. In another embodiment, R^{a} and R^{b} when on adjacent atoms together with the atoms to which they are attached form a 5- to 6-membered heteroaryl ring optionally substituted with one or more -CO₂H. In another embodiment, R^{a} and R^{b} when on adjacent atoms together with the atoms to which they are attached form a 5- or 6-membered heteroaryl ring optionally substituted with one to two - CO₂H. In another embodiment, R^{a} and R^{b} when on adjacent atoms together with the atoms to which they are attached form a 5-membered heteroaryl ring optionally substituted with one or more -CO₂H. In another embodiment, R^{a} and R^{b} when on adjacent atoms together with the atoms to which they are attached form a furan ring optionally substituted with one or more - CO₂H.

In Formulas (II), (IIa), (IIb), (IIc), (IId), (IIf), (IIg), and (IIi), R^{c} is -CN or NO₂. In some embodiments, R^{c} is NO₂. In another embodiment, R^{c} is -CN.

In Formulas (II), (IIa), (IIb), (IIc), (IId), (IIf), (IIg), and (IIi), each R^{d} is independently at each occurrence absent or H. In another embodiment, each R^{d} is independently at each occurrence H.

In some embodiments of Formula (II), (IIa), (IIb), (IIc), (IId), (IIf), (IIg), and (IIi), each R^{e} is independently at each occurrence C₁-C₃ alkyl, C₂-C₃ alkenyl, or C₂-C₃ alkynyl, halogen, C₁-C₃ haloalkyl, -NHR^{z}, -OH, or -CN. In another embodiment, each R^{e} is independently at each occurrence C₁-C₆ alkyl, C₂-C₆ alkenyl, or C₂-C₆ alkynyl. In another embodiment, each R^{e} is independently at each occurrence halogen, C₁-C₆ haloalkyl, -NHR^{z},-OH, or -CN. In another embodiment, each R^{e} is independently at each occurrence C₁-C₆ alkyl, C₂-C₆ alkenyl, or C₂-C₆ alkynyl halogen, or C₁-C₆ haloalkyl. In another embodiment, each R^{e} is independently at each occurrence halogen, -NHR^{z}, -OH, or -CN. In another embodiment, each R^{e} is independently at each occurrence halogen or -OH.

In some embodiments of Formula (II), (IIa), (IIb), (IIc), (IId), (IIf), (IIg), and (IIi), each R^{f} is independently H or C₁-C₃ alkyl. In another embodiment, each R^{f} is C₁-C₄ alkyl. In yet another embodiment, each R^{f} is H or C₁-C₂ alkyl. In another embodiment, each R^{f} is H, methyl, or ethyl. In yet another embodiment, each R^{f} is H or methyl. In another embodiment, each R^{f} is independently each H.

In some embodiments of Formula (II), (IIa), (IIb), (IIc), (IId), (IIf), (IIg), and (IIi), R^{g} is C₁-C₃ alkyl or OH. In another embodiment, R^{g} is -S(O)₂(C₁-C₃ alkyl), or S(O)₂N(C₁-C₃ alkyl)₂. In another embodiment, R^{g} is H or C₁-C₃ alkyl. In another embodiment, R^{g} is OH, - S(O)₂(C₁-C₃ alkyl), or -S(O)₂N(C₁-C₃ alkyl)₂. In another embodiment, R^{g} is H, C₁-C₃ alkyl, OH, -S(O)₂(C₁-C₃ alkyl), or -S(O)₂N(C₁-C₃ alkyl)₂. In another embodiment, R^{g} is H, C₁-C₂ alkyl, OH, -S(O)₂(C₁-C₂ alkyl), or -S(O)₂N(C₁-C₂ alkyl)₂. In another embodiment, R^{g} is H, methyl, OH, -S(O)₂CH₃, or -S(O)₂N(CH₃)₂.

In some embodiments of Formula (II), (IIa), (IIb), (IIc), (IId), (IIf), (IIg), and (IIi), R^{x} is H or C₁-C₃ alkyl. In another embodiment, R^{x} is C₁-C₄ alkyl. In yet another embodiment, R^{x} is H or C₁-C₂ alkyl. In another embodiment, R^{x} is H, methyl, or ethyl. In yet another embodiment, R^{x} is H or methyl. In another embodiment, R^{x} is H.

In some embodiments of Formula (II), (IIa), (IIb), (IIc), (IId) (IIf), (IIg), and (IIi), m is 0. In another embodiment, m is 1. In yet another embodiment, m is 2. In another embodiment, m is 0 or 1. In yet another embodiment, m is 1 or 2.

In some embodiments of Formula (II), (IIa), (IIb), (IIc), (IId), (IIf), (IIg), and (IIi), p is 0. In another embodiment, p is 1. In yet another embodiment, p is 2. In another embodiment, p is 0 or 1. In yet another embodiment, p is 1 or 2.

In some embodiments of Formula (II), (IIa), (IIb), (IIc), (IId), (IIf), (IIg), and (IIi), r is 0. In another embodiment, r is 1. In yet another embodiment, r is 2. In another embodiment, r is 0 or 1. In yet another embodiment, r is 1 or 2.

In some embodiments of Formula (II), (IIa), (IIb), (IIc), (IId), (IIf), (IIg), and (IIi), o is 1. In another embodiment, o is 2. In another embodiment, o is 3. In another embodiment, o is 4. In another embodiment, o is 1, 2, or 3. In another embodiment, o is 1 or 2. In another embodiment, o is 2 or 3. In another embodiment, o is 3 or 4.

In some embodiments of Formula (II), (IIa), (IIb), (IIc), (IId), (IIf), (IIg), and (IIi), the dotted line is a double bond. In other embodiments, the dotted line is absent.

In some embodiments of Formula (II), (IIa), (IIb), (IIc), (IId), (IIf), (IIg), and (IIi), R^{c} is -CN.

In some embodiments of Formula (II), (IIa), (IIb), (IIc), (IId), (IIf), (IIg), and (IIi), R^{d} is H.

In some embodiments of Formula (II), (IIa), (IIb), (IIc), (IId), (IIf), (IIg), and (IIi), R¹ is C₆-C₁₀ aryl substituted with R^{a} and R^{b}, and optionally substituted with one to two R^{e}. In another embodiment, R¹ is phenyl substituted with R^{a} and R^{b}, and optionally substituted with one to two R^{e}.

In some embodiments of Formula (II), (IIa), (IIb), (IIc), (IId), (IIf), (IIg), and (IIi), R¹ is C₆-C₁₀ aryl substituted with R^{a} and R^{b}. In another embodiment, R¹ is phenyl substituted with R^{a} and R^{b}.

In some embodiments of Formula (II), (IIa), (IIb), (IIc), (IId), (IIf), (IIg), and (IIi), R¹ is heteroaryl substituted with R^{a} and R^{b}, and optionally substituted with one to two R^{e}. In another embodiment, R¹ is pyridinyl substituted with R^{a} and R^{b}, and optionally substituted with one to two R^{e}.

In some embodiments of Formula (II), (IIa), (IIb), (IIc), (IId), (IIf), (IIg), and (IIi), R¹ is heteroaryl. In another embodiment, R¹ is pyridinyl or thiophenyl. In yet another embodiment, R¹ is thiophenyl. In another embodiment, R¹ is pyridinyl.

In some embodiments of Formula (II), (IIa), (IIb), (IIc), (IId), (IIf), (IIg), and (IIi), R¹ is heteroaryl substituted with R^{a} and R^{b}. In another embodiment, R¹ is pyridinyl substituted with R^{a} and R^{b}.

In some embodiments of Formula (II), (IIa), (IIb), (IIc), (IId), (IIf), (IIg), and (IIi), R^{a} is H and R^{b} is -(C(R^{f})₂)ᵣCO₂R^{x}, -O(C(R^{f})₂)ᵣCO₂R^{x},
-(C(R^{f})₂)ᵣC₆-C₁₀ aryl. In another embodiment, R^{a} is H and R^{b} is -CO₂H, -CH₂CO₂H,-OCH₂CO₂R^{x}, -OCH(CH₃) CO₂R^{x},-OC(CH₃)₂CO₂R^{x}, or

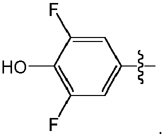

In some embodiments of Formula (II), (IIa), (IIb), (IIc), (IId), (IIf), (IIg), and (IIi), R^{a} is OR^{y} and R^{b} is -O(C(R^{f})₂)ᵣCO₂R^{x}, or -O(C(R^{f})₂)ᵣheteroaryl. In another embodiment, R^{a} is H and R^{b} is -(C(R^{f})₂)ᵣCO₂R^{x}, -Y²(C(R^{f})₂)ᵣCO₂R^{x}, -(C(R^{f})₂)ᵣS-C₆-C₁₀ aryl, -(C(R^{f})₂)ᵣheteroaryl,-(C(R^{f})₂)ᵣP(O)₂OH, -(C(R^{f})₂)ᵣS(O)₂OH, or-CH=CHCO₂R^{x}, wherein the aryl and heteroaryl are substituted with one to three substituents selected from halogen and OH; or R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H.

In some embodiments of Formula (II), (IIa), (IIb), (IIc), (IId), (IIf), (IIg), and (IIi), R^{a} is H, -CH₃, -OCH₃,-OH, -OCH₂CH₃, -OCH₂CH₂CH₃, -OCH₂CH(CH₃)₂, -O-cyclopropyl, -O-pyrrolidinyl, or ₋OCH₂cyclopropyl, or R^{a} and R^{b} when on adjacent atoms together with the atoms to which they are attached form a 5- to 6-membered heteroaryl ring optionally substituted with one or more -CO₂H; and R^{b} is -CH₃,-CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

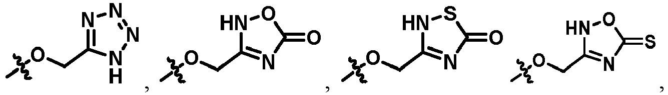

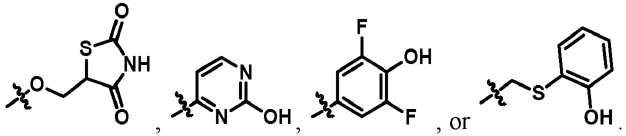

In some embodiments of Formula (II), (IIa), (IIb), (IIc), (IId), (IIf), (IIg), and (IIi), R^{a} is H, -OCH₃, -OH, or -OCH₂CH₃, or R^{a} and R^{b} when on adjacent atoms together with the atoms to which they are attached form a 5- to 6-membered heteroaryl ring optionally substituted with one or more -CO₂H; and R^{b} is -CO₂H, -(CH₂)CO₂H, -O(CH₂)CO₂H, -S(CH₂)CO₂H, -NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

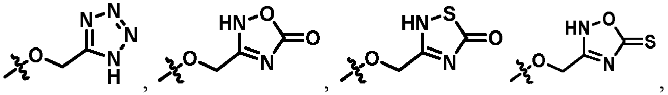

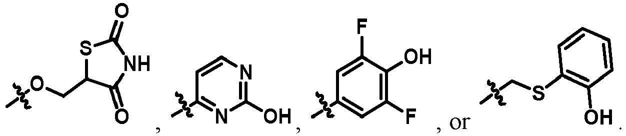

In some embodiments of Formula (II), (IIa), (IIb), (IIc), (IId), (IIf), (IIg), and (IIi), R^{a} is H, -OCH₃, or -OCH₂CH₃; and R^{b} is -CO₂H, -(CH₂)CO₂H, -O(CH₂)CO₂H, -S(CH₂)CO₂H,-NH(CH₂)CO₂H, -O(CH₂CH₂)CO₂H, -C(CH₂)₂CO₂H, -CH(CH₂)CO₂H, -O(CH₂)CO₂CH₂CH₃, -CH=CHCO₂H, -O-CH₂CH₂OH, -O(CH₂CH₂)P(O)₂OH, -O(CH₂CH₂)S(O)₂OH, - O(CH₂)C(O)NH₂, -O(CH₂)C(O)NHOH, -O(CH₂)C(O)NS(O)₂N(CH₃)₂,-O(CH₂)C(O)NS(O)₂CH₃,

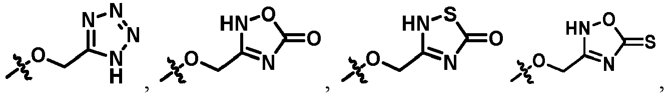

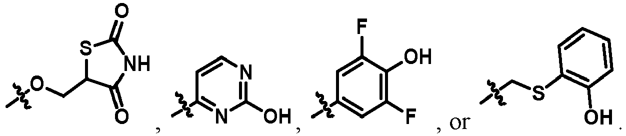

In some embodiments of Formula (II), (IIa), (IIb), (IIc), (IId), (IIf), (IIg), and (IIi), R^{a} is H or -OCH₃; and R^{b} is -CO₂H, -(CH₂)CO₂H, -O(CH₂)CO₂H, -S(CH₂)CO₂H, -NH(CH₂)CO₂H, -O(CH₂CH₂)CO₂H, -CH=CHCO₂H, -O-CH₂CH₂OH, -O(CH₂CH₂)P(O)₂OH, -O(CH₂CH₂)S(O)₂OH, -O(CH₂)C(O)NH₂, -O(CH₂)C(O)NHOH, -O(CH₂)C(O)NS(O)₂N(CH₃)₂, -O(CH₂)C(O)NS(O)₂CH₃,

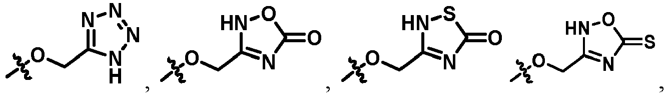

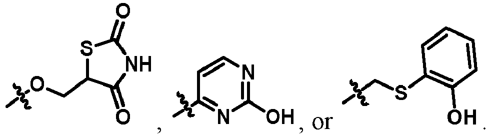

In some embodiments of Formula (II), (IIa), (IIb), (IIc), (IId), (IIf), (IIg), and (IIi), R^{a} is H, -OCH₃, or -OCH₂CH₃; and R^{b} is -CO₂H, -(CH₂)CO₂H, -O(CH₂)CO₂H, -C(CH₂)₂CO₂H, -CH(CH₂)CO₂H, or

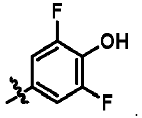

In some embodiments of Formula (II), (IIa), (IIb), (IIc), (IId), (IIf), (IIg), and (IIi), R^{a} is H, -OH, -OCH₃, or -OCH₂CH₃; and R^{b} is -CO₂H, -(CH₂)CO₂H, -O(CH₂)CO₂H, -C(CH₂)₂CO₂H, -CH(CH₂)CO₂H, -O(CH₂)CO₂CH₂CH₃,

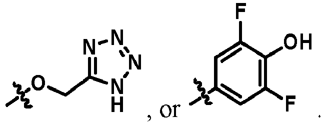

In some embodiments of Formula (II), (IIa), (IIb), (IIc), (IId), (IIf), (IIg), and (IIi), R^{a} is H or -OCH₃; and R^{b} is -O(CH₂)CO₂H, -C(CH₂)₂CO₂H, -CH(CH₂)CO₂H, or

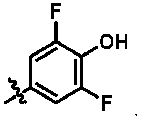

In some embodiments of Formula (II), (IIa), (IIb), (IIc), (IId), (IIf), (IIg), and (IIi), one of R^{a} or R^{b} is a carboxylic acid or a carboxylic acid bioisostere.

In some embodiments of Formula (II), (IIa), (IIb), (IIc), (IId), (IIf), (IIg), and (IIi), R^{a} is -CO₂H,-(CH₂)CO₂H, or -OCH₂CO₂H. In other embodiments, R^{a} is -CO₂CH₃,-CO₂CH₂CH₃, -CO₂CH₂CH₂CH₃, -CO₂CH(CH₃)₂, -(CH₂)CO₂CH₃, -(CH₂)CO₂CH₂CH₃,-(CH₂)CO₂CH₂CH₂CH₃, or -(CH₂)CO₂CH(CH₃)₂.

In some embodiments of Formula (II), (IIa), (IIb), (IIc), (IId), (IIf), (IIg), and (IIi), R^{a} is -P(O)(OH)OH, -(CH₂)P(O)(OH)OH, -P(O)(OH)OCH₃, -P(O)(OH)OCH₂CH₃,-P(O)(OH)OCH₂CH₂CH₃, -P(O)(OH)OCH(CH₃)₂, -(CH₂) P(O)(OH)OCH₃, -(CH₂)P(O)(OH)OCH₂CH₃, -(CH₂)P(O)(OH)OCH₂CH₂CH₃, or -(CH₂)P(O)(OH)OCH(CH₃)₂.

In some embodiments of Formula (II), (IIa), (IIb), (IIc), (IId), (IIf), (IIg), and (IIi), R^{a} is -S(O)₂OH, -(CH₂)S(O)₂OH, -C(O)NHCN, or
-(CH₂)C(O)NHCN.

In some embodiments of Formula (II), (IIa), (IIb), (IIc), (IId), (IIf), (IIg), and (IIi), R^{a} is -C(O)NHS(O)₂CH₃, -C(O)NHS(O)₂CH₂CH₃, -C(O)NHS(O)₂CH₂CH₂CH₃, -C(O)NHS(O)₂CH(CH₃)₂, -(CH₂)C(O)NHS(O)₂CH₃, -(CH₂)C(O)NHS(O)₂CH₂CH₃, -(CH₂)C(O)NHS(O)₂CH₂CH₂CH₃, or -(CH₂)C(O)NHS(O)₂CH(CH₃)₂.

In some embodiments of Formula (II), (IIa), (IIb), (IIc), (IId), (IIf), (IIg), and (IIi), R^{a} is 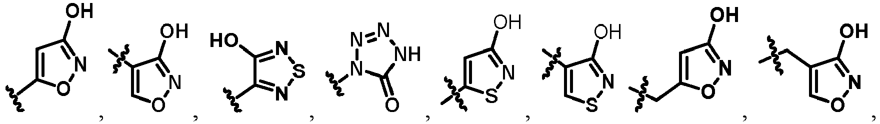 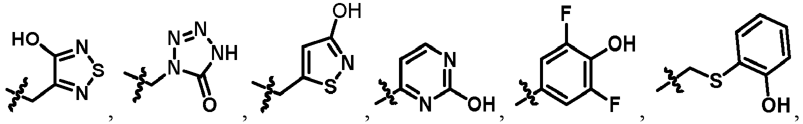 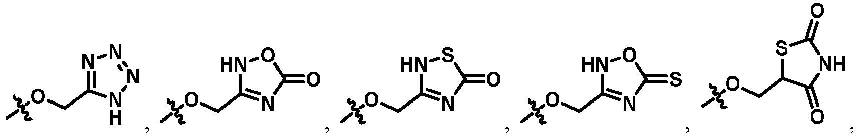 or

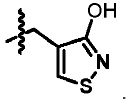

In some embodiments of Formula (II), (IIa), (IIb), (IIc), (IId), (IIf), (IIg), and (IIi), R^{a} is

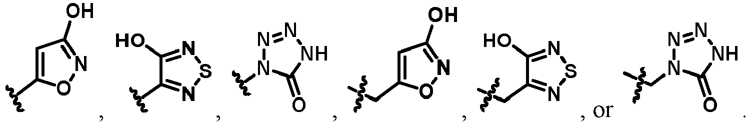

In some embodiments of the Formula above, R^{a} is

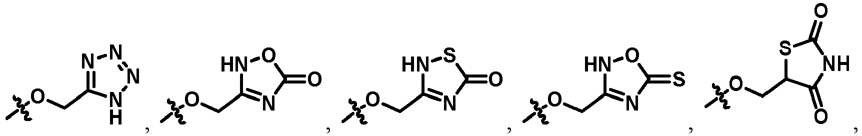

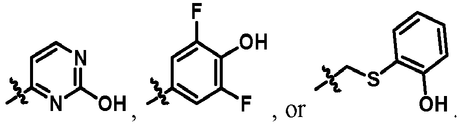

In some embodiments of Formula (II), (IIa), (IIb), (IIc), (IId), (IIf), (IIg), and (IIi), R^{b} is -CO₂H, -(CH₂)CO₂H, or -OCH₂CO₂H. In other embodiments, R^{b} is -CO₂CH₃,-CO₂CH₂CH₃, -CO₂CH₂CH₂CH₃, -CO₂CH(CH₃)₂, -(CH₂)CO₂CH₃, -(CH₂)CO₂CH₂CH₃,-(CH₂)CO₂CH₂CH₂CH₃, or -(CH₂)CO₂CH(CH₃)₂.

In some embodiments of Formula (II), (IIa), (IIb), (IIc), (IId), (IIf), (IIg), and (IIi), R^{b} is -P(O)(OH)OH, -(CH₂)P(O)(OH)OH, -P(O)(OH)OCH₃, -P(O)(OH)OCH₂CH₃,-P(O)(OH)OCH₂CH₂CH₃, -P(O)(OH)OCH(CH₃)₂, -(CH₂) P(O)(OH)OCH₃, -(CH₂)P(O)(OH)OCH₂CH₃, -(CH₂)P(O)(OH)OCH₂CH₂CH₃, or -(CH₂)P(O)(OH)OCH(CH₃)₂.

In some embodiments of Formula (II), (IIa), (IIb), (IIc), (IId), (IIf), (IIg), and (IIi), R^{b} is -S(O)₂OH,-(CH₂)S(O)₂OH, -C(O)NHCN, or
-(CH₂)C(O)NHCN.

In some embodiments of Formula (II), (IIa), (IIb), (IIc), (IId), (IIf), (IIg), and (IIi), R^{b} is -C(O)NHS(O)₂CH₃, -C(O)NHS(O)₂CH₂CH₃, -C(O)NHS(O)₂CH₂CH₂CH₃, -C(O)NHS(O)₂CH(CH₃)₂, -(CH₂)C(O)NHS(O)₂CH₃, -(CH₂)C(O)NHS(O)₂CH₂CH₃, -(CH₂)C(O)NHS(O)₂CH₂CH₂CH₃, or -(CH₂)C(O)NHS(O)₂CH(CH₃)₂.

In some embodiments of Formula (II), (IIa), (IIb), (IIc), (IId), (IIf), (IIg), and (IIi), R^{b} is 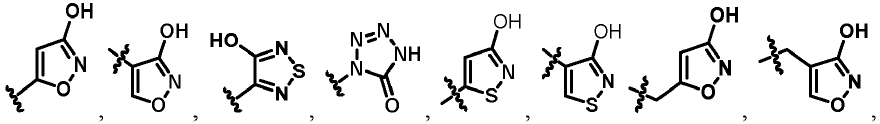 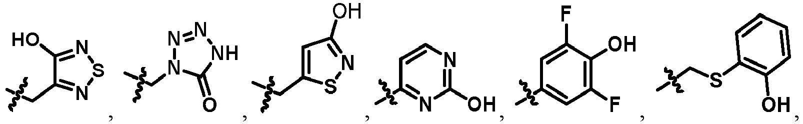 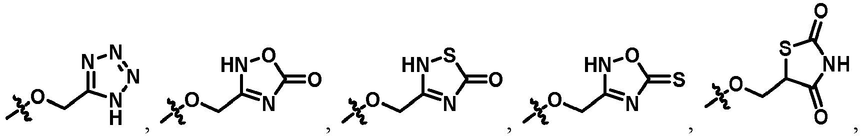 or

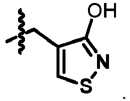

In some embodiments of Formula (II), (IIa), (IIb), (IIc), (IId), (IIf), (IIg), and (IIi), R^{b} is

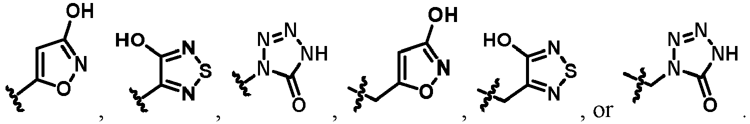

In some embodiments of Formula (II), (IIa), (IIb), (IIc), (IId), (IIf), (IIg), and (IIi), R^{b} is

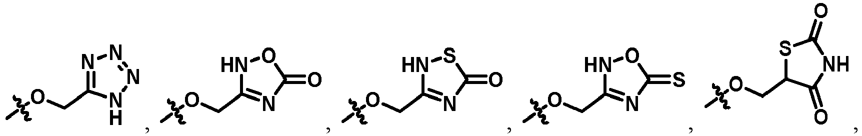

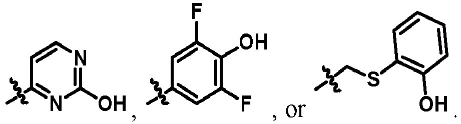

In some embodiments, the compound of Formula (II) used in the methods of the present disclosure is a compound selected from:

| **Cmpd No.** | **Structure** | **Chemical Name** |
|---|---|---|
| **I-1** | 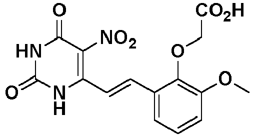 | (E)-2-(2-methoxy-6-(2-(5-nitro-2,6-dioxo-1,2,3,6-tetrahydropyrimidin-4-yl)vinyl)phenoxy)acetic acid |
| **I-2** | 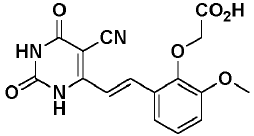 | (E)-2-(2-(2-(5-cyano-2, 6-dioxo-1,2,3,6-tetrahydropyrimidin-4-yl)vinyl)-6-methoxyphenoxy)acetic acid; |
| **I-3** | 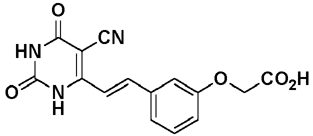 | (E)-2-(3-(2-(5-cyano-2,6-dioxo-1,2,3,6-tetrahydropyrimidin-4-yl)vinyl)phenoxy)acetic acid; |
| **I-4** | 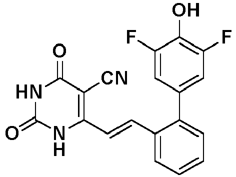 | (E)-6-(2-(3',5'-difluoro-4'-hydroxy-[1,1'-biphenyl]-2-yl)vinyl)-2,4-dioxo-1,2,3,4-tetrahydropyrimidine-5-carbonitrile; |
| **I-5** | 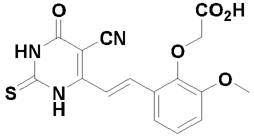 | (E)-2-(2-(2-(5-cyano-6-oxo-2-thioxo-1,2,3,6-tetrahydropyrimidin-4-yl)vinyl)-6-methoxyphenoxy)acetic acid; |
| **I-6** | 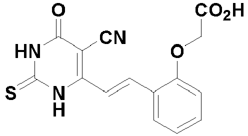 | (E)-2-(2-(2-(5-cyano-6-oxo-2-thioxo-1,2,3,6-tetrahydropyrimidin-4-yl)vinyl)phenoxy)acetic acid; |
| **I-7** | 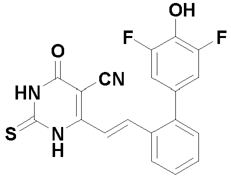 | (E)-6-(2-(3',5'-difluoro-4'-hydroxy-[1,1'-biphenyl]-2-yl)vinyl)-4-oxo-2-thioxo-1,2,3,4-tetrahydropyrimidine-5-carbonitrile; |
| **I-8** | 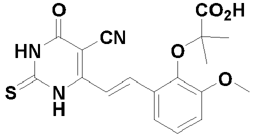 | (E)-2-(2-(2-(5-cyano-6-oxo-2-thioxo-1,2,3,6-tetrahydropyrimidin-4-yl)vinyl)-6-methoxyphenoxy)-2-methylpropanoic acid; |
| **I-9** | 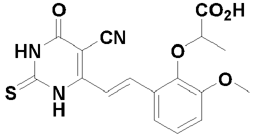 | (E)-2-(2-(2-(5-cyano-6-oxo-2-thioxo-1,2,3,6-tetrahydropyrimidin-4-yl)vinyl)-6-methoxyphenoxy)propanoic acid; |
| **I-11** | 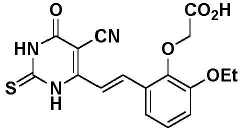 | (E)-2-(2-(2-(5-cyano-6-oxo-2-thioxo-1,2,3,6-tetrahydropyrimidin-4-yl)vinyl)-6-ethoxyphenoxy)acetic acid; |
| **I-12** | 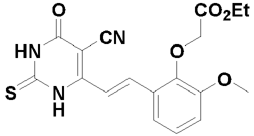 | ethyl (E)-2-(2-(2-(5-cyano-6-oxo-2-thioxo-1,2,3,6-tetrahydropyrimidin-4-yl)vinyl)-6-methoxyphenoxy)acetate; |
| **I-14** | 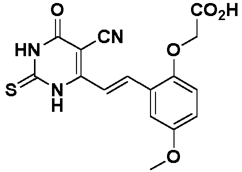 | (E)-2-(2-(2-(5-cyano-6-oxo-2-thioxo-1,2,3,6-tetrahydropyrimidin-4-yl)vinyl)-4-methoxyphenoxy)acetic acid; |
| **I-15** | 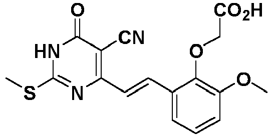 | (E)-2-(2-(2-(5-cyano-2-(methylthio)-6-oxo-1,6-dihydropyrimidin-4-yl)vinyl)-6-methoxyphenoxy)acetic acid; |
| **I-17** | 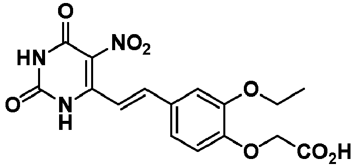 | (E)-2-(2-ethoxy-4-(2-(5-nitro-2,6-dioxo-1,2,3,6-tetrahydropyrimidin-4-yl)vinyl)phenoxy)acetic acid |
| **I-18** | 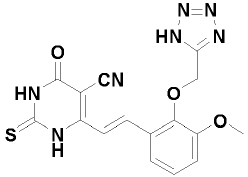 | (E)-6-(2-((1H-tetrazol-5-yl)methoxy)-3-methoxystyryl)-4-oxo-2-thioxo-1,2,3,4-tetrahydropyrimidine-5-carbonitrile; and |
| **I-19** | 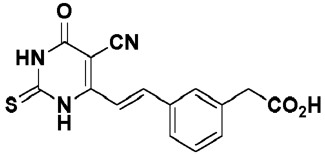 | (E)-2-(3-(2-(5-cyano-6-oxo-2-thioxo-1,2,3,6-tetrahydropyrimidin-4-yl)vinyl)phenyl)acetic acid; |
| **I-20** | 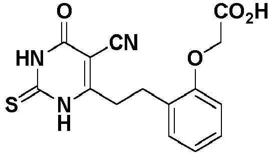 | 2-(2-(2-(5-cyano-6-oxo-2-thioxo-1,2,3,6-tetrahydropyrimidin-4-yl)ethyl)phenoxy)acetic acid; |
| **I-23** | 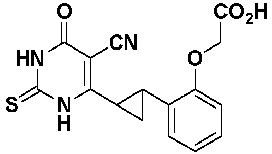 | 2-(2-(2-(5-cyano-6-oxo-2-thioxo-1,2,3,6-tetrahydropyrimidin-4-yl)cyclopropyl)phenoxy)acetic acid; |
| **I-25** | 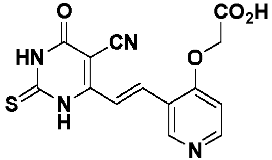 | (E)-2-((3-(2-(5-cyano-6-oxo-2-thioxo-1,2,3,6-tetrahydropyrimidin-4-yl)vinyl)pyridin-4-yl)oxy)acetic acid; |
| **I-26** | 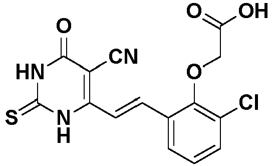 | (E)-2-(2-chloro-6-(2-(5-cyano-6-oxo-2-thioxo-1,2,3,6-tetrahydropyrimidin-4-yl)vinyl)phenoxy)acetic acid; |
| **I-27** | 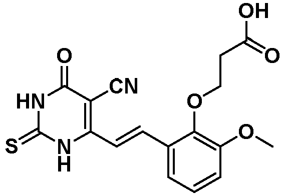 | (E)-3-(2-(2-(5-cyano-6-oxo-2-thioxo-1,2,3,6-tetrahydropyrimidin-4-yl)vinyl)-6-methoxyphenoxy)propanoic acid; |
| **I-28** | 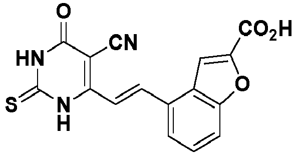 | (E)-4-(2-(5-cyano-6-oxo-2-thioxo-1,2,3,6-tetrahydropyrimidin-4-yl)vinyl)benzofuran-2-carboxylic acid; |
| **I-30** | 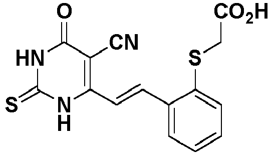 | (E)-2-((2-(2-(5-cyano-6-oxo-2-thioxo-1,2,3,6-tetrahydropyrimidin-4-yl)vinyl)phenyl)thio)acetic acid; |
| **I-31** | 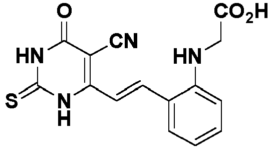 | (E)-(2-(2-(5-cyano-6-oxo-2-thioxo-1,2,3,6-tetrahydropyrimidin-4-yl)vinyl)phenyl)glycine; |
| **I-32** | 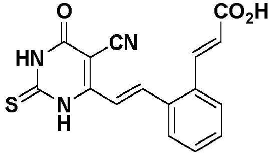 | (E)-3-(2-((E)-2-(5-cyano-6-oxo-2-thioxo-1,2,3,6-tetrahydropyrimidin-4-yl)vinyl)phenyl)acrylic acid; |
| **I-35** | 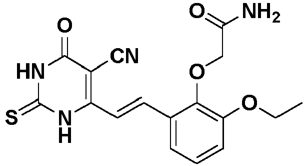 | (E)-2-(2-(2-(5-cyano-6-oxo-2-thioxo-1,2,3,6-tetrahydropyrimidin-4-yl)vinyl)-6-ethoxyphenoxy)acetamide |
| **I-36** | 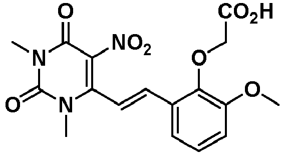 | (E)-2-(2-(2-(1,3-dimethyl-5-nitro-2,6-dioxo-1,2,3,6-tetrahydropyrimidin-4-yl)vinyl)-6-methoxyphenoxy)acetic acid. |
| **I-37** | 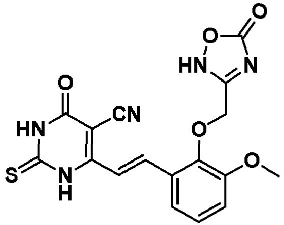 | (E)-6-(3-methoxy-2-((5-oxo-2, 5-dihydro-1,2,4-oxadiazol-3-yl)methoxy)styryl)-4-oxo-2-thioxo-1,2,3,4-tetrahydropyrimidine-5-carbonitrile |
| **I-38** | 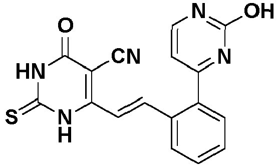 | (E)-6-(2-(2-hydroxypyrimidin-4-yl)styryl)-4-oxo-2-thioxo-1,2,3,4-tetrahydropyrimidine-5-carbonitrile; |
| **I-39** | 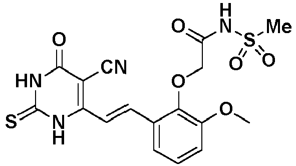 | (E)-2-(2-(2-(5-cyano-6-oxo-2-thioxo-1,2,3,6-tetrahydropyrimidin-4-yl)vinyl)-6-methoxyphenoxy)-N-(methylsulfonyl)acetamide; |
| **I-40** | 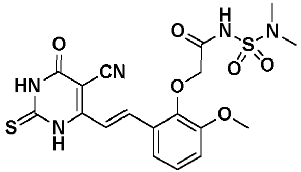 | (E)-2-(2-(2-(5-cyano-6-oxo-2-thioxo-1,2,3,6-tetrahydropyrimidin-4-yl)vinyl)-6-methoxyphenoxy)-N-(N,N-dimethylsulfamoyl)acetamide; |
| **I-41** | 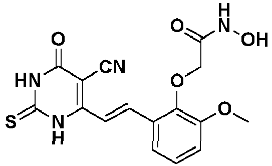 | (E)-2-(2-(2-(5-cyano-6-oxo-2-thioxo-1,2,3,6-tetrahydropyrimidin-4-yl)vinyl)-6-methoxyphenoxy)-N-hydroxy acetamide; |
| **I-42** | 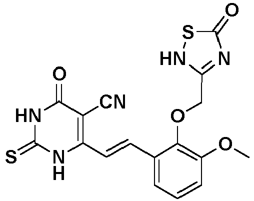 | (E)-6-(3-methoxy-2-((5-oxo-2,5-dihydro-1,2,4-thiadiazol-3-yl)methoxy)styryl)-4-oxo-2-thioxo-1,2,3,4-tetrahydropyrimidine-5-carbonitrile; |
| **I-43** | 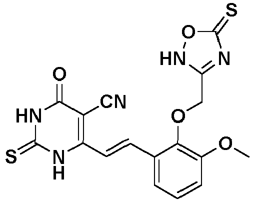 | (E)-6-(3-methoxy-2-((5-thioxo-2,5-dihydro-1,2,4-oxadiazol-3-yl)methoxy)styryl)-4-oxo-2-thioxo-1,2,3,4-tetrahydropyrimidine-5-carbonitrile; |
| **I-44** | 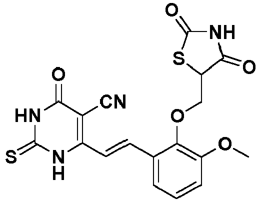 | (E)-6-(2-((2,4-dioxothiazolidin-5-yl)methoxy)-3-methoxystyryl)-4-oxo-2-thioxo-1,2,3,4-tetrahydropyrimidine-5-carbonitrile; |
| **I-45** | 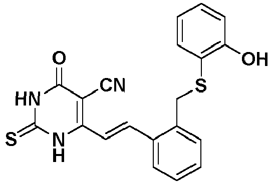 | (E)-6-(2-(((2-hydroxyphenyl)thio)methyl)styryl)-4-oxo-2-thioxo-1,2,3,4-tetrahydropyrimidine-5-carbonitrile; |
| **I-46** | 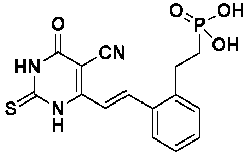 | (E)-(2-(2-(5-cyano-6-oxo-2-thioxo-1,2,3,6-tetrahydropyrimidin-4-yl)vinyl)phenethyl)phosphonic acid; |
| **I-47** | 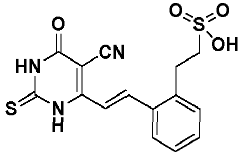 | (E)-2-(2-(2-(5-cyano-6-oxo-2-thioxo-1,2,3,6-tetrahydropyrimidin-4-yl)vinyl)phenyl)ethane-1-sulfonic acid; |
| **I-48** | 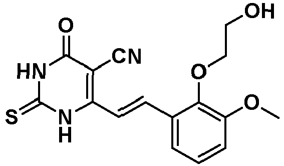 | (E)-6-(2-(2-hydroxyethoxy)-3-methoxystyryl)-4-oxo-2-thioxo-1,2,3,4-tetrahydropyrimidine-5-carbonitrile; |
| **I-49** | 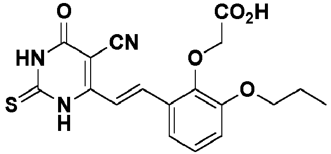 | (E)-2-(2-(2-(5-cyano-6-oxo-2-thioxo-1,2,3,6-tetrahydropyrimidin-4-yl)vinyl)-6-propoxyphenoxy)acetic acid |
| **I-50** | 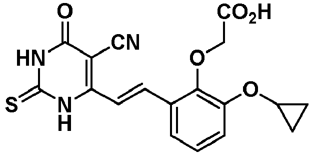 | (E)-2-(2-(2-(5-cyano-6-oxo-2-thioxo-1,2,3,6-tetrahydropyrimidin-4-yl)vinyl)-6-cyclopropoxyphenoxy)acetic acid |
| **I-51** | 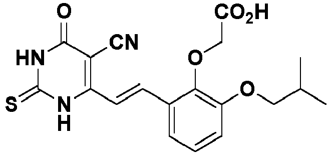 | (E)-2-(2-(2-(5-cyano-6-oxo-2-thioxo-1,2,3,6-tetrahydropyrimidin-4-yl)vinyl)-6-isobutoxyphenoxy)acetic acid |
| **I-52** | 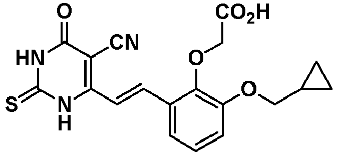 | (E)-2-(2-(2-(5-cyano-6-oxo-2-thioxo-1,2,3,6-tetrahydropyrimidin-4-yl)vinyl)-6-(cyclopropylmethoxy)phenoxy)acetic acid |
| **I-53** | 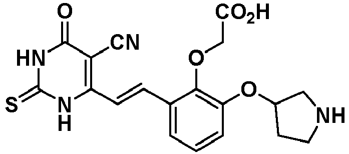 | (E)-2-(2-(2-(5-cyano-6-oxo-2-thioxo-1,2,3,6-tetrahydropyrimidin-4-yl)vinyl)-6-(pyrrolidin-3-yloxy)phenoxy)acetic acid |
| **I-59** | 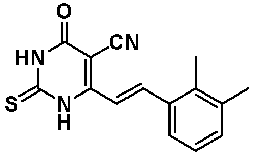 | (E)-6-(2,3-dimethylstyryl)-4-oxo-2-thioxo-1,2,3,4-tetrahydropyrimidine-5-carbonitrile |
| **I-60** | 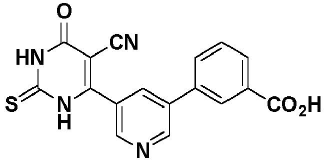 | 3-(5-(5-cyano-6-oxo-2-thioxo-1,2,3,6-tetrahydropyrimidin-4-yl)pyridin-3-yl)benzoic acid |
| **I-61** | 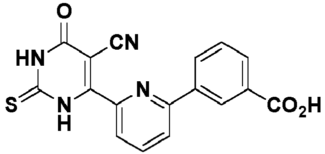 | 3-(6-(5-cyano-6-oxo-2-thioxo-1,2,3,6-tetrahydropyrimidin-4-yl)pyridin-2-yl)benzoic acid |
| **I-62** | 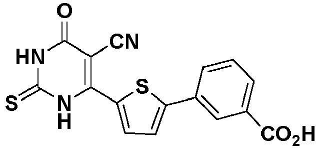 | 3-(5-(5-cyano-6-oxo-2-thioxo-1,2,3,6-tetrahydropyrimidin-4-yl)thiophen-2-yl)benzoic acid |
| **I-76** | 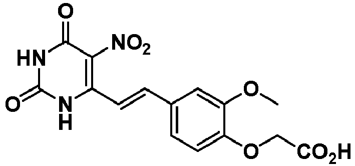 | (E)-2-(2-methoxy-4-(2-(5-nitro-2,6-dioxo-1,2,3,6-tetrahydropyrimidin-4-yl)vinyl)phenoxy)acetic acid; and |
| **I-77** | 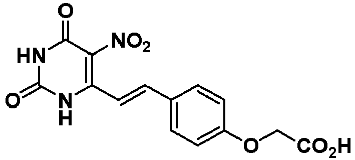 | (E)-2-(4-(2-(5-nitro-2,6-dioxo-1,2,3,6-tetrahydropyrimidin-4-yl)vinyl)phenoxy)acetic acid; and |
| **I-78** | 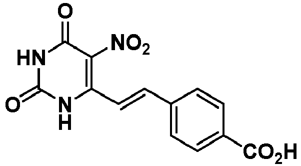 | (E)-4-(2-(5-nitro-2,6-dioxo-1,2,3,6-tetrahydropyrimidin-4-yl)vinyl)benzoic acid. |

It should be understood, that such references are intended to encompass not only the above general formula, but also each and every of the embodiments, etc. discussed in the following. It should also be understood, that unless stated to the opposite, such references also encompass pharmaceutically acceptable salts and solvates of the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi).

### Definitions

The term "alkyl" as used herein refers to a saturated, straight or branched hydrocarbon chain. The hydrocarbon chain preferably contains from one to eight carbon atoms (C₁₋₈-alkyl), more preferred from one to six carbon atoms (C₁₋₆-alkyl), in particular from one to four carbon atoms (C₁₋₄-alkyl), including methyl, ethyl, propyl, isopropyl, butyl, isobutyl, secondary butyl, tertiary butyl, pentyl, isopentyl, neopentyl, tertiary pentyl, hexyl, isohexyl, heptyl and octyl. In a preferred embodiment "alkyl" represents a C₁₋₄-alkyl group, which may in particular include methyl, ethyl, propyl, isopropyl, butyl, isobutyl, secondary butyl, and tertiary butyl. Correspondingly, the term "alkylene" means the corresponding biradical (-alkyl-).

The term "cycloalkyl" or "carbocycle" as used herein refers to a cyclic alkyl group, preferably containing from three to ten carbon atoms (C₃₋₁₀-cycloalkyl or C₃₋₁₀-carbocycle), such as from three to eight carbon atoms (C₃₋₈-cycloalkyl or C₃₋₁₀-carbocycle), preferably from three to six carbon atoms (C₃₋₆-cycloalkyl or C₃₋₁₀-carbocycle), including cyclopropyl, cyclobutyl, cyclopentyl, cyclohexyl, cycloheptyl and cyclooctyl. Furthermore, the term "cycloalkyl" as used herein may also include polycyclic groups such as for example bicyclo[2.2.2]octyl, bicyclo[2.2.1]heptanyl, decalinyl and adamantyl. Correspondingly, the term "cycloalkylene" means the corresponding biradical (-cycloalkyl-). Alkyl and cycloalkyl groups may be optionally substituted with 1-4 substituents. Examples of substituents on alkyl groups include, but are not limited to, alkyl, alkenyl, alkynyl, halogen, haloalkyl, alkoxy, heteroaryl, aryl, carbocyclyl, hydroxyl, carbamoyl, oxo, and -CN.

The term "alkenyl" as used herein refers to a straight or branched hydrocarbon chain or cyclic hydrocarbons containing one or more double bonds, including di-enes, tri-enes and poly-enes. Typically, the alkenyl group comprises from two to eight carbon atoms (C₂₋₈-alkenyl), such as from two to six carbon atoms (C₂₋₆-alkenyl), in particular from two to four carbon atoms (C₂₋₄-alkenyl), including at least one double bond. Examples of alkenyl groups include ethenyl; 1- or 2-propenyl; 1-, 2- or 3-butenyl, or 1,3-but-dienyl; 1-, 2-, 3-, 4- or 5-hexenyl, or 1,3-hex-dienyl, or 1,3,5-hex-trienyl; 1-, 2-, 3-, 4-, 5-, 6-, or 7-octenyl, or 1,3-octadienyl, or 1,3,5-octatrienyl, or 1,3,5,7-octatetraenyl, or cyclohexenyl. Correspondingly, the term "alkenylene" means the corresponding biradical (-alkenyl-). Alkenyl groups may be optionally substituted with 1-4 substituents. Examples of substituents on alkenyl groups include, but are not limited to, alkyl, alkenyl, alkynyl, halogen, haloalkyl, alkoxy, heteroaryl, aryl, carbocyclyl, hydroxyl, carbamoyl, oxo, and -CN.

The term "alkynyl" as used herein refers to a straight or branched hydrocarbon chain containing one or more triple bonds, including di-ynes, tri-ynes and poly-ynes. Typically, the alkynyl group comprises of from two to eight carbon atoms (C₂₋₈-alkynyl), such as from two to six carbon atoms (C₂₋₆-alkynyl), in particular from two to four carbon atoms (C₂₋₄-alkynyl), including at least one triple bond. Examples of preferred alkynyl groups include ethynyl; 1-or 2-propynyl; 1-, 2- or 3-butynyl, or 1,3-but-diynyl; 1-, 2-, 3-, 4- or 5-hexynyl, or 1,3-hex-diynyl, or 1,3,5-hex-triynyl; 1-, 2-, 3-, 4-, 5-, 6-, or 7-octynyl, or 1,3-oct-diynyl, or 1,3,5-oct-triynyl, or 1,3,5,7-oct-tetraynyl. Correspondingly, the term "alkynylene" means the corresponding biradical (-alkynyl-). Alkynyl groups may be optionally substituted with 1-4 substituents. Examples of substituents on alkynyl groups include, but are not limited to" alkyl, alkenyl, alkynyl, halogen, haloalkyl, alkoxy, heteroaryl, aryl, carbocyclyl, hydroxyl, carbamoyl, oxo, and -CN.

The terms "halo" and "halogen" as used herein refer to fluoro, chloro, bromo or iodo. Thus a trihalomethyl group represents, e.g., a trifluoromethyl group, or a trichloromethyl group. Preferably, the terms "halo" and "halogen" designate fluoro or chloro.

The term "haloalkyl" as used herein refers to an alkyl group, as defined herein, which is substituted one or more times with one or more halogen. Examples of haloalkyl groups include, but are not limited to, trifluoromethyl, difluoromethyl, pentafluoroethyl, trichloromethyl, etc.

The term "alkoxy" as used herein refers to an "alkyl-O-" group, wherein alkyl is as defined above.

The term "hydroxyalkyl" as used herein refers to an alkyl group (as defined hereinabove), which alkyl group is substituted one or more times with hydroxy. Examples of hydroxyalkyl groups include HO-CH₂-, HO-CH₂-CH₂- and CH₃-CH(OH)-.

The term "oxy" as used herein refers to an "-O-" group.

The term "oxo" as used herein refers to an "=O" group.

The term "amine" as used herein refers to primary (R-NH₂, R ≠ H), secondary ((R)₂-NH, (R)₂ ≠ H) and tertiary ((R)₃-N, R ≠ H) amines. A substituted amine is intended to mean an amine where at least one of the hydrogen atoms has been replaced by the substituent.

The term "carbamoyl" as used herein refers to a "H₂N(C=O)-" group.

The term "aryl", as used herein, unless otherwise indicated, includes carbocyclic aromatic ring systems derived from an aromatic hydrocarbon by removal of a hydrogen atom. Aryl furthermore includes bi-, tri- and polycyclic ring systems. Examples of preferred aryl moieties include phenyl, naphthyl, indenyl, indanyl, fluorenyl, biphenyl, indenyl, naphthyl, anthracenyl, phenanthrenyl, pentalenyl, azulenyl, and biphenylenyl. Preferred "aryl" is phenyl, naphthyl or indanyl, in particular phenyl, unless otherwise stated. Any aryl used may be optionally substituted. Correspondingly, the term "arylene" means the corresponding biradical (-aryl-). Aryl groups may be optionally substituted with 1-4 substituents. Examples of substituents on aryl groups include, but are not limited to, alkyl, alkenyl, alkynyl, halogen, haloalkyl, alkoxy, heteroaryl, aryl, carbocyclyl, hydroxyl, and -CN.

The term "heteroaryl", as used herein, refers to aromatic groups containing one or more heteroatoms selected from O, S, and N, preferably from one to four heteroatoms, and more preferably from one to three heteroatoms. Heteroaryl furthermore includes bi-, tri- and polycyclic groups, wherein at least one ring of the group is aromatic, and at least one of the rings contains a heteroatom selected from O, S, and N. Heteroaryl also include ring systems substituted with one or more oxo moieties. Examples of preferred heteroaryl moieties include N-hydroxytetrazolyl, N-hydroxytriazolyl, N-hydroxyimidazolyl, furanyl, triazolyl, pyranyl, thiadiazinyl, benzothiophenyl, dihydro-benzo[b]thiophenyl, xanthenyl, isoindanyl, acridinyl, benzisoxazolyl, quinolinyl, isoquinolinyl, phteridinyl, azepinyl, diazepinyl, imidazolyl, thiazolyl, carbazolyl, pyridinyl, pyridazinyl, pyrimidinyl, pyrazolyl, pyrazinyl, tetrazolyl, furyl, thienyl, isoxazolyl, oxazolyl, isothiazolyl, pyrrolyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, azaindolyl, pyrazolinyl, 1,2,4-oxadiazol-5(4H)-one, and pyrazolidinyl. Non-limiting examples of partially hydrogenated derivatives are 1,2,3,4-tetrahydronaphthyl, 1,4-dihydronaphthyl, and 1-octalin. Correspondingly, the term "heteroarylene" means the corresponding biradical (-heteroaryl-). Heteroaryl groups may be optionally substituted with 1-4 substituents. Examples of substituents on heteroaryl groups include, but are not limited to, alkyl, alkenyl, alkynyl, halogen, haloalkyl, alkoxy, heteroaryl, aryl, carbocyclyl, hydroxyl, and -CN.

The term "heterocyclyl" as used herein, refers to cyclic saturated or partially unsaturated non-aromatic groups containing one or more heteroatoms selected from O, S, and N, preferably from one to four heteroatoms, and more preferably from one to three heteroatoms. Heterocyclyl furthermore includes bi-, tri- and polycyclic non-aromatic groups, and at least one of the rings contains a heteroatom selected from O, S, and N. Heterocyclyl also include ring systems substituted with one or more oxo moieties. Examples of heterocyclic groups are oxetane, pyrrolidinyl, pyrrolyl, 3H-pyrrolyl, oxolanyl, furanyl, thiolanyl, thiophenyl, pyrazolyl, pyrazolidinyl, imidazolyl, imidazolidinyl, 3H-pyrazolyl, 1,2-oxazolyl, 1,3-oxazolyl, 1,2-thiazolyl, 1,3-thiazolyl, 1,2,5-oxadiazolyl, piperidinyl, pyridinyl, oxanyl, 2-H-pyranyl, 4-H-pyranyl, thianyl, 2H-thiopyranyl, pyridazinyl, 1,2-diazinanyl, pyrimidinyl, 1,3-diazinanyl, pyrazinyl, piperazinyl, 1,4-dioxinyl, 1,4-dioxanyl, 1,3-diazinanyl, 1,4-oxazinyl, morpholinyl, thiomorpholinyl, 1,4-oxathianyl, benzofuranyl, isobenzofuranyl, indazolyl, benzimidazolyl, quinolinyl, isoquinolinyl, chromanyl, isochromanyl, 4H-chromenyl, 1H-isochromenyl, cinnolinyl, quinazolinyl, quinoxalinyl, phthalazinyl, purinyl, naphthyridinyl, pteridinyl, indolizinyl, 1H-pyrrolizinyl, 4H-quinolizinyl and aza-8-bicyclo[3.2.1]octane. Correspondingly, the term "heterocyclylene" means the corresponding biradical (-heterocyclyl-). Heterocyclyl groups may be optionally substituted with 1-4 substituents. Examples of substituents on heterocyclyl groups include, but are not limited, to alkyl, alkenyl, alkynyl, halogen, haloalkyl, alkoxy, heteroaryl, aryl, carbocyclyl, hydroxyl, and -CN.

The term "N-heterocyclic ring" as used herein, refers to a heterocyclyl or a heteroaryl, as defined hereinabove, having at least one nitrogen atom, and being bound via a nitrogen atom. Examples of such N-heterocyclic rings are pyrrolidinyl, pyrrolyl, 3H-pyrrolyl, pyrazolyl, pyrazolidinyl, imidazolyl, imidazolidinyl, 3H-pyrazolyl, 1,2-oxazolyl, 1,2-thiazolyl, 1,3-thiazolyl, piperidinyl, pyridinyl, pyridazinyl, pyrazinyl, piperazinyl, morpholinyl, pyridinyl, pyridazinyl, pyrimidinyl, pyrazolyl, pyrazinyl, tetrazolyl, etc.

### Isomers

In the present specification, the structural formula of the compound represents a certain isomer for convenience in some cases, but the present disclosure includes geometrical isomers, optical isomers based on an asymmetrical carbon, stereoisomers, and tautomers. Accordingly, it should be understood that the definition of compounds of Formulae (I), (Ia), (Ib), (Ic), (Id), (Ie), (If), (Ig), (Ih), (Ii), (Ij), (II) (IIa), (IIb), (IIc), (IId), (IIf), (IIg) and (IIi) include cis-trans isomers, stereoisomers and tautomers, as well as racemic mixtures of these and pharmaceutically acceptable salts thereof. Hence, the definition of compounds of Formulae (I), (Ia), (Ib), (Ic), (Id), (Ie), (If), (Ig), (Ih), (Ii), (Ij), (II) (IIa), (IIb), (IIc), (IId), (IIf), (IIg), and (IIi) are also intended to encompass all R- and S-isomers of a chemical structure in any ratio, *e.g.*, with enrichment (*i.e.*, enantiomeric excess or diastereomeric excess) of one of the possible isomers and corresponding smaller ratios of other isomers. In addition, a crystal polymorphism may be present for the compounds represented by Formulae (I), (Ia), (Ib), (Ic), (Id), (Ie), (If), (Ig), (Ih), (Ii), (Ij), (II) (IIa), (IIb), (IIc), (IId), (IIf), (IIg), and (IIi). It is noted that any crystal form, crystal form mixture, or anhydride or hydrate thereof is included in the scope of the present disclosure.

"Isomerism" means compounds that have identical molecular formulae but differ in the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images of each other are termed "enantiomers" or sometimes optical isomers. A mixture containing equal amounts of individual enantiomeric forms of opposite chirality is termed a "racemic mixture".

A carbon atom bonded to four non-identical substituents is termed a "chiral center".

"Chiral isomer" means a compound with at least one chiral center. Compounds with more than one chiral center may exist either as an individual diastereomer or as a mixture of diastereomers, termed "diastereomeric mixture". When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the Sequence Rule of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

Diastereoisomers, *i.e.*, non-superimposable stereochemical isomers, can be separated by conventional means such as chromatography, distillation, crystallization or sublimation. The optical isomers can be obtained by resolution of the racemic mixtures according to conventional processes, for example by formation of diastereoisomeric salts by treatment with an optically active acid or base. Examples of appropriate acids include, without limitation, tartaric, diacetyltartaric, dibenzoyltartaric, ditoluoyltartaric and camphorsulfonic acid. The mixture of diastereomers can be separated by crystallization followed by liberation of the optically active bases from these salts. An alternative process for separation of optical isomers includes the use of a chiral chromatography column optimally chosen to maximize the separation of the enantiomers. Still another available method involves synthesis of covalent diastereoisomeric molecules by reacting compounds of Formulae (I), (Ia), (Ib), (Ic), (Id), (Ie), (If), (Ig), (Ih), (Ii), (Ij), (II) (IIa), (IIb), (IIc), (IId), (IIf), (IIg), and (IIi) with an optically pure acid in an activated form or an optically pure isocyanate. The synthesized diastereoisomers can be separated by conventional means such as chromatography, distillation, crystallization or sublimation, and then hydrolyzed to obtain the enantiomerically pure compound. The optically active compounds of Formulae (I), (Ia), (Ib), (Ic), (Id), (Ie), (If), (Ig), (Ih), (Ii), (Ij), (II) (IIa), (IIb), (IIc), (IId), (IIf), (IIg), and (IIi) can likewise be obtained by utilizing optically active starting materials and/or by utilizing a chiral catalyst. These isomers may be in the form of a free acid, a free base, an ester or a salt. Examples of chiral separation techniques are given in Chiral Separation Techniques, A Practical Approach, 2nd ed. by G. Subramanian, Wiley-VCH, 2001.

"Geometric isomer" means the diastereomers that owe their existence to hindered rotation about double bonds. These configurations are differentiated in their names by the prefixes cis and trans, or Z and E, which indicate that the groups are on the same or opposite side of the double bond in the molecule according to the Cahn-Ingold-Prelog rules.

Furthermore, the structures and other compounds discussed in this disclosure include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques; it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solid form, usually one tautomer predominates. In solutions where tautomerization is possible, a chemical equilibrium of the tautomers will be reached. The exact ratio of the tautomers depends on several factors, including temperature, solvent and pH. The concept of tautomers that are interconvertable by tautomerizations is called tautomerism.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ringshaped) form as exhibited by glucose.

Common tautomeric pairs are: ketone-enol, amide-nitrile, lactam-lactim, amide-imidic acid tautomerism in heterocyclic rings (*e.g.*, in nucleobases such as guanine, thymine and cytosine), amine-enamine and enamine-enamine. It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form.

The term "crystal polymorphs", "polymorphs" or "crystal forms" means crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

Additionally, the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

As used herein, a "subject" or "subject in need thereof" is a subject having a disease or disorder associated with α-amino-β-carboxymuconate-ε-semialdehyde decarboxylase (ACMSD) dysfunction. A "subject" includes a mammal. The mammal can be *e.g.*, any mammal, *e.g.*, a human, primate, bird, mouse, rat, fowl, dog, cat, cow, horse, goat, camel, sheep or a pig. Preferably, the mammal is a human.

The present disclosure is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

Throughout the description and claims of this specification, the words "comprise" and "contain" and variations of the words, for example "comprising" and "comprises", mean "including but not limited to" and do not exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

No admission is made that any reference constitutes prior art. Further, no admission is made that any of the prior art constitutes part of the common general knowledge in the art.

### Method of treatment

In another aspect, the present disclosure relates to a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, for use in a method of treating, preventing, reducing the risk of a disease or disorder associated with α-amino-β-carboxymuconate-ε-semialdehyde decarboxylase (ACMSD), the method comprising administering to a subject suffering from or susceptible to developing a disease or disorder associated with ACMSD dysfunction a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Another aspect of the present disclosure relates to a pharmaceutical composition comprising one or more compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and at least one of a pharmaceutically acceptable carrier, diluent, or excipient, for use in a method of treating, preventing, or reducing the risk of a disease or disorder associated with α-amino-β-carboxymuconate-ε-semialdehyde decarboxylase (ACMSD), the method comprising administering to a subject suffering from or susceptible to developing a disease or disorder associated with ACMSD dysfunction a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency..

In another aspect, the present disclosure relates to a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, for use in a method of treating, preventing, or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

In another aspect, the present disclosure relates to a pharmaceutical composition comprising one or more compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and at least one of a pharmaceutically acceptable carrier, diluent, or excipient, for use in a method of treating, preventing, or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Another aspect of the present disclosure relates to a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, for use in a method of treating, preventing, or reducing the risk of a disorder associated with mitochondrial dysfunction, the method comprising administering to a subject suffering from or susceptible to developing a metabolic disorder a therapeutically effective amount of the compound, wherein the disorder associated with mitochondrial dysfunction is an inherited mitochondrial disease, a common metabolic disorder, a neurodegenerative disease, an aging related disorder, a kidney disorder, or a chronic inflammatory disease. In a preferred embodiment, the disorder associated with mitochondrial dysfunction is a common metabolic disorder such as obesity or type II diabetes. In one embodiment, the disorder associated with mitochondrial dysfunction is a metabolic disorder, a neurodegenerative disease, a chronic inflammatory disease, a fatty liver disease, a kidney disorder, or an aging related disorder.

Another aspect of the present disclosure relates to a pharmaceutical composition comprising one or more compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and at least one of a pharmaceutically acceptable carrier, diluent, or excipient, for use in a method of treating, preventing, or reducing the risk of a disorder associated with mitochondrial dysfunction, the method comprising administering to a subject suffering from or susceptible to developing a metabolic disorder a therapeutically effective amount of the compound, wherein the disorder associated with mitochondrial dysfunction is an inherited mitochondrial disease, a common metabolic disorder, a neurodegenerative disease, an aging related disorder, a kidney disorder, or a chronic inflammatory disease. In a preferred embodiment, the disorder associated with mitochondrial dysfunction is a common metabolic disorder such as obesity or type II diabetes.

In some embodimens, the metabolic disorder is type II diabetes, obesity, hyperglycemia, glucose intolerance, insulin resistance, hypercholesterolemia, hypertension, hyperlipoproteinemia, hyperlipidemia, hypertriglylceridemia, cardiovascular disease, atherosclerosis, peripheral vascular disease, kidney disease, ketoacidosis, thrombotic disorders, nephropathy, diabetic neuropathy, diabetic retinopathy, sexual dysfunction, dermatopathy, dyspepsia, hypoglycaemia, cancer, or edema. In some embodiments, the neurodegenerative disease is photoreceptor degeneration, dementia, Alzheimer's disease, Parkinson's disease, or Huntington's disease. In some embodiments, the chronic inflammatory disease is celiac disease, vasculitis, lupus, chronic obstructive pulmonary disease (COPD), irritable bowel disease, atherosclerosis, arthritis, or psoriasis. In some embodiments, the kidney disease is acute kidney injury (AKI) or chronic kidney disease (CKD). In some embodiments, the disease associated with aging is cancer, dementia, cardiovascular disease, hypertension, diabetes mellitus (type I or type II), arthritis, cataracts, Alzheimer's disease, macular degeneration, or osteoporosis.

Also described (but not encompassed by the claims) is a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), , or a pharmaceutically acceptable salt thereof, that increases intracellular nicotinamide adenine dinucleotide (NAD⁺), for use in a method of promoting oxidative metabolism, the method comprising administering to a subject suffering from or susceptible to developing a metabolic disorder a therapeutically effective amount of one or more of said compounds.

Also described (but not encompassed by the claims) is a pharmaceutical composition comprising one or more compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), andFormula (IIi), , or a pharmaceutically acceptable salt thereof, and at least one of a pharmaceutically acceptable carrier, diluent, or excipient, that increases intracellular nicotinamide adenine dinucleotide (NAD⁺), for use in a method of promoting oxidative metabolism, the method comprising administering to a subject suffering from or susceptible to developing a metabolic disorder a therapeutically effective amount of the pharmaceutical composition.

Also described (but not encompassed by the claims) is a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof for use in promoting oxidative metabolism.

Also described (but not encompassed by the claims) is a pharmaceutical composition comprising one or more compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and at least one of a pharmaceutically acceptable carrier, diluent, or excipient, for use in promoting oxidative metabolism.

In some embodiments, the disease or disorder associated with reduced nicotinamide adenine dinucleotide (NAD⁺) levels is a chronic liver disease including, but is not limited to, primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher' s disease, hemochromatosis, and alpha 1-antitrypsin deficiency. In one embodiment, the common metabolic disorder is obesity or type II diabetes.

In some embodiments, the disorder associated with mitochondrial dysfunction is an inherited mitochondrial disease, a common metabolic disorder, a neurodegenerative disease, an aging related disorder, a kidney disorder, or a chronic inflammatory disease. In some embodiments, the disorder associated with mitochondrial dysfunction is a metabolic disorder, a neurodegenerative disease, a chronic inflammatory disease, a fatty liver disease, a kidney disorder, or an aging related disorder.

The present disclosure also relates to a compound for use in a method of treating, preventing, reducing the risk of, or ameliorating a disease or disorder associated with α-amino-β-carboxymuconate-ε-semialdehyde decarboxylase (ACMSD) dysfunction, the method comprising administering to a subject in need thereof, a therapeutically effective amount of the compound or pharmaceutical composition comprising the compound, wherein the compound has one the following Formulae:

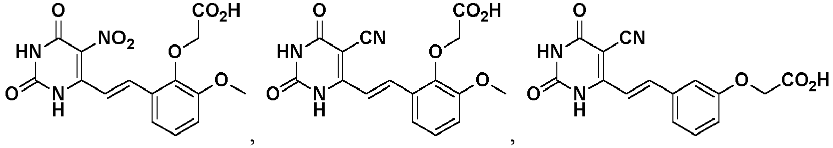

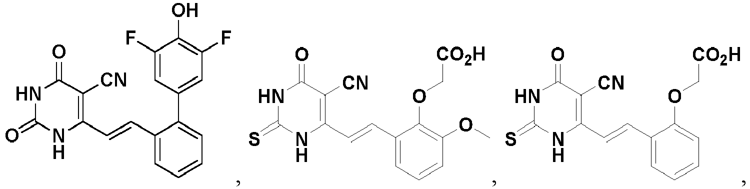

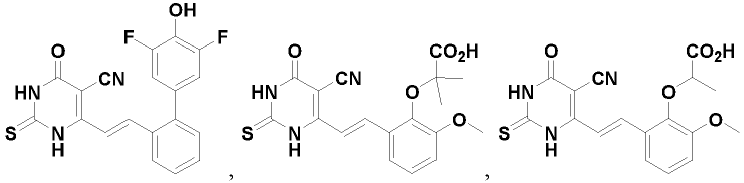

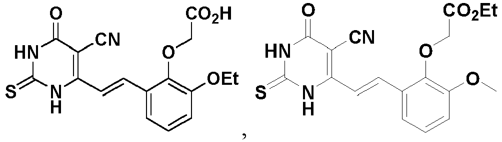

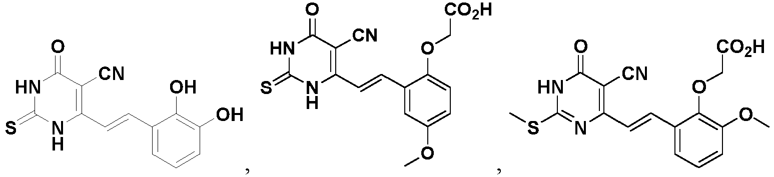

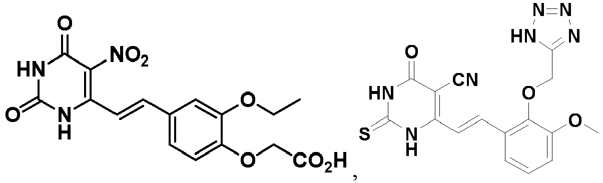

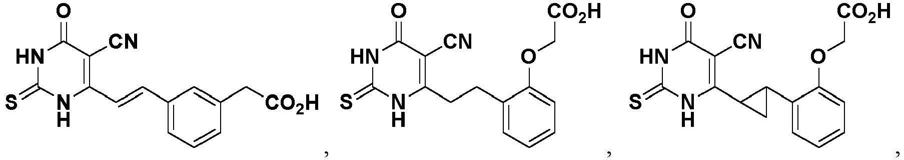

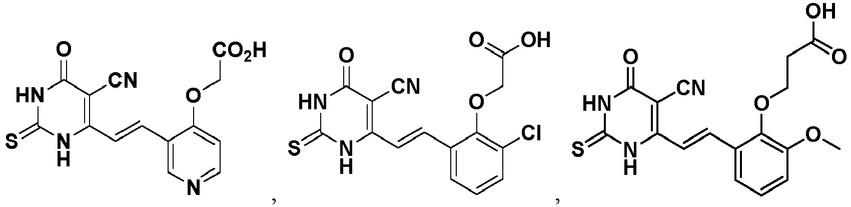

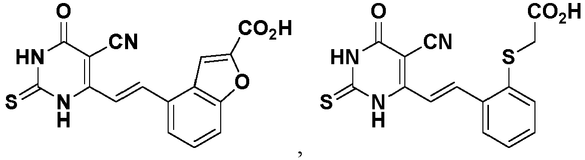

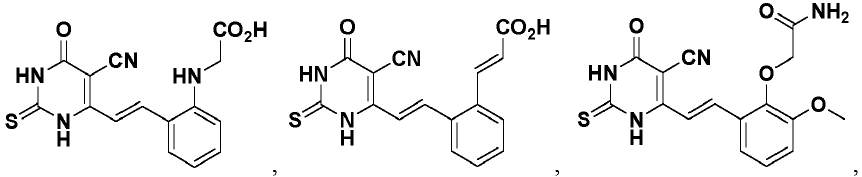

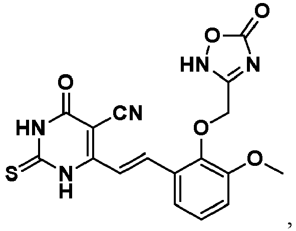

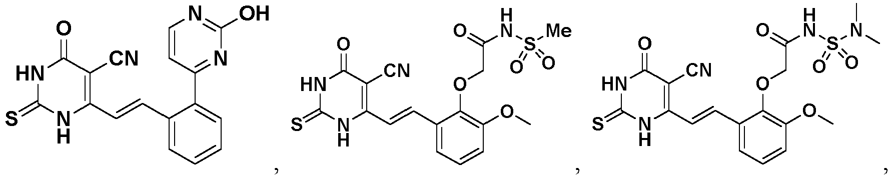

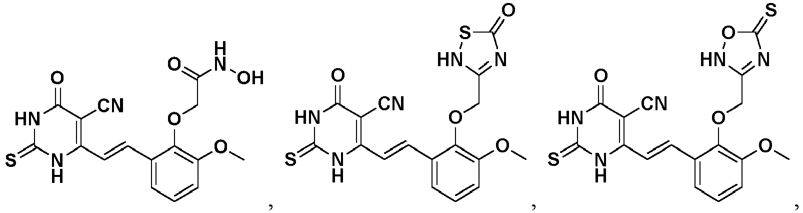

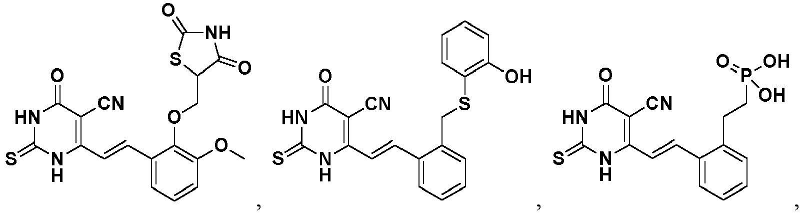

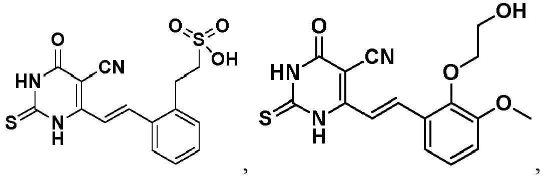

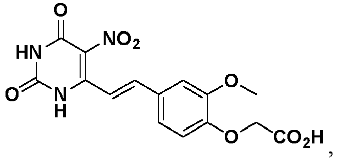

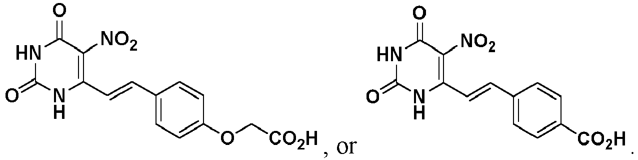

As used herein, "treating" or "treat" describes the management and care of a patient for the purpose of reversing, inhibiting, or combating a disease, condition, or disorder and includes the administration of a compound of the present disclosure (*i.e.*, a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi)), or a pharmaceutically acceptable salt, polymorph or solvate thereof, to reverse the disease, condition, or disorder, eliminate the disease, condition, or disorder, or inhibit the process of the disease, condition, or disorder.

A compound of the present disclosure (*i.e.*, a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi)), or a pharmaceutically acceptable salt, polymorph or solvate thereof, can also be used to prevent a disease, condition, or disorder or one or more symptoms of such disease, condition, or disorder. As used herein, "preventing" or "prevent" describes reducing or eliminating the onset of the symptoms or complications of the disease, condition, or disorder.

A compound of the present disclosure (*i.e.*, a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi)), or a pharmaceutically acceptable salt, polymorph or solvate thereof, can also be used to alleviate one or more symptoms of such disease, condition, or disorder. As used herein, the term "alleviate" is meant to describe a process by which the severity of a sign or symptom of a disorder is decreased. Importantly, a sign or symptom can be alleviated without being eliminated. Preferably treatment is curative or ameliorating.

### Methods for the Preparation of Compounds

The compounds of the present disclosure (*e.g.*, compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi)) can be prepared in a number of ways well known to those skilled in the art of organic synthesis. By way of example, compounds of the present disclosure can be synthesized using the methods described below, together with synthetic methods known in the art of synthetic organic chemistry, or variations thereon as appreciated by those skilled in the art. Preferred methods include but are not limited to those methods described below. The final products of the reactions described herein may be isolated by conventional techniques, *e.g.*, by extraction, crystallization, distillation, chromatography, etc.

Compounds of the present disclosure can be synthesized by following the steps outlined in General Scheme **A** to **E** which comprise different sequences of assembling intermediates **Ia-Ih** and **Ij-Io.** Starting materials are either commercially available or made by known procedures in the reported literature or as illustrated. Useful steps that may be used in the preparation steps of the compounds will be known to the skilled person. The method below is given as a non-limiting example on how the compounds may be prepared. 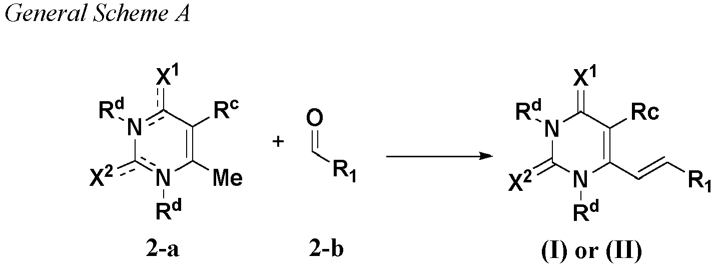 wherein L is -CH=CH-, and R¹, R^{c}, R^{d}, X¹, and X² are defined as in Formula (I).

The general way of preparing compounds of Formula (I) and (II) wherein L is - CH=CH- by using intermediates **2-a**, and **2-b** is outlined in General Scheme A. Knoevenagel type reaction between intermediate **2-a** and aldehyde **2-b** using a base, *i.e.*, piperidine, and in a solvent (*i.e.*, n-butanol (*n*-BuOH), ethanol (EtOH), etc.) provides the desired product of Formula (I) or (II). Pure final compounds can be obtained in acceptable yield after flash chromatography purification or trituration with the appropriate solvent. Bases that can be used include, but are not limited to, piperidine. Solvents used in the coupling reaction can be polar or non-polar solvents. 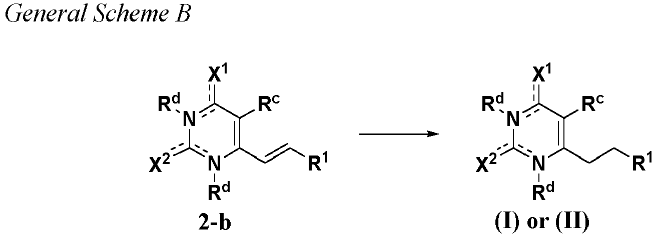 wherein L is -CH₂CH₂- and R^{c}, R^{d}, X¹, and X² are defined as in Formula (I).

The general way of preparing compounds of Formula (I) and (II) wherein L is - CH₂CH₂- by using intermediates **2-c** and **2-d** is outlined in General Scheme B. Hydrogenation of **2-b** using a metal catalyst (*e.g.*, palladium on calcium carbonate) and hydrogen gas (H₂) in a solvent (*e.g.*, tetrahydrofuran (THF) and/or EtOH) provides the desired compound of Formula (I) or (II). Pure final compounds can be obtained in acceptable yield after flash chromatography purification or trituration with the appropriate solvent. 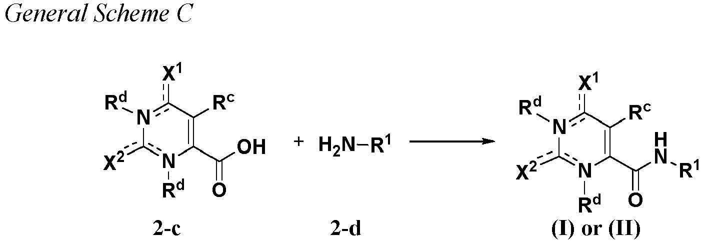 wherein L is -C(O)NH- and R¹, R^{c}, R^{d}, X¹, and X² are defined as in Formula (I).

Alternatively, compounds of Formula (I) and (II) wherein L is -C(O)NH-by using intermediates **2-c** and **2-d** can be prepared as outlined in General Scheme C. Coupling of **2-c** and **2-d** under standard coupling conditions using a coupling agent (*e.g.*, *N*-Ethyl-*N'*-(3-dimethylaminopropyl)carbodiimide (EDC) and Hydroxybenzotriazole (HOBt) or 1-[bis(dimethylamino)methylene]-1H-1,2,3-triazolo[4,5-b]pyridinium3-oxid hexafluorophosphate (HATU)) and a base (e.g., triethylamine (Et₃N) or N,N-diisopropylethylamine (DIPEA)) in a solvent (*e.g.*, dichloromethane (DCM) or N,N-dimethylformamide (DMF)) provides the desired compound of Formula (I) or (II). Pure final compounds can be obtained in acceptable yield after flash chromatography purification or trituration with the appropriate solvent. 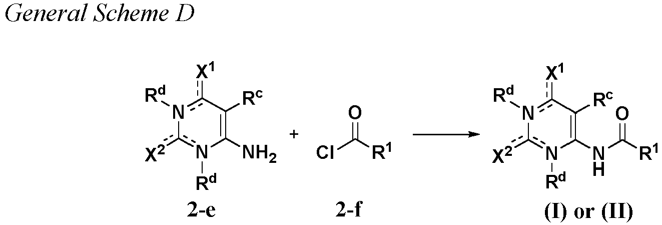 wherein L is -NHC(O)- and R¹, R^{c}, R^{d}, X¹, and X² are defined as in Formula (I).

The general way of preparing compounds of Formula (I) and (II) wherein L is - NHC(O)- by using intermediates **2-e** and **2-f** is outlined in General Scheme D. Acylation of **2-e** with acid chloride **2-f** using a base, *i.e.*, triethylamine or DIPEA, and in solvent, *e.g.*, DCM, provides the desired compound of Formula (I) or (II). Pure final compounds can be obtained in acceptable yield after flash chromatography purification or trituration with the appropriate solvent. 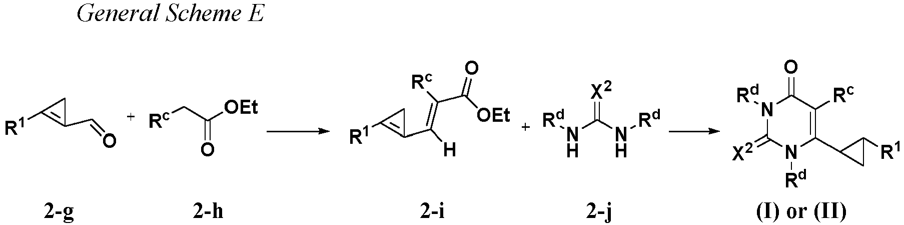 wherein L is 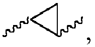 X¹ is O, and R¹, R^{c}, R^{d}, and X² are defined as in Formula (I).

Alternatively, compounds of Formula (I) and (II) wherein L is 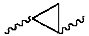 and X¹ is O by using intermediates **2-g, 2-h, 2-i,** and **2-j** can be prepared as outlined in General Scheme E. Hydrogenation of **2-c** using a metal catalyst (*e.g.*, palladium on calcium carbonate) and hydrogen gas (H₂) in a solvent (*e.g.*, tetrahydrofuran (THF) and/or EtOH) provides intermediate **2-d**. Hydrolysis of ester **2-d** in the presence of a base (*e.g.*, sodium hydroxide (NaOH)) and in a solvent (*e.g.*, EtOH) provides the desired compound of Formula (I) or (II). Pure final compounds can be obtained in acceptable yield after flash chromatography purification or trituration with the appropriate solvent.

A mixture of enantiomers, diastereomers, cis/trans isomers resulting from the process described above can be separated into their single components by chiral salt technique, chromatography using normal phase, reverse phase or chiral column, depending on the nature of the separation.

It should be understood that in the description and formula shown above, the various groups R¹, R², R³, R⁴, X¹, X², L, Y¹, Y², R^{a}, R^{b}, R^{c}, R^{d}, R^{e}, R^{f}, R^{g}, R^{x}, R^{y}, R^{z}, m, n, o, p, q, r and other variables are as defined herein above, except where otherwise indicated. Furthermore, for synthetic purposes, the compounds of General Schemes A-E are mere representative with elected radicals to illustrate the general synthetic methodology of the compounds of Formula (I) as defined herein.

### Biological Assays and Animals Studies

### Method of ScreeningACMSD1 Inhibition

The activity of compounds as inhibitors of ACMSD1 is determined in a spectrophotometrical in vitro assay. The pre-assay mixture is incubated and a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and ACMSD 1 solution is then added. The effect of ACMS concentration on the enzyme activity is investigated by varying 3-hydroxyanthranilic acid (3OH-HA) concentration in the pre-assay mixture. Kinetic parameters are calculated from the initial velocity data using a Lineweaver-Burk plot.

### Cellular Assay Methods

The mouse hepatocytes cell lines are grown and plated. The cells are maintained in culture at 37 °C and once the cells are attached, different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, or DMSO are added. Primary hepatocytes are harvested about 24 hrs later.

### Determination of ACMSD-1 Modulation in HEK293T Cells.

HEK293T cells are seeded and transfected to transiently express ACMSD. The cells are then stimulated with different concentrations of Compound 1, and then lysed to measure the ACMSD activity in a spectrophotometrical in vitro assay. The amount of the whole protein content in cell lysates is detected by Bradford analysis and used to get the specificity activity of the enzyme normalized in all samples.

### Determination of NAD⁺ content in Human Primary Hepatocytes

Primary hepatocytes are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, or MEHP (control) after seeding. The compound is replaced every 24 hours, and then cells are directly harvested and lysed to detect NAD⁺ content through LC MS/MS (liquid chromatography mass spectrometry/mass spectroscopy).

### Modulation of SOD 2 activity in AML12 cells and Murine Primary Hepatocytes

Primary hepatocytes or AML-12 cells are lysed and total protein concentration is determined using the Bradford assay. SOD2 activity is determined at indicated times after treatment with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, using a SOD Assay Kit. Absorbance is determined and results are expressed in U/ml/mg of protein according to the standard curve and measured protein concentration.

### Determination of NAD⁺ content in Murine Primary Hepatocytes

NAD⁺ is extracted using acidic extraction method and samples are collected and homogenized. After insoluble protein parts are pelleted, the samples are separated by high-performance liquid chromatography (HPLC) and analyzed by mass-spectrometry. The proteins in the pellet are quantified by Bradford assay and are used for normalization.

### RNA preparation and RT-qPCR analysis of ACMSD and SIRT1-regulated Genes in Cells,

Cells (AML-12, Hepa-1.6, HEK-293, primary human and murine hepatocytes) are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof and the gene expression of ACMSD, *Pgc1a*, *Sod1*, and *Sod2* (MnSOD) is determined using RT-qPCR. Total RNA is extracted from cells and the extracted RNA is treated with DNase and used for reverse transcription (RT).

### Modulation of Caspase 3/7 Activity in MDCK Cells

MDCK cells are cultured in base medium to a final concentration of 10%. Cells are plated into 96 wells and 24 hours after cell plating the medium is changed with fresh medium supplemented with 1% FBS. Cisplatin is then used to induce cell injury. Different concentrations of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof (in DMSO) are added in combination with cisplatin or prior to adding cisplatin. Caspase 3/7 activity (Promega) is determined according to standard procedures using a luminescent signal readout on a plate reader. Each experiment/condition is performed in triplicate. Caspase activity is analyzed as percentage effect normalized to the cisplatin alone and vehicle treated cells.

### Cytotoxicity and hERG screening

HePG2 and AML-12 cells are seeded and a dose-response of the compound is performed at various concentrations. Cells are stimulated and the supernatant is used to perform LDH release as a measure of necrosis while the cells are lysed to detect ATP levels for determining cell viability.

The Predictor^{™} hERG assay kit is stably transfected with hERG potassium channel and a high-affinity red fluorescent hERG channel ligand and is used for the determination of hERG channel affinity binding of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof. Compounds that bind to the hERG channel protein (competitors) are identified by their ability to displace the tracer which results in a lower fluorescence polarization.

### Anti-diabetic Effects studies in C57BL/6J and KK-Ay mice

Mice are fed with regular chow or a high fat diet (HFD). A compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, is dosed daily and blood and tissues are harvested for RNA isolation, lipid measurements and histology. Oxygen consumption is measured and histological analysis and transmission electron microscopy are performed. An oral glucose tolerance test and an intraperitoneal insulin tolerance test are also performed to quantify glucose and to measure plasma insulin concentrations.

### Anti-diabetic and Anti-obesity studies in db/db Mice with LepR Mutation

Animals are fed a high-fat diet (HFD). For subchronic intervention, the animals are treated once/day with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, for 14 days. Blood samples are collected and glucose concentrations of each blood sample are determined. For acute intervention, initial blood samples are collected and then compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, are administered. Diet-access is then restricted, and a second blood sample is collected. The mice are subjected to an oral glucose tolerance test and blood glucose concentrations are determined.

For the euglycemic-hyperinsulinemic clamps assay, the animals receive a primed-continuous [3-³H]glucose infusion and a blood sample is then collected to determine plasma insulin, glucose and [3-³H]glucose concentrations and to calculate basal endogenous glucose appearance rates. The mice then receive vehicle or a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, *via* gavage. Subsequently, the animals receive a [3-³H]glucose infusion containing insulin causing a moderate net-increase in plasma insulin concentrations. Blood glucose concentrations are measured and target glycemia is established by adjusting the rate of glucose infusion. 2-deoxy-D-[1-¹⁴C] glucose is then given intravenously and blood samples are collected. The mice are then sacrificed. Gastrocnemius muscle and epididymal adipose tissue are collected and plasma [³H]- and [¹⁴C]-radioactivity is determined in deproteinized plasma.

Body weights are assessed and brown adipose tissue (BAT) and gonadal white adipose tissue (WAT) are dissected and weighed. Volume oxygen (VO₂) and volume carbon dioxide production (VCO₂) are measured and are reported as average VO₂ per hour normalized to body weight (mL/h/kg). Activity counts by infrared beam interruptions and food intake are simultaneously measured.

### Non-alcoholic fatty liver disease (NAFLD) and non-alcoholic steatohepatitis (NASH) Studies in Male C57BL/6J mice

Mice are fed a 'Western' HF-HSD (high fat-high sucrose diet) or normal chow diet (NCD) as control. The animals are then treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, for 4, 12 or 20 weeks, and then sacrificed. Body weight and food intake are monitored weekly and total fat mass is analyzed. An intraperitoneal glucose tolerance test (IPGTT) is also performed and tail vein glucose levels are measured after glucose administration. Insulin resistance is calculated using the Homeostasis Model of Insulin Resistance. The mice are then sacrificed by blood sampling via cardiac puncture. Plasma is obtained and tissues were collected together with the plasma for further biochemical and molecular analyses or for histological analysis.

### Non-alcoholic fatty liver disease (NAFLD) and non-alcoholic steatohepatitis (NASH) Studies in in Methionine and Choline Deficient mice

Mice weighing 25 g are either fed a methionine- and choline-deficient diet (MCD to induce NASH) or chow diet (as a control). Animal experiments and evaluation of NAFLD and NASH are conducted as described above in for C57BL/6J mice fed the high fat and high sucrose diet.

### Atherosclerosis Studies in High Cholesterol Fed LDL-R Knockout mice

LDL-R knockout (KO) mice are sacrificed about 12 weeks after the initiation of the atherogenic diet, after which the heart and aorta are perfused with PBS and subsequently fixed. Atherosclerosis and biochemistry parameters are measured with the appropriate commercially available kits. For the *in vivo* lipopolysaccharide (LPS) study, mice are intraperitoneally injected with LPS, and blood is taken from the tail vein. TNFα levels are quantified with a Mouse TNFα ELISA assay. Blood cell counts are determined.

### Inherited Mitochondrial Disease Studies in Sco2^{KO/}^{KI} mice

Compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, are dissolved in water and added to a standard powder diet at the appropriate concentration. The diet supply is changed every three days and administered *ad libitum* for one month. Tissues are collected for histological analysis. For the muscle quadriceps samples, the spectrophotometric activity of cI, cII, cIII, and cIV, as well as CS, is measured. NAD⁺ is extracted from tissues using acidic and alkaline extraction methods, respectively, and analyzed with mass spectrometry.

### Inherited Mitochondrial Disease Studies in Deletor mice

Deletor and WT male mice are administered either chow diet (CD) or a compound of Formula (I), Formula (Ia), Formula (Ib), Formula (Ic), Formula (Id), Formula (Ie), Formula (If), Formula (Ig), Formula (Ih), Formula (Ii), Formula (Ij),or Formula (II), or a pharmaceutically acceptable salt or tautomer thereof, admixed with the CD. The mice are regularly monitored for weight, food consumption, and physical endurance and their exercise capability is measured. Oxygen consumption and carbon dioxide production, as well as spontaneous moving and feeding activities, are recorded. Tissue sections are collected and prepared from the quadriceps, liver, and BAT. Frozen sections from quadriceps are assayed for *in situ* histochemical COX and succinate dehydrogenase (SDH) activities, crista content in both BAT and muscle is determined from electron micrographs and skeletal muscle samples are analyzed for citrate synthase activity.

### Kidney Disease Studies

C57BL/6J WT mice are fed a standard commercial diet and divided into four groups: control; cisplatin; a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and cisplatin; and a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, alone. The mice are sacrificed and tissue samples and serum are collected. Serum creatinine and BUN levels are measured and the proinflammatory cytokines TNF-α, IL-1β, and IL-6 from serum or homogenates from kidney tissue are quantified. Mouse kidneys are collected and stained for analysis. Tubular damage is examined and scored based on the percentage of cortical tubular necrosis. Neutrophil infiltration is quantitatively assessed on stained tissue by counting the number of neutrophils per high-power field.

Alternatively, C57BL/6J WT mice are numbered and kept in acclimatization for a period and then randomized into different treatment groups based on their body weight. Different groups are maintained on a specified diet for a period of time. Body weight measurements are taken and food consumption is evaluated. Blood is collected by retro-orbital puncture under mild anesthesia and used for analysis of basal blood urea nitrogen levels (BUN).

Mice are anesthetized and placed on a surgical platform. Both kidneys are exposed through incisions and renal pedicles are occluded using vascular clamps. The clamp is then removed and the surgical site is sutured. The sham-operated group is subjected to similar surgical procedures, except that the occluding clamp is not applied. Animals are monitored until recovery from anesthesia and returned to their home cage. Animals are observed every day for general clinical signs and symptoms and mortality.

One day prior to termination, animals are individually housed in metabolic cages and urine is collected for estimation of urea, creatinine, sodium and potassium. Blood is also collected by retro orbital puncture under mild anesthesia and plasma is used for analysis of blood urea nitrogen levels (BUN) and serum creatinine. Animals are then euthanized and organs are collected. One kidney is fixed and the other is flash frozen and used for the estimation of lipid peroxidation, GSH, MPO and SOD levels.

### Ischemia/Reperfusion-induced Acute Kidney Injury Studies

CD-1 (ICR) mice are treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, by oral gavage once per day. CD-1 mice are divided into four groups: (1) young mice with sham injury; (2) young mice with ischemic/ reperfusion (I/R) injury; (3) adult mice with sham injury; and (4) adult mice with I/R injury. An additional 27 adult mice are randomized into two groups: mice receiving a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, and mice receiving the vehicle as a control. The serum creatinine level is measured and BUN measurements are recorded. Renal tissue is then evaluated and tubular injury is scored.

### Determination of the Effects on FoxO1 Phosphorylation levels

AML-12 cells are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof. Cells are then lysed, and analyzed by SDS-PAGE/western blot. Blocking and antibody incubations are then done and each protein present is detected with its specific antibody.

### Inhibitory effect

The present disclosure also relates to a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein, in a method for inhibiting the activity of ACMSD. The method includes contacting a cell with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof. In a related embodiment, the method further provides that the compound is present in an amount effective to produce a concentration sufficient to selectively inhibit ACMSD in the cell.

Thus, preferably in an assay for ACMSD inhibition (*i.e.*, an ACMSD assay described herein, *e.g.*, Example 29, or an ACMSD assays known in the literature), the preferred compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, are compounds capable of reducing or preferably inhibiting ACMSD and increasing NAD⁺ levels and/or activating SIRTs and the downstream targets of SIRTs, such as PGC-1α, FoxO1 and/or SOD. Preferably, said inhibition is determined as the IC₅₀ of said compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, with respect to said ACMSD inhibition assay. Preferred compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, have an IC₅₀ at or below 1 µM, more preferably less than 300 nM, for example less than 100 nM, such as less than 50 nM with respect to inhibition of ACMSD.

### Pharmaceutically acceptable salts

The compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), may be provided in any form suitable for the intended administration, in particular including pharmaceutically acceptable salts and solvates of the compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi)

Pharmaceutically acceptable salts refer to salts of the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), which are considered to be acceptable for clinical and/or veterinary use. Typical pharmaceutically acceptable salts include those salts prepared by reaction of the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), and a mineral or organic acid or an organic or inorganic base. Such salts are known as acid addition salts and base addition salts, respectively. It will be recognized that the particular counter-ion forming a part of any salt is not of a critical nature, so long as the salt as a whole is pharmaceutically acceptable and as long as the counter-ion does not contribute undesired qualities to the salt as a whole. These salts may be prepared by methods known to the skilled person. Pharmaceutically acceptable salts are, *e.g.*, those described and discussed in Remington's Pharmaceutical Sciences, 17. Ed. Alfonso R. Gennaro (Ed.), Mack Publishing Company, Easton, PA, U.S.A., 1985 and more recent editions and in Encyclopedia of Pharmaceutical Technology.

Examples of pharmaceutically acceptable addition salts include acid addition salts formed with inorganic acids, *e.g.*, hydrochloric, hydrobromic, sulfuric, nitric, hydroiodic, metaphosphoric, or phosphoric acid; and organic acids *e.g.*, succinic, maleic, acetic, fumaric, citric, tartaric, benzoic, trifluoroacetic, malic, lactic, formic, propionic, glycolic, gluconic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), ethanesulfonic, pantothenic, stearic, sulfinilic, alginic and galacturonic acid; and arylsulfonic, for example benzenesulfonic, p-toluenesulfonic, methanesulfonic or naphthalenesulfonic acid; and base addition salts formed with alkali metals and alkaline earth metals and organic bases such as N,N-dibenzylethylenediamine, chloroprocaine, choline, diethanolamine, ethylenediamine, meglumine (N-methylglucamine), lysine and procaine; and internally formed salts. It should be understood that all references to pharmaceutically acceptable salts include solvent addition forms (solvates) or crystal forms (polymorphs) as defined herein, of the same salt.

The compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, may be provided in dissoluble or indissoluble forms together with a pharmaceutically acceptable solvent such as water, ethanol, and the like. Dissoluble forms may also include hydrated forms such as the mono-hydrate, the dihydrate, the hemihydrate, the trihydrate, the tetrahydrate, and the like.

The term "prodrug" used herein is intended to mean a compound which - upon exposure to certain physiological conditions - will liberate the compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, which then will be able to exhibit the desired biological action. A typical example is a labile carbamate of an amine.

Since prodrugs are known to enhance numerous desirable qualities of pharmaceuticals (*e.g.*, solubility, bioavailability, manufacturing, etc.), the compounds of the present disclosure can be delivered in prodrug form. "Prodrugs" are intended to include any covalently bonded carriers that release an active parent drug of the present disclosure *in vivo* when such prodrug is administered to a subject. Prodrugs in the present disclosure are prepared by modifying functional groups present in the compound in such a way that the modifications are cleaved, either in routine manipulation or *in vivo*, to the parent compound. Prodrugs include compounds of the present disclosure wherein a hydroxy, amino, sulfhydryl, carboxy or carbonyl group is bonded to any group that may be cleaved *in vivo* to form a free hydroxyl, free amino, free sulfhydryl, free carboxy or free carbonyl group, respectively.

Examples of prodrugs include, but are not limited to, esters (*e.g.*, acetate, dialkylaminoacetates, formates, phosphates, sulfates and benzoate derivatives) and carbamates (*e.g.*, N,N-dimethylaminocarbonyl) of hydroxy functional groups, esters (*e.g*.,C₁₋₆ alkyl esters, *e.g.*, methyl esters, ethyl esters, 2-propyl esters, phenyl esters, 2-aminoethyl esters, morpholinoethanol esters, etc.) of carboxyl functional groups, N-acyl derivatives (*e.g.*, N-acetyl) N-Mannich bases, Schiff bases and enaminones of amino functional groups, oximes, acetals, ketals and enol esters of ketone and aldehyde functional groups in compounds of the disclosure, and the like. *See* Bundegaard, H., Design of Prodrugs, p1-92, Elesevier, New York-Oxford (1985).

The compounds, or pharmaceutically acceptable salts, or esters thereof, are administered orally, nasally, transdermally, pulmonary, inhalationally, buccally, sublingually, intraperintoneally, subcutaneously, intramuscularly, intravenously, rectally, intrapleurally, intrathecally and parenterally. In one embodiment, the compound is administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Techniques for formulation and administration of the disclosed compounds of the disclosure can be found in Remington: the Science and Practice of Pharmacy, 19th edition, Mack Publishing Co., Easton, PA (1995). In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

In one aspect of this disclosure, there is provided a pharmaceutical composition comprising at, as an active ingredient, at least one compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein, and optionally one or more pharmaceutically acceptable excipients, diluents and/or carriers. The compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, may be administered alone or in combination with pharmaceutically acceptable carriers, diluents or excipients, in either single or multiple doses. Suitable pharmaceutically acceptable carriers, diluents and excipients include inert solid diluents or fillers, sterile aqueous solutions and various organic solvents.

A "pharmaceutical composition" is a formulation containing the compounds of the present disclosure in a form suitable for administration to a subject. The pharmaceutical compositions may be formulated with pharmaceutically acceptable carriers or diluents as well as any other known adjuvants and excipients in accordance with conventional techniques such as those disclosed in Remington: The Science and Practice of Pharmacy, 21st Edition, 2000, Lippincott Williams & Wilkins.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

The pharmaceutical compositions formed by combining a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein, with pharmaceutically acceptable carriers, diluents or excipients can be readily administered in a variety of dosage forms such as tablets, powders, lozenges, syrups, suppositories, injectable solutions and the like. In powders, the carrier is a finely divided solid such as talc or starch which is in a mixture with the finely divided active component. In tablets, the active component is mixed with the carrier having the necessary binding properties in suitable proportions and compacted in the shape and size desired.

The pharmaceutical compositions may be specifically prepared for administration by any suitable route such as the oral and parenteral (including subcutaneous, intramuscular, intrathecal, intravenous and intradermal) route. It will be appreciated that the preferred route will depend on the general condition and age of the subject to be treated, the nature of the condition to be treated and the active ingredient chosen.

Pharmaceutical compositions for oral administration include solid dosage forms such as capsules, tablets, dragees, pills, lozenges, powders, and granules. Where appropriate, they can be prepared with coatings such as enteric coatings or they can be prepared so as to provide controlled release of the active ingredient such as sustained or prolonged release according to methods well known in the art.

For oral administration in the form of a tablet or capsule, a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein, may suitably be combined with an oral, non-toxic, pharmaceutically acceptable carrier such as ethanol, glycerol, water, or the like. Furthermore, suitable binders, lubricants, disintegrating agents, flavoring agents, and colorants may be added to the mixture, as appropriate. Suitable binders include, e.g., lactose, glucose, starch, gelatin, acacia gum, tragacanth gum, sodium alginate, carboxymethylcellulose, polyethylene glycol, waxes, or the like. Lubricants include, *e.g.*, sodium oleate, sodium stearate, magnesium stearate, sodium benzoate, sodium acetate, sodium chloride, or the like. Disintegrating agents include, *e.g.*, starch, methyl cellulose, agar, bentonite, xanthan gum, sodium starch glycolate, crospovidone, croscarmellose sodium, or the like. Additional excipients for capsules include macrogels or lipids.

For the preparation of solid compositions such as tablets, the active compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, is mixed with one or more excipients, such as the ones described above, and other pharmaceutical diluents such as water to make a solid pre-formulation composition containing a homogenous mixture of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof. The term "homogenous" is understood to mean that the compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, is dispersed evenly throughout the composition so that the composition may readily be subdivided into equally effective unit dosage forms such as tablets or capsules.

Liquid compositions for either oral or parenteral administration of the compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, include, *e.g.*, aqueous solutions, syrups, elixirs, aqueous or oil suspensions and emulsion with edible oils such as cottonseed oil, sesame oil, coconut oil, or peanut oil. Suitable dispersing or suspending agents for aqueous suspensions include synthetic or natural gums such as tragacanth, alginate, acacia, dextran, sodium carboxymethylcellulose, gelatin, methylcellulose, or polyvinylpyrrolidone.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use.

For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

The preparation of all these solutions under sterile conditions is readily accomplished by standard pharmaceutical techniques well known to those skilled in the art.

For example, sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof. Depot injectable compositions are also contemplated as being within the scope of the present disclosure.

For parenteral administration, solutions containing a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The oily solutions are suitable for intra-articular, intra-muscular and subcutaneous injection purposes.

In addition to the aforementioned ingredients, the compositions of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, may include one or more additional ingredients such as diluents, buffers, flavouring agents, colourant, surface active agents, thickeners, preservatives, *e.g.*, methyl hydroxybenzoate (including anti-oxidants), emulsifying agents and the like.

The term "therapeutically effective amount", as used herein, refers to an amount of a pharmaceutical agent to treat, ameliorate, or prevent an identified disease, disorder, or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or disorder to be treated is a disease or disorder associated with α-amino-β-carboxymuconate-ε-semialdehyde decarboxylase (ACMSD) dysfunction.

For any compound, the therapeutically effective amount can be estimated initially either in cell culture assays, *e.g.*, in cells, or in animal models, usually rats, mice, rabbits, dogs, or pigs. The animal model may also be used to determine the appropriate concentration range and route of administration. Such information can then be used to determine useful doses and routes for administration in humans. Therapeutic/ prophylactic efficacy and toxicity may be determined by standard pharmaceutical procedures in cell cultures or experimental animals, *e.g.*, ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

A suitable dosage of the compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, will depend on the age and condition of the patient, the severity of the disease to be treated and other factors well known to the practicing physician. The compound may be administered for example either orally, parenterally or topically according to different dosing schedules, e.g., daily or with intervals, such as weekly intervals. In general a single dose will be in the range from 0.01 to 500 mg/kg body weight, preferably from about 0.05 to 100 mg/kg body weight, more preferably between 0.1 to 50 mg/kg body weight, and most preferably between 0.1 to 25 mg/kg body weight. The compound may be administered as a bolus (*i.e.*, the entire daily dose is administered at once) or in divided doses two or more times a day. Variations based on the aforementioned dosage ranges may be made by a physician of ordinary skill taking into account known considerations such as weight, age, and condition of the person being treated, the severity of the affliction, and the particular route of administration.

The compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, may also be prepared in a pharmaceutical composition comprising one or more further active substances alone, or in combination with pharmaceutically acceptable carriers, diluents, or excipients in either single or multiple doses. The suitable pharmaceutically acceptable carriers, diluents and excipients are as described herein above, and the one or more further active substances may be any active substances, or preferably an active substance as described in the section "combination treatment" herein below.

### Clinical conditions and other uses of compounds

The compounds according to Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable form thereof, compositions, medicaments, and compounds for use, as defined herein, are useful for treatment of a disease or disorder in which α-amino-β-carboxymuconate-ε-semialdehyde decarboxylase (ACMSD) modulation plays a role. The compounds may be used either in human or in veterinary medicine and the patient may be any mammal, but especially a human. The treatment may include administering to any mammal, but especially a human, suffering from a disease or disorder in which α-amino-β-carboxymuconate-ε-semialdehyde decarboxylase (ACMSD) modulation plays a role, a therapeutically effective amount of a compound according to Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein.

The present disclosure also relates to a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, as defined herein, for use in a disease or disorder associated with α-amino-β-carboxymuconate-ε-semialdehyde decarboxylase (ACMSD) dysfunction, such as obesity, type II diabetes and its complications (e.g., diabetic retinopathy and nephropathy), non-alcoholic fatty liver disease (NAFLD), non-alcoholic steatohepatitis (NASH), or chronic kidney disease.

By the term "disease or disorder associated with α-amino-β-carboxymuconate-ε-semialdehyde decarboxylase (ACMSD) dysfunction" is meant any disease characterized by reduced nicotinamide adenine dinucleotide (NAD⁺) expression and/or activity in at least in some instances of the disease, or a disease which is ameliorated by elevation of the levels of NAD⁺.

The methods, medicaments and compounds for use of the present disclosure are useful to treat, alleviate the symptoms of, or delay the onset of a disorder associated with aberrant mitochondrial function. Disorders associated with aberrant mitochondrial function include, for example, metabolic disorders, neurodegenerative disorders, aging related disorders, and chronic inflammatory disorders. Mitochondrial disorders also include diseases with inherited and/or acquired mitochondrial dysfunction (*i.e.*, Charcot-Marie-Tooth disease, Type 2A2, Mitochondrial Encephalopathy Lactic Acidosis and Stroke (MELAS), Leigh syndrome, Barth syndrome, and Leber's optic neuropathy), fatty acid oxidation disorders, inherited forms of deafness and blindness, and metabolic abnormalities induced by exposure to toxic chemicals and/or drugs (e.g., cisplatin induced deafness).

Metabolic disorders include, for example, type II diabetes, obesity, hyperglycemia, glucose intolerance, insulin resistance (*i.e.*, hyperinsulinemia, metabolic syndrome, syndrome X), hypercholesterolemia, hypertension, hyperlipoproteinemia, hyperlipidemia (e.g., dyslipidemia), hypertriglyceridemia, cardiovascular disease, atherosclerosis, peripheral vascular disease, kidney disease, ketoacidosis, thrombotic disorders, nephropathy, diabetic neuropathy, diabetic retinopathy, sexual dysfunction, dermatopathy, dyspepsia, hypoglycemia, cancer, and edema.

Neurodegenerative disorders include diseases such as photoreceptor degeneration (*i.e.*, retinitis pigmentosa), Dementia, Alzheimer's disease, Parkinson's disease, and Huntington's disease.

Chronic inflammatory diseases include diseases such as celiac disease, vasculitis, lupus, chronic obstructive pulmonary disease (COPD), irritable bowel disease, atherosclerosis, arthritis, and psoriasis.

Aging related disorders include diseases such as cancer, dementia, cardiovascular disease (*i.e.*, arteriosclerosis), hypertension, diabetes mellitus (type I or type II), arthritis, cataracts, Alzheimer's disease, macular degeneration, and osteoporosis.

The subject can be suffering from or susceptible to developing a metabolic disorder. Subjects suffering from or at risk of developing a metabolic disorder are identified by methods known in the art. For example, diabetes can be diagnosed by measuring fasting blood glucose levels or insulin or by glucose tolerance test. Normal adult glucose levels are between about 60-126 mg/dl. Normal insulin levels are about 7 mU/mL ± 3mU. Hypertension can be diagnosed by a blood pressure reading consistently at or above about 140/90. Cardiovascular disease can be diagnosed by measuring cholesterol levels. For example, LDL cholesterol above about 137 or total cholesterol above about 200 is indicative of cardiovascular disease. Hyperglycemia can be diagnosed by a blood glucose level higher than about 10 mmol/l (180 mg/dl). Glucose intolerance can be diagnosed by glucose levels of 140 to 199 mg per dL (7.8 to 11.0 mmol) after conducting a 75 g oral two-hour glucose tolerance test. Insulin resistance can be diagnosed by a fasting serum insulin level of greater than approximately 60 pmol/L. Hypoglycemia can be diagnosed by a blood glucose level lower than about 2.8 to 3.0 mmol/L (50 to 54 mg/dl). Obesity can be diagnosed, for example, by body mass index. Body mass index (BMI) is measured in kg/m² (or lb/in² X 704.5). Alternatively, waist circumference (estimates fat distribution), waist-to-hip ratio (estimates fat distribution), skinfold thickness (if measured at several sites, estimates fat distribution), or bioimpedance (based on principle that lean mass conducts current better than fat mass (*i.e.*, fat mass impedes current), estimates % fat) can be measured. The parameters for normal, overweight, or obese individuals are as follows: Underweight: BMI < 18.5; Normal: BMI about 18.5 to about 24.9; Overweight: BMI = about 25 to about 29.9. Overweight individuals are characterized as having a waist circumference of > 94 cm for men or > 80 cm for women and waist to hip ratios of ≥ 0.95 in men and ≥ 0.80 in women. Obese individuals are characterized as having a BMI of 30 to 34.9, being greater than 20% above "normal" weight for height, having a body fat percentage > 30% for women and 25% for men, and having a waist circumference >102 cm (40 inches) for men or 88 cm (35 inches) for women. Individuals with severe or morbid obesity are characterized as having a BMI of ≥ 35.

The methods described herein may lead to a reduction in the severity or the alleviation of one or more symptoms of a metabolic disorder. For example, symptoms of diabetes include elevated fasting blood glucose levels, blood pressure at or above 140/90 mm/Hg; abnormal blood fat levels, such as high-density lipoproteins (HDL) less than or equal to 35 mg/dL, or triglycerides greater than or equal to 250 mg/dL (mg/dL = milligrams of glucose per deciliter of blood). Efficacy of treatment is determined in association with any known method for diagnosing the metabolic disorder. Alleviation of one or more symptoms of the metabolic disorder indicates that the compound confers a clinical benefit.

The methods of the present disclosure are useful to treat, alleviate the symptoms of, or delay the onset of a kidney disorder. Kidney disorders include acute kidney injury (AKI) and chronic kidney disease (CKD).

The subject can be suffering from or susceptible to developing acute kidney injury (AKI). The acute kidney injury can be characterized by one or more clinical criteria or conditions *(i.e.,* an abrupt decrease in the ability of the kidneys to excrete nitrogenous waste products from the blood, resulting in azotemia). Subjects suffering from or at risk of developing acute kidney injury (AKI) are identified by methods known in the art. For example, the acute kidney injury can be characterized by an increase in serum creatinine by at least 50% over baseline, an absolute increase in serum creatinine of at least 0.3 mg/dL over baseline, a reduction in glomerular filtration rate of at least 25% compared to baseline, a decrease in urine output to 0.5 ml per kilogram of body weight or less per hour persisting for at least 6 hours, or any combination thereof. An acute kidney injury may be caused by ischemia, drugs or toxic agents *(i.e.,* radiocontrast media, a non-steroidal anti-inflammatory drug (NSAID), alcohol, or a chemotherapy agent), viruses, and obstruction.

The subject can be suffering from or susceptible to developing chronic kidney disease (CKD). Chronic kidney disease (CKD) is defined as either (1) having kidney damage as defined by structural or functional abnormalities of the kidney for 3 months or longer with or without a decreased glomerular filtration rate (GFR) or (2) having a GFR of less than 60 mL/min/1.73 m² for 3 months or longer with or without kidney damage. Subjects suffering from or at risk of developing a chronic kidney disease (CKD) are identified by methods known in the art. Structural or functional abnormalities are manifested by symptoms such as either pathologic abnormalities or markers of kidney damage, including abnormalities identified in imaging studies or the composition of blood or urine.

For example, CKD can be diagnosed by testing for specific marker. For example, markers of kidney damage include a plasma creatinine concentration of above about 1.6 mg/dL and a blood urea nitrogen (BUN) concentration of above about 20 mg/dL. Typically, both of these markers are elevated in individuals with CKD. Additional markers of kidney damage can include hematuria (i.e., any detectable amount of blood in the urine), proteinuria (i.e., protein concentrations in urine above about 100 mg/dL), albuminuria (i.e., albumin concentrations in urine above about 100 mg/dL), an intact parathyroid hormone (PTH) concentration in the blood above about 150 pg/mL, or blood phosphate levels of above about 4.5 mg/dL. One specific marker of kidney disease is a GFR rate above normal *(i.e.,* a GFR above about 90 mL/min/1.73 m²), however a below normal GFR also indicates CKD.

The methods of the present disclosure are useful to treat, alleviate the symptoms of, or delay the onset of non-alcoholic fatty liver disease (NAFLD) and/or non-alcoholic steatohepatitis (NASH). The subject can be suffering from or susceptible to developing non-alcoholic fatty liver disease (NAFLD) and/or non-alcoholic steatohepatitis (NASH). Subjects suffering from or at risk of developing a non-alcoholic fatty liver disease (NAFLD) and/or non-alcoholic steatohepatitis (NASH) are identified by methods known in the art. For example, NAFLD and/ or NASH can be diagnosed by liver biopsy.

Non-alcoholic fatty liver disease (NAFLD), as defined herein, is a disease with fat deposition in the liver, which occurs in patients whose alcohol ingestion history is not long enough to cause liver injury. Non-alcoholic fatty liver disease (NAFLD) can be further classified into simple fatty liver, steatohepatitis and cirrhosis. Nonalcoholic steatohepatitis (NASH) refers to a pathology associated with inflammation, liver cell necrosis, ballooning and fibrosis. The onset of nonalcoholic simple fatty liver is induced by fat deposition in liver cells, and this fat accumulation is defined by the balance between increasing factors (influx and synthesis of fats in liver cells) and decreasing factors (catabolism of fats and their release from liver cells). Once damage of liver cells occurs, in addition to this fat deposition, nonalcoholic simple fatty liver will progress to nonalcoholic steatohepatitis. Nonalcoholic steatohepatitis is progressive and may finally progress to cirrhosis and hepatocellular carcinoma.

### Combination treatment

In another aspect, the disclosure includes a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, for use in a combination therapy. A compound, compositions, medicaments and compounds for use of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, may also be used to advantage in combination with one or more other therapeutic agents. Such therapeutic agents include, but are not limited to other ACMSD inhibitors; anti-diabetic agents such as PPAR-γ agonists, PPAR-α/γ dual agonists, PPAR-δ agonists, biguanides, protein tyrosine phosphatase-1B (PTP-1B), dipeptidyl peptidase IV (DPP-IV) inhibitors, sulfonylureas, meglitinides, alpha glucoside hydrolase inhibitors, alpha-amylase inhibitors, insulin secreatagogues, A2 antagonists, insulin or insulin mimetics, glycogen phosphorylase inhibitors, GLP-1 agonists, non-thiazolidinediones, glycokinase, and 11 β HSD-1 inhibitor; anti-obesity agents such as uncoupling Protein (UCP-1, UCP-2, and UCP-3) activators, β3 adrenergic receptor (β3), thyroid hormone β agonists, fatty acid synthase (PAS) inhibitors, phosphodieterase (PDE) inhibitors, lipase inhibitors, serotonin reuptake inhibitors, monoamine reuptake inhibitors, Mc4r agonists, 5HT2c agonists, growth hormone secretagogue (GHS) agonists, CNTF derivatives, ciliary neurotrophic factors (CNTh), cholecystokinin-A (CCK-A) agonists, opioid antagonists, orexin antagonists, acyl-estrogens, leptin, NPY 5 antagonists, neuropeptide Y5 (NPY5) antagonists, neuropeptide Y2 (NPY2) agonists, melanin-concentrating hormone receptor (MCHLR) antagonists and melanin-concentrating hormone 2 receptor (MCH2R), MCH1R antagonists, neuropeptide Y1, ghrelin antagonists, cannabinoid receptor 1 (CB-1), serotonin (5HT) transport inhibitors, CCK-A agonists and histamine 3 (H3) antagonist/inverse agonists; cholesterol lower agents such as 3-hydroxy-3-methylglutaryl-coenzyme A (HMG CoA) reductase inhibitors, HMG-CoA synthase inhibitors, squalene epoxidase inhibitors, fibric acids, bile acid-binding resins probucol and niacin (nicotinic acid) ; compounds that boost NAD⁺ levels such as NAD⁺ precursors *(i.e.,* nicotinamide ribose (NA), nicotinamide mononucleotide (NMN), nicotinic acid (NA) and nicotinamide); and compounds that inhibit NAD⁺ consumption such as PARP inhibitors and CD38 inhibitors.

PPAR-γ agonists useful in the present disclosure include, but are not limited to, glitazones (e.g., balaglitazone, ciglitazone, darglitazone, englitazone, isaglitazone (MCC-555), pioglitazone, rosiglitazone, troglitazone, CLX-0921, 5-BTZD, and the like); GW-0207, LG-100641, LY-300512, LY-519818, R483 (Roche), T131 (Tularik), and compounds disclosed in WO97/27857, 97/28115, 97/28137 and 97/27847; and pharmaceutically acceptable salts or esters thereof. PPAR-α/γ dual agonists useful in the present disclosure, include, but are not limited to, CLX-0940, GW1536, GW1929, GW2433, KRP-297, L-796449, LR-90, MK-0767, SB 219994, and muraglitazar, and pharmaceutically acceptable salts or esters thereof. KRP-297 is 5-[(2,4- Dioxo-5-thiazolidinyl)methyl]-2-methoxy-N-[[4-(trifluoromethyl) phenyl] methyl]benzamide, and pharmaceutically acceptable salts or esters thereof. PPAR-δ agonists useful in the present disclosure include, but are not limited to, GW 501516, GW 590735, and compounds disclosed in JP 10237049, and WO 02/14291; and pharmaceutically acceptable salts or esters thereof.

Biguanides useful in the present disclosure include, but are not limited to, buformin, metformin, and phenformin, and pharmaceutically acceptable salts or esters thereof. Metformin (Glucophage^{®}) is indicated for patients with non-insulin dependent diabetes mellitus, particularly those with refractory obesity. Physician's Desk Reference® page 1080-1086, (56th ed. 2002).

Protein tyrosine phosphatase-1B (PTP-1B) inhibitors useful in the present disclosure include, but are not limited to, A-401,674, KR 61639, OC-060062, OC-83839, OC-297962, MC52445, MC52453, and the compounds disclosed in WO 02/26707, WO 02/26743, JP 2002114768, and pharmaceutically acceptable salts or esters thereof.

Dipeptidyl peptidase IV (DPP-IV) inhibitors, such as isoleucine thiazolidide; NVP-DPP728; P32/98; and LAP 237, P 3298, TSL 225, valine pyrrolidide, TMC-2A/2B/2C, CD-26 inhibitors, FE 999011, P9310/K364, VIP 0177, DPP4, SDZ 274A444; and the compounds disclosed in WO 03/00449; WO 03/004496; EP 1 258 476; WO 02/083128; WO 021062764; WO 02/062764; WO 03/000250; WO 03/002530; WO 03/002531; WO 03/002553; WO 03/002593; WO 03/000180; and WO 03/000181.

Sulfonylureas useful in the present disclosure include, but are not limited to, acetohexamide, chloropropamide, diabinese, glibenclamide, glipizide, glyburide, glimepiride, gliclazide, glipentide, gliquidone, glisolamide, tolazamide, and tolbutamide, pharmaceutically acceptable salts or esters thereof. Meglitinides useful in the present disclosure include, but are not limited to, repaglinide and nateglinide, and pharmaceutically acceptable salts or esters thereof.

Alpha glucoside hydrolase inhibitors (or glucoside inhibitors) useful in the present disclosure include, but are not limited to, acarbose, adiposine, camiglibose, emiglitate, miglitol, voglibose, pradimicin-Q, salbostatin, CKD-711, MDL-25,637, MDL-73,945, and MOR 14, and pharmaceutically acceptable salts or esters thereof, and the compounds disclosed in U.S. Pat. Nos. 4,062,950, 4,174,439, 4,254,256, 4,701,559, 4,639,436, 5,192,772, 4,634,765, 5,157,116, 5,504,078, 5,091,418, 5,217,877, and 5,091,524. Alpha-amylase inhibitors useful in the present disclosure include, but are not limited to, tendamistat, trestatin, and A1-3688, and pharmaceutically acceptable salts and esters thereof, and the compounds disclosed in U.S. Pat. Nos. 4,451,455, 4,623,714, and 4,273,765.

Insulin secreatagogues useful in the present disclosure include, but are not limited to, linogliride and A-4166, and pharmaceutically acceptable salts and esters thereof.

Fatty acid oxidation inhibitors useful in the present disclosure include, but are not limited to, clomoxir, and etomoxir, and pharmaceutically acceptable salts and esters thereof. A2 antagonists useful in the present disclosure include, but are not 'limited to, midaglizole, isaglidole, deriglidole, idazoxan, earoxan, fluparoxan, and pharmaceutically acceptable salts and esters thereof. Insulin or insulin mimetics useful in the present disclosure include, but are not limited to, biota, LP-100, novarapid, insulin detemir, insulin lispro, insulin glargine, insulin zinc suspension (lente and ultralente), Lys-Pro insulin, GLP-1 (73-7) (insulintropin), and GLP-1 (7-36)-NH2), and pharmaceutically acceptable salts or esters thereof.

Glycogen phosphorylase inhibitors useful in the present disclosure include, but are not limited to, CP-368, 296, CP-316,819, BAYR3401, and compounds disclosed in WO 01/94300, and WO 02/20530, and pharmaceutically acceptable salts or esters thereof. GLP-1 agonists useful in the present disclosure include, but are not limited to, exendin-3 and exendin-4, and compounds disclosed in US 2003087821 and NZ 504256, and pharmaceutically acceptable salts or esters thereof.

Non-thiazolidinediones useful in the present disclosure include, but are not limited to, JT-501, and farglitazar (GW2570/GI262579), and pharmaceutically acceptable salts or esters thereof. Glycokinase activators useful in this disclosure, include, but are not limited to, fused heteroaromatic compounds such as those disclosed in US 2002103199, and isoindolin-1-one-substituted propionamide compounds such as those disclosed in WO 02/48106.

Serotonin (5HT) transport inhibitors useful in this disclosure include, but are not limited to, paroxetine, fluoxetine, fenfluramine, fluvoxamine, sertraline, and imipramine. Norepinephrine (NE) transport inhibitors useful in this disclosure include, but are not limited to, GW 320659, despiramine, talsupram, and nomifensine. Cannabinoid receptor 1 (CB-1) antagonist/inverse agonists useful in the present disclosure include: U.S. Pat. Nos. 5,532,237, 4,973,587, 5,013,837, 5,081,122, 5,112,820, 5,292,736, 5,624,941 and U.S. Pat. No. 6,028,084, and PCT Application Nos. WO 96/33159, WO 98/33765, WO98/43636, WO98/43635, WO 01/09120, WO 98/31227, WO 98/41519, WO 98/37061, WO 00/10967, WO 00/10968, WO 97/29079, WO 99/02499, WO 01/58869, WO 02/076949, WO 01/64632, WO 01/64633, WO 01/64634, and WO 03/007887, and EPO Application No. EP-658546. Specific CB-1 antagonists/inverse agonists useful in the present disclosure include, but are not limited to, rimonabant (Sanofi Synthelabo), SR-147778 (Sanofi Synthelabo), BAY 65-2520 (Bayer), and SLY 319 (Solvay). CCK-A agonists useful in the present disclosure include GI 181771, and SR 146,131. Ghrelin antagonists useful in the present disclosure, include: PCT Application Nos. WO 01/87335, and WO 02/08250. Histamine 3 (H3) antagonist/inverse agonists useful in the present disclosure include: PCT Application No. WO 02/15905, and O-[3-(1H-imidazol4-yl)propanol]carbamates (Kiec-Kononowicz, K. et al., Pharmazie, 55:349-55 (2000)), piperidine-containing histamine H3-receptor antagonists (Lazewska, D. et al., Pharmazie, 56:927-32 (2001), benzophenone derivatives and related compounds (Sasse, A. et al. Arch. Pharm.(Weinheim) 334:45-52 (2001)), substituted N - phenyl carbamates (Reidemeister, S. et al., Pharmazie, 55:83-6 (2000)), and proxifan derivatives (Sasse, A. et al., J. Med. Chem. 43:3335-43 (2000)). Specific H3antagonists/inverse agonists useful in the present disclosure include, but are not limited to, thioperamide, 3-(1H-imidazol-4-yl)propyl N-4-pentenyl)carbamate, clobenpropit, iodophenpropit, imoproxifan, GT2394 (Gliatech), and A331440.

Melanin-concentrating hormone receptor (MCHLR) antagonists and melanin-concentrating hormone 2 receptor (MCH2R) agonist/antagonists useful in the present disclosure include PCT Patent Application Nos. WO 01/82925, WO 01/87834, WO 02/06245, WO 02/04433, and WO 02/51809, and Japanese Patent Application No. JP 13226269. Specific MCH1R antagonists useful in the present disclosure include, but are not limited to, T-226296 (Takeda), SB 568849, and SNAP 7941. Neuropeptide Y1 (NPY1) antagonists useful in the present disclosure, include: U.S. Pat. No. 6,001,836, and PCT Application Nos. WO 96/14307, WO 01/23387, WO 99/51600, WO 01/85690, WO 01/85098, WO 01/85173, and WO 01/89528. Specific examples of NPY1 antagonists useful in the present disclosure include, but are not limited to, BIBP3226, J-1 15814, BIBO 3304, LY-357897, CP-671906, and GI264879A. Neuropeptide Y2 (NPY2) agonists useful in the present disclosure, include, but are not limited to, peptide YY (PYY), and PYY3_36, peptide YY analogs, PYY agonists, and the compounds disclosed in WO 03/026591, WO 03/057235, and WO 03/027637. Neuropeptide Y5 (NPY5) antagonists useful in the present disclosure, include, but are not limited to, the compounds described in: U.S. Pat. Nos. 6,140,354, 6,191,160, 6,258,837, 6,313,298, 6,337,332, 6,329,395, and 6,340,683, U.S. Pat. Nos. 6,326,375, 6,329,395, 6,337,332, 6,335,345, European Patent Nos. EP-01010691, and EP 01044970, and PCT-International Patent Publication Nos. WO 97/19682, WO 97/20820, WO 97/20821, WO 97/20822, WO 97/20823, WO 98/27063, WO 00/107409, WO00/185714, WO 00/185730, WO 00/64880, WO 00/68197, WO 00/69849, WO 01/09120, WO 01/85714, WO 01/85730, WO 01/07409, WO 01/02379, WO 01/02379, WO 01/23388, WO 01/23389, WO 01/44201, WO 01/62737, WO 01/62738, WO 01/09120, WO 02/20488, WO 02/22592, WO 02/48152, WO 02/49648, and WO 01/14376. Specific NPY5 antagonists useful in the combinations of the present disclosure, include, but are not limited to GW569180A, GW594884A, GW587081X, GW548118X, FR 235,208, FR226928, FR 240662, FR252384, 1229U91, GI-264879A, CGP71683A, LY-377897, LY366377, PD-160170, SR-120562A, SR-120819A, JCF-104, and H409/22. Additional specific NPY5 antagonists useful in the combinations of the present disclosure, include, but are not limited to the compounds described in Norman et al., J. Med. Chem. 43:42884312 (2000). Leptin includes, but is not limited to, recombinant human leptin (PEG-OB, Hoffman La Roche) and recombinant methionyl human leptin (Amgen). Leptin derivatives (e.g., truncated forms of leptin) useful in the present disclosure include: Pat. Nos. 5,552,524, 5,552,523, 5,552,522, 5,521,283, and PCT International Publication Nos. WO 96/23513, WO 96/23514, WO 96/23515, WO 96/23516, WO 96/23517, WO 96/23518, WO 96/23519, and WO 96/23520.

Opioid antagonists useful in the present disclosure include: PCT Application No. WO 00/21509. Specific opioid antagonists useful in the present disclosure include, but are not limited to, nalmefene (Revex^{®}), 3-methoxynaltrexone, naloxone, and naltrexone. Orexin antagonists useful in the present disclosure include: PCT Patent Application Nos. WO 01/96302, WO 01/68609, WO 02/51232, WO 02/51838, and WO 03/023561. Specific orexin antagonists useful in the present disclosure include, but are not limited to, SB-334867-A. Acyl-estrogens useful in the present disclosure include oleoyl-estrone (del Mar-Grasa, M. et al., Obesity Research, 9:202-9 (2001)). Cholecystokinin-A (CCK-A) agonists useful in the present disclosure include U.S. Pat. No. 5,739,106. Specific CCK-A agonists include, but are not limited to, AR-R 15849, GI181771, JMv-180, A-71378, A-71623 and SR146131. Specific ciliary neurotrophic factors (CNTh) useful in the present disclosure include, but are not limited to, GI181771 (Glaxo-SmithKline), SR146131 (Sanofi Synthelabo), butabindide, PD170,292, PD 149164 (Pfizer). CNTF derivatives useful in the present disclosure include, but are not limited to, axokine (Regeneron), and PCT Application Nos. WO 94/09134, WO 98/22128, and WO 99/43813. Growth hormone secretagogue (GHS) agonists useful in the present disclosure include: U.S. Pat. No. 6,358, 951, and U.S. Patent Application Nos. 2002/049196 and 2002/022637, and PCT Application Nos. WO 01/56592, and WO 02/32888. Specific GHS agonists include, but are not limited to, NN703, hexarelin, MK-0677, SM-130686, CP424 391, L-692,429 and L-163,255.

5HT2c agonists useful in the present disclosure include: U.S. Pat. No. 3,914,250, and PCT Application Nos. WO 02/36596, WO 02/48124, WO 02/10169, WO 01/66548, WO 02/44152, WO 02/51844, WO 02/40456, and WO 02/40457. Specific 5HT2c agonists useful in this disclosure include, but are not limited to, BVT933, DPCA37215, 1K264, PNU 22394, WAY161503, R-1065, and YM 348.

Mc4r agonists useful in the present disclosure include: PCT Application Nos. WO 99/64002, WO 00/74679, WO 01/991752, WO 01/74844, WO 01/70708, WO 01/70337, WO 01/91752, WO 02/059095, WO 02/059107, WO 02/059108, WO 02/059117, wo 02/12166, WO 02111715, WO 02/12178, WO 02/15909, WO 02/068387, WO 02/068388, WO 02/067869, WO 03/007949, and WO 03/009847. Specific Mc4r agonists useful in the present disclosure include CIR86036 (Chiron), ME-10142, and ME-10145 (Melacure).

Monoamine reuptake inhibitors useful in the present disclosure include: PCT Application Nos. WO 01/27068, and WO 01/62341. Specific monoamine reuptake inhibitors useful in the present disclosure include, but are not limited to, sibutramine (Meridia O /Reductil^{®}) disclosed in U.S. Pat. Nos. 4,746,680, 4,806,570, and 5,436,272, and U.S. Patent Publication No. 2002/0006964.

Serotonin reuptake inhibitors, and releasers, useful in the present disclosure include: dexfenfluramine, fluoxetine, and other serotonin reuptake inhibitors, including, but not limited to, those in U.S. Pat. No. 6,365,633, and PCT Patent Application Nos. WO 01/27060, and WO 01/162341.

11 β HSD-1 inhibitor useful in the present disclosure include, but are not limited to, BVT 3498, BVT 2733, and those compounds disclosed in WO 01/90091, WO 01/90090, WO 01/90092. Uncoupling Protein (UCP-1, UCP-2, and UCP-3) activators useful in the present disclosure include: PCT Patent Application No. WO 99/00123. Specific uncoupling protein (UCP-1, UCP-2, and UCP-3) activators useful in the present disclosure include, but are not limited to, phytanic acid, 4-[ (E)-2-(5 ,6, 7,8-tetrahydro-5,5 ,8,8-tetramethyl-2-napthalenyl)-1-propenyl]benzoic acid (TTNPB), and retinoic acid.

β3 adrenergic receptor (β3) agonists useful in the present disclosure include: U.S. Pat. No. 5,705,515 and U.S. Pat. No. 5,451,677 and PCT Patent Application Nos. WO 01/74782, and WO 02/32897. Specific β agonists useful in the present disclosure include, but are not limited to, AD9677/TAK677 (Dainippon/Takeda), CL-316,243, SB 418790, BRL-37344, L-796568, BMS-196085, BRL- 35135A, CGP12177A, BTA-243, GW 427353, Trecadrine, Zeneca D7114, and SR 59119A.

Thyroid hormone β agonists useful in the present disclosure include: PCT Application No. WO 02/15845 and Japanese Patent Application No. JP 2000256190. Specific thyroid hormone β agonists useful in the present disclosure include, but are not limited to, KB-2611 (KaroBioBMS). Specific fatty acid synthase (PAS) inhibitors useful in the present disclosure, include, but are not limited to, Cerulenin and C75. Specific phosphodieterase (PDE) inhibitors useful in the present disclosure, include, but are not limited to, theophylline, pentoxifylline, zaprinast, sildenafil, arnrinone, milrinone, cilostamide, rolipram, and cilomilast.

Lipase inhibitors useful in the present disclosure include, but are not limited to, those disclosed in PCT Application No. WO 01/77094, and U.S. Pat. Nos. 4,598,089, 4,452,813, 5,512,565, 5,391,571, 5,602,151, 4,405,644, 4,189,438, and 4,242,453. Specific lipase inhibitors useful in the present disclosure include, but are not limited to, tetrahydrolipstatin (orlistat/Xenical^{®}), Triton WR1339, RHC80267, lipstatin, teasaponin, and diethylumbelliferyl phosphate, FL-386, WAY-121898, Bay-N-3176, valilactone, esteracin, ebelactone A, ebelactone B, and RHC 80267.

Examples of HMG-CoA reductase inhibitors include, but are not limited to, lovastatin, simvastatin, pravastatin and fluvastatin. Examples of HMG-CoA synthase inhibitors are the beta-lactone derivatives disclosed in U.S. Patents 4,806,564, 4,816,477, 4,847,271, and 4,751,237; the beta-lactam derivatives disclosed in U.S. 4,983,597 and U.S.S.N. 07/540,992 filed June 20, 1990; and the substituted oxacyclopropane analogues disclosed in European Patent Publication EP 0 411 703. Examples of squalene epoxidase inhibitors are disclosed in European Patent Publication EP 0 318 860 and in Japanese Patent Publication J02 169-571A. Examples of LDL-receptor gene inducer molecules are disclosed in U.S. Patent 5,182,298 filed March 18, 1991. Other cholesterol lowering agents that may be administered include niacin, probucol, fibric acids *(i.e.,* clofibrate and gemfibrozil), and LDL-receptor gene inducers.

Examples of PARP inhibitors include, but are not limited to, iodonitocoumarin, 5-iodo-6-nitrocoumarin, 3,4-dihydro-5-methyl-isoquinolinone, 4-amino-1,8-naphthalimide, 3-methoxybenzamide, 8-hydroxy-2-methyl-3- hydro-quinazolin-4-one, 2-{3-[4-(4-fluorophenyl)-3,6-dihydro-1(2H)-pyridinyl]propyl}-8-methyl-4(3H)-quinazolinone, 5-fluoro-1-[4-(4-phenyl-3,6-dihydropyridin-1(butyl]quinazoline-2,4(1H,3H)-dione, 3-(4-chlorophenyl) quinoxaline-5-carboxamide, 2-(3'-methoxyphenyl)benzirnidazole-4-carboxam, 2-(3'-methoxyphenyl)-1H-benzimidazole-4-carboxamide, benzamide, 3-aminobenzamide, 3-aminophtalhydrazide, and 1,5-dihydroxyisoquinoline.

The above-mentioned compounds, which can be used in combination with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), and Formula (IIi), or a pharmaceutically acceptable salt thereof, can be prepared and administered as described in the art such as in the documents cited above.

The above compounds are only illustrative of the ACMSD inhibitors, anti-diabetic agents, anti-obesity agents, cholesterol lower agent, compounds that boost NAD⁺ levels, compounds that inhibit NAD⁺ consumption that can be used in the compositions of the present disclosure. As this listing of compounds is not meant to be comprehensive, the methods of the present disclosure may employ any anti-obesity agent and any anti-diabetic agent, and are not limited to any particular structural class of compounds.

As used herein, "combination therapy" includes the administration of a compound of the present disclosure, or a pharmaceutically acceptable salt, polymorph or solvate thereof, and at least a second agent as part of a specific treatment regimen intended to provide the beneficial effect from the co-action of these therapeutic agents. The beneficial effect of the combination includes, but is not limited to, a cooperative, e.g., synergistic, effect and/or a pharmacokinetic or pharmacodynamic co-action, or any combination thereof, resulting from the combination of therapeutic agents. Administration of these therapeutic agents in combination typically is carried out over a defined time period (usually minutes, hours, days or weeks depending upon the combination selected). "Combination therapy" may be, but generally is not, intended to encompass the administration of two or more of these therapeutic agents as part of separate monotherapy regimens that incidentally and arbitrarily result in the combinations of the present disclosure.

"Combination therapy" is intended to embrace administration of these therapeutic agents in a sequential manner, wherein each therapeutic agent is administered at a different time and in any order, or in alternation and in any order, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical.

All percentages and ratios used herein, unless otherwise indicated, are by weight. Other features and advantages of the present disclosure will become apparent from the different examples. The provided examples illustrate different components and methodology useful in practicing the present disclosure. Generally speaking, the disclosure extends to any novel one, or any novel combination, of the features disclosed in this specification (including the accompanying claims and drawings). The examples do not limit the claimed disclosure. Thus, features, integers, characteristics, compounds or chemical moieties described in conjunction with a particular aspect, embodiment or example of the disclosure are to be understood to be applicable to any other aspect, embodiment or example described herein, unless incompatible therewith. Based on the present disclosure the skilled artisan can identify and employ other components and methodology useful for practicing the present disclosure. Moreover, unless stated otherwise, any feature disclosed herein may be replaced by an alternative feature serving the same or a similar purpose.

### Exemplary Embodiments

Embodiment 1-1. A compound represented by Formula (I): 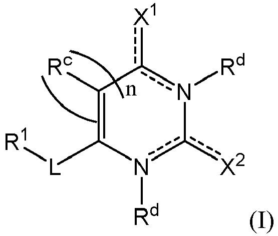 or a pharmaceutically acceptable salt or tautomer thereof,
wherein:
X¹ is O, S, OR² or SH;
X² is O, S, OR² or SR²;
L is -(CH₂)ₘCH=CH(CH₂)ₚ-, -(CH₂)*ₒ*-, 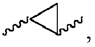 -(CH₂)mC=(O)NR³(CH₂)ₚ-,-(CH₂)ₘNR³C=(O)(CH₂)ₚ-,pyridinyl, or thiophenyl;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -O(C(R^{f})₂)ᵣ(C₃-C₇)cycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R)₂)P(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is-CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

Embodiment 1-2. The compound of Embodiment 1-1, wherein:
X¹ is O or OR²;
X² is S or OR²;
L is -(CH₂)ₘCH=CH(CH₂)ₚ-, -(CH₂)*ₒ*-, 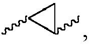 , -(CH₂)ₘC=(O)NR³(CH₂)ₚ-, or -(CH₂)ₘNR³C=(O)(CH₂)ₚ-;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(W)₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, or -CH=CHCO₂R^{x}, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

Embodiment I-3. The compound of Embodiment I-1, wherein:
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, or -(C(R^{f})₂)ᵣC₆-C₁₀ aryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

Embodiment 1-4. The compound of Embodiment 1-1, wherein:
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, -O(C(R^{f})₂)ᵣheteroaryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO₂R^{x}, -(C(R^{f})₂)ᵣC₆-C₁₀ aryl, or -O(C(R^{f})₂)ᵣheteroaryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

Embodiment 1-5. The compound of Embodiment 1-1, wherein the compound is represented by Formula (Ia), (Ib), (Ic), (Id), (Ie), (If), (Ig), (Ih), (Ii), or (Ij): 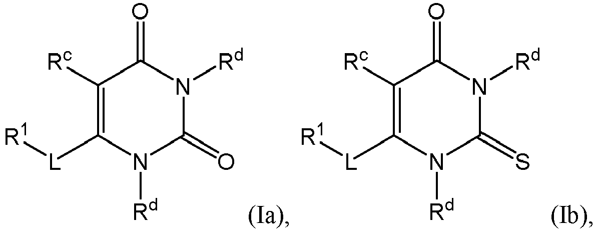 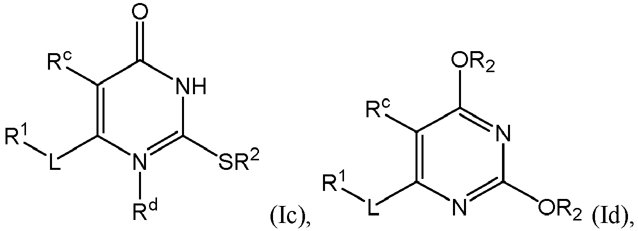 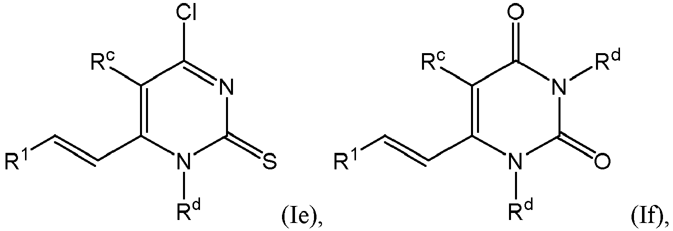 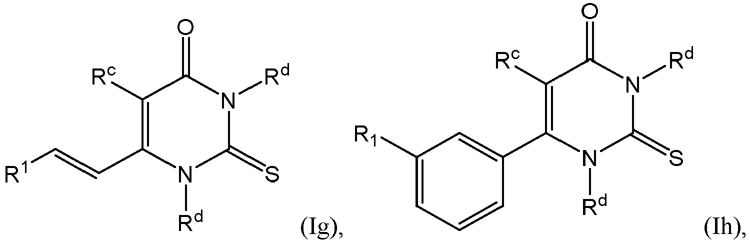 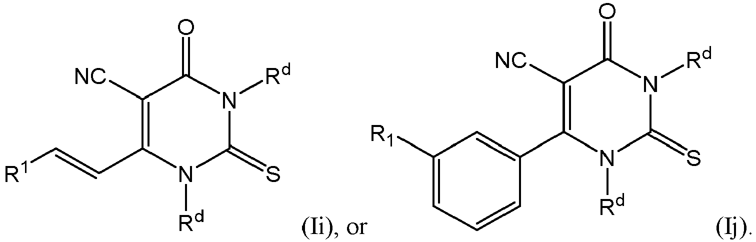 or a pharmaceutically acceptable salt thereof, or tautomer thereof.

Embodiment 1-6. The compound of any one of the preceding embodiments, wherein R^{d} is H.

Embodiment 1-7. The compound of any one of the preceding embodiments, wherein R¹ is C₆-C₁₀ aryl substituted with R^{a} and R^{b}, and optionally substituted with one to two R^{e}.

Embodiment 1-8. The compound of any one of the preceding embodiments, wherein R¹ is phenyl substituted with R^{a} and R^{b}, and optionally substituted with one to two R^{e}.

Embodiment 1-9. The compound of any one of the preceding embodiments, wherein R¹ is heteroaryl comprising one 5- to 7-membered ring and 1-4 heteroatoms selected from N, O and S, and substituted with R^{a} and R^{b}, and optionally substituted with one to two R^{e}.

Embodiment 1-10. The compound of any one of the preceding embodiments, wherein R¹ is pyridinyl substituted with R^{a} and R^{b}, and optionally substituted with one to two R^{e}.

Embodiment 1-11. The compound of any one of the preceding embodiments, wherein R^{a} is H and R^{b} is -(C(R^{f})₂)ᵣCO₂R^{x}, -O(C(R^{f})₂)ᵣCO₂R^{x}, or -(C(R^{f})₂)ᵣC₆-C₁₀ aryl.

Embodiment I-12. The compound of any one of the preceding embodiments, wherein R^{a} is H and R^{b} is -CO₂H, -CH₂CO₂H, - -OCH₂CO₂R^{x}, -OCH(CH₃) CO₂R^{x},-OC(CH₃)₂CO₂R^{x}, or

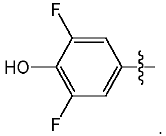

Embodiment I-13. The compound of any one of the preceding embodiments, wherein R^{a} is OR^{y} and R^{b} is -O(C(R^{f})₂)ᵣCO₂R^{x}, or -O(C(R^{f})₂)ᵣheteroaryl.

Embodiment 1-14. The compound of any one of the preceding embodiments, wherein R^{a} is H and R^{b} is -(C(R^{f})₂)ᵣCO₂R^{x}, -Y²(C(R^{f})₂)ᵣCO₂R^{x}, -(C(R^{f})₂)ᵣS-C₆-C₁₀ aryl,-(C(R^{f})₂)ᵣheteroaryl, -(C(R^{f})₂)ᵣP(O)₂OH, -(C(R^{f})₂)ᵣS(O)₂OH, or-CH=CHCO₂R^{x}, wherein the aryl and heteroaryl are substituted with one to three substituents selected from halogen and OH; or R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H.

Embodiment 1-15. A pharmaceutical composition comprising a compound of any one of Embodiments I-1 to I-14, or a pharmaceutically acceptable salt thereof, and at least one of a pharmaceutically acceptable carrier, diluent, or excipient.

Embodiment I-16. The pharmaceutical composition according to Embodiment I-15, which comprises one or more further therapeutic agents.

Embodiment I-17. A compound of any one of Embodiment I-1 to I-15, or a pharmaceutical composition of Embodiment I-16, for use in a method of treating, preventing or reducing the risk of a disease or disorder associated with α-amino-β-carboxymuconate-ε-semialdehyde decarboxylase (ACMSD) dysfunction, the method comprising administering to the subject suffering from or susceptible to developing a disease or disorder associated with ACMSD dysfunction a therapeutically effective amount of the compound, wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

Embodiment 1-18. A compound of any one of Embodiment I-1 to 1-15, or a pharmaceutical composition of Embodiment 1-16, for use in a method of treating, preventing or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound, wherein the disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher' s disease, hemochromatosis, and alpha 1-antitrypsin deficiency.

Embodiment 1-19. A compound of any one of Embodiment I-1 to 1-15, or a pharmaceutical composition of Embodiment 1-16, for use in a method of treating, preventing or reducing the risk of a disorder associated with mitochondrial dysfunction, the method comprising administering to the subject suffering from or susceptible to developing a metabolic disorder a therapeutically effective amount of the compound, wherein said disorder associated with mitochondrial dysfunction is an inherited mitochondrial disease, a common metabolic disorder, a neurodegenerative disease, an aging related disorder, a kidney disorder, or a chronic inflammatory disease..

Embodiment I-20. The method of Embodiment I-19, wherein the common metabolic disorder is obesity or type II diabetes.

Embodiment I-45. A compound for use in a method of treating, preventing, or reducing the risk of a disease or disorder associated with α-amino-β-carboxymuconate-ε-semialdehyde decarboxylase (ACMSD) dysfunction comprising administering to the subject suffering from or susceptible to developing a disease or disorder associated with ACMSD dysfunction a therapeutically effective amount of the compound, wherein the compound is represented by Formula (II): 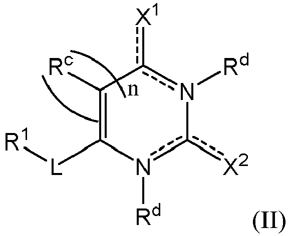 or a pharmaceutically acceptable salt or tautomer thereof, wherein:
X¹ is H, O, S, OR², or SH;
X² is O, S, OR², or SR²;
L is -(CH₂)ₘCH=CH(CH₂)p-, -(CH₂)*ₒ*-, 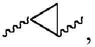 -(CH₂)ₘC=(O)NR³(CH₂)ₚ-,-(CH₂)ₘNR³C=(O)(CH₂)ₚ-, pyridinyl, or thiophenyl;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -O(C(R^{f})₂)ᵣ(C₃-C₇)cycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, - O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl,-(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)P(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is-CN, or -NO₂;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond;
wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

### EXEMPLIFICATION

The Disclosure will now be described by way of example only with reference to the Examples below:

### COMPOUND PREPARATION

### General Methods and Materials

All chemicals were purchased from Sigma-Aldrich, Alfa Aesar. ¹H NMR spectra were recorded at 200 and 400 MHz and ¹³C NMR spectra were recorded at 100.6 and 50.3 MHz by using deuterated solvents indicated below. TLC were performed on aluminium backed silica plates (silica gel 60 F254). All the reactions were performed under nitrogen atmosphere using distilled solvents. All tested compounds were found to have > 95% purity determined by HPLC analysis. HPLC-grade water was obtained from a tandem Milli-Ro/Milli-Q apparatus. The analytical HPLC measurements were made on a Shimadzu LC-20AProminence equipped with a CBM-20A communication bus module, two LC-20AD dual piston pumps, a SPD-M20A photodiode array detector and a Rheodyne 7725i injector with a 20 µL stainless steel loop.

Abbreviations used in the following examples and elsewhere herein are:
- AC₂O: acetic anhydride
- AcOH: acetic acid
- AIBN: Azobisisobutyronitrile
- atm: atmosphere
- br: broad
- DIPEA: *N*,*N*-diisopropylethylamine
- DCM: dichloromethane
- DME: dimethoxyethane
- DMF: *N*,*N*-dimethylformamide
- DMSO: dimethyl sulfoxide
- EDC: *N*-(3-Dimethylaminopropyl)-*N'*-ethylcarbodiimide hydrochloride
- ESI: electrospray ionization
- EtOAc: ethyl acetate
- EtO₂: diethyl ether
- EtOH: ethanol
- EtO⁻Na⁺: sodium ethoxide
- Et₃NH⁺Cl⁻: triethylamine hydrochloride
- h: hour(s)
- HPLC: high-performance liquid chromatography
- LCMS: liquid chromatography-mass spectrometry
- m: multiplet
- MeI: methyl iodide
- MeOH: methanol
- MHz: megahertz
- min: minutes
- MS: molecular sieves
- MTBE: 2-methoxy-2-methylpropane
- MW: microwave
- NBS: N-bromosuccinamide
- NMR: nuclear magnetic resonance
- PET: petroleum ether
- ppm: parts per million
- p-TSA: para-toluenesulfonic acid
- r.t.: room temperature
- TLC: thin layer chromatography

### Example 1: Intermediate 1.2- 6-methyl-5-nitropyrimidine-2,4(1H,3H)-dione

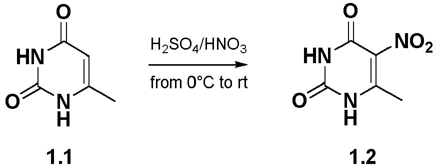

To a stirred mixture of 96% H₂SO₄ (15 mL) and of 70% HNO₃ (15 mL) was added 6-methyl pyrimidine-2,4-(1H,3H)-dione (2.5 g, 19.8 mmol). The solution was kept at 50 °C for 10h. The mixture was cooled to room temperature and poured into a large volume of ice water. The solid was collected and dried *in vacuo.* Recrystallization with MeOH gave the final compound (2.7g, 16.2 mmol) as yellows solid. ¹H NMR (200 MHz, DMSO) δ 2.31 (s, 3H), 11.82 (s, 1H), 11.85 (s, 1H).

### Example 2: Intermediate 2.2- ethyl 2-(2-formyl-6-methoxyphenoxy)acetate

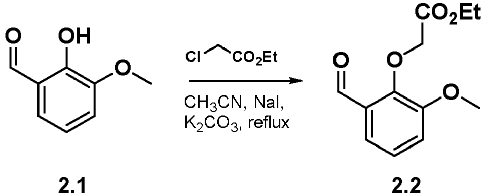

To a suspension of the starting compound **2.1** (3 g, 19.7 mmol) in CH₃CN (40 mL) was added K₂CO₃ (4.1 g, 29.5 mmol) and NaI (443 mg, 2.96 mmol). Stirring was continued at reflux 30 min and then ethyl chloroacetate (2.6 mL, 24.6 mmol) was added dropwise to the mixture. Stirring was continued at reflux additional 6 h. The solvent was removed *in vacuo.* The crude was taken up with water and extracted with EtOAc (3 x 20 ml). The organic phase was washed with brine and dried over Na₂SO₄. Evaporation of the solvent afforded the title compound 2.2 (3.2 g, 13.4 mmol) as pure white solid after shredding with Et₂O. Yield 68%; ¹H NMR (400 MHz, CDCl₃) δ 1.25 (t, *J* = 7.2 Hz, 3H), 3.88 (s, 3H), 4.20 (q, *J* = 7 Hz, 2H); 4.8 (s, 2H), 7.13 (m, 2H), 7.43 (t, *J=* 5 Hz, 1H), 10.6 (s, 1H).

### Example 3: Intermediate 3.2- 6-methyl-2,4-dioxo-1,2,3,4-tetrahydropyrimidine-5-carbonitrile

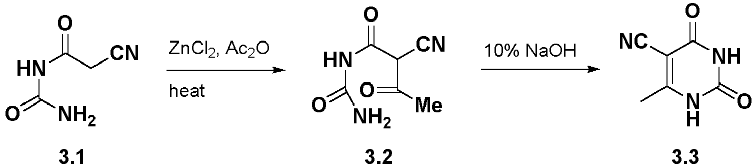

### Step 1. N-carbamoyl-2-cyano-3-oxobutanamide (3.2)

A mixture of intermediate 3.1 (5 g, 39.3 mmol) Ac₂O (20 mL) and molten ZnCl₂ (500 mg, mmol), was heated gently for several minutes until a solution was obtained. The solution was immediately cooled in an ice bath. The solidified product was filtered off, washed with Et₂O, and dried *in vacuo* affording intermediate 3.2 (4.5 g, 26.6 mmol) as colorless needles. Yield 68%. mp. 161 °C (dec).

### Step 2. 6-methyl-2,4-dioxo-1,2,3,4-tetrahydropyrimidine-5-carbonitrile (3.3)

Intermediate **3.2** (4.0 g, 2.36 mmol) was mixed with 10% NaOH (20 mL, 50 mmol). The mixture was shaken to give a solution, which solidified after a few minutes. The solidified mixture was heated at 60 °C for 5 minutes on a water bath, cooled to room temperature and acidified with 50% AcOH. The resulting precipitate was collected, dried *in vacuo* affording intermediate **3.3** (2.8 g, 18.5 mmol), as off white solid. ¹H NMR (400 MHz, DMSO) δ 2.08 (s, 3H), 10.05 (brs, 1H); ¹³C NMR (100 MHz, DMSO) δ 23.8, 79.9, 120.1, 159.4, 165.6, 173.7.

### Example 4: Intermediate 4.2-

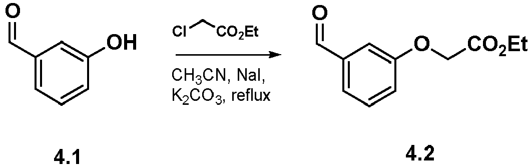

To a suspension of the starting compound 4.1 (500 mg, 3.12 mmol) in CH₃CN (40 mL) was added K₂CO₃ (645 mg, 3.74 mmol) and NaI (70 mg, 0.47 mmol). Stirring was continued at reflux 30 min and then ethyl chloroacetate (0.4 mL, 3.74 mmol) was added dropwise to the mixture. Stirring was continued at reflux additional 3 h. The solvent was removed *in vacuo.* The crude was taken up with water and extracted with EtOAc (3 x 20 ml). The organic phase was washed with brine and dried over Na₂SO₄. Evaporation of the solvent afforded the title compound 4.2 (620 mg, 3 mmol) as colorless oil. Yield 96%; =

### Example 5: Intermediate 5.4- ethyl 2-(3-formylphenoxy)acetate

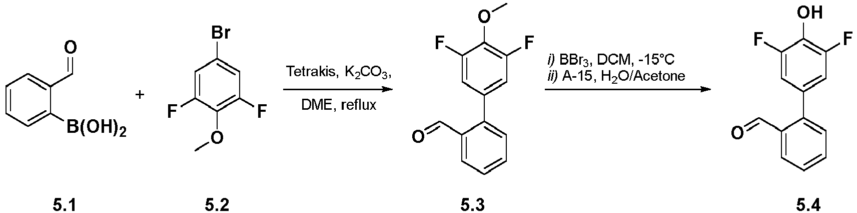

### Step 1. 3',5'-difluoro-4'-methoxy-[1,1'-biphenyl]-2-carbaldehyde (5.3)

To a solution of compound **5.2** (0.15 mL, 1.22 mmol) in DME (7 mL) was added tetrakis triphenylphosphine palladium (78 mg, 0.067 mmol). Stirring was continued at r.t. 5 min. 2-formyl phenyl boronic acid **5.1** (202 mg, 1.35 mmol) and K₂CO₃ (745 mg, 3.56 mmol) were added in turn. Stirring was continued at reflux 4h. The solvent was removed *in vacuo.* The crude was taken up with water and extracted with EtOAc (3 x 20 ml). The organic phase was washed with brine and dried over Na₂SO₄. Flash chromatography purification (eluent PET/EtOAc) of the reaction crude afforded the title compound **5.3** (180 mg, 0.72 mmol) as brownish oil. ¹H NMR (200 MHz, CDCl₃) δ 4.20 (s, 3H), 6.95 (d, *J* = 6.7 Hz, 2H), 7.40 (d, *J* = 7.7 Hz, 2H), 7.53 (d, *J=* 8.6 Hz, 1H), 7.64-7.70 (m, 1H), 8.03 (d, *J=* 7.8 Hz, 1H), 10.02 (s, 1H).

### Step 2. ethyl 2-(3-formylphenoxy)acetate (5.4)

To a solution of intermediate 5.3 (400 mg, 1.92 mmol) in DCM (15 mL) was added dropwise at -15 °C a 1 M solution of BBr₃ (3.84 mL) in DCM. Stirring was continued at -15 °C 2 h, then 16 h at r.t. The reaction was quenched by the addition of water. The organic phase was collected, washed with brine and dried over Na₂SO₄. The crude aldehyde hydrate (290 mg, 1.14 mmol) was solubilized in a mixture of H₂O/Acetone (3/6 mL) and A-15 (200 mg). Stirring was continued at reflux gently 72 h. The reaction mixture was filtered. The solvent was removed *in vacuo.* The crude was taken up with water, extracted with EtOAc (3 x 20 mL). The organic phase was washed with brine and dried over Na₂SO₄ affording the title compound 5.4 (130 mg, 0.55 mmol) as white solid; Yield 48%. ¹H NMR (200 MHz, CDCl₃) δ 6.95 (d, *J* = 8.2 Hz, 2H), 7.42 (d, *J* = 7.8 Hz, 1H), 7.59 (t, *J* = 7.4 Hz, 1H), 7.68 (t, *J* = 6.1 Hz, 1H), 8.04 (t, *J=* 7.6 Hz, 1H), 10.03 (s, 1H).

### Example 6: Intermediate 6.4- 6-methyl-4-oxo-2-thioxo-1,2,3,4-tetrahydropyrimidine-5-carbonitrile

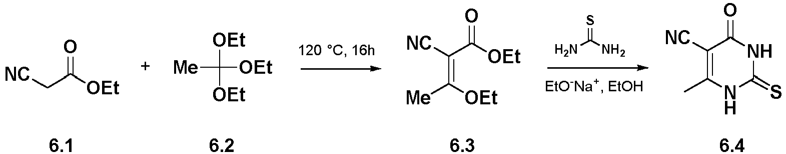

### Step 1. ethyl (Z)-2-cyano-3-ethoxybut-2-enoate (6.3)

To a mixture of ethyl cyanoacetate 6.1 (3.76 mL, 35.36 mmol) and triethyl orthoacetate **6.2** (9.7 mL, 53.04 mmol) was added a catalytic amount of AcOH (0.24 mL). Stirring was continued at reflux 18 h. The solid that was formed was collected and dried *in vacuo.* Compound **6.3** (4.7 g, 25.6 mmol) was obtained as yellowish powder. ¹H NMR (200 MHz, CDCl₃) δ 1.29 (t, *J=* 7.1 Hz, 3H), 1.42 (t, *J=* 7.1 Hz, 3H), 2.60 (s, 3H), 4.20 (q, *J=* 7.1 Hz, 2H), 4.20 (q, *J=* 7.1 Hz, 2H).

### Step 2. 6-methyl-4-oxo-2-thioxo-1,2,3,4-tetrahydropyrimidine-5-carbonitrile (6.4)

To a solution of intermediate 6.3 (4.7 g, 25.6 mmol) in EtOH (40 mL) was added thiourea (1.95 g, 26 mmol) and freshly prepared EtO⁻Na⁺ (1.77 g, 26 mmol). Stirring was continued at reflux for 18 h. The solvent was removed *in vacuo,* the crude was taken up with water, washed twice with EtOAc. The pH was adjusted to 5 by the addition of 3N HCl. The precipitate was collected and dried *in vacuo.* Intermediate 6.4 (2.6g, 15.8 mmol) was obtained as light brown powder. ¹H NMR (200 MHz, DMSO) δ 2.31 (s, 3H), 13.05 (brs, 1H), 13.1 (brs, 1H).

### Example 7: Intermediate 7.2- ethyl 2-(2-formylphenoxy)acetate

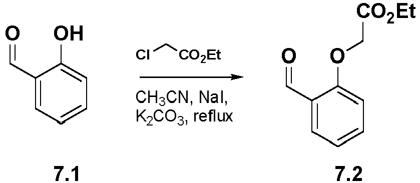

To a suspension of the starting compound 7.1 (1.5 g, 12.28 mmol) in CH₃CN (40 mL) was added K₂CO₃ (1.7 g, 12.28 mmol) and NaI (271 mg, 1.87 mmol). Stirring was continued at reflux for 30 min and then ethyl chloroacetate (1.31 mL, 12.28 mmol) was added dropwise to the mixture. Stirring was then continued at reflux for an additional 6 h. The solvent was removed *in vacuo.* The crude was taken up with water and extracted with EtOAc (3 x 20 ml). The organic phase was washed with brine and dried over Na₂SO₄. Evaporation of the solvent afforded the title compound **7.2** (1.8 g, 9.2 mmol) as colorless oil. Yield 75%; ¹H NMR (400 MHz, CDCl₃) δ 1.29 (t, *J=* 7.2 Hz, 3H), 4.28 (q, *J=* 7.2 Hz, 2H), 4.75 (s, 2H), 6.87 (t, *J* = 8.4, 1H), 7.54 (m, 1H), 7.88 (dd, *J_{d}* = 7.7 Hz, *J_{d}* = 1.8 Hz, 1H), 10.5 (s, 1H).

### Example 8: Intermediate 8.1- ethyl 2-(2-formyl-6-methoxyphenoxy)-2-methyl-propanoate

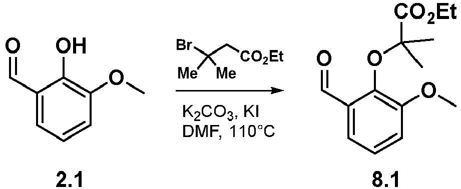

To a solution of the starting compound 2.1 (500 mg, 3.29 mmol) in DMF (10 mL) was added K₂CO₃ (500 mg, 3.62 mmol), KI (142 mg, 0.86 mmol) and ethyl 3-bromo-3-methyl-butyrate (0.73 mL, 5 mmol). Stirring was continued at 110 °C for an additional 4 h. The reaction was poured into water, extracted with EtOAc (3 x 20 ml). The organic phase was washed with brine and dried over Na₂SO₄. Flash chromatography purification (eluent PET/EtOAc, 0-20%) of the reaction mixture afforded the title compound 8.1 (570 mg, 2.13 mmol) as colorless oil. Yield 63%; ¹H NMR (400 MHz, CDCl₃) δ 1.34 (t, *J=* 7.1 Hz, 3H), 1.54 (s, 6H), 3.78 (s, 3H), 4.29 (q, *J* = 7.1 Hz, 2H), 7.1 (d, *J* = 1.8 Hz, 1H), 7.12 (d, *J* = 7.5 Hz, 1H), 7.44 (dd, *J_{d} =* 7.5 Hz, *J_{d}* = 1.9 Hz, 1h), 10.50 (s, 1H).

### Example 9: Intermediate 9.1 - methyl 2-(2-formyl-6-methoxyphenoxy)propanoate

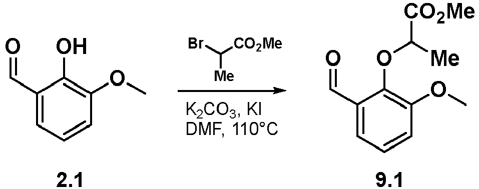

To a solution of the starting compound 2.1 (500 mg, 3.29 mmol) in DMF (10 mL) was added K₂CO₃ (500 mg, 3.62 mmol), KI (110 mg, 0.66 mmol) and methyl 2-bromo-propionate (0.64 mL, 4.94 mmol). Stirring was continued at 110 °C for an additional 4 h. The reaction was poured in water, extracted with EtOAc (3 x 20 ml). The organic phase was washed with brine and dried over Na₂SO₄. Flash chromatography purification (eluent PET/EtOAc, 0-20%) of the reaction mixture afforded the title compound 9.1 (626 mg, 2.63 mmol) as colorless oil. Yield 80%; ¹H NMR (400 MHz, CDCl₃) δ 1.22 (t, *J* = 7.1 Hz, 3H), 1.64 (d, *J* = 6.8 Hz, 3H), 3.88 (s, 3H), 4.16 (q, *J* = 7.1 Hz, 2H), 7.11-7.13 (m, 2H), 7.43-7.45 (m, 1H), 10.6 (1H).

### Example 10: Intermediate 10.2 - ethyl 2-(2-ethoxy-6-formylphenoxy)acetate

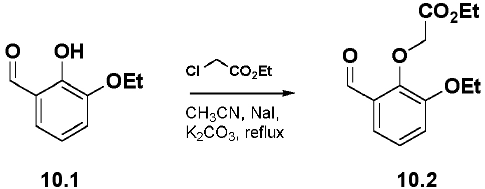

To a suspension of the starting compound 10.1 (500 mg, 3 mmol) in CH₃CN (25 mL) was added K₂CO₃ (455 mg, 3.3 mmol) and NaI (65 mg, 0.45 mmol). Stirring was continued at reflux for 30 min and then ethyl chloroacetate (0.35 mL, 3.3 mmol) was added dropwise to the mixture. Stirring was continued at reflux for an additional 16 h. The solvent was removed *in vacuo.* The crude was taken up with water and extracted with EtOAc (3 x 20 ml). The organic phase was washed with brine and dried over Na₂SO₄. Flash chromatography purification (eluent PET/EtOAc, 0-20%) of the reaction mixture afforded the title compound **10.2** (650 mg, 2.7 mmol) as yellowish oil. Yield 90%; ¹H NMR (400 MHz, CDCl₃) δ 1.28 (t, *J* = 7.1 Hz, 3H), 1.48 (t, *J* = 7 Hz, 3H), 4.11 (q, *J* = 6.9 Hz, 2H), 4.23 (q, *J* = 7.1 Hz, 2H), 4.86 (s, 2H), 7.12-7.14 (m, 2H), 7.44 (dd, *J_{d}* = 5.8 Hz, *J_{d}* = 3.5 Hz, 1H), 10.6 (s, 1H).

### Example 11: Intermediate 11.2 - ethyl 2-(2-formyl-4-methoxyphenoxy)acetate

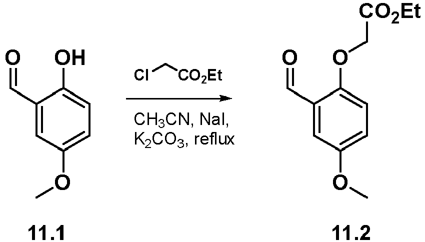

To a suspension of the starting compound **11.1** (0.25mL, 2 mmol) in CH₃CN (10 mL) was added K₂CO₃ (414 mg, 3 mmol) and NaI (45 mg, 0.3 mmol). Stirring was continued at reflux for 30 min and then ethyl chloroacetate (0.27 mL, 2.5 mmol) was added dropwise to the mixture. Stirring was continued at reflux for an additional 16 h. The solvent was removed *in vacuo.* The crude was taken up with water and extracted with EtOAc (3 × 20 ml). The organic phase was washed with brine and dried over Na₂SO₄. Flash chromatography purification (eluent PET/EtOAc, 0-20%) of the reaction mixture afforded the title compound **11.2** (450 mg, 1.88 mmol) as colorless oil. Yield 94%; ¹H NMR (400 MHz, CDCl₃) δ 1.20 (t, *J* = 7.2 Hz, 3H), 3.81 (s, 3H), 4.27 (q, *J* = 7.1 Hz, 2H), 4.72 (s, 2H), 6.86 (d, *J* = 9.1 Hz, 1H), 7.11 (dd, *J_{d} =* 9.4 Hz, *J_{d}* = 3.2 Hz, 1H), 7.36 (d, *J* = 3.2 Hz, 1H), 10.5 (s, 1H).

### Example 12: Intermediate 12.3 - ethyl 3'-formyl-[1,1'-biphenyl]-3-carboxylate

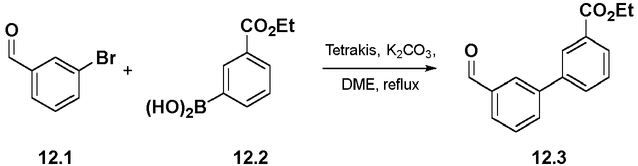

Tetrakis triphenylphosphine palladium (59 mg, 0.05 mmol) and intermediate 12.1 (0.19 mL, 1.70 mmol) were dissolved in DME (5 mL). To another flask was added K₂CO₃, compound **12.2** (300 mg, 1.54 mmol), and DME (5 mL) and the resulting mixture was stirred 10 min. The solutions were mixed together via cannula. Stirring was continued at 110 °C for 20 h. The crude reaction mixture was poured in water, acidified with 3N HCl and extracted with EtOAc (3 x 20 mL). The organic phase was washed with brine and dried over Na₂SO₄. Flash chromatography purification (eluent PET/EtOAc, 0-20%) of the reaction mixture afforded the title compound **12.3** (280 mg, 1.1 mmol) as colored oil. Yield 65%; ¹H NMR (400 MHz, CDCl₃) δ 1.44 (t, *J* = 7.1 Hz, 3H), 4.44 (q, *J* = 7.1 Hz, 2H), 7.56 (t, *J* = 7.7 Hz, 1H), 7.65 (t, *J* = 7.6 Hz, 1H), 7.82 (d, *J* = 7.7 Hz, 1H), 7.91 (d, 7.6 Hz, 2H), 8.09 (d, *J* = 7.8 Hz, 1H), 8.14 (s, 1H), 8.07 (s, 1H), 10.11 (s, 1H).

### Example 13: Intermediate 13.2 - 2-(2-ethoxy-4-formylphenoxy)acetic acid

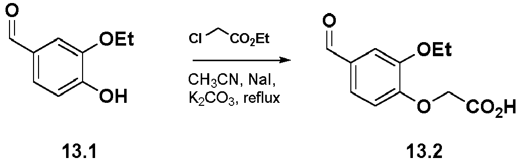

To a suspension of the starting compound 13.1 (500 mg, 3 mmol) in CH₃CN (20 mL) was added K₂CO₃ (621 mg, 4.5 mmol) and NaI (67 mg, 0.45 mmol). Stirring was continued at reflux for 30 min and then ethyl chloroacetate (0.4 mL, 3.75 mmol) was added dropwise to the mixture. Stirring was continued at reflux for an additional 16 h. The solvent was removed *in vacuo.* The crude was taken up with water and extracted with EtOAc (3 x 20 ml). The organic phase was washed with brine and dried over Na₂SO₄. Flash chromatography purification (eluent PET/EtOAc, 0-20%) of the reaction mixture afforded the title compound **13.2** (848 mg, 2.25 mmol) as colorless oil. Yield 75%; ¹H NMR (200 MHz, CDCl₃) δ 1.33 (t, *J* = 7.2 Hz, 3H), 1.53 (t, *J* = 7 Hz, 3H), 4.16 (q, *J* = 4.8 Hz, 2H), 4.31 (q, *J* = 7.2 Hz, 2H), 4.82 (s, 2H), 6.92 (d, *J* = 7.9 Hz, 1H), 7.45 (d, *J* = 7.7 Hz, 2H), 9.89 (s, 1H).

### Example 14: Intermediate 14.3- 2-((1H-tetrazol-5-yl)methoxy)-3-methoxybenzaldehyde

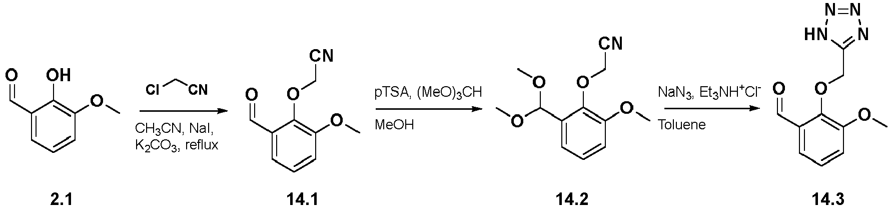

### Step 1. 2-(2-formyl-6-methoxyphenoxy)acetonitrile (14.1)

To a suspension of the starting compound **2.1** (1 g, 6.57 mmol) in CH₃CN (35 mL) was added K₂CO₃ (997 mg, 7.23 mmol) and NaI (148 mg, 0.99 mmol). Stirring was continued at reflux for 30 min and then ethyl cyanoacetate (0.5 mL, 7.23 mmol) was added dropwise to the mixture. Stirring was continued at reflux an additional 16 h. The solvent was removed *in vacuo.* The crude mixture was taken up with water and extracted with EtOAc (3 x 20 ml). The organic phase was washed with brine and dried over Na₂SO₄ affording compound **14.1** (1.14 g, 6 mmol) as brownish solid. Yield 91%; ¹H NMR (200 MHz, CDCl₃) δ 3.95 (s, 3H), 5.0 (s, 3H), 7.24 (d, *J* = 6.4 Hz, 2H), 7.48 (dd, *J_{d} =* 7 Hz, *J_{d}* = 2.2 Hz, 1H), 10.4 (s, 1H).

### Step 2. 2-(2-(dimethoxymethyl)-6-methoxyphenoxy)acetonitrile (14.2)

To a solution of the starting compound **14.1** (1.14 g, 5.96 mmol) in MeOH (40 mL) was added trimethyl orthoformate (6.5 mL, 59.6 mmol) and *p*-TSA (113.4 mg, 0.59 mmol). Stirring was continued at reflux for 24 h. The solvent was removed *in vacuo.* And the crude mixture was taken up with Et₂O (50 mL). The organic extract was washed with NaHCO₃ (sat. solution) and brine and dried over Na₂SO₄. The title compound **14.2** (1.3 g, 5 mmol) was obtained as brown oil. Yield 83%. ¹H NMR (200 MHz, CDCl₃) δ 3.39 (s, 6H), 3.90 (s, 3H), 4.84 (s, 2H), 5.66 (s, 1H), 6.96 (d, *J* = 4.8 Hz, 1H), 7.15 (m, 1H), 7.18 (m, 1H).

### Step 3. 2-((1H-tetrazol-5-yl)methoxy)-3-methoxybenzaldehyde (14.3)

To a solution of intermediate 14.2 (1.1 g, 4.6 mmol) in toluene (45 ml) was added Et₃NH⁺Cl⁻ (1.9 g, 13.9 mmol) and NaN₃ (902 mg, 13.9 mmol). Stirring was continued at reflux for 16 h. The reaction mixture was poured into water and then stirred 10 min. The organic phase was collected. The pH of the organic phase was adjusted to 4 with 3N HCl followed by extraction with EtOAc (3 × 20 mL). The organic phase was washed with brine and dried over Na₂SO₄ affording compound **14.3** (560 mg, 2.4 mmol) as brown oil. Yield 52%. ¹H NMR (400 MHz, CDCl₃) δ 3.88 (s, 3H), 5.54 (s, 2H), 7.27 (t, *J* = 4.19 Hz, 2H), 7.44 (d, *J* = 6 Hz, 1H), 10.2 (s, 1H).

### Example 15: Intermediate 15.3 - 2-(3-formylphenyl)acetic acid

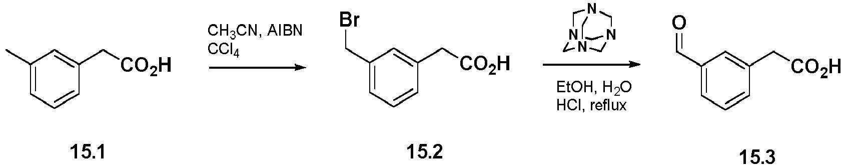

### Step 1. 2-(3-(bromomethyl)phenyl)acetic acid (15.2)

To a solution of the starting compound 15.1 (2 g, 13.3 mmol) in CCl₄ (30 mL) were added NBS (2.6 g, 14.7 mmol) and AIBN (11 mg, 0.066 mmol). Stirring was continued at reflux for 16 h. The solvent was removed *in vacuo.* The crude was taken up with water and extracted with EtOAc (3 × 20 mL). The organic phase was washed with brine, dried over Na₂SO₄ and concentrated under reduced pressure. The title compound 15.2 (2.9 g, 12.7 mmol) was obtained as white solid after shredding with Et₂O. Yield 95%. ¹H NMR (400 MHz, CDCl₃) δ 1.83 (s, 2H), 4.47 (s, 2H), 7.24 (m, 1H), 7.32-7.36 (m, 3H), 9.30 (s, 1H). *Step 2.* **2-(3-formylphenyl)acetic acid (15.3)**

Intermediate **15.2** (1 g, 4.4 mmol) was dissolved in a 1:1 mixture of EtOH (10 mL) and water (10 mL) and hexamethylentetramine (1.66 g, 11.88 mmol) was added. The mixture was heated to reflux for 4 h. Concentrated HCl (2 mL) was added cautiously to the mixture at reflux. Stirring was continued at this temperature for an additional 30 min and then reaction mixture was allowed to cool. The solvent was removed *in vacuo.* The crude was taken up with water and the pH was adjusted to 8 by the addition of NaHCO₃. The aqueous phase was washed twice with EtOAc (2 × 20 mL). Then the pH was adjusted to 3 by the addition of 3N HCl. The crude mixture was extracted with EtOAc (3 × 20 ml). The organic phase was washed with brine, dried over Na₂SO₄, filtered, and concentrated under reduced pressure. The title compound **15.3** (520 mg, 3.2 mmol) was obtained as white solid. Yield 72%. ¹H NMR (200 MHz, CDCl₃) δ 3.71 (s, 2H), 7.49 (m, 2H), 7.61 (s, 2H), 10.03 (s, 1H).

### Example 16: Intermediate 35. 1-2-(2-Ethoxy-6-formyl-phenoxy)-acetamide

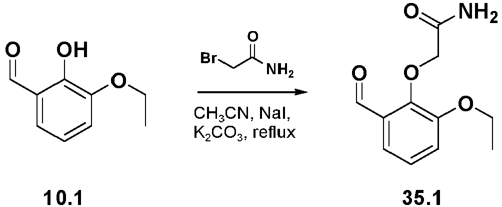

To a stirred solution of the starting compound 10.1 (300 mg, 1.81 mmol) in CH₃CN (10 mL) was added K₂CO₃ (375 mg, 2.72 mmol), NaI (41 mg, 0.27 mmol) and ethyl 2-bromoacetamide (312 mg, 2.26 mmol) and stirring was continued at reflux for an additional 16 h. The solvent was removed *in vacuo.* The crude was taken up with water and extracted with EtOAc (3 × 20 ml). The organic phase was washed with brine, dried over Na₂SO₄, filtered and concentrated to afford the title compound 35.1 (650 mg, 2.7 mmol) as yellowish powder. Yield 89%; ¹H NMR (400 MHz, CDCl₃) δ 1.48 (t, *J=* 6.9 Hz, 3H), 4.11 (q, *J=* 6.9 Hz, 1H), 4.67 (s, 1H), 7.14-7.21 (m, 2H), 7.34-7.38 (m, 1H), 7.85 (brs, 1H), 10.2 (s, 1H).

### Example 17: Intermediate 17. 2 -3'-Formyl-biphenyl-2-carboxylic acid ethyl ester

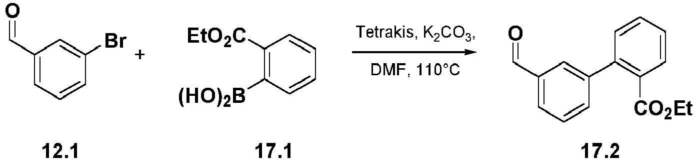

Tetrakis triphenylphosphine palladium (73 mg, 0.069 mmol) and intermediate 12.1 (0.27 mL, 2.3 mmol) were dissolved in DMF (4 mL). K₂CO₃ (492 mg, 3.57 mmol), compound 17.1 (400 mg, 2.1 mmol), and DMF (5 mL) were dissolved in a separate flask and the resulting mixture was stirred 10 min. The two solutions were then mixed together via cannula and stirring was continued at 110 °C for 20 h. The crude reaction mixture was poured in water, acidified with 3N HCl and extracted with EtOAc (3 × 20 mL). The organic phase was washed with brine and dried over Na₂SO₄. Flash chromatography purification (eluent PET/EtOAc, 0-20%) of the crude reaction mixture afforded the title compound 17.2 (350 mg, 1.1 mmol) as white solid. Yield 60%; ¹H NMR (400 MHz, CDCl₃) δ 1.03 (t, *J* = 7.1 Hz, 3H), 4.11 (q, *J* = 7.1 Hz, 2H), 7.38 (d, *J* = 7.5 Hz, 1H), 7.48 (t, *J* = 7.5 Hz, 1H), 7.57-7.60 (m, 3H), 7.85 (s, 1H), 7.89-7.91 (m, 1H), 7.94 (d, *J* = 7.6 Hz, 1H), 10 (s, 1H).

### Example 18: (E)-2-(2-methoxy-6-(2-(5-nitro-2,6-dioxo-1,2,3,6-tetrahydropyrimidin-4-yl)vinyl)phenoxy)acetic acid (Compound I-1)

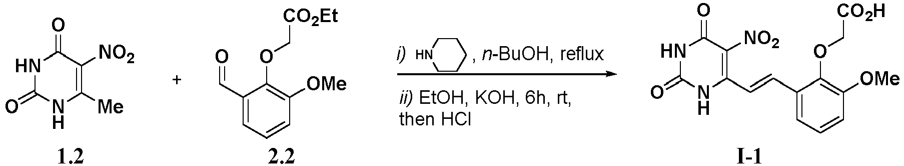

To a stirred suspension of intermediate **1.2** (200 mg, 1.16 mmol) and intermediate **2.2** (304 mg, 1.27 mmol) in *n*-butanol (6 mL) was added piperidine (0.12 mL, 1.16 mmol). Stirring was continued overnight at reflux. Upon cooling the solid was collected, washed with Et₂O and dried *in vacuo.* The solid was taken up with EtOH, the resulting solution was basified with 2 molar solution of KOH and then stirred an additional 6 h. The volatiles were removed *in vacuo.* The crude was taken up with water, acidified to pH 3 with 3N HCl solution. Compound **I-1** (300 mg, 0.79 mmol) was obtained as yellowish solid. ¹H NMR (400 MHz, DMSO) δ 3.81 (s, 3H), 4.58 (s, 3H), 7.04 (d, *J* = 16.2 Hz, 1H), 7.14 (s, 2H), 7.27 (s, 1H), 7.97 (d, *J* = 16.1 Hz, 1H), 11.68 (brs, 1H); ¹³C NMR (100 MHz, DMSO) δ 56.2, 69.3, 115.5, 115.5, 119.4, 124.8, 126.6, 128.1, 137, 146.1, 148.3, 149.6, 152.3, 156.8, 170.3; HPLC: 96.1%.

### Example 19: (E)-2-(2-(2-(5-cyano-2,6-dioxo-1,2,3,6-tetrahydropyrimidin-4-yl)vinyl)-6-methoxyphenoxy)acetic acid (Compound I-2)

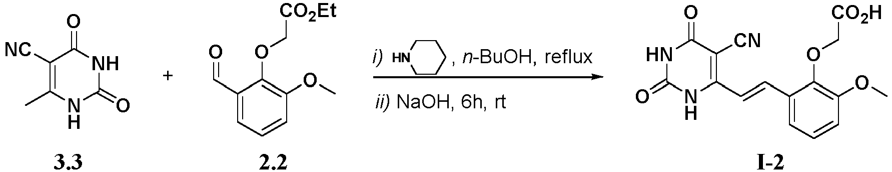

To a stirred suspension of intermediate 3.3 (400 mg, 2.64 mmol) and intermediate 2.2 (964 mg, 3.96 mmol) in *n*-butanol (15 mL) was added piperidine (0.29 mL, 2.9 mmol). Stirring was continued overnight at reflux. Upon cooling the solid was collected, washed with Et₂O, and dried *in vacuo.* The solid was taken up with a 2 M solution of NaOH and stirred an additional 6 h. The crude was diluted with cold water, acidified to pH 3 with 3N HCl solution affording compound 1-2 (300 mg, 0.87 mmol) as yellowish solid. Yield 33%. ¹H NMR (400 MHz, DMSO) δ 3.82 (s, 3H), 4.60 (s, 2H), 6.98 (d, *J=* 16.4 Hz, 1H), 7.18 (m, 2H), 7.3 (m, 1H), 8.18 (d, *J* = 16.5 Hz, 1H), 11.90 (s, 1H), 12 (brs, 1H), 12.95 (brs, 1H); ¹³C NMR (100 MHz, DMSO) δ 56.4, 69.4, 86, 115.2, 115.8, 117.6, 119.2, 125.1, 128.2, 138.1, 146.3, 150.3, 152.4, 157.8, 161.9, 170.5; HPLC: 95.01%.

### Example 20: (E)-2-(3-(2-(5-cyano-2,6-dioxo-1,2,3,6-tetrahydropyrimidin-4-yl)vinyl) phenoxy)acetic acid (Compound I-3)

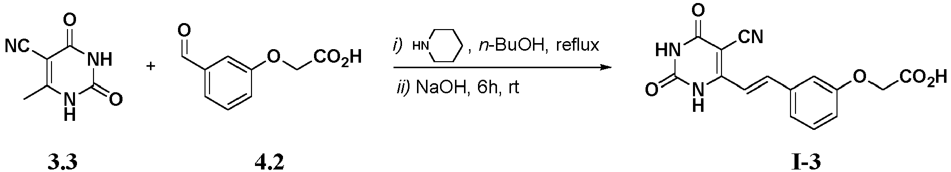

To a stirred suspension of intermediate 3.3 (200 mg, 1.34 mmol) and intermediate 4.2 (335 mg, 1.6 mmol) in *n*-butanol (10 mL) was added piperidine (0.15 mL, 1.47 mmol). Stirring was continued overnight at reflux. Upon cooling the solid was collected, washed with cold Et₂O and dried *in vacuo.* The solid was taken up with a 2 molar solution of NaOH and the resulting solution was then stirred for an additional 6 h. The crude mixture was diluted with cold water and then the aqueous solution was acidified to pH 3 with 3N HCl solution affording compound **I-3** (180 mg, 0.87 mmol) as yellowish solid. Yield 43%. ¹H NMR (400 MHz, DMSO) δ 4.76 (s, 2H), 6.97 (d, *J* = 16.3 Hz, 1H), 7.03 (d, *J* = 7.7 Hz, 1H), 7.15 (s, 1H), 7.24 (d, *J* = 7.4 Hz, 1H); 7.39 (t, *J* = 7.7 Hz, 1H), 7.89 (d, *J* = 16.3 Hz, 1H), 11.7 (s, 1H), 11.9 (s, 1H), 13 (brs, 1H); ¹³C NMR (100 MHz, DMSO) δ 64.8, 86.1, 114.1, 115.1, 116.9, 117.6, 121.5, 130.7, 135.7, 142.6, 150.2, 157.4, 158.5, 161.8, 170.3; HPLC: 98.3%.

### Example 21: (E)-6-(2-(3',5'-difluoro-4'-hydroxy-[1,1'-biphenyl]-2-yl)vinyl)-2,4-dioxo-1,2,3,4-tetrahydropyrimidine-5-carbonitrile (Compound I-4)

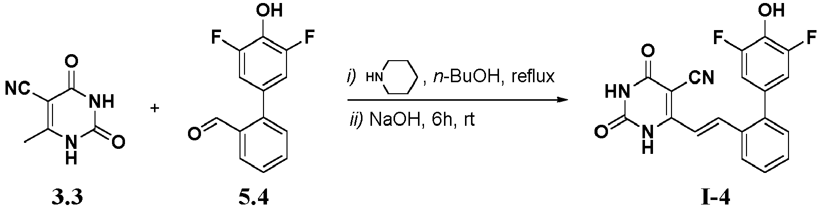

To a stirred suspension of intermediate **3.4** (77 mg, 0.5 mmol) and intermediate **5.4** (130 mg, 1.2 mmol) in *n*-butanol (10 mL) was added piperidine (0.05 mL, 0.55 mmol). Stirring was continued overnight at reflux. Upon cooling the solid was collected, washed with cold Et₂O and dried *in vacuo.* The solid was taken up with a 1 molar solution of NaOH and stirred for an additional 6 h. The crude was diluted with cold water, acidified to pH 3 with 3N HCl solution affording compound **I-4** (40 mg, 0.11 mmol) as yellowish solid. Yield 22%. ¹H NMR (400 MHz, DMSO) δ 6.90 (d, *J* = 16.2 Hz, 1H), 7.02 (d, *J* = 7.6 Hz, 2H), 7.40 (d, *J* = 6.9 Hz, 1H), 7.5 (m, 2H), 7.82 (d, *J* = 7.2 Hz, 1H), 7.88 (d, *J* = 16.2 Hz, 1H), 10.5 (s, 1H), 11.6 (s, 1H), 11.90 (s, 1H); ¹³C NMR (100 MHz, DMSO) δ 87.7, 115.1, 115.3 (*²J_{CF}* = 22 Hz), 116.6, 119.7, 128.7, 130.2, 131.5, 132.4 (*³J_{CF}* = 9 Hz), 134.2, 135.15, 135.4 (*²J_{CF}* = 16 Hz), 142.4, 142.6, 151.8, 153.8 (*¹J_{CF}* = 242 Hz), 153.9 (*¹J_{CF}*= 242 MHz), 158.5, 163.2; HPLC: 98.3%.

### Example 22: (E)-2-(2-(2-(5-cyano-6-oxo-2-thioxo-1,2,3,6-tetrahydropyrimidin-4-yl) vinyl)-6-methoxyphenoxy)acetic acid (Compound I-5)

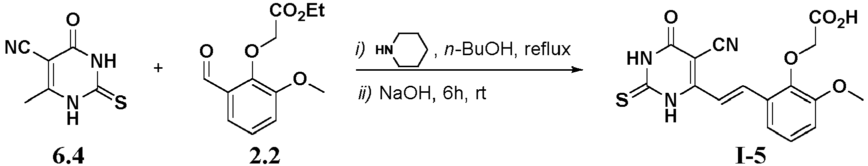

To a stirred suspension of intermediate **6.4** (250 mg, 0.89 mmol) and intermediate **2.2** (238 mg, 0.98 mmol) in *n*-butanol (10 mL) was added piperidine (0.1 mL, 0.55 mmol). Stirring was continued overnight at reflux. Upon cooling the solid was collected, washed with cold Et₂O and dried *in vacuo.* The solid was taken up with a 1M solution of NaOH and stirred for an additional 6h. The crude mixture was diluted with cold water, acidified to pH 3 with 3N HCl solution affording an orange solid. The crude product was boiled in a mixture of MeOH/DCM-1:2 affording the title compound **I-5** (200 mg, 0.55 mmol) as yellowish solid. Yield 62%. ¹H NMR (400 MHz, DMSO) δ 3.82 (s, 3H), 4.61 (s, 2H), 6.99 (d, *J=* 16.5 Hz, 1H), 7.18 (m, 2H), 7.27 (m, 1H), 8.27 (d, *J=* 16.5 Hz, 1H), 12.9 (brs, 1H), 13.0 (s, 2H); ¹³C NMR (100 MHz, DMSO) δ 56.4, 69.5, 88.9, 114.9, 115.9, 117.4, 119.2, 125.0, 128.2, 139.1, 146.4, 152.4, 156.9, 158.9, 170.4, 176.4. HPLC: 92.4%.

### Example 23: (E)-2-(2-(2-(5-cyano-6-oxo-2-thioxo-1,2,3,6-tetrahydropyrimidin-4-yl) vinyl)phenoxy)acetic acid (Compound I-6)

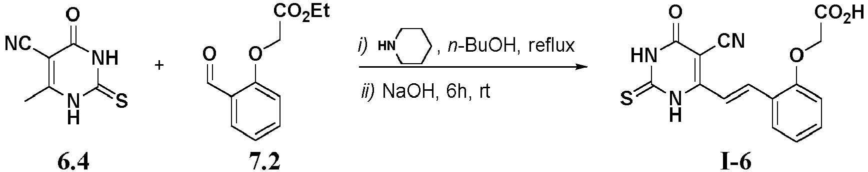

To a stirred suspension of intermediate **6.4** (200 mg, 1.19 mmol) and intermediate **7.2** (300 mg, 1.44 mmol) in *n*-butanol (8 mL) was added piperidine (0.15 mL, 1.44 mmol). Stirring was continued overnight at reflux. The solid was collected, washed with cold Et₂O and dried *in vacuo.* The solid was taken up with a 1M solution of NaOH (20 mL) and stirred for an additional 6h. The crude product was diluted with cold water, acidified to pH 3 with 3N HCl solution affording the title compound **I-6** (170 mg, 0.51 mmol) as yellowish solid. Yield 43%. ¹H NMR (400 MHz, DMSO) δ 4.84 (s, 2H), 7.02 (d, *J* = 8.4 Hz, 1H); 7.07 (t, *J* = 7.4 Hz, 1H), 7.14 (d, *J* = 7.4 Hz, 1H), 7.14 (d, *J* = 16.4 Hz, 1H), 7.43 (t, *J* = 7.3 Hz, 1H); 7.62 (d, *J* = 7.6 Hz, 1H) 8.19 (d, *J* = 16.4 Hz, 1H), 13 (s, 2H); ¹³C NMR (100 MHz, DMSO) δ 55.3, 65.3, 88.7, 113.2, 115.1, 117.5, 121.9, 123.4, 129.8, 132.7, 139.5, 157.2, 159.1, 170.1, 176.6; HPLC: 96.02%.

### Example 24: (E)-6-(2-(3',5'-difluoro-4'-hydroxy-[1,1'-biphenyl]-2-yt)vinyl)-4-oxo-2-thioxo-1,2,3,4-tetrahydropyrimidine-5-carbonitrile (Compound I-7)

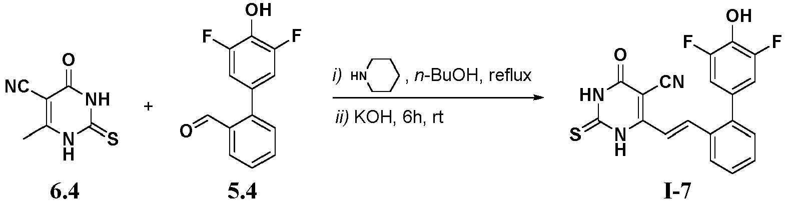

To a stirred suspension of intermediate **6.4** (84 mg, 0.51 mmol) and intermediate **5.4** (130 mg, 0.56 mmol) in *n*-butanol (10 mL) was added piperidine (0.05 mL, 0.51 mmol). Stirring was continued overnight at 120 °C. The solid was collected, washed with cold Et₂O and dried *in vacuo.* The solid was taken up with a 1M solution of KOH (20 mL) and stirred for an additional 6 h. The crude mixture was diluted with cold water, acidified to pH 3 with 3N HCl solution affording the title compound I-7 (35 mg, 0.09 mmol) as yellowish solid. Yield 18%. ¹H NMR (400 MHz, DMSO) δ 6.9 (d, *J* = 16.3 Hz, 1H), 7.04 (d, *J* = 6.9 Hz, 2H), 7.42 (s, 1H), 7.53 (m, 2H), 7.8 (d, *J* = 5.4 Hz, 1 H), 7.98 (d, *J* = 16.3 Hz, 1H), 10.5 (s, 1H), 12.9 (brs, 1H), 13 (s, 1H); HPLC: 96.4%.

### Example 25: (E)-2-(2-(2-(5-cyano-6-oxo-2-thioxo-1,2,3,6-tetrahydropyrimidin-4-yl)vinyl)-6-methoxyphenoxy)-2-methylpropanoic acid (Compound I-8)

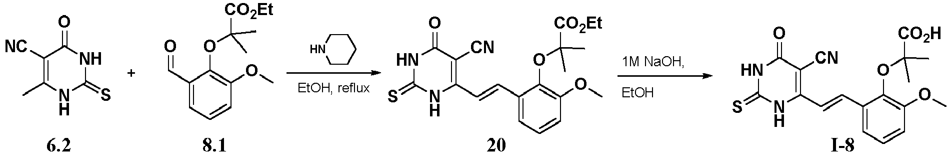

### Step 1. ethyl (E)-2-(2-(2-(5-cyano-6-oxo-2-thioxo-1,2,3,6-tetrahydropyrimidin-4-yl) vinyl)-6-methoxyphenoxy)-2-methylpropanoate (20)

To a stirred solution of intermediate **6.2** (200 mg, 1.19 mmol) and intermediate **8.1** (350 mg, 1.31 mmol) in ethanol (15 mL) was added piperidine (0.12 mL, 1.19 mmol) and molecular sieves. Stirring was continued at reflux overnight. Upon cooling, the solid was collected washed with Et₂O and dried *in vacuo.* Intermediate **20** (250 mg, 0.6 mmol) was obtained as a yellowish solid. Yield 51%. ¹H NMR (400 MHz, DMSO) δ 1.23 (t, *J=* 7.1 Hz, 3H), 1.42 (s, 6H), 3.69 (s, 3H), 4.17 (q, *J=* 7.1 Hz, 2H), 7 (d, *J=* 16.5 Hz, 1H), 7.17 (m, 2H), 7.29 (m, 1H), 8.17 (d, *J=* 16.5 Hz, 1H), 13.05 (brs, 2H).

### Step 2. (E)-2-(2-(2-(5-cyano-6-oxo-2-thioxo-1,2,3,6-tetrahydropyrimidin-4-yl)vinyl)-6-methoxyphenoxy)-2-methylpropanoic acid (I-8)

To a solution of compound 20 (210 mg, 0.51 mmol) in EtOH (20 mL) was added 1M NaOH (1 mL). Stirring was continued at r.t. 16 h. The solvent was removed *in vacuo.* The crude was taken up with water, washed twice with EtOAc (2 × 20 mL). The pH was adjusted to 3 by the addition of 1N HCl followed by extraction with EtOAc (3 × 20 mL). The organic phase were washed with brine, dried over Na₂SO₄ and concentrated under vacuum. Shredding with a mixture of acetone/Et₂O afforded the title compound 1-8 (115 mg, 0.3 mmol) as yellowish solid. Yield 58% ¹H NMR (400 MHz, DMSO) δ 1.38 (s, 6H), 3.7 (s, 3H), 6.98 (d, *J* = 16.5 Hz, 1H), 7.13 (m, 2H), 7.26 (m, 1H); 8.19 (d, *J* = 16.5 Hz, 1H), 12.45 (brs, 1H), 12.95 (brs, 1H), 13.04 (s, 1H); ¹³C NMR (100 MHz, DMSO) δ 25.4, 25.4, 55.5, 81.3, 88.2, 115.2, 117.7, 119.3, 124.6, 129.3, 139.3, 143.9, 152.9, 156.9, 159.1, 174.8, 176.3; HPLC: 96.1%

### Example 26: (E)-2-(2-(2-(5-cyano-6-oxo-2-thioxo-1,2,3,6-tetrahydropyrimidin-4-yl) vinyl)-6-methoxyphenoxy)propanoic acid (Compound I-9)

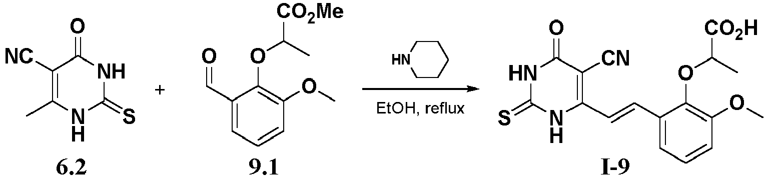

To a stirred suspension of intermediate **6.2** (180 mg, 1.08 mmol) and intermediate **9.1** (300 mg, 0.56 mmol) in *n*-butanol (10 mL) was added piperidine (0.1 mL, 1.08 mmol). Stirring was continued overnight at 120 °C. The mixture was cooled in an ice bath. The resulting solid was collected, washed with cold Et₂O, and dried *in vacuo.* The solid was then taken up in a 1M solution of KOH (20 mL) and stirred for an additional 6 h. The crude material was diluted with cold water, acidified to pH 3 with 3N HCl solution and the yellow gel was collected. Shredding with a mixture of acetone/Et₂O afforded the title compound **I-9** (40 mg, 0.11 mmol) as yellowish solid. Yield 10%. ¹H NMR (400 MHz, DMSO) δ 1.48 (d, *J* = 6.6 Hz, 3H), 3.80 (s, 3H), 4.88 (q, *J* = 6.6 Hz, 1H), 7.07 (d, *J* = 16.5 Hz, 1H), 7.14 (m, 2H), 7.24 (d, *J=* 6.6 Hz, 1H), 8.35 (d, *J=* 16.5 Hz, 1H), 12.80 (brs, 1H), 13.01 (s, 2H); ¹³C NMR (100 MHz, DMSO) δ 18.9, 56.4, 76.5, 88.1, 115.1, 115.9, 117.5, 119.7, 124.4, 128.3, 139.9, 145.9, 152.9, 156.9, 159.1, 173.1, 176.4; HPLC: 95.2%.

### Example 27: (E)-6-(3-methoxystyryl)-4-oxo-2-thioxo-1,2,3,4-tetrahydropyrimidine-5-carbonitrile (Compound I-10) (compound not in accordance with the invention)

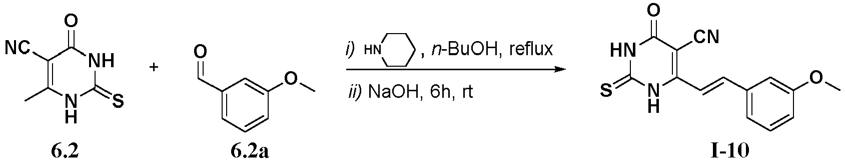

To a stirred suspension of intermediate **6.2** (200 mg, 1.19 mmol) and 3-methoxy benzaldehyde (**6.2a**, 0.17 mL, 1.43 mmol) in *n*-butanol (10 mL) was added piperidine (0.13 mL, 1.31 mmol). Stirring was continued overnight at reflux. The solid was collected, washed with cold Et₂O, and dried *in vacuo.* The solid was taken up with a 1M solution of NaOH (20 mL) and stirred for an additional 6 h. The crude mixture was diluted with cold water, acidified to pH 3 with 3N HCl solution affording the title compound **I-10** (188 mg, 0.55 mmol) as yellowish solid. Yield 55%. ¹H NMR (400 MHz, DMSO) δ 3.79 (s, 3H), 6.97 (d, *J* = 16.4 Hz, 1H), 7.06 (d, *J* = 6.1 Hz, 1H), 7.18 (s, 1H), 7.23 (d, *J* = 7.5 Hz, 1H), 7.40 (t, *J* = 7.8 Hz, 1H), 7.97 (d, *J=* 16.4 Hz, 1H), 12.9 (brs, 1H), 13.05 (s, 1H); ¹³C NMR (100 MHz, DMSO) δ 55.6, 89.1, 113.3, 114.8, 116.6, 117.3, 121, 130.7, 135.8, 143.6, 156.4, 158.8, 160, 176.3; HPLC: 98.7%.

### Example 28: (E)-2-(2-(2-(5-cyano-6-oxo-2-thioxo-1,2,3,6-tetrahydropyrimidin-4-yl)vinyl)-6-ethoxyphenoxy)acetic acid (Compound I-11)

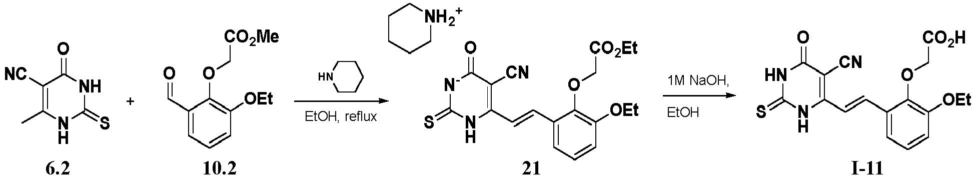

### Step 1. piperidin-1-ium (E)-5-cyano-4-(3-ethoxy-2-(2-ethoxy-2-oxoethoxy)styryl)-6-oxo-2-thioxo-3,6-dihydro-2H-pyrimidin-1-ide (21)

To a stirred solution of intermediate **6.2** (250 mg, 1.49 mmol) and intermediate **10.2** (415 mg, 1.64 mmol) in ethanol (15 mL) was added piperidine (0.22 mL, 2.23 mmol) and molecular sieves. Stirring was continued at reflux overnight. Upon cooling the solid was collected, solubilized with a mixture of DCM/MeOH and filtered again to remove molecular sieves. Then the crude was washed with Et₂O and dried *in vacuo.* The piperidine salt **21** (580 mg, 1.44 mmol) was obtained as a yellowish solid. Yield 96%. ¹H NMR (400 MHz, DMSO) δ 1.18 (t, *J* = 7.1 Hz, 3H), 1.33 (t, *J* = 6.9 Hz, 3H), 1.54 (m, 2H), 1.62 (m, 4H), 3.01 (m, 4H), 4.06 (q, *J=* 6.9 Hz, 3H), 4.17 (q, *J=* 7.1 Hz, 3H), 4.76 (s, 2H), 7.02 (d, *J=* 15.6 Hz, 1H), 7.07 (s. 1H), 7.08 (d, *J* = 3.4 Hz, 1H), 7.23 (dd, *J_{d}* = 6.4 Hz, *J_{d}* = 2.9 Hz, 1H), 8.15 (d, *J* = 15.6 Hz, 1H), 11.4 (brs, 1H).

### Step 2. (E)-2-(2-(2-(5-cyano-6-oxo-2-thioxo-1,2,3,6-tetrahydropyrimidin-4-yl)vinyl)-6-ethoxyphenoxy)acetic acid (I-11)

To a solution of compound **21** (500 mg, 1.24 mmol) in EtOH (30 mL) was added 1M NaOH (4.3 mL). Stirring was continued at r.t. for 16 h. The solvent was removed *in vacuo.* The crude was taken up with water, washed twice with EtOAc (2 × 20 mL). The pH was adjusted to 3 by the addition of 1N HCl resulting in the formation of an orange powder. The solid was collected, washed with Et₂O, and dried *in vacuo* affording the title compound **I-11** (360 mg, 0.96 mmol), as orange solid. Yield 77%. ¹H NMR (400 MHz, DMSO) δ 1.34 (t, *J* = 6.8 Hz, 3H), 4.07 (q, *J* = 6.9, 2H), 4.64 (s, 2H), 6.98 (d, *J=* 16.5 Hz, 1H), 7.16 (m, 2H), 7.26 (d, *J=* 6.8 Hz, 1H), 8.27 (d, *J=* 16.5 Hz, 1H), 13 (s, 2H); ¹³C NMR (100 MHz, DMSO) δ 14.7, 64.4, 69.4, 88.7, 114.8, 116.6, 117.2, 119.1, 124.7, 127.9,. 139.1, 146.5, 151.3, 156.8, 158.8, 170.4, 176.3; HPLC: 94.2%

### Example 29: ethyl (E)-2-(2-(2-(5-cyano-6-oxo-2-thioxo-1,2,3,6-tetrahydropyrimidin-4-yl)vinyl)-6-methoxyphenoxy)acetate (Compound I-12)

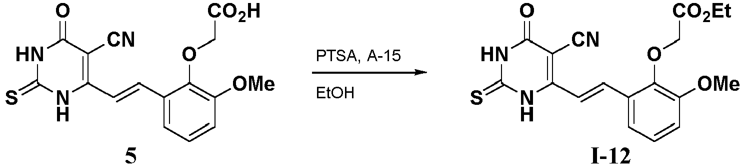

To a stirred suspension of compound **5** (560 mg, 1.49 mmol) in ethanol (50 mL) was added Amberlyst^{®} 15 (100 mg) and molecular sieves. Stirring was continued at reflux overnight. Upon cooling the solvent was removed *in vacuo.* The crude ester was solubilized with a mixture of DCM/MeOH and filtered again to remove molecular sieves. Then the mixture was purified by flash chromatography (eluent DCM/MeOH) affording the title compound **1-12** (440mg, 0.9 mmol) as yellow solid. Yield 61%. ¹H NMR (400 MHz, DMSO) δ 1.19 (t*, J* = 7.1 Hz, 3H), 3.82 (s, 3H), 4.15 (q, *J* = 7.1 Hz, 2H), 4.70 (s, 2H), 7 (d*, J* = 16.5 Hz, 1H) 7.19 (m, 2H), 7.28 (m, 1H), 8.27 (d, *J=* 16.57 Hz, 1H), 13.04 (brs, 2H); ¹³C NMR (100 MHz, DMSO) δ 14.2, 56.3, 60.8, 69.6, 88.7, 114.8, 115.8, 117.4, 119.1, 124.9, 127.9, 138.9, 146.2, 152.1, 156.8, 158.8, 168.9, 176.3; HPLC: 94.5%.

### Example 30: (E)-6-(2,3-dihydroxystyryl)-4-oxo-2-thioxo-1,2,3,4-tetrahydropyrimidine-5-carbonitrile (Compound 1-13) (compound not in accordance with the invention)

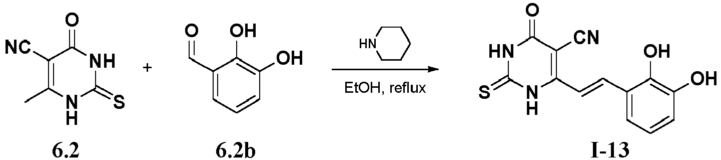

To a stirred solution of intermediate **6.2** (300 mg, 1.79 mmol) and 2,3-dihydroxy benzaldehyde **(6.2b,** 273 mg, 1.97 mmol) in ethanol (15 mL) was added piperidine (0.23 mL, 2.33 mmol) and molecular sieves. Stirring was continued at reflux overnight. The solvent was removed *in vacuo.* The crude was solubilized with a mixture of DCM/MeOH and filtered again to remove molecular sieves. The solid was taken up with water and the pH was adjusted to 3 by the addition of 3N HCl followed by extraction with EtOAc (3 × 20 mL). The organic phase were washed with brine, dried over Na₂SO₄ and concentrated under vacuum. Flash chromatography purification of the crude (eluent DCM/MeOH) afforded the title compound **1-13** (250 mg, 0.49 mmol) as red solid. Yield 48%. ¹H NMR (400 MHz, DMSO) δ 6.72 (t*, J* = 7.8 Hz, 1H), 6.87 (d, *J=* 7.7 Hz, 1H), 6.96 (d, *J=* 7.9 Hz, 1H), 7.07 (d, *J=* 16.3 Hz, 1H), 8.22 (d, *J=* 16.3 Hz, 1H), 9.36 (s, 1H), 9.76 (s, 1H), 12.88 (brs, 2H); ¹³C NMR (100 MHz, DMSO) δ 87.9, 115.2, 115.8, 117.7. 119.6, 119.6, 121.8, 140.6, 146, 157.3, 159.1, 176.5; HPLC: 94.5%.

### Example 31: (E)-2-(2-(2-(5-cyano-6-oxo-2-thioxo-1,2,3,6-tetrahydropyrimidin-4-yl)vinyl)-4-methoxyphenoxy)acetic acid (Compound 1-14)

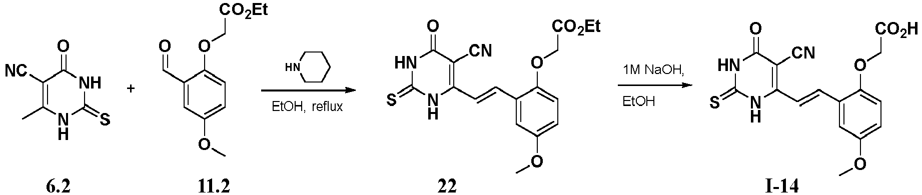

### Step 1. ethyl (E)-2-(2-(2-(5-cyano-6-oxo-2-thioxo-1,2,3,6-tetrahydropyrimidin-4-yl) vinyl)-4-methoxyphenoxy)acetate (Compound 1-22)

To a stirred solution of intermediate **6.2** (300 mg, 1.8 mmol) and intermediate **11.2** (476 mg, 1.1 mmol) in ethanol (15 mL) was added piperidine (0.23 mL, 2.34 mmol) and molecular sieves. Stirring was continued at reflux overnight. Upon cooling the solid was collected, solubilized with a mixture of DCM/MeOH and filtered again to remove molecular sieves. The solvent was removed and the solid was taken up with ice cold water. The pH was adjusted to 3, the resulting yellow precipitate was collected and dried *in vacuo.* Intermediate **22** (500 mg, 1.29 mmol) was obtained as a yellowish solid. Yield 71%. ¹H NMR (200 MHz, DMSO) δ 1.20 (t, *J=* 6.9 Hz, 3H), 2.76 (s, 3H), 4.16 (q, *J=* 7 Hz, 2H), 4.86 (s, 2H), 7.01-7.17 (m, 4H), 8.14 (d, *J=* 13.3 Hz, 1H), 13.05 (brs, 2H).

### Step 2. (E)-2-(2-(2-(5-cyano-6-oxo-2-thioxo-1,2,3,6-tetrahydropyrimidin-4-yl)vinyl)-4-methoxyphenoxy)acetic acid (1-14)

To a solution of compound **22** (480 mg, 1.24 mmol) in EtOH (20 mL) was added 1M NaOH (5 mL). Stirring was continued at r.t. for 16 h. The solvent was removed *in vacuo.* The crude product was taken up with water and washed twice with EtOAc (2 × 20 mL). The pH of the solution was adjusted to 3 by the addition of IN HCl resulting in the formation of an orange powder. The solid was collected, washed with Et₂O and dried *in vacuo* affording the title compound **1-14** (330 mg, 0.91 mmol), as orange solid. Yield 74%. ¹H NMR (400 MHz, DMSO) δ 3.76 (s, 3H), 4.76 (s, 2H), 6.99 (m, 2H), 7.14 (m, 2H), 8.14 (d, *J=* 16.4 Hz, 1H), 13 (brs, 2H); ¹³C NMR (100 MHz, DMSO) δ 55.8, 65.8, 88.6, 113.6, 114.6, 114.8, 117.8, 118.3, 124, 139.2, 151.5, 153.8, 156.8, 158.8, 170.2, 176.3; HPLC: 95.06%

### Example 32: (E)-2-(2-(2-(5-cyano-2-(methylthio)-6-oxo-3,6-dihydropyrimidin-4-yl) vinyl)-6-methoxyphenoxy)acetic acid (Compound I-15)

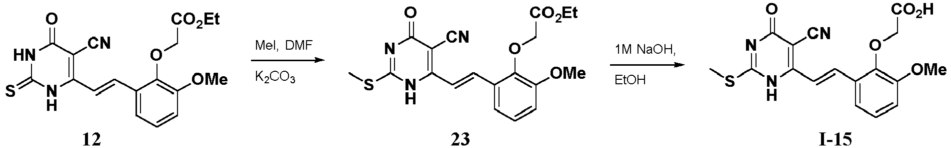

### Step 1. ethyl (E)-2-(2-(2-(5-cyano-2-(methylthio)-6-oxo-3,6-dihydropyrimidin-4-yl)vinyl)-6-methoxyphenoxy)acetate (23)

To a solution of compound **12** (100 mg, 0.26 mmol) in DMSO (3mL) was added DIPEA (0.02 mL, 0.13 mmol) and Mel (0.01 mL, 0.13 mmol). Stirring was continued at r.t. overnight. The resulting mixture was poured into water. The solid that precipitated from solution was collected, dried under reduced pressure, and purified by flash chromatography (eluent DCM/MeOH) affording the title compound 23 (100 mg, 0.25 mmol). Yield 96%. *Step 2.* **(E)-2-(2-(2-(5-cyano-2-(methylthio)-6-oxo-3,6-dihydropyrimidin-4-yl) vinyl)-6-methoxyphenoxy)acetic acid (1-15)**

To a suspension of compound **23** (170 mg, 0.42 mmol) in EtOH (20 mL) was added 1M NaOH (1.5 mL). Stirring was continued at r.t. for 16 h. The solvent was removed *in vacuo.* The crude was taken up with water and washed twice with EtOAc (2 × 20 mL). The pH of the solution was adjusted to 3 by the addition of IN HCl resulting in the formation of a yellow precipitate. The resulting solid was collected, washed with DCM, MeOH and acetone and dried *in vacuo* affording the title compound **1-15** (20 mg, 0.05 mmol), as yellowish solid. Yield 12%. ¹H NMR (400 MHz, DMSO) δ 2.65 (s, 3H), 3.84 (s, 3H), 4.64 (s, 2H), 7.15 (m, 3H), 7.38 (m, 1H), 8.63 (d, *J=* 15.5 Hz, 1H), 12.90 (brs, 1H), 13.45 (brs, 1H); ¹³C NMR (100 MHz, DMSO) δ 13.6, 56.3, 69.6, 93.4, 115.4, 115.5, 118.9, 122.6, 124.9, 128.5, 137.8, 146.5, 152.5, 160.7, 163.9, 165.9, 170.6; HPLC: 92.7%

### Example 33: 3'-(5-cyano-6-oxo-2-thioxo-1,2,3,6-tetrahydropyrimidin-4-yl)-[1,1'-biphenyl]-3-carboxylic acid (Compound I-16) (compound not in accordance with the invention)

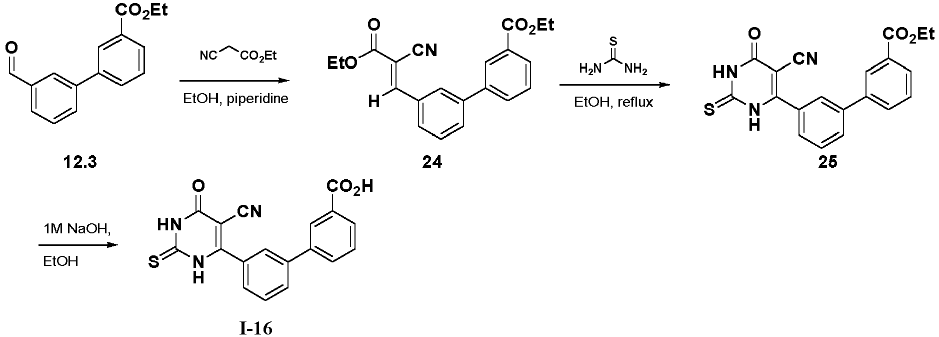

### Step 1. ethyl (E)-3'-(2-cyano-3-ethoxy-3-oxoprop-1-en-1-yl)-[1,1'-biphenyl]-3-carboxylate (24)

To a solution of intermediate **12.3** (254 mg, 1.01 mmol) and ethyl cyanoacetate (0.11 mL, 1.01 mmol) in ethanol (9 mL) was added piperidine (3 drops). Stirring was continued at r.t. overnight. The solvent was removed *in vacuo.* The crude was taken up with water, acidified with IN HCl, and extracted with EtOAc (3 × 20 mL). The combined organic phases were washed with brine, dried over Na₂SO₄, filtered, and concentrated under vacuum affording the title compound **24** (350 mg, 1 mmol), as colorless oil. Yield 98%. ¹H NMR (400 MHz, DMSO) δ 1.44 (t, *J* = 6.1 Hz, 3), 4.43 (q, *J* = 5.7 Hz, 2H), 7.56 (d, *J* = 7.7 Hz, 1H), 7.64 (d, *J=* 7.7 Hz, 1H), 7.83 (m, 2H), 8.05-8.11 (m, 2H), 8.19 (m, 1H), 8.31 (m, 1H), 8.35 (s, 1H).

### Step 2. ethyl 3'-(5-cyano-6-oxo-2-thioxo-1,2,3,6-tetrahydropyrimidin-4-yl)-[1,1'-biphenyl]-3-carboxylate (25)

To a solution of intermediate **24** (350 mg, 1 mmol) in ethanol (15 mL) was added K₂CO₃ (165.6 mg, 1.2 mmol) and thiourea (91.3 mg, 1.2 mmol). Stirring was continued at reflux for 4 h. The solvent was removed under reduced pressure. The crude was taken up in water and the resulting solution was acidified with IN HCl and extracted with EtOAc (3 × 20 mL). The combined organic phases were washed with brine and dried over Na₂SO₄. The crude was purified by flash chromatography, eluting with DCM/MeOH (2% for product) affording pure intermediate **25** (100 mg, 0.29 mmol) as a yellow powder. Yield 28%. ¹H NMR (400 MHz, DMSO) δ 1.35 (t, *J=* 7.1 Hz, 3H), 4.37 (q, *J* = 7.1 Hz, 2H), 7.67 (d, *J=* 2.6 Hz, 1H), 7.69-7.75 (m, 2H), 7.97-8.04 (m, 4H), 8.32 (s, 1H), 13.22 (s, 1H), 1343 (brs, 1H).

### Step 3. 3'-(5-cyano-6-oxo-2-thioxo-1,2,3,6-tetrahydropyrimidin-4-yl)-[1,1'-biphenyl]-3-carboxylic acid (1-16)

To a solution of compound **25** (80 mg, 0.21 mmol) in EtOH (10 mL) was added 1M NaOH (0.84 mL). Stirring was continued at 80°C for 6 h. The solvent was removed *in vacuo.* The crude was taken up in water and washed twice with EtOAc (2 × 20 mL). The pH of the solution was adjusted to 3 by the addition of IN HCl and then extracted with EtOAc (3 × 20 mL). The combined organic phases were washed with brine, dried over Na₂SO₄, filtered, and concentrated *in vacuo.* Shredding with Et₂O afforded the title compound 1-16 (45 mg, 0.13 mmol) as yellowish solid. Yield 61%. ¹H NMR (400 MHz, DMSO) δ 7.65 (m, 1H), 7.68 (m, 1H), 7.74 (d, *J=* 7.7 Hz, 1H), 7.99 (m, 4H), 8.35 (s, 1H), 13.2 (s, 1H), 13.3 (brs, 1H); ¹³C NMR (100 MHz, DMSO) δ 91.1,127.9, 127.9, 128.4, 129.1, 129.5, 129.7, 130.1, 130.7, 131.5, 131.9, 139.5, 139.7, 158.7, 160.7, 167.4, 172.2. 176.5;HPLC: 94.12%

### Example 34: (E)-2-(2-ethoxy-4-(2-(5-nitro-2,6-dioxo-1,2,3,6-tetrahydropyrimidin-4-yl)vinyl)phenoxy)acetic acid (Compound 1-17)

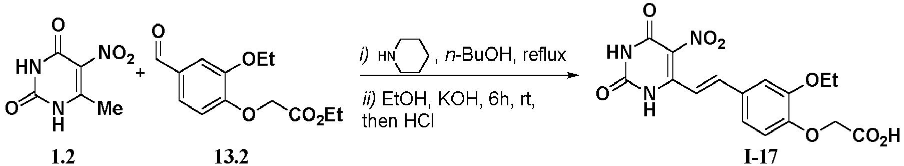

To a stirred suspension of intermediate **1.2** (305 mg, 1.78 mmol) and intermediate **13.2** (500 mg, 3.56 mmol) in *n*-butanol (6 mL) was added piperidine (0.19 mL, 1.95 mmol). Stirring was continued overnight at reflux. Upon cooling, the solid was collected, washed with Et₂O and dried *in vacuo.* The solid was taken up with EtOH and the resulting solution was basified with a 2 molar solution of KOH and then stirred for an additional 6 h. The volatiles were removed *in vacuo.* The crude product was taken up with water and the resulting solution was acidified to pH 3 with 3N HCl solution. Compound **1-17** (300 mg, 0.79 mmol) was obtained as yellowish solid. Yield 45%. ¹H NMR (400 MHz, DMSO) δ 1.34 (t, *J* = 6.8 Hz, 3H), 4.08 (q, *J* = 6.8 Hz, 2H) 4.75 (s, 2H), 6.88 (m, 2H), 7.12 (d, *J* = 8.1 Hz, 1H), 7.25 (s, 1H), 7.67 (d*, J* = 16.1 Hz, 1H), 11.5 (brs, 1H), 11.78 (s, 1H), 12.98 (brs, 1H). ¹³C NMR (100 MHz, DMSO) δ 151, 64.5, 65.2, 112.0, 112.3, 113.5, 123.4, 126.4, 128.1, 142.5, 148.2, 148.7, 149.7, 150.4, 157.1, 170.3; HPLC: 99.6%.

The following compounds can be synthesized according the procedures used herein above for Compound 1-17.

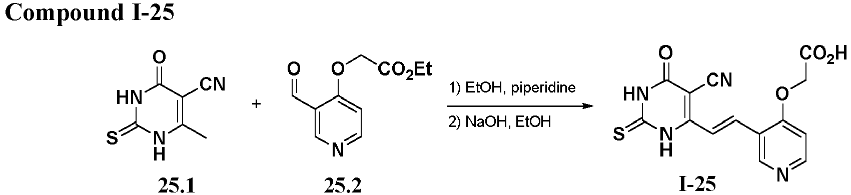

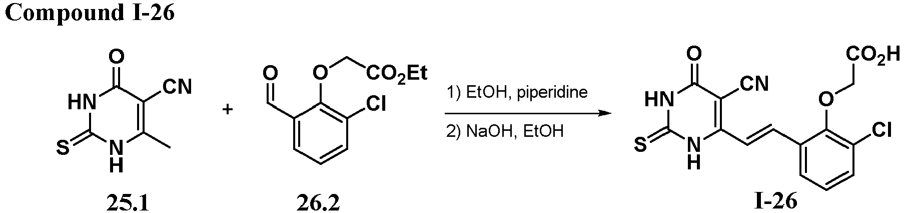

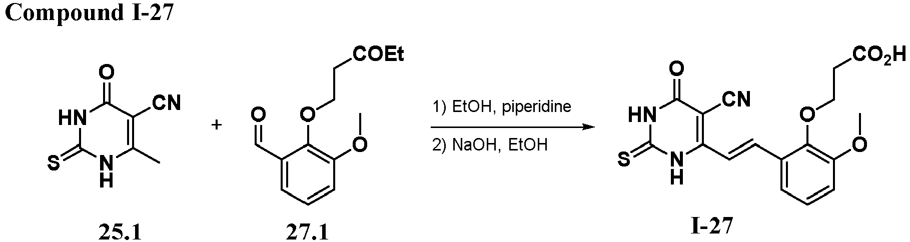

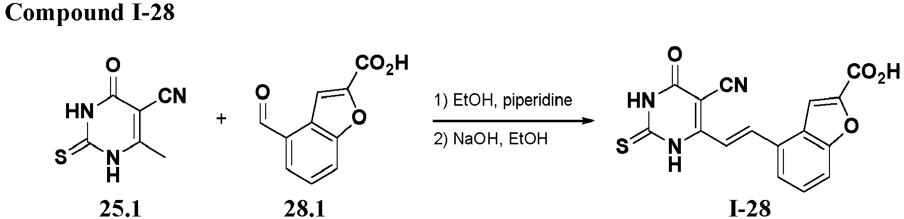

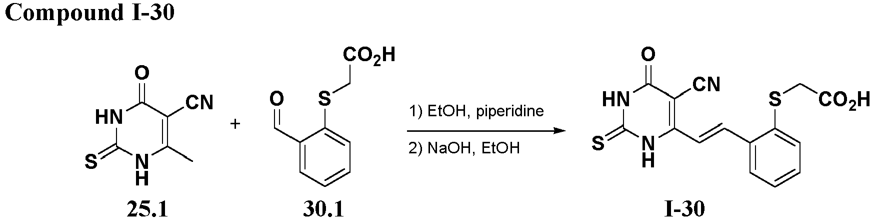

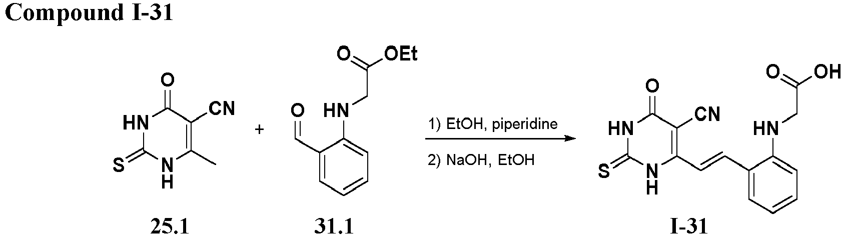

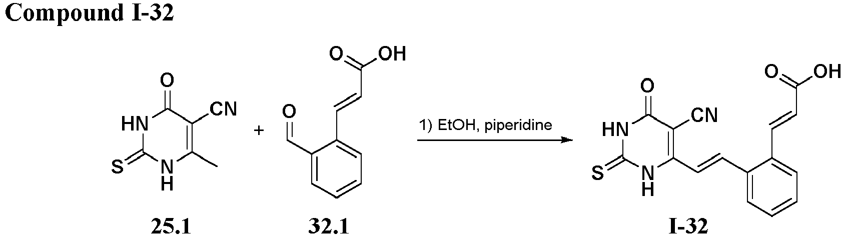

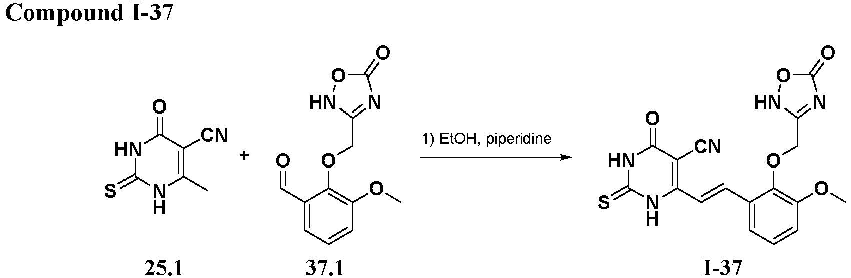

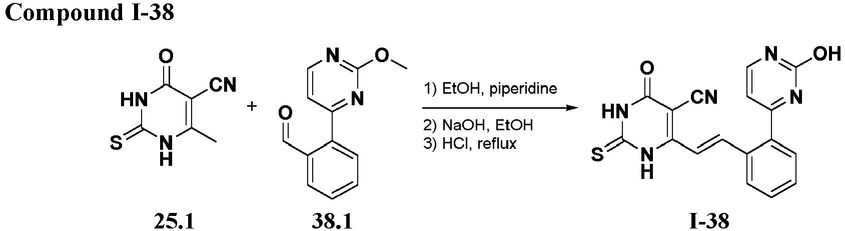

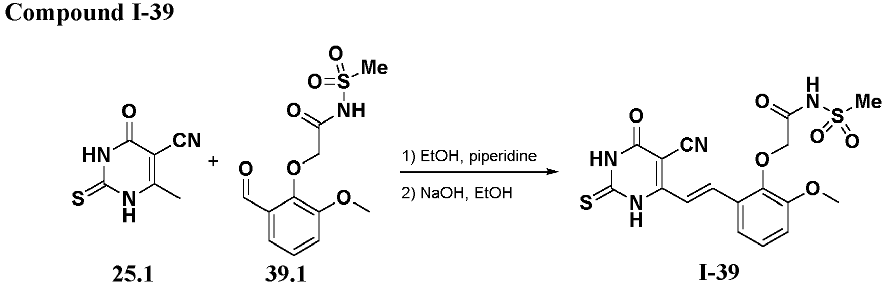

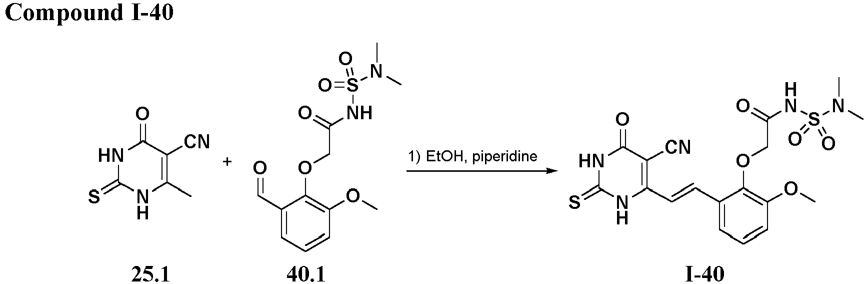

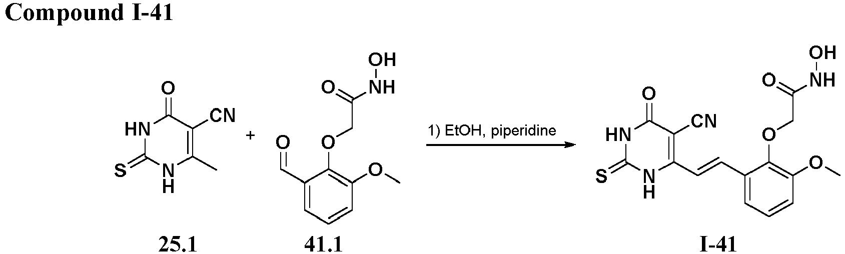

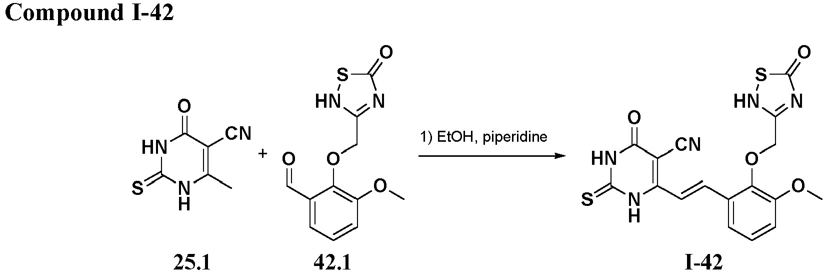

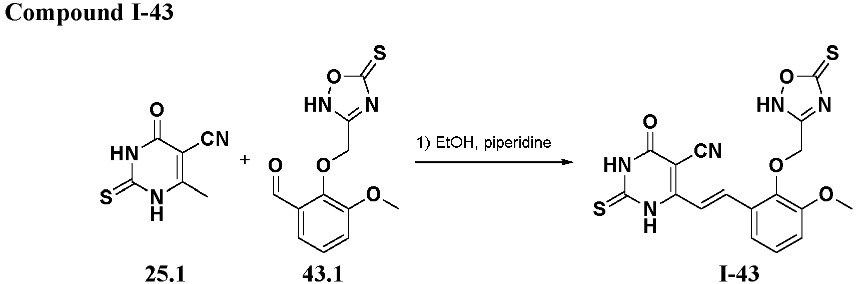

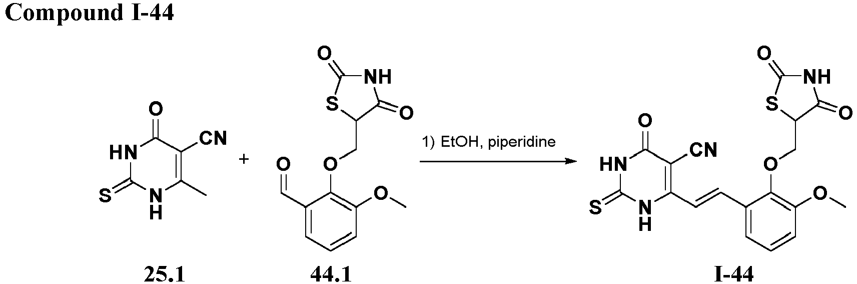

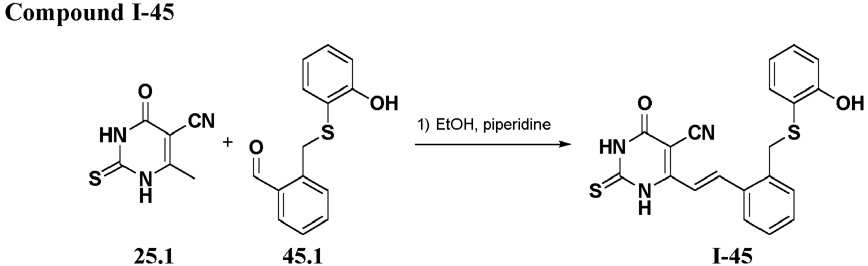

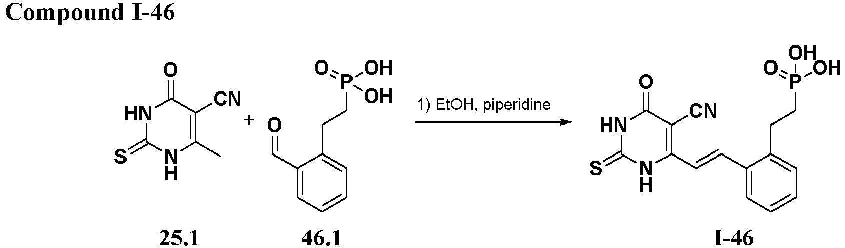

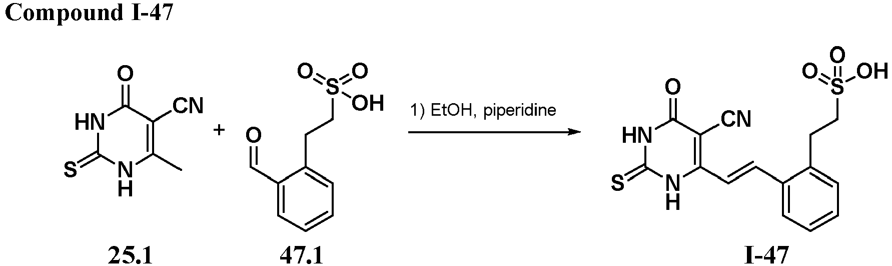

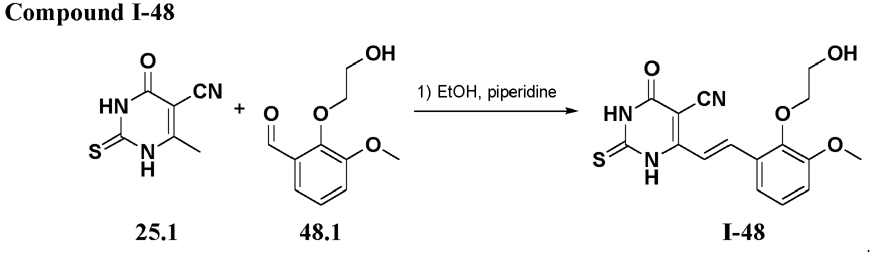

### Example 35: (E)-6-(2-((1H-tetrazol-5-yl)methoxy)-3-methoxystyryl)-4-oxo-2-thioxo-1,2,3,4-tetrahydropyrimidine-5-carbonitrile (Compound I-18)

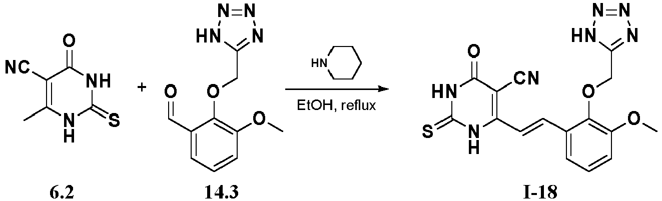

To a stirred solution of intermediate 6.2 (200 mg, 1.2 mmol) and intermediate 14.3 (309 mg, 3.56 mmol) in ethanol (15 mL) was added piperidine (0.26 mL, 2.6 mmol). Stirring was continued overnight at reflux. The solvent was removed *in vacuo.* The crude was taken up in H₂O and the resulting solution was basified with NaOH pellets and washed twice with EtOAc (20 mL). The pH of the solution was adjusted to 7 by the addition of 3N HCl and the solution was washed again with EtOAc. The aqueous phase was then acidified to pH 4 and the resulting solid was collected. The title compound **1-18** (200 mg, 0.52 mmol) was obtained as pure yellowish powder after shredding with EtOAc. Yield 43%. ¹H NMR (400 MHz, DMSO) δ 3.79 (s, 3H), 5.48 (s, 2H), 6.93 (d, *J=* 16.5 Hz, 1H), 7.21-7.29 (m, 3H), 8.1 (d, *J* = 16.5 Hz, 1H), 13.1 (s, 1H); ¹³C NMR (100 MHz, DMSO) δ 56.2, 64.1, 89.0, 114.8, 115.6, 117.8, 118.8, 125.9, 128.5, 137.9, 145.7, 152.8, 156.5, 158.8, 176.2; HPLC 95.1%.

### Example 36: (E)-2-(3-(2-(5-cyano-6-oxo-2-thioxo-1,2,3,6-tetrahydropyrimidin-4-yl) vinyl)phenyl)acetic acid (Compound I-19)

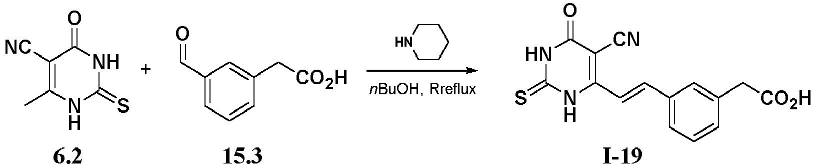

To a stirred solution of intermediate 6.2 (250 mg, 1.15 mmol) and intermediate **15.3** (189 mg, 1.15 mmol) in n-butanol (10 mL) was added piperidine (0.22 mL, 2.3 mmol). Stirring was continued overnight at reflux. The solvent was removed *in vacuo.* The crude was taken up with H₂O and the resulting aqueous solution was basified with NaOH pellets and washed twice with EtOAc (20 mL). The pH of the solution was adjusted to 5 by the addition of 3N HCl and the solution was then washed again with EtOAc. The aqueous phase was acidified to pH 4 and the resulting solid was collected. The mixture was purified by flash chromatography eluting with (DCM/MeOH). The title compound **1-19** (60 mg, 0.19 mmol) was obtained as pure yellowish powder after shredding with EtOAc. Yield 17%. ¹H NMR (400 MHz, DMSO) δ 3.66 (s, 2H), 6.95 (d, *J=* 16.3 Hz, 1H), 7.35 (d, *J=* 7.5 Hz, 1H), 7.44 (t, *J* = 7.5 Hz, 1H), 7.5 (d, *J* = 7.5 Hz, 1H), 7.57 (s, 1H), 8.0 (d, *J* = 16 Hz, 1H), 12.4 (s, 1H), 12.99 (brs, 2H); ¹³C NMR (100 MHz, DMSO) δ 39.1, 88.9, 115.0, 116.4, 127.0, 129.4, 129.4, 132.5, 134.3, 136.4, 143.3, 156.8, 159.0, 172.8, 176.6.

### Example 37: 2-(2-(2-(5-cyano-6-oxo-2-thioxo-1,2,3,6-tetrahydropyrimidin-4-yl)ethyl) phenoxy)acetic acid (1-20)

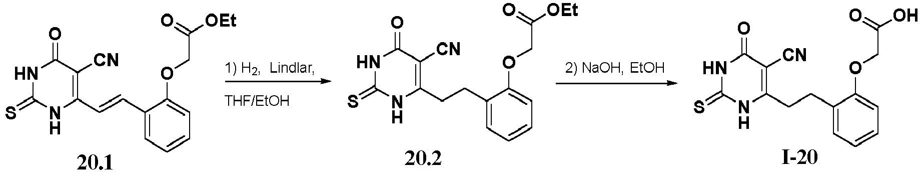

A mixture of intermediate **20.1,** Lindlar's catalyst (palladium on calcium carbonate poisoned with lead), THF and EtOH was stirred under an atmosphere of hydrogen. Workup and purification provides Intermediate **20.2.**

### Example 38: 2-(2-((6-oxo-2-thioxo-1,2,3,6-tetrahydropyrimidin-4-yl)carbamoyl) phenoxy)acetic acid (compound not in accordance with the invention)

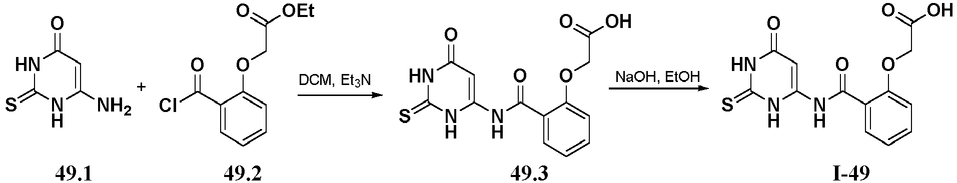

Acylation of intermediate **49.1** with chloride **49.2** using triethylamine in DCM provides intermediate **49.3.** Hydrolysis of ester **49.3** using sodium hydroxide in ethanol provide Compound **1-49.**

### Example 39: 2-(2-(2,6-dioxo-1,2,3,6-tetrahydropyrimidine-4-carboxamido)phenoxy) acetic acid (compound not in accordance with the invention)

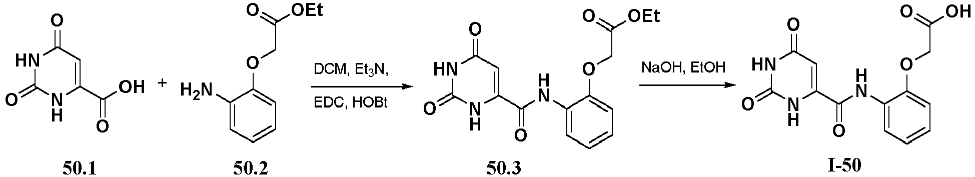

Coupling of **50.1** and **50.1** using a *N*-ethyl-*N'*-(3-dimethylaminopropyl)carbodiimide (EDC), Hydroxybenzotriazole (HOBt), and triethylamine (Et₃N) in a solvent (*e*.*g*., dichloromethane (DCM) provides intermediate **50.3.** Hydrolysis of ester **50.3** using sodium hydroxide in ethanol provides Compound **1-50.**

### Example 40: 2-(2-(2-(5-cyano-6-oxo-2-thioxo-1,2,3,6-tetrahydropyrimidin-4-yl) cyclopropyl)phenoxy)acetic acid (I-23)

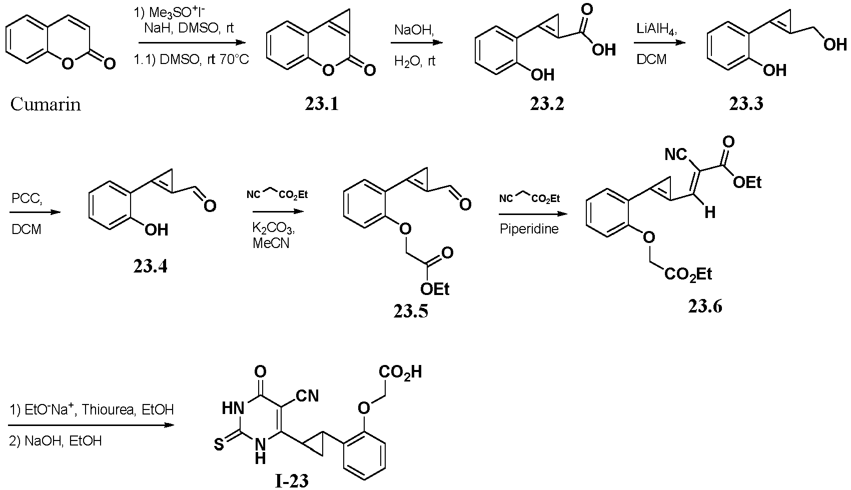

Compound **I-23** is synthesized as depicted herein above starting from Cumarin.

### Example 41: 2-((3'-(5-cyano-6-oxo-2-thioxo-1,2,3,6-tetrahydropyrimidin-4-yl)-[1,1'-biphenyl]-3-yl)oxy)acetic acid (1-24) (compound not in accordance with the invention)

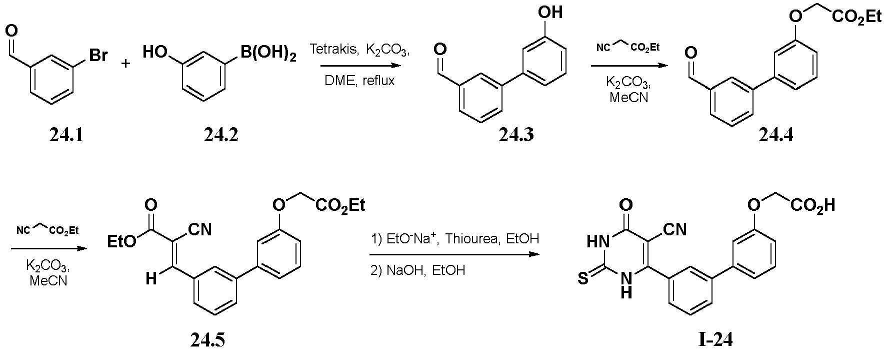

Compound 1-24 is synthesized as depicted herein above starting from 24.1 and 24.2.

### Example 42: (E)-2-(2-(2-(2,6-dimethoxypyrimidin-4-yl)vinyl)-6-methoxyphenoxy)acetic acid (1-33) (compound not in accordance with the invention)

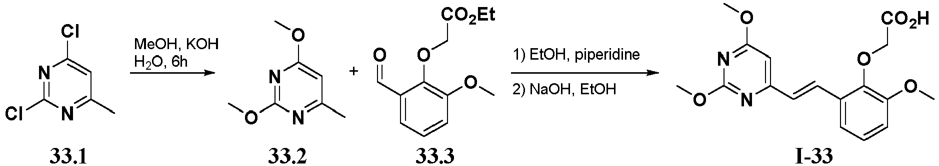

### Example 43: (E)-2-(2-(2-(6-chloro-5-cyano-2-thioxo-2,3-dihydropyrimidin-4-yl)vinyl)-6-methoxyphenoxy)acetic acid (1-34) (compound not in accordance with the invention)

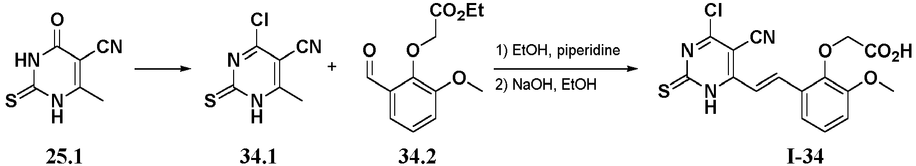

Chlorination of intermediate **25.1** followed by Knoevenagel type reaction between intermediate **34.1** and aldehyde **34.2** using piperidine in *n*-BuOH provides the Compound **I-34**.

### Example 44: 2-{2-[2-(5-Cyano-6-oxo-2-thioxo-1,2,3,6-tetrahydro-pyrimidin-4-yl)-vinyl]-6-ethoxy-phenoxy}-acetamide (Compound I-35)

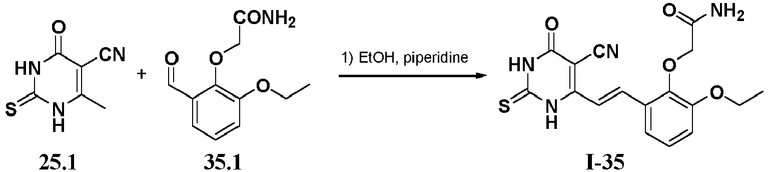

To a stirred solution of intermediate **25.1** (200 mg, 1.2 mmol) and intermediate **35.1** (295 mg, 1.32 mmol) in ethanol (15 mL) was added piperidine (0.2 mL, 1.80 mmol) and molecular sieves. Stirring was continued at reflux overnight. Upon cooling, the solid was collected washed with Et₂O and dried *in vacuo.* The piperidine salt was dissolved in 10 % aqueous KOH solution and stirred an additional 2 h. Then the pH was adjusted to 4 by the addition of 3N HCl. The resulting solid was collected and washed with cold water and acetone to afford the title compound **1-35** (303 mg, 0.84 mmol) as a yellowish solid. Yield 70 %. ¹H NMR (400 MHz, DMSO) δ 1.34 (t, *J* = 6.9 Hz, 3H), 4.07 (d, *J* = 6.8 Hz, 2H), 4.64 (s, 2H), 7 (d, *J=* 16.5 Hz, 1H), 7.12-7.17 (m, 2H), 7.26 (d, *J=* 6.8 Hz, 1H), 8.27 (d, *J=* 16.5 Hz, 1H), 12.95 (brs, 1H), 13.04 (s, 1H). ¹³C NMR (100 MHz, DMSO) δ 14.7, 40.1, 64.5, 69.4, 88.7, 114.8, 116.6, 117.3, 119.1, 128, 139.1, 146.5, 151.3, 156.9, 158.8, 170.4, 176.3; HPLC: 95.2%.

### Example 45: 4-Oxo-6-styryl-2-thioxo-1,2,3,4-tetrahydro-pyrimidine-5-carbonitrile (Compound I-54) (compound not in accordance with the invention)

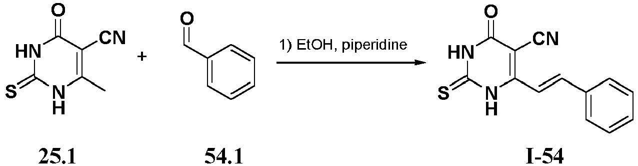

To a stirred solution of intermediate **25.1** (250 mg, 1.15 mmol) and intermediate **54.1** (0.13 mL, 1.27 mmol) in ethanol (15 mL) was added piperidine (0.23 mL, 2.3 mmol) and molecular sieves. Stirring was continued at reflux overnight. Upon cooling the solid was collected, solubilized with a mixture of DCM/MeOH, and filtered again to remove molecular sieves. The crude product was washed with Et₂O and dried *in vacuo* affording the title compound **1-54** (250 mg, 0.97 mmol) as yellowish solid. Yield 85%. ¹H NMR (400 MHz, DMSO) δ 6.95 (d, *J=* 16.4 Hz, 1H), 7.49 (m. 3H), 7.66 (m, 2H), 8.02 (d, *J=* 16.4 Hz, 1H), 12.9 (brs, 1H), 13.10 (s, 1H). ¹³C NMR (100 MHz, DMSO) δ 89.1, 114.8, 116.1, 128.5, 128.5, 129.1, 129.5, 129.5, 134.3, 143.7, 156.4, 158.8, 176.3; HPLC: 96.4%.

### Example 46: 6-[2-(3-Ethoxy-2-hydroxy-phenyl)-vinyl]-4-oxo-2-thioxo-1,2,3,4-tetrahydro-pyrimidine-5-carbonitrile (Compound 1-56) (compound not in accordance with the invention)

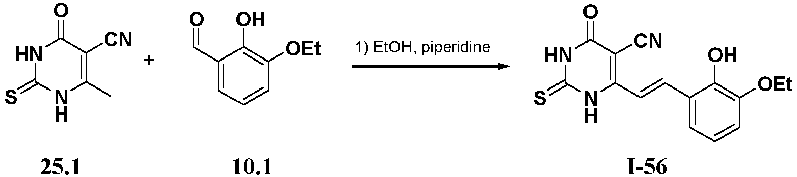

To a stirred solution of intermediate **25.1** (250 mg, 1.15 mmol) and intermediate **10.1** (211 mg, 1.27 mmol) in ethanol (15 mL) was added piperidine (0.23 mL, 2.3 mmol) and molecular sieves. Stirring was continued at reflux overnight. Upon cooling the solid was collected, solubilized with a mixture of DCM/MeOH, and filtered again to remove molecular sieves. The crude product was washed with Et₂O and dried *in vacuo.* The isolated piperidine salt product was dissolved in 10 % KOH and stirred an additional 2 h. The pH was adjusted to 4 by the addition of 3N HCl and the resulting solid was collected and dried *in vacuo.* The title compound **1-54** (250 mg, 0.97 mmol) was obtained as yellowish solid after shredding with acetone. Yield 85%. ¹H NMR (400 MHz, DMSO) δ 1.36 (t, *J=* 6.9 Hz, 3H), 4.08 (q, *J* = 6.8 Hz, 3H), 6.85 (t, *J=* 7.9 Hz, 1H), 7.08 (d, *J=* 7.9 Hz, 1H), 7.1 (d, *J=* 7.9 Hz, 1H), 7.14 (d, *J=* 16.4 Hz, 1H), 8.24 (d, *J=* 16.4 Hz, 1H), 9.56 (s, 1H), 12.97 (brs, 1H), 13.01 (s, 1H). ¹³C NMR (100 MHz, DMSO) δ 14.8, 64.6, 88.3, 115.1, 115.4, 116, 119.7, 121, 121.4, 140.4, 147.3, 157, 158.9, 176.3; HPLC: 94.4%.

### Example 47: 6-[2-(2,3-Dimethyl-phenyl)-vinyl]-4-oxo-2-thioxo-1,2,3,4-tetrahydropyrimidine-5-carbonitrile (Compound I-59)

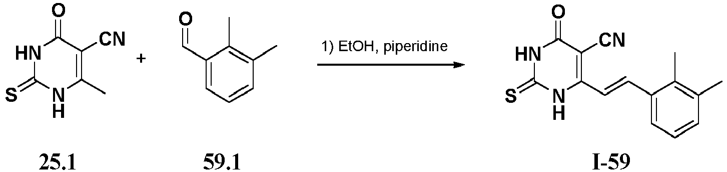

To a stirred suspension of intermediate **25.1** (200 mg, 1.2 mmol) and 2,3-dimethyl benzaldehyde **(59.1,** 0.2 mL, 1.32 mmol) in *n*-butanol (10 mL) was added piperidine (0.12 mL, 1.31 mmol) and stirring was continued overnight at reflux. The resulting solid was collected, washed with cold Et₂O, and dried *in vacuo.* The solid was taken up with a 1M solution of KOH (20 mL) and stirred for an additional 6 h. The crude mixture was diluted with cold water, acidified to pH 3 with 3N HCl, and the resulting solid was collected. The title compound **1-59** (68 mg, 0.24 mmol) was obtained as yellowish solid after flash chromatography purification of the crude (eluent DCM/MeOH). Yield 20%. ¹H NMR (400 MHz, DMSO) δ 2.28 (s, 3H), 2.34 (s, 3H), 6.80 (d, *J=* 16.2 Hz, 1H), 7.2 (t, *J=* 7.6 Hz, 1H), 7.27 (d, *J=* 7.2 Hz, 1H), 7.48 (d, *J=* 7.6 Hz, 1H), 8.3 (d, *J=* 16.2 Hz, 1H), 13.1 (s, 2H); ¹³C NMR (100 MHz, DMSO) δ 15.5, 20.4, 89, 115.1, 117.4, 124.2, 126.4, 132.4, 133.5, 137.2, 137.8, 142.1, 156.4, 158.8, 176.3. HPLC: 98.6%.

### Example 48: 3'-(5-Cyano-6-oxo-2-thioxo-1,2,3,6-tetrahydro-pyrimidin-4-yl)-biphenyl-2-carboxylic acid (Compound 1-66) (compound not in accordance with the invention)

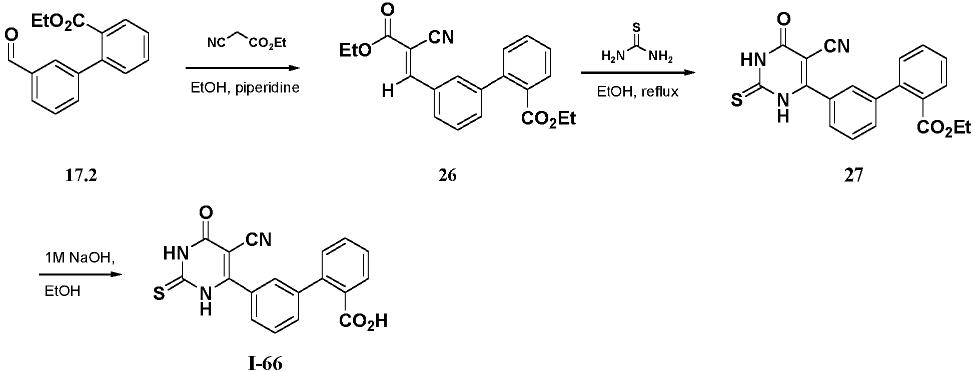

### Step 1. 3'-(2-Cyano-2-ethoxycarbonyl-vinyl)-biphenyl-2-carboxylic acid ethyl ester (26)

To a solution of intermediate **17.2** (350 mg, 1.46 mmol) and ethyl cyanoacetate (0.15 mL, 1.46 mmol) in ethanol (15 mL) was added piperidine (3 drops) and stirring was continued at r.t. overnight. The solvent was removed *in vacuo.* The crude residue was taken up with water, acidified with IN HCl, and extracted with EtOAc (3 × 20 mL). The combined organic phases were washed with brine, dried over Na₂SO₄, filtered, and concentrated *in vacuo* to afford the title compound **26** (430 mg, 1.23 mmol), as yellowish oil. Yield 84%. ¹H NMR (400 MHz, CDCl₃) δ 1.06 (t, *J* = 7.1 Hz, 3H), 1.41 (t, *J* = 7.1 Hz, 3H), 4.13 (q, *J* = 7.1 Hz, 2H), 4.40 (q, *J=* 7.1 Hz, 2H), 7.37 (d, *J=* 7.6 Hz, 1H), 7.46-7.59 (m, 4H), 7.87 (s, 1H), 7.94 (d, *J* = 7.7 Hz, 1H), 8.08 (d, *J* = 7.5 Hz, 1H), 8.3 (s, 1H).

### Step 2. 3'-(5-Cyano-6-oxo-2-thioxo-1,2,3,6-tetrahydro-pyrimidin-4-yl)-biphenyl-2-carboxylic acid ethyl ester (27)

To a stirred solution of intermediate **26** (400 mg, 1.05 mmol) in ethanol (15 mL) was added K₂CO₃ (362 mg, 2.63 mmol) and thiourea (80 mg, 1.05 mmol) and stirring was continued at reflux for 4 h. The solvent was removed *in vacuo.* The crude residue was taken up in water and the resulting solution was acidified with IN HCl and extracted with EtOAc (3 × 20 mL). The combined organic phases were washed with brine, dried over Na₂SO₄, filtered and concentrated *in vacuo.* The crude product was purified by flash chromatography, eluting with DCM/MeOH (2% for product) to afford pure intermediate **27** (165 mg, 0.43 mmol) as a yellow powder. Yield 41%. ¹H NMR (400 MHz, DMSO) δ 0.94 (t, *J* = 7.1 Hz, 3H), 4.04 (q, *J* = 7.07 Hz, 2H), 7.49-7.62 (m, 4H), 7.69 (t, J = 7.66 Hz, 2H), 7.82 (d, J = 7.6 Hz, 1H), 13.2 (s, 1H), 13.39 (brs, 1H).

### Step 3. 3'-(5-Cyano-6-oxo-2-thioxo-1,2,3,6-tetrahydro-pyrimidin-4-yl)-biphenyl-2-carboxylic acid (1-66)

To a stirred solution of compound **27** (150 mg, 0.4 mmol) in EtOH (10 mL) was added 1M NaOH (4 mL) and stirring was continued at 80°C for 6 h. The solvent was removed *in vacuo* and the crude residue was taken up in water and washed twice with EtOAc (2 × 20 mL). The pH of the solution was adjusted to 3 by the addition of IN HCl and then extracted with EtOAc (3 × 20 mL). The combined organic phases were washed with brine, dried over Na₂SO₄, filtered, and concentrated *in vacuo.* The title compound **1-66** (40 mg, 0.11 mmol) was obtained as white solid after flash chromatography purification (eluent DCM/MeOH). Yield 29%. ¹H NMR (400 MHz, DMSO) δ 7.52 (t, *J* = 6.1 Hz, 2H), 7.58 (m, 2H), 7.64 (d, *J* = 7.5 Hz, 1H), 7.70 (m, 2H), 7.82 (d, *J* = 7.3 Hz, 1H), 12.9 (s, 1H), 13.2 (s, 1H), 13.4 (s, 1H); ¹³C NMR (100 MHz, DMSO) δ 90.7, 115.3, 127.9, 128.1, 128.3, 128.7, 129.8, 131.1, 131.5, 132, 132.7, 140.2, 141.1, 158.9, 160.9, 169.4; HPLC: 98.5%.

### I. BIOLOGICAL ACTIVITY

### Example 49: Determination of ACMSD1 inhibition

The inhibition activity of ACMSD1 of compounds disclosed herein was determined by measuring the conversion of 30H-Anthranilic Acid into product *(i.e.,* ACMS) in a spectrophotometrical in vitro assay.

The pre-assay mixture consisting of 3-hydroxyanthranilic acid (3OH-HA), 3-hydroxyanthranilic acid, 3,4-diOxygenase (HAO), and a dialyzed crude extract of *E. coli* BL21 (DE3) cells expressing the recombinant enzyme, was incubated at 25 °C with monitoring of the increase in absorbance at 360 nm due to the formation of ACMS from 3OH-HA. After the reaction was completed within ~ 2 mins, an aliquot of ACMSD1 solution (prepared and purified from *Pichia Pastoris* overexpressing the recombinant enzyme) was added, and the decrease in absorbance at 360 nm was followed at 15 second intervals. The effect of ACMS concentration on the enzyme activity was investigated by varying 3OH-HA concentration from 2 to 20 µM. Kinetic parameters were calculated from the initial velocity data by using the Lineweaver-Burk plot.

The rate of the decrease in absorbance caused by ACMSD1 was calculated by subtracting that of the control reaction mixture without ACMSD from that described above. One unit of ACMSD activity was indicated as the amount of enzyme that converts 1 mmol of ACMS per minute at 25 °C. The absence or a reduction of ACMSD1 activity (e.g., by using ACMSD inhibitors) results in a slow ACMS-spontaneous degradation (i.e., cyclization to form quinolic acid).

The enzymatic activity was determined at a HAA concentration of 10µM in the presence of the compounds in Table 1 below. The compounds were tested at the concentration of about 5 µM and 10µM and the IC₅₀ was calculated for compounds showing inhibitory activity higher than 50%. The results are shown in Table 1.

The ACMSD activity is shown in Table 2. "A" indicates an IC₅₀ of > 0.1 µM, "B" indicates IC₅₀ of between about 0.1 µM to about 1µM, and "C" indicates an IC₅₀ of about 1 µM to about 2 µM.

**TABLE 1:**

| **Compound No.** | **Structure** | **Activity hACMSD IC₅₀** |
|---|---|---|
| **I-1** | 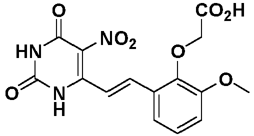 | A |
| **I-2** | 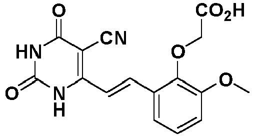 | B |
| **I-3** | 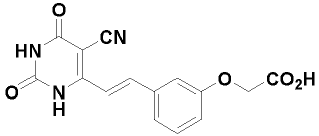 | C |
| **I-4** | 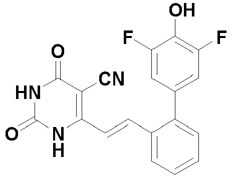 | B |
| **I-5** | 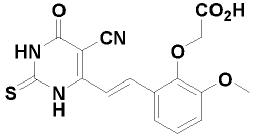 | A |
| **I-6** | 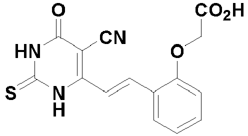 | B |
| **I-7** | 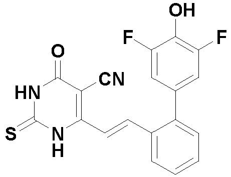 | A |
| **I-8** | 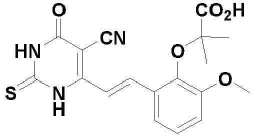 | B |
| **I-9** | 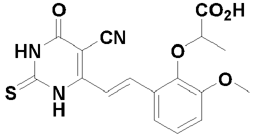 | B |
| **I-10*** | 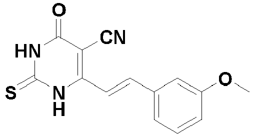 | B |
| **I-11** | 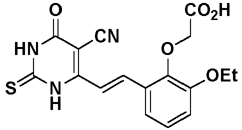 | A |
| **I-13*** | 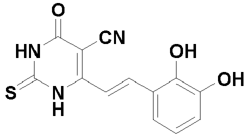 | A |
| **I-15** | 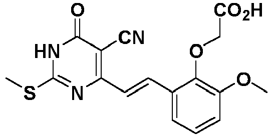 | B |
| **I-16*** | 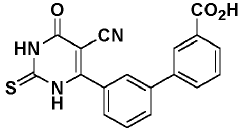 | B |
| **I-17** | 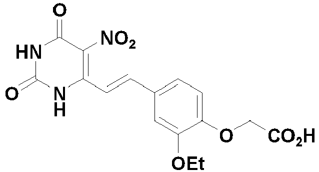 | C |

| | | |
|---|---|---|
| *compound which is not in accordance with the present invention | | |

### Example 50: Determination of ACMSD-1 modulation in HEK293T cells

HEK293T cells (ATCC) are seeded in six-well plates and transfected using Fugene HD to express transiently ACMSD. 24 hrs post transfection, the cells are stimulated for 48 hrs to 72 hrs with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, and then lysed to measure the ACMSD activity, by measuring the conversion of 3OH-Anthranilic Acid into product (*i.e.* α-amino-beta-carboxymuconate-ε-semialdehyde, ACMS) in a spectrophotometrical *in vitro* assay. The amount of the whole protein content in cell lysates is detected by Bradford analysis. This value is used to get the specificity activity of the enzyme normalized in all samples (mU/ml or ΔE/Δt/mg of total protein).

ACMSD-1 enzyme is known to be expressed in liver, kidney and brain; available cell lines for these cell types were therefore tested to determine the expression levels of ACMSD. We determine whether ACMSD-1 is not expressed in transformed cell lines from liver and kidney, such as HepG2, HEK293T, Hep3B etc. Transfection of ACMSD was performed to express the enzyme in different cellular backgrounds such as COS-7, HEK293T, and HepG2. The HEK293T cellular background proved to be the best system, with the highest protein production allowing robust measurement ACMSD1 enzyme activity. This is probably due to the better transfection efficacy observed in HEK293T.

Having determined the optimum stimulation time and transfection protocol cells are stimulated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), (about 50 nM to about 5 uM).

### Example 51: Determination of NAD⁺ content in Human Primary Hepatocytes Treated with a Compound of The Disclosure

The NAD⁺ concentration or content is determined in human primary hepatocytes treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), orFormula (IIi), or a pharmaceutically acceptable salt thereof,. Vehicle (NT) was used as a control.

At least three experiments are run treating primary hepatocytes with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, (0.5 µM and 5 µM) after 48 hrs from seeding. The compounds are replaced every 24 hrs, and then cells are directly harvested and lysed with ACN/H₂O (ratio 5:1). LCMS/MS is used to detect and measure NAD⁺ concentration/content.

### Example 52: Determination of NAD⁺ content in Human Primary Hepatocytes Treated with a Compound of the Disclosure

The NAD⁺ concentration or content is determined in human primary hepatocytes treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, and MEHP, a known ACMSD inhibitor. MEHP is used as a control.

At least three experiments are run treating primary hepatocytes with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, (0.5 µM, 5 µM, and 50 µM) after 48 hrs from seeding. The compounds are replaced every 24 hrs, and then cells are directly harvested and lysed with ACN/H₂O (ratio 5:1). LCMS/MS is used to detect and measure NAD⁺ concentration/content.

### Example 53: Modulation of SOD2 activity in AML-12 cells and Murine Primary Hepatocytes

The modulation of SOD-2 activity in AML-12 cells and murine primary hepatocytes treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc),Formula (IId), Formula (IIf), Formula (IIg), or Formula (IIi or a pharmaceutically acceptable salt thereof, is measured.

The mouse hepatocytes cell line AML-12 (alpha mouse liver 12) is obtained from ATCC and grown at 37 °C in a humidified atmosphere of 5% CO₂/95% air in Dulbecco's Modified Eagle Medium / Nutrient Mixture F-12 (DMEM / F-12) supplemented with 0.005 mg/ml insulin, 0.005 mg/ml transferrin, 5 ng/ml selenium, 40 ng/ml dexamethasone and 1% gentamycin. ACMSD inhibitors are initially diluted from powder in DMSO to a stock concentration of 1 mM. This stock is further diluted with water to a concentration of 100 µM which was used for the cell treatments.

Primary hepatocytes are prepared from 8-12-week-old C57BL/6J mice by collagenase perfusion method. Mouse livers are perfused with Hank's balanced salt solution

(HBSS, KCl, 5.4 mM; KH₂PO₄, 0.45 mM; NaCl, 138 mM; NaHCO₃, 4.2 mM; Na₂HPO₄, 0.34 mM; glucose, 5.5 mM; HEPES, 1 M; EGTA, 50 mM; CaCl₂, 50 mM; pH 7.4). Livers are then washed at a rate of 5 ml/min through the portal vein. After washing, livers are perfused with collagenase (0.025%) solution. Cell viability is assessed by the trypan blue method. Isolated primary hepatocytes are plated with DMEM medium (Gibco) including 10% FCS, 10 units per ml penicillin and HEPES for buffering. The cells are maintained in culture at 37 °C in a humidified atmosphere of 5% CO₂/95% air. After 6-8 hrs of attachment, this medium is replaced with media containing different concentrations of an ACMSD inhibitor *(i.e.,* compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof,) or with the corresponding concentration of DMSO (as a control). Primary hepatocytes are harvested about 24 hrs later if not indicated differently.

Primary hepatocytes or AML-12 cells are then lysed in a 20 mM HEPES buffer (Gibco), pH 7.2, containing 1 mM EGTA (Sigma), 210 mM mannitol (Sigma), and 70 mM sucrose (AMRESCO). Total protein concentration is determined using the Bradford assay (BioRad). SOD-2 activity is determined at various times after ACMSD inhibitor treatment by the SOD Assay Kit (Cayman Chemical) according to the manufacturer's instructions. In order to specifically detect the SOD2 activity 2 mM potassium cyanide is added to the assay, which inhibits both Cu/Zn-SOD and extracellular SOD, resulting in the detection of only MnSOD (SOD-2) activity. Absorbance is determined with a Victor X4 multi-label plate reader (Perkin-Elmer) at 450 nm. Results are expressed in U/ml/mg of protein according to the standard curve and measured protein concentration.

The oxidative stress resistance pathway is explored by measuring the activity of SOD2.

### Example 54: Determination of NAD⁺ Content in Murine Primary Hepatocytes

NAD⁺ levels are determined in human primary hepatocytes treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof.

NAD⁺ is extracted using acidic extraction method. Samples are collected and homogenized in 70% ice-cold perchloric acid (HClO₄). After insoluble protein parts are pelleted by adding potassium carbonate (K₂CO₃), the samples are separated by high-performance liquid chromatography (HPLC) and analyzed by mass-spectrometry. The proteins in the pellet are quantified by Bradford assay and were used for normalization.

The exposure of primary hepatocytes to 5 nM, 10 nM and 50 nM of an ACMSD inhibitor of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, for 24 hours is examined for significant and dose-dependent increases in intra-cellular NAD⁺ levels.

### Example 55: RT-qPCR analysis of SIRT1-regulated genes in AML-12 cells, Hepa-1.6 cells and Primary Murine Hepatocytes treated with a compound of the Disclosure

Gene expression of ACMSD and genes known to be regulated by SIRT1, (an enzyme that is strictly NAD⁺ dependent) such as *Pgc1a,* Sod1, *Sod2* (MnSOD), are analyzed in AML-12 cells, Hepa-1.6 cells and primary murine hepatocytes treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof,.

Cells (AML-12, Hepa-1.6, HEK-293, primary human and murine hepatocytes) are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof. Total RNA is extracted from cells using TRIzol (Invitrogen) according to the manufacturer's instructions. The RNA is treated with DNase, and 2 µg of RNA is used for reverse transcription (RT). 50X diluted cDNA is used for RT-quantitative PCR (RT-qPCR) reactions. The RT-qPCR reactions are performed using the Light-Cycler system (Roche Applied Science) and a qPCR Supermix (QIAGEN) with the indicated primers. The average of at least three technical repeats is used for each biological data point.

A dose-dependent increase in mRNA expression levels of genes is known to be regulated by SIRT1, (an enzyme that is strictly NAD⁺ dependent) such as *Pgc1a, Sod2* (MnSOD), but not *Sod1* (Cu-Zn SOD).Primary mouse hepatocytes are treated for 24 hrs with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc),Formula (IId), Formula (IIf), Formula (IIg), or Formula (IIi), (5 nM - 500 nM range) and are observed for changes in expression levels and mRNA levels of *Pgc1a* and *Sod2* (MnSOD). Changes in mRNA expression are compatible with the activation of SIRT1, subsequent to the induction in NAD⁺ levels by inhibition of **ACMSD1** activity.

### Example 56: Modulation of Caspase 3/7 Activity in MDCK Cells

An *in vitro* study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on Acute Kidney Injury in MDCK cells.

MDCK cells (MDCK (NBL-2) ATCC^{®} CCL-34^{™}) are cultured in base medium ATCC-formulated Eagle's Minimum Essential Medium, Catalog No. 30-2003 with fetal bovine serum (FBS) to a final concentration of 10%. 10,000 cells are plated into 96 wells and 24 hours after cell plating the medium is changed with fresh medium supplemented with 1% FBS. Cisplatin (50µM for 16 hrs) is then used to induce cell injury. Different concentrations (about 1µM to about 125µM) of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, (in 1% DMSO) are added in combination with cisplatin or 1 hour prior adding cisplatin.

Caspase 3/7 activity (Promega) is determined according to standard procedures using a luminescent signal readout on a Victor V plate reader (PerkinElmer). Each experiment/condition is performed in triplicate.

Caspase activity is analyzed as percentage effect normalized to the cisplatin alone (100%) and vehicle treated cells as 0% of caspase activity. Data are analyzed by GraphPad Software. One-way analysis of variance (Dunnett's Multiple Comparison test) is used for statistical analyses.

MDCK cells are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof.

### Example 57: Cytotoxicity and hERG screening

Cytotoxicity: 20000 HePG2 and AML-12 cells are seeded in 96 well plate (Viewplate PerkinElmer). Dose-response of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, is performed using HP D300 digital dispenser, ranging from 10 nM to 300 µM with constant DMSO 1% in medium. Cells are stimulated for 4 hrs at 37 °C; the supernatant is used to perform LDH release (Cytotox-one, Promega) as a measure of necrosis while the cells are lysed to detect ATP level for determining cell viability (Celltiter-glo, Promega) according to manufacturer's instructions.

The Predictor hERG assay kit (Invitrogen), containing membrane preparations from Chinese hamster ovary cells stably transfected with hERG potassium channel and a high-affinity red fluorescent hERG channel ligand (tracer), is used for the determination of hERG channel affinity binding of the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof. Compounds that bind to the hERG channel protein (competitors) are identified by their ability to displace the tracer, resulting in a lower fluorescence polarization. The final concentration of DMSO in each well is maintained at 1%. The assays are performed according to the manufacturer's protocol (Invitrogen).

### Example 58: Anti-diabetic Effects in C57BL/6J and KK-Ay Mice

A glucose tolerance test is performed on male C57BL/6J and KK-Ay mice to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on glucose and insulin levels.

Male C57BL/6J and KK-Ay mice, 6-7 weeks of age, are obtained, e.g., from Charles River Laboratories France and CLEA Japan, respectively. Mice are fed from the age of 8 weeks onwards with regular chow (CD-Harlan 2018), a high fat diet (HFD-Harlan 06414). A compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, is mixed with the HFD at 180 mg kg⁻¹ of food. On the basis of their daily food intake, this results in a daily dose of about 15 mg kg⁻¹ body weight. The mice are fasted for 4 hrs before blood and tissues are harvested for RNA isolation, lipid measurements and histology. Oxygen consumption is measured with the Oxymax apparatus (Columbus Instruments). Histological analysis and transmission electron microscopy are performed.

An oral glucose tolerance test is performed in the animals that are fasted overnight. Glucose is administered by gavage at a dose of 2 g/kg. An intraperitoneal insulin tolerance test is performed in animals fasted for 4 hrs. Insulin is injected at a dose of 0.75 U/kg body weight. Glucose is quantified with the Maxi Kit Glucometer 4 (Bayer Diagnostic) or Glucose RTU (bioMerieux Inc.) and plasma insulin concentrations are measured by ELISA (Cristal Chem Inc.). Statistical differences are determined by either ANOVA or Student's t-test.

### Example 59: Anti-diabetic and Obesity Effects in db/db Mice with LepR Mutation

A study of the anti-diabetic effects of the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, is conducted in genetically obese *Leprdb* /J (db/db) mice.

Animals are bred and housed in a temperature- and humidity-controlled environment in compliance with FELASA-protocols. From an age of three weeks, mice are fed a high-fat diet (HFD) (Harlan 06414). Most pharmacological studies are started in diabetic eight-week-old db/db and wild type (wt) references.

### Subchronic intervention

db/db mice are treated once/day with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, for 14 days between 5-6 PM before dark-phase onset (6 PM). Blood samples are collected after 4 hrs of fasting the mice prior to the first dose and at 18 ± 2 hrs after the last dose. Glucose concentrations of each blood sample are determined.

### Acute intervention Glucose

Initial blood samples are collected in random-fed db/db mice between 6-8 AM after light-phase-onset (6 AM), then compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, are administered, diet-access is restricted, and the second blood sample is collected 4 hrs post-treatment. Thereafter, mice are subjected to an oral glucose tolerance test (OGTT1: 1 g glucose/kg body mass) and blood glucose concentrations are determined at 0.5, 1, 2, 3, and 4 hrs after each glucose challenge.

### Euglycemic-hyperinsulinemic Clamps Assay

db/db mice receive a permanent jugular vein catheter under ketamine/xylazine anesthesia. For six to seven days, later (after 6 AM) food-access is restricted. Conscious mice are placed in oversized rat-restrainers and warmed by warming pads. Catheter-ends are then connected to syringes in CMA402-pumps (Axel Semrau, Sprockhoevel, Germany). After 110 minutes of primed-continuous [3-³H]glucose infusion (1.85 kBq/min), a blood sample is collected to determine plasma insulin, glucose and [3-³ H]glucose concentrations and to calculate basal endogenous glucose appearance rates. The mice then receive vehicle or a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, *via* gavage.

Subsequently, glucose-1 clamps are started with a [3-³H]glucose infusion (3.7 kBq/min) containing insulin (36 pmol/kg^{∗}min⁻¹; HumulinR, Lilly, USA) causing a moderate net-increase in plasma insulin concentrations. Blood glucose concentrations are measured every 10 minutes and target glycemia is established by adjusting the rate of a 20% glucose infusion (GIR). At minute 120, 2-deoxy-D-[1-¹⁴ C]glucose (370 kBq) is given intravenously. Blood samples are collected at minute 30, 60, 90, 100, 110, 120, 122, 125, 130, and 140. The mice are then sacrificed *(i.e.,* through an intravenous ketamine/xylazine-overdose). Gastrocnemius muscle and epididymal adipose tissue are collected, immediately snap-frozen in liquid nitrogen, and stored at -80 °C. 2-[¹⁴C]deoxyglucose-6-phosphate is extracted from the tissue and glucose uptake rates (Rg) are calculated .

Plasma [³H]- and [¹⁴C]-radioactivity is determined in deproteinized plasma after [³H₂O] evaporation. Glucose fluxes under basal conditions and between glucose clamp minute 60 to 90 and 90 to 120 are estimated as follows: whole-body glucose disappearance rate (Rd) = [3-³H]GIR (dpm/min)/plasma [3-³H]glucose specific activity (dpm/min^{∗}mol); basal Endo Ra=[3-³H]GIR (dpm/min)/plasma [3-³H]glucose specific activity (dpm/min^{∗}mol); glucose-clamp Endo Ra = GIR-Rd. Ultima-Gold scintillation-cocktail, radioisotopes, and a Tri-Carb2910TR are obtained from Perkin Elmer (Germany).

### Assays from blood, plasma, urine

Blood samples are collected from lateral tail veins. Blood glucose is measured with a glucometer (Contour, Bayer Vital, Germany), urine and plasma glucose with a colorimetric Glucose LabAssay (Wako, Germany), and HbAlc with A1cNow+ (Bayer Vital) or Clover Analyzer (Inopia, South Korea).

### Analyses of disease onset and survival

Disease onset is defined as the last day of individual peak body weight before gradual loss occurs. The stages of disease are defined as follows: the early stage of disease is defined as the duration of time between peak body weight until loss of 10% of peak body weight. The late stage of disease is defined as the duration of time between 10% loss of peak body weight until the end stage of disease. The end stage of disease is defined as the day when an animal could no longer right itself within 30 s for three consecutive trials when placed on its side. Animals are euthanized at the end stage of disease.

### Body composition measurements

Body weights are assessed weekly for at least 13 weeks. Brown adipose tissue (BAT) and gonadal white adipose tissue (WAT) are dissected and weighed at the indicated age. Total lean mass, % of WAT and BMD (bone mineral density) are determined by DEXA (PIXImus DEXA; GE).

### Indirect calorimetry, food intake and activity

Animals are initially weighed and acclimated to the test cage. Volume oxygen (VO₂) and volume carbon dioxide production (VCO₂) are measured every 20 min using the Oxymax Comprehensive Laboratory Animal Monitoring System (CLAMS) (Columbus Instruments) and are reported as average VO₂ per hour normalized to body weight (mL/h/kg). Using the CLAMS machine, activity counts by infrared beam interruptions and food intake are simultaneously measured. More specifically, food intake is measured by deducting the weight of powderized food pellets at the end of experimentation from the starting weight at the beginning of experimentation. To complement this experiment and to control for a novel environment that may affect feeding behaviour, we also perform a more 'manual' experiment, wherein a set weight of food pellets is placed at the same time each day into a clean home cage, which holds a mouse. The next day the weight of the remaining pellets is recorded and deducted from the starting weight. This experiment is performed for 14 days straight. The body weight of each mouse is also recorded daily. Results for each genotype are similar to that acquired from the CLAMS.

### Statistical analyses.

Considering a 1-β larger than 0.9 statistically powerful, we estimate appropriate group numbers from pilot studies *a priori.* One- or two-way Analyses of Variance (Bonferroni post-tests) or t-tests are performed.

### Example 60: Effects on Non-alcoholic Fatty Liver Disease (NAFLD) and Non-alcoholic Steatohepatitis (NASH) in Mice

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), orFormula (IIi), or a pharmaceutically acceptable salt thereof, on non-alcoholic fatty liver disease (NAFLD) and non-alcoholic steatohepatitis (NASH) in male C57BL/6J fed a high fat and high sucrose diet.

Male C57BL/6J mice (The Jackson Laboratory, Bar Harbor, Maine, USA) are housed under a 14 hrs light-10 hrs dark cycle at 21-23°C and have ad libitum access to water during the entire experiment. From the age of 6 weeks, mice are fed a 'Western' HF-HSD with 44.6% of kcal derived from fat (of which 61% saturated fatty acids) and 40.6% of kcal derived from carbohydrates (primarily sucrose 340 g/kg diet) (TD.08811, 45% kcal Fat Diet, Harlan Laboratories Inc., Madison, Wisconsin, USA) or normal chow diet (NCD) as control (V1534-000 ssniff R/M-H, ssniff Spezialdiäten GmbH, Soest, Germany). The animals are then treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, or a control for 4, 12 or 20 weeks (n = 8 per group for every time point), after which they are sacrificed.

Body weight and food intake are monitored weekly on the same day. After sedation with sodium pentobarbital (intraperitoneal injection, 50 mg/kg body weight), total fat mass is analyzed by dual-energy X-ray absorptiometry (DEXA) (PIXImus densitometer, Lunar Corp., Madison, Wisconsin, USA). Intraperitoneal glucose tolerance test (IPGTT) is performed in 6 hrs fasted mice. Tail vein glucose levels are measured with a Bayer Contour glucometer immediately before (time point 0 min) and 15, 30, 60, 90 and 150 min after glucose administration (1 g glucose/kg body weight). Insulin resistance is calculated using the Homeostasis Model of Insulin Resistance (HOMA-IR) index: (fasting insulin (ng/mL) × fasting glucose (mg/dL))/405.

### Sacrifice

After a 6 hrs fasting period, mice are anaesthetised with sodium pentobarbital (intraperitoneal injection, 50 mg/kg body weight) and sacrificed by blood sampling via cardiac puncture. Plasma is obtained by centrifugation of blood (6000 rpm for 5 min at 4 °C) that is collected in heparinised syringes. Tissues are either snap frozen in liquid nitrogen or stored at -80°C together with the plasma until further biochemical and molecular analyses or preserved for histological analysis.

### Histological analyses

Liver samples are routinely fixed in buffered formalin (4%) and embedded in paraffin. Serial 4 mm thick sections are stained with H&E and picrosirius red to assess fibrosis. Frozen liver sections are stained with Oil Red O to assess lipid accumulation. All liver biopsies are analyzed by an expert liver pathologist, blinded to the dietary condition or surgical intervention. Steatosis, activity and fibrosis are semiquantitatively scored according to the NASH-Clinical Research Network criteria. The amount of steatosis (percentage of hepatocytes containing fat droplets) is scored as 0 (<5%), 1 (5-33%), 2 (>33-66%) and 3 (>66%). Hepatocyte ballooning is classified as 0 (none), 1 (few) or 2 (many cells/prominent ballooning). Foci of lobular inflammation are scored as 0 (no foci), 1 (<2 foci per 200× field), 2 (2-4 foci per 200×field) and 3 (>4 foci per 200×field). Fibrosis is scored as stage F0 (no fibrosis), stage F1a (mild, zone 3, perisinusoidal fibrosis), stage F1b (moderate, zone 3, perisinusoidal fibrosis), stage F1c (portal/periportal fibrosis), stage F2 (perisinusoidal and portal/periportal fibrosis), stage F3 (bridging fibrosis) and stage F4 (cirrhosis). Diagnosis of NASH is based on accepted histological criteria. Severity of the disease is assessed using the NAS (NAFLD activity score) as the unweighted sum of scores of steatosis, hepatocyte ballooning and lobular inflammation. Percentage of fibrosis is quantitated by morphometry from digitalised sirius red stained sections using the Aperio system after tuning the threshold of fibrosis detection under visual control. Results are expressed as collagen proportional area.

### Example 61: Effects on Non-alcoholic Fatty Liver Disease (NAFLD) and Non-alcoholic Steatohepatitis (NASH) in Methionine and Choline Deficient mice

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on non-alcoholic fatty liver disease (NAFLD) and non-alcoholic steatohepatitis (NASH) in male wildtype mice fed a methionine- and choline-deficient diet.

Wildtype mice housed in 12-hour light/dark cycles, with free access to food and water are used. At least 5 animals per time point are analyzed. All experiments are repeated at least three times. For dietary treatment, 8-12 weeks old male mice weighing 25 g are either fed a methionine- and choline-deficient diet (MCD to induce NASH) or chow diet (as a control). Animal experiments and evaluation of NAFLD and NASH as described above in Example 40 for mice fed the high fat and high sucrose diet.

### Example 62: Effects on Atherosclerosis in High Cholesterol Fed LDL-R Knockout mice

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on atherosclerosis in high cholesterol fed LDL-R knockout mice.

LDL-R knockout (KO) mice are backcrossed for ten generations with the C57BL/6J strain, yielding congenic C57BL/6J animals. The controls that are used are littermates in all experiments. The animals are treated with a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or pharmaceutically acceptable salt thereof, or a control. Mice are sacrificed 12 weeks after the initiation of the atherogenic diet (TD94059; Harlan), after which the heart and aorta are perfused with PBS and subsequently fixed (Shandon Formal Fixx, Thermo Scientific). Atherosclerosis is assessed by an Oil red O staining of the aortic root and quantified with MetaMorph software. Biochemistry parameters are measured with the appropriate kits in the COBAS C111 (Roche). For the *in vivo* lipopolysaccharide (LPS) study, mice are intraperitoneally injected with 100 mg of LPS, and blood is taken from the tail vein. TNFα levels are quantified with Mouse TNFα ELISA Ready-SET-Go! (eBioscience) assay. Blood cell counts are determined with Advia2120 (Siemens Healthcare Diagnostics).

The Student's t test is used to calculate the statistical significance. In case of multiple testing *(i.e.,* the comparison of more than two groups), this test is preceded by the ANOVA test. P < 0.05 is **considered** statistically significant. Results represent the mean ± SEM.

### Example 63: Effects on Inherited Mitochondrial Disease in Sco2^{KO/KI} mice

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on inherited mitochondrial disease in Sco2^{KO/KI} mice.

Anti-COI, anti-COX5a, anti-Ndufa9, anti-SDH-HA, and anti-Core 2 are from Invitrogen; anti-GAPDH is from Millipore; anti-FoxO1 and anti-acetylated-FoxO1 are from Cell Signaling and Santa Cruz, respectively. Anti-mouse secondary antibodies are from Amersham. Chemicals are from Sigma. Oligonucleotides are from PRIMM, Italy.

Compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc),Formula (IId), Formula (IIf), Formula (IIg), or Formula (IIi), or a pharmaceutically acceptable salt thereof, are dissolved in water and added to a standard powder diet (Mucedola, Italy) at the appropriate concentration of 50 mg/Kg/day. Pellets containing the compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, or the vehicles are reconstituted by hand and kept frozen at - 20°C until needed. The diet supply is changed every three days, and only the amount needed is thawed at each time and administered *ad libitum* for one month. Sco2^{KO/KI} mice are maintained in a temperature- and humidity-controlled animal-care facility, with a 12 hrs light/dark cycle and free access to water and food. Animals are sacrificed by cervical dislocation.

### Morphological Analysis

For histochemical analysis, tissues are frozen in liquid-nitrogen precooled isopentane. Series of 8 mm thick sections are stained for COX and SDH.

### Biochemical Analysis of MRC Complexes

Muscle quadriceps samples stored in liquid nitrogen are homogenized in 10 mM phosphate buffer (pH 7.4), and the spectrophotometric activity of cI, cII, cIII, and cIV, as well as CS, is measured as described. Note that in all panels the activity of cII is multiplied by 10 for visualization clarity.

### NAD⁺ Determination

NAD⁺ is extracted using acidic and alkaline extraction methods, respectively. Tissue NAD⁺ is analyzed with mass spectrometry as previously described.

### Example 64: Effects on Inherited Mitochondrial Disease in Deletor mice

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on inherited mitochondrial disease in Deletor mice.

The Deletor mouse model is generated in C57BL/6 congenic background and has been previously characterized (Tyynismaa et al, 2005); WT mice are littermates from the same congenic mouse strain C57BL/6J. Deletor and WT male mice are administered either chow diet (CD) or a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, admixed with the CD at the appropriate concentration. The food pellets are manually prepared by mixing a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, into the powdered food as described for the Sco2^{KO/KI} mice in Example 43 and stored at -20°C. The mice are housed in standard animal facility, under a 12 hrs dark/light cycle. They have ad libitum access to food and water. The pre-manifestation group consists of 12 Deletors and 12 WT mice, and the post-manifestation group of 24 Deletors and 24 WT mice, receiving either a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, or CD diet. During the intervention, the mice are regularly monitored for weight, food consumption, and physical endurance. Their exercise capability is measured twice by treadmill exercise test (Exer-6M Treadmill, Columbus Instruments) at the start and the end of the diet. The exercise test protocol consists of the initial running speed of 7 m/s which is increased every 2 min by 2 m/s and continued until the animal is unable to run or repeatedly falls from the belt at the stimulus site.

Oxygen consumption and carbon dioxide production, as well as spontaneous moving and feeding activities, are recorded by Oxymax Lab Animal Monitoring System (CLAMS; Columbus Instruments, OH, USA). The mice are kept in individual cages inside a CLAMS chamber for 3 days; the first day and night is a nonrecording adjustment period followed by a 24 hrs recording at thermoneutrality (+30 °C). The results of O₂ consumption and CO₂ production are used to calculate respiratory exchange rate and analyzed separately from the light (inactive) and dark (active) periods of the day.

### Morphologic analysis

Tissue sections are prepared from the quadriceps, liver, and BAT. Samples are embedded with OCT Compound Embedding Medium (Tissue-Tek) and snap-frozen in 2-methylbutane in liquid nitrogen. Frozen sections (12 lm) from quadriceps are assayed for *in situ* histochemical COX and succinate dehydrogenase (SDH) activities simultaneously. The activities from the quadriceps sections, the COX-negative and the COX-negative plus SDH positive and normal fibres are calculated. Approximately 2000 fibres are counted from each mouse sample. The intensity of COX histochemical activity from quadriceps for both oxidative and non-oxidative fibres is measured with Image J software. Frozen sections (8 µm) from liver and BAT are stained with Oil Red O. For plastic embedding, quadriceps, liver, and BAT samples are fixed in 2.5% glutaraldehyde, treated with 1% osmium tetroxide, dehydrated in ethanol, and embedded in epoxy resin. Semi-thin (1 µm) sections are stained with methyl blue (0.5% w/v) and boric acid (1% w/v). The interesting areas for the ultrastructural analyses are selected by inspection of the light microscopic sections. For transmission electron microscopy, ultrathin (60-90 nm) sections are cut on grids and stained with uranyl acetate and lead citrate and viewed with a Transmission Electron Microscope. Crista content in both BAT and muscle is determined from electron micrographs, utilizing a 1 µm "intra-mitochondrial measuring stick," placed perpendicular to cristae. Skeletal muscle samples are also analyzed for citrate synthase activity.

### Example 65: Effects on Kidney Disease

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on kidney disease in C57BL/6J WT mice. (Wei, Q., et al., "Mouse model of ischemic acute kidney injury: technical notes and tricks" American Journal of Physiology-Renal Physiology, 303(11), F1487-F1494)

C57BL/6J WT mice are purchased from Charles-River. All mice are fed a standard commercial diet while housed at an ambient temperature of 20-22 °C with a relative humidity of 50 ± 5% under 12/12 hrs light-dark cycle in a specific pathogen-free facility. The experimental mice are 8 weeks old and are divided into four groups: control (n = 5); cisplatin (20 mg/kg; Sigma Chemical, St Louis, MO; n = 5); a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, and cisplatin (n = 5); and a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, alone (40 mg/kg; n = 5). The dose and time of cisplatin treatment for nephrotoxicity are chosen according to a published method. A compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, is administered orally once a day for 4 days. Cisplatin is injected once at 12 hrs after the first administration of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof. The mice are sacrificed at 72 hrs after the single cisplatin injection.

### Assays for renal functional markers and proinflammatory cytokines

For renal function analysis, serum is isolated and stored at -80 °C until use. Serum creatinine and BUN levels are measured using an assay kit according to the manufacturer's instructions (BioVision, Milpitas, CA). In addition, the proinflammatory cytokines TNF-α, IL-1b, and IL-6 from serum or homogenates from kidney tissue are quantified by ELISA (Quantikine Kit; R&D Systems, Minneapolis, MN) according to the manufacturer's instructions. For measuring cytokines, kidney tissue is homogenized in phosphate buffered saline containing 0.05% Tween-20. Aliquots containing 300 mg of total protein are used. A metabolic cage is used for collecting urine to analyze the level of urinary cytokines. The sample size for each group is five.

### Alternative Study of the Effects on Kidney Disease

Alternatively, C57BL/6J WT mice are numbered and kept in acclimatization for a period of 5-7 days before initiation of the experiment. (Wei, Q., et al.. "Mouse model of ischemic acute kidney injury: technical notes and tricks" American Journal of Physiology-Renal Physiology, 303(11), F1487-F1494) Mice are randomized into different treatment groups based on their body weight. Different groups are maintained on Harlan diet 2916. Mice are then maintained on the respective diets for 10 days prior to bilateral Ischemic kidney injury. Body weight measurement is made once at randomization and once on day 7. Food consumption is evaluated once on day 7. Blood is collected by retro-orbital puncture under mild Isoflurane anesthesia and used for analysis of basal blood urea nitrogen levels (BUN) on day 9.

Mice are anesthetized with ketamine (80 mg/kg i.p) and/or Xylazine (10 mg/kg, i.p.) and placed on a surgical platform in a dorsal position. Both kidneys are exposed through flank incisions and renal pedicles are occluded using vascular clamps for 25 minutes. The clamp is then removed and the surgical site is sutured. 1ml of physiological saline is administered intra-peritoneally after closing the wound to prevent dehydration. The sham-operated group is subjected to similar surgical procedures, except that the occluding clamp is not applied. Animals are monitored until recovery from anesthesia and returned to their home cage. Animals are observed every day for general clinical signs and symptoms and mortality.

One day prior to termination, animals are individually housed in metabolic cages for 12h and urine is collected for estimation of urea, creatinine, sodium and potassium.

On days 12, 14, & 16 blood is collected by retro orbital puncture under mild isoflurane anesthesia and plasma is used for analysis of blood urea nitrogen levels (BUN) and serum creatinine. Animals are then euthanized by CO₂ inhalation and organs are collected. One kidney is fixed in 10% neutral buffered formalin and the other is flash frozen in liquid nitrogen, stored at -80°C and used for the estimation of lipid peroxidation, GSH, MPO and SOD levels.

### Histological analysis and neutrophil counting

Mouse kidneys are fixed in 4% formaldehyde and embedded in paraffin wax. The 5-mm-thick sections are deparaffinised in xylene and rehydrated through graded concentrations of ethanol. H&E and PAS staining are performed using standard protocols. Images are collected and analyzed using a light microscope (IX71, Olympus, Tokyo, Japan) with DP analyzer software (DP70-BSW, Tokyo, Japan). Tubular damage in PAS-stained kidney sections is examined under a light microscope and scored based on the percentage of cortical tubular necrosis: 0 = normal, 1 = 1-10, 2 = 11-25, 3 = 26-45, 4 = 46-75, and 5 = 76-100%. Slides are scored in a blinded manner, and results are means ± s.d. of 10 representative fields/group. Severity criterion for tubular necrosis displaying the loss of the proximal tubular brush border and cast formation are used to classify samples. The sample size for each group is 10. Neutrophil infiltration is quantitatively assessed on PAS stained tissue by a renal pathologist by counting the number of neutrophils per high-power field (x400). At least 10 fields are counted in the outer stripe of the outer medulla for each slide.

All values are represented as mean ± s.d. One-way analysis of variance is used to calculate the statistical significance of the results of all assays and P-values < 0.05 are considered statistically significant.

### Example 66: Effects on Ischemia/Reperfusion-induced Acute Kidney Injury

A study is performed to determine the effects of compounds of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, on Ischemia/Reperfusion-induced (I/R-induced) Acute Kidney Injury in CD-1 (ICR) mice.

CD-1 (ICR) mice are purchased from Charles River Laboratory (Wilmington, MA). Mice are housed in a temperature- and humidity-controlled environment with a 12:12 hrs light-dark cycle and are allowed freely access to standard rodent chow (TekLad, Madison, WI) and tap water.

Mice are subjected to a midline back incision, and both renal pedicles are clamped for 45 min with microaneurysm clamps (00396-01; Fine Science Tools, Foster City, CA). After removal of the clamp, the kidneys are inspected for the restoration of blood flow. The animals are allowed to recover, and they are sacrificed 48 hrs after reperfusion. Mice are treated with 100 mg/kg of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, by oral gavage once per day. CD-1 mice are divided into four groups: (1) young mice with sham injury (n = 4) (6-7 weeks old); (2) young mice with I/R injury (n = 8); (3) adult mice with sham injury (n = 4) (20-24 weeks old); and (4) adult mice with I/R injury (n = 11). An additional 27 adult mice (20-24 weeks old) are randomized into two groups: 13 mice received a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc), Formula (IId), Formula (IIf), Formula (IIg), or Formula (IIi), or a pharmaceutically acceptable salt thereof, and the other 14 mice received the vehicle as a control.

The serum creatinine level is measured using the QuantiChrom Creatinine Assay Kit (DICT-500, BioAssay Systems, Hayward, CA). BUN measurements are recorded using the Infinity Urea (Nitrogen) Liquid Stable Reagent (TR12421; ThermoTrace, Victoria, AU).

### Evaluation of renal tissue

Kidneys are fixed in 4% paraformaldehyde, embedded in paraffin, and stained with hematoxylin and eosin (4 mm thick). Tubular injury is scored on a scale of 0-4 on the basis of the percentage of tubules with necrosis, dilatation, or cell swelling: 0, less than 5%; 1, 5-25%; 2, 25-50%; 3, 50-75%; and 4, over 75%. All high-power fields (x 400) in the cortex and outer medulla are evaluated by a pathologist in a blinded manner.

All values are expressed as mean ± s.e. Statistical analysis is carried out using GraphPad Prism 4.00 (San Diego, CA) with unpaired Student's t testing for two sets of data and an analysis of variance with a Bonferroni post-test for multiple groups. P < 0.05 was considered significant.

### Example 67: Effects on FoxO1 Phosphorylation levels

AML-12 cells are treated with different concentrations of a compound of Formula (I), Formula (Ia), Formula (Ib), Formula (Ic), Formula (Id), Formula (Ie), Formula (If), Formula (Ig), Formula (Ih), Formula (Ii), Formula (Ij), Formula (II), Formula (IIa), Formula (IIb), Formula (IIc),Formula (IId), Formula (IIf), Formula (IIg), or Formula (IIi), or a pharmaceutically acceptable salt thereof, for 24 hours. Cells are then lysed in lysis buffer (50 mM Tris, 150 mM KCl, EDTA ImM, NP40 1%) containing protease and phosphatase inhibitors, and analyzed by SDS-PAGE/western blot. Blocking and antibody incubations were done in 5% milk. Each protein present is detected with its specific antibody. Tubulin antibody is obtained from Sigma Inc, FoxO1 and phopho-FoxO1 (Ser256) antibodies were obtained from Cell Signaling. Antibody detection reactions are developed by enhanced chemiluminescence (Advansta, CA, USA) using x-ray films.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice of testing the present disclosure, suitable methods and materials are described below. The references cited herein are not admitted to be prior art of the claimed disclosure. In the case of conflict, the present specification, including definitions, will control. In addition, the materials, methods, and examples are illustrative only and are not intended to be limiting.

### EQUIVALENTS

Those skilled in the art will recognize, or be able to ascertain using no more than routine experimentation, many equivalents to the specific embodiments and methods described herein. Such equivalents are intended to be encompassed by the scope of the present disclosure, but are not necessarily part of the invention. The scope of the invention is defined solely by the appended claims.

## Claims

1. A compound represented by Formula (I): 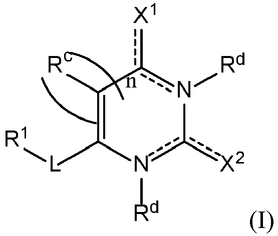 or a pharmaceutically acceptable salt or tautomer thereof, wherein:
X¹ is O, S, OR², or SH;
X² is O, S, OR², or SR²;
L is -(CH₂)ₘCH=CH(CH₂)ₚ-, -(CH₂)*ₒ-* ; 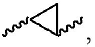 -(CH₂)ₘC=(O)NR³(CH₂)ₚ-, -(CH₂)ₘNR³C=(O)(CH₂)ₚ-, pyridinyl, or thiophenyl;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -O(C(R^{f})₂)_{f}(C₃-C₇)cycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, - O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

2. The compound of claim 1, wherein:
X¹ is O or OR²;
X² is S or OR²;
L is -(CH₂)ₘCH=CH(CH₂)ₚ-, -(CH₂)*ₒ*-, 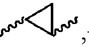 -(CH₂)ₘC=(O)NR³(CH₂)ₚ-, or -(CH₂)ₘNR³C=(O)(CH₂)ₚ-;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R^{a} is H, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, -OR^{y}, or -CH=CHCO₂R^{x}, wherein the aryl and heteroaryl are optionally substituted with one to three substituents each independently selected from halogen and OH, and wherein the heterocycloalkyl is substituted with one to two =O or =S;
R^{b} is -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl, -(C(R^{f})₂)ᵣP(O)₂OH, -(C(R^{f})₂)ᵣS(O)₂OH, -O(C(R^{f})₂)ᵣOH, or -CH=CHCO₂R^{x}, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

3. The compound of claim 1, wherein:
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC₆-C₁₀ aryl, or -OR^{y}, wherein the aryl is substituted with one to three substituents selected from halogen and OH;
R^{b} is -(C(R^{f})₂)ᵣCO₂R^{x}, -O(C(R^{f})₂)ᵣCO2R^{x}, or -(C(R^{f})₂)ᵣC₆-C₁₀ aryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

4. The compound of claim 1, wherein:
X¹ is O;
X² is O, S, or SR²;
L is -(CH₂)ₘCH=CH(CH₂)ₚ-;
Y² is O, NH or S;
R¹ is C₆-C₁₀ aryl substituted with R^{a} and R^{b}, and optionally substituted with one to two R^{e};
R² is H or C₁-C₄ alkyl;
R^{a} is H, -(C(R^{f})₂)ᵣCO₂R^{x}, -O(C(R^{f})₂)ᵣCO₂R^{x}, -(C(R^{f})₂)ᵣC6-C₁₀ aryl, -O(C(R^{f})₂)ᵣheteroaryl, or -OR^{y}, wherein the aryl is substituted with one to three substituents selected from halogen and OH;
R^{b} is -(C(R_{f})₂)ᵣCO₂R^{x}, -O(C(R^{f})₂)ᵣCO2R^{x}, -(C(R^{f})₂)ᵣC6-C₁₀ aryl, or -O(C(R^{f})₂)ᵣheteroaryl, wherein the aryl is substituted with one to three substituents selected from halogen and OH;
R^{c} is -CN;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond.

5. The compound of claim 1, wherein the compound is represented by Formula (Ia), (Ib), (Ic), (Id), (Ie), (If), (Ig), (Ih), (Ii), or (Ij): 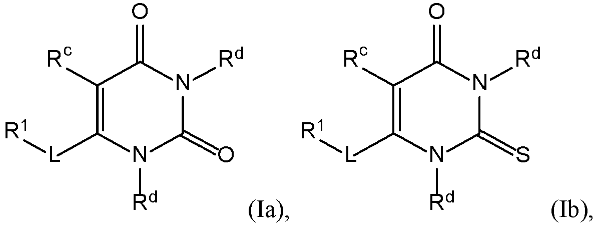 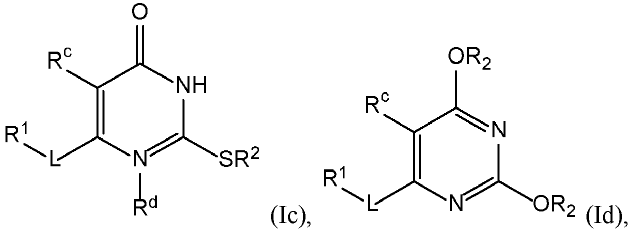 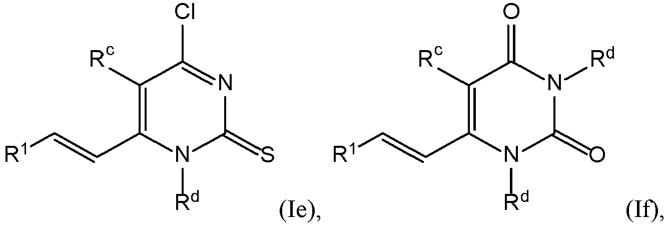 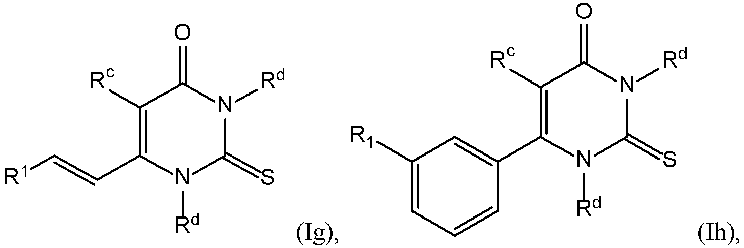 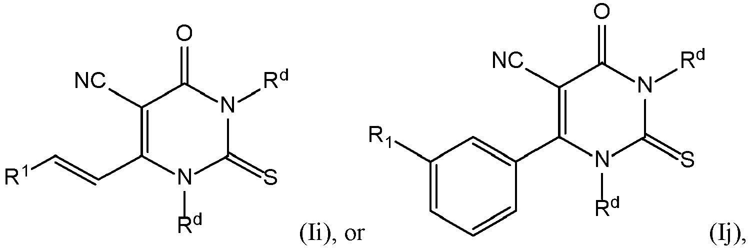 or a pharmaceutically acceptable salt thereof, or tautomer thereof.

6. The compound of any one of claims 1 to 5, wherein R¹ is C₆-C₁₀ aryl substituted with R^{a} and R^{b}, and optionally substituted with one to two R^{e}.

7. The compound of any one of claims 1 to 5, wherein R¹ is phenyl substituted with R^{a} and R^{b}, and optionally substituted with one to two R^{e}.

8. The compound of any one of claims 1 to 5, wherein R¹ is heteroaryl comprising one 5-to 7-membered ring and 1-4 heteroatoms selected from N, O and S, and substituted with R^{a} and R^{b}, and optionally substituted with one to two R^{e}.

9. The compound of any one of claims 1 to 5, wherein R¹ is pyridinyl substituted with R^{a} and R^{b}, and optionally substituted with one to two R^{e}.

10. The compound of any one of claims 1 to 9, wherein R^{a} is H and R^{b} is -(C(R_{f})₂)ᵣCO₂R^{x}, -O(C(R^{f})₂)ᵣCO₂R^{x}, or -(C(R^{f})₂)ᵣC₆-C₁₀ aryl.

11. The compound of any one of claims 1 to 9, wherein R^{a} is H and R^{b} is -CO₂H, -CH₂CO₂H, -OCH₂CO₂R^{x}, -OCH(CH₃) CO₂R^{x},-OC(CH₃)₂CO₂R^{x}, or

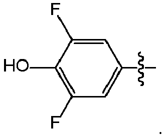

12. The compound of any one of claims 1 to 9, wherein R^{a} is OR^{y} and R^{b} is -O(C(R^{f})₂)ᵣCO2R^{x}, or -O(C(R^{f})₂)ᵣheteroaryl.

13. The compound of any one of claims 1 to 9, wherein R^{a} is H and R^{b} is -(C(R^{f})₂)ᵣCO₂R^{x}, -Y²(C(R^{f})₂)ᵣCO₂R^{x}, -(C(R^{f})₂)ᵣS-C₆-C₁₀ aryl, -(C(R^{f})₂)ᵣheteroaryl, -(C(R^{f})₂)ᵣP(O)₂OH, -(C(R^{f})₂)ᵣS(O)₂OH, or-CH=CHCO₂R^{x}, wherein the aryl and heteroaryl are substituted with one to three substituents selected from halogen and OH; or R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more -CO₂H.

14. The compound of any one of claims 1-13, having any one of the following formula:

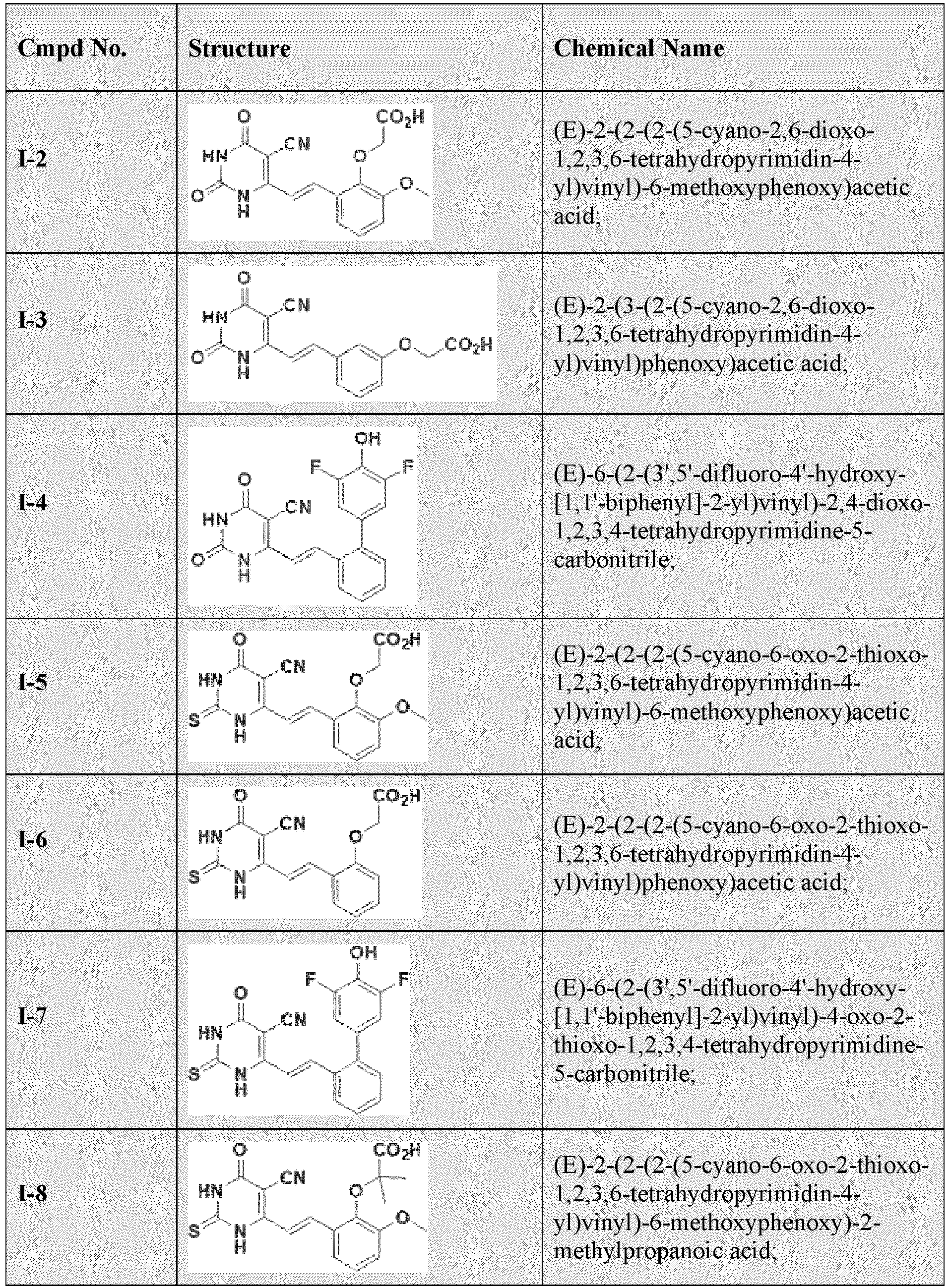

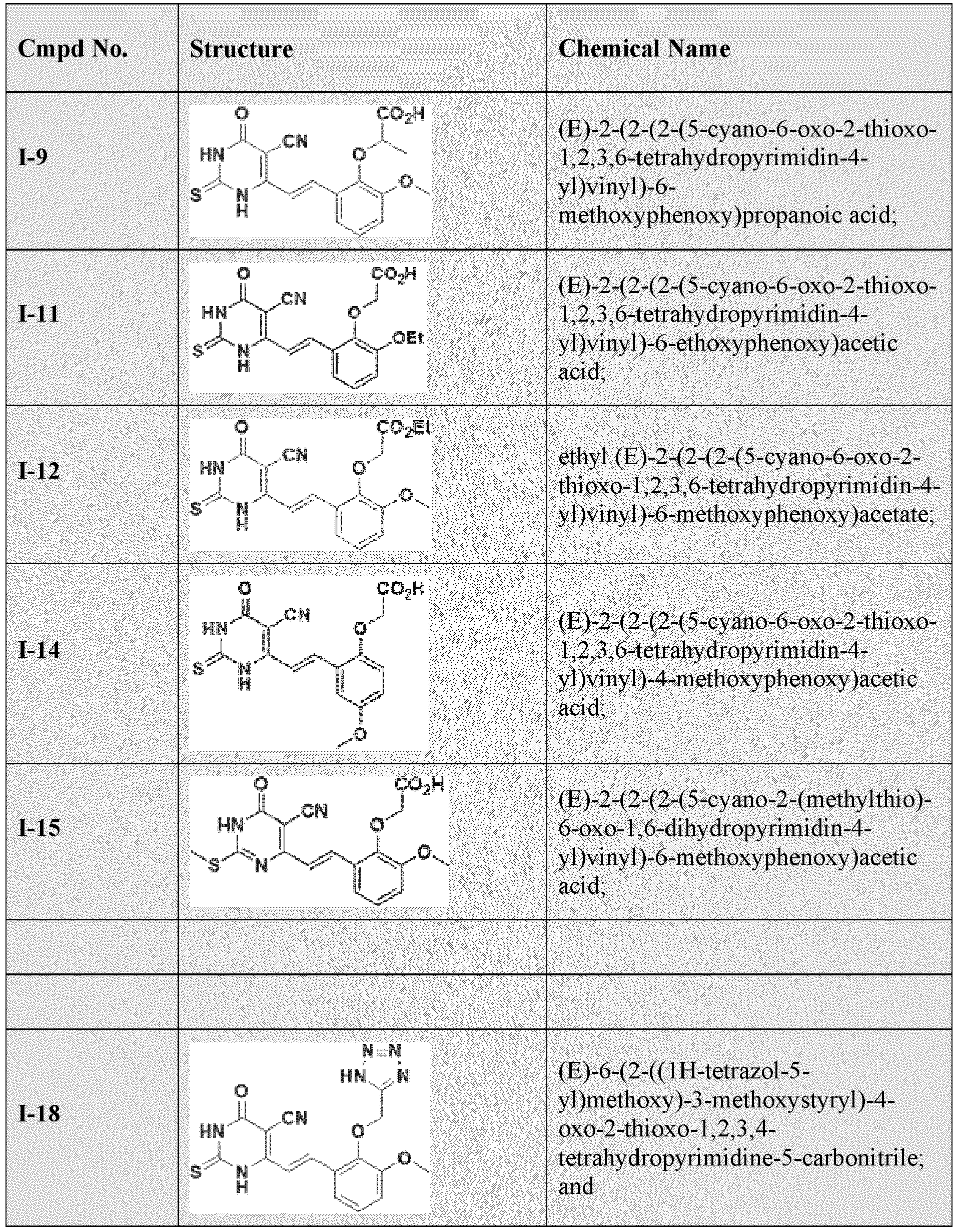

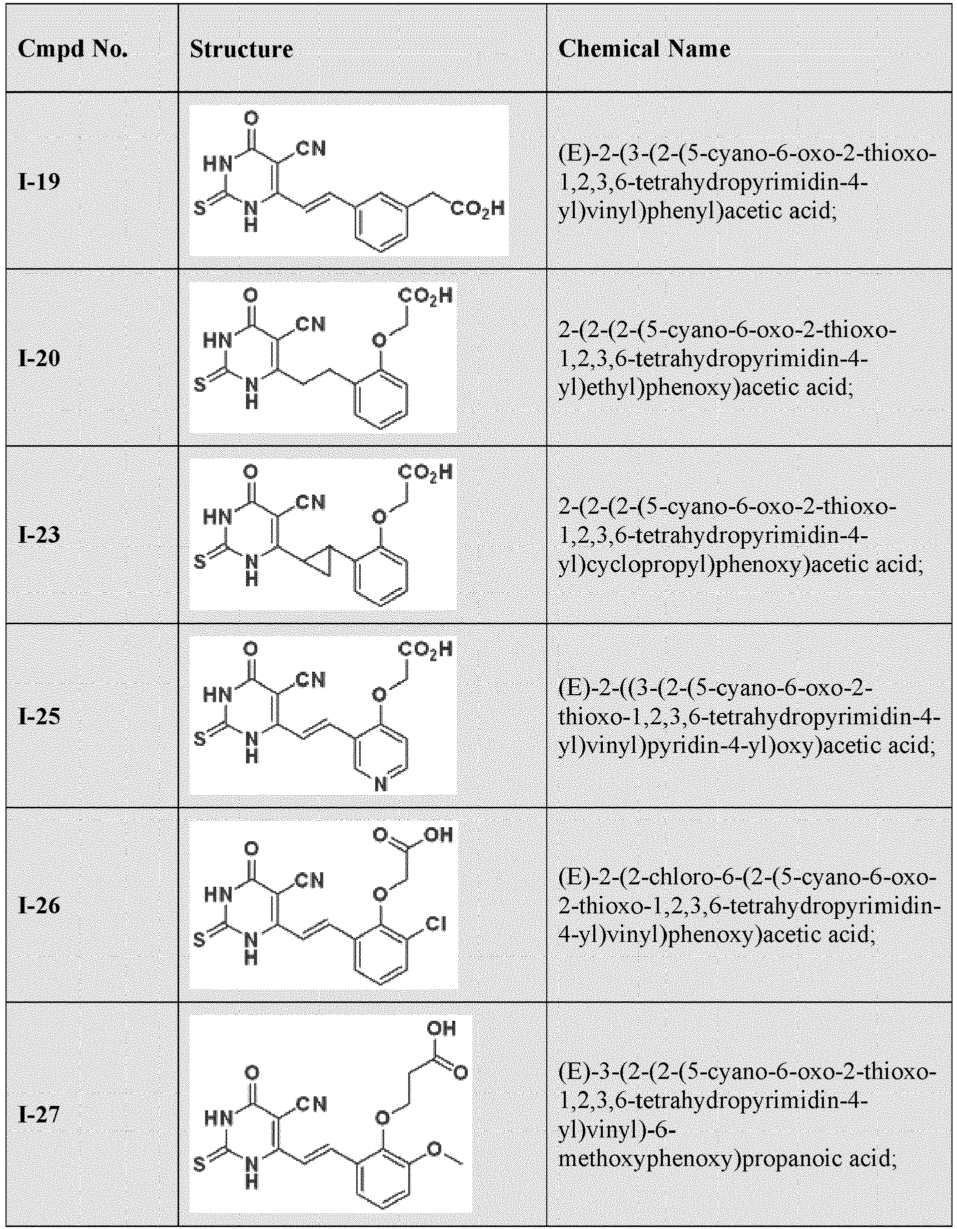

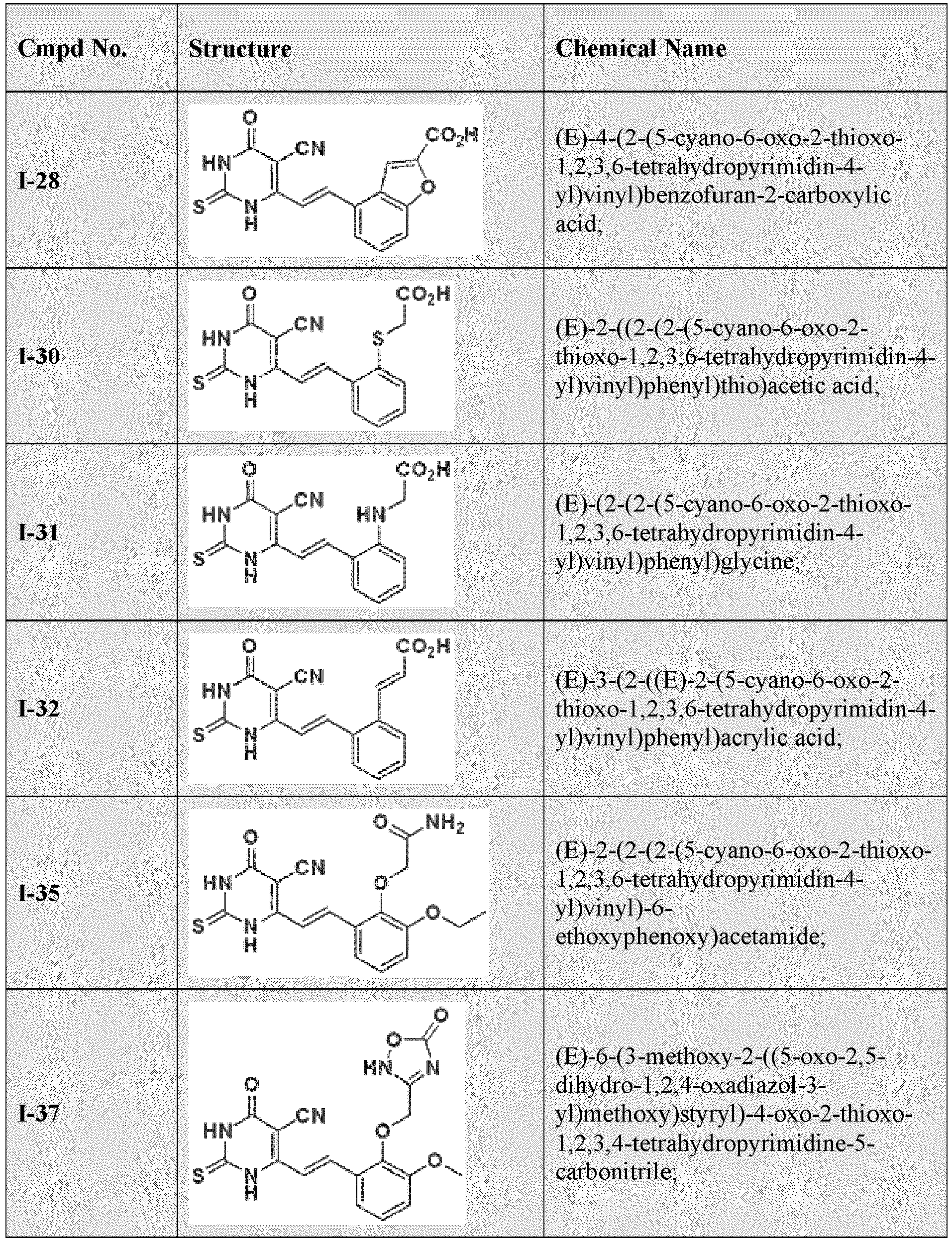

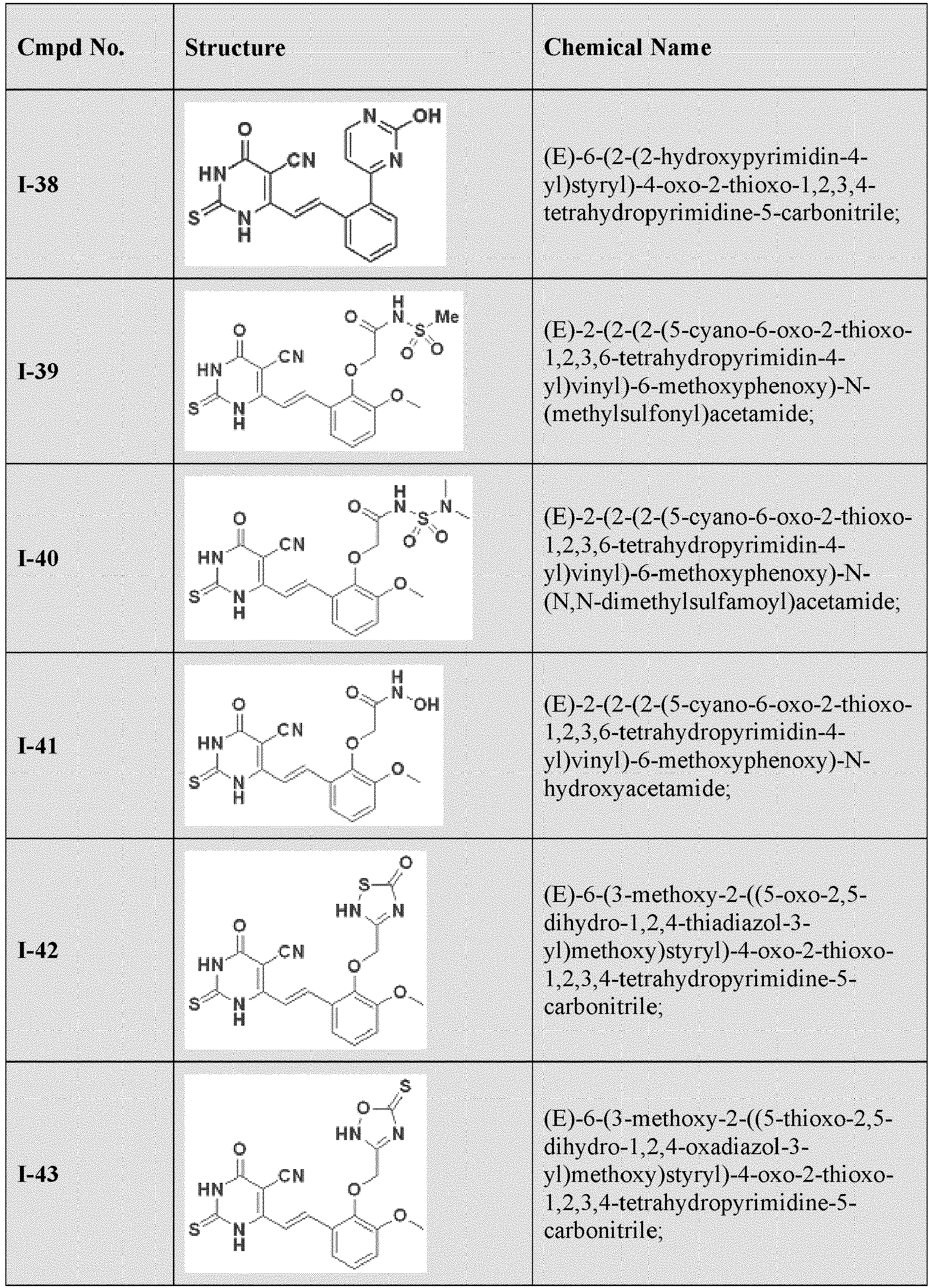

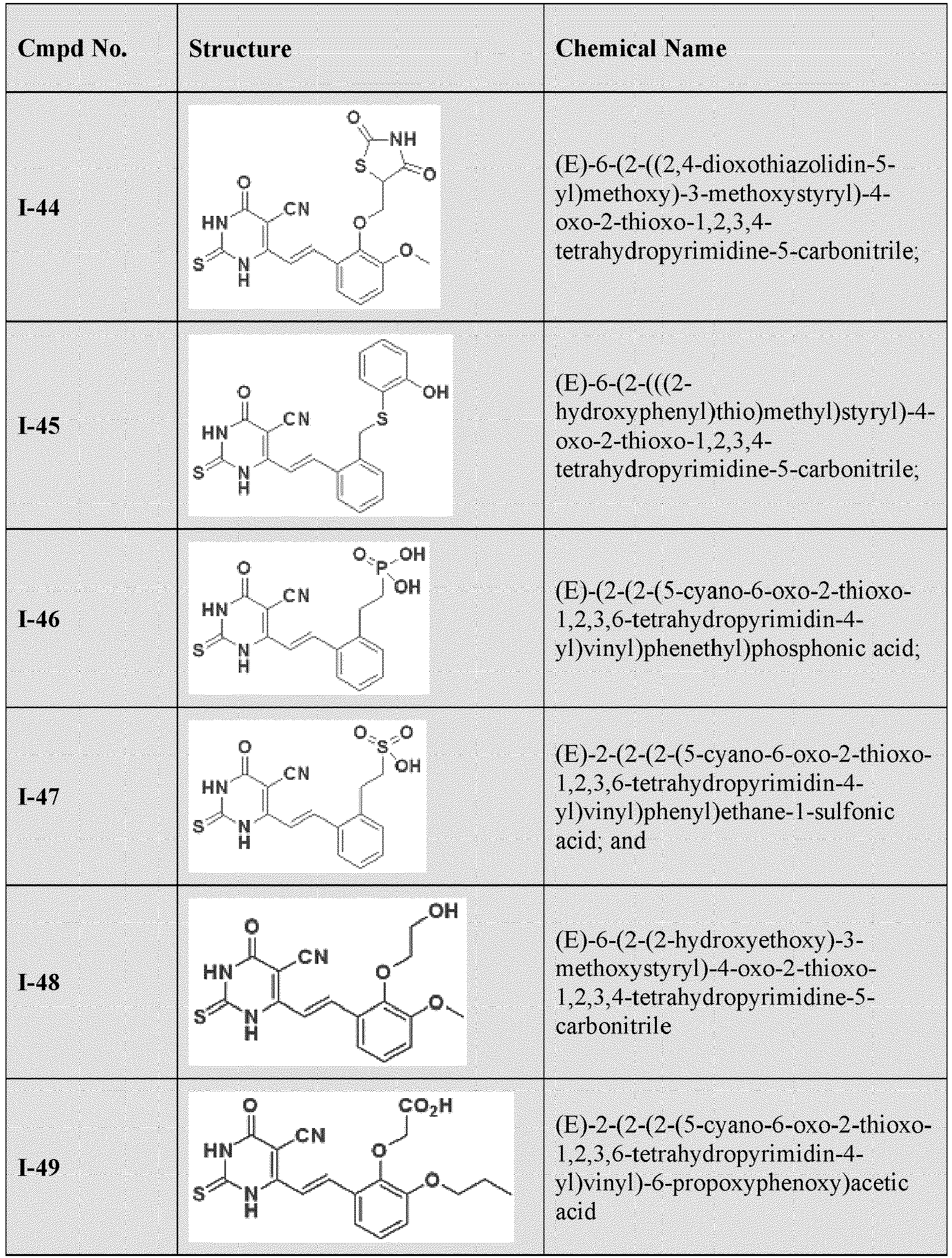

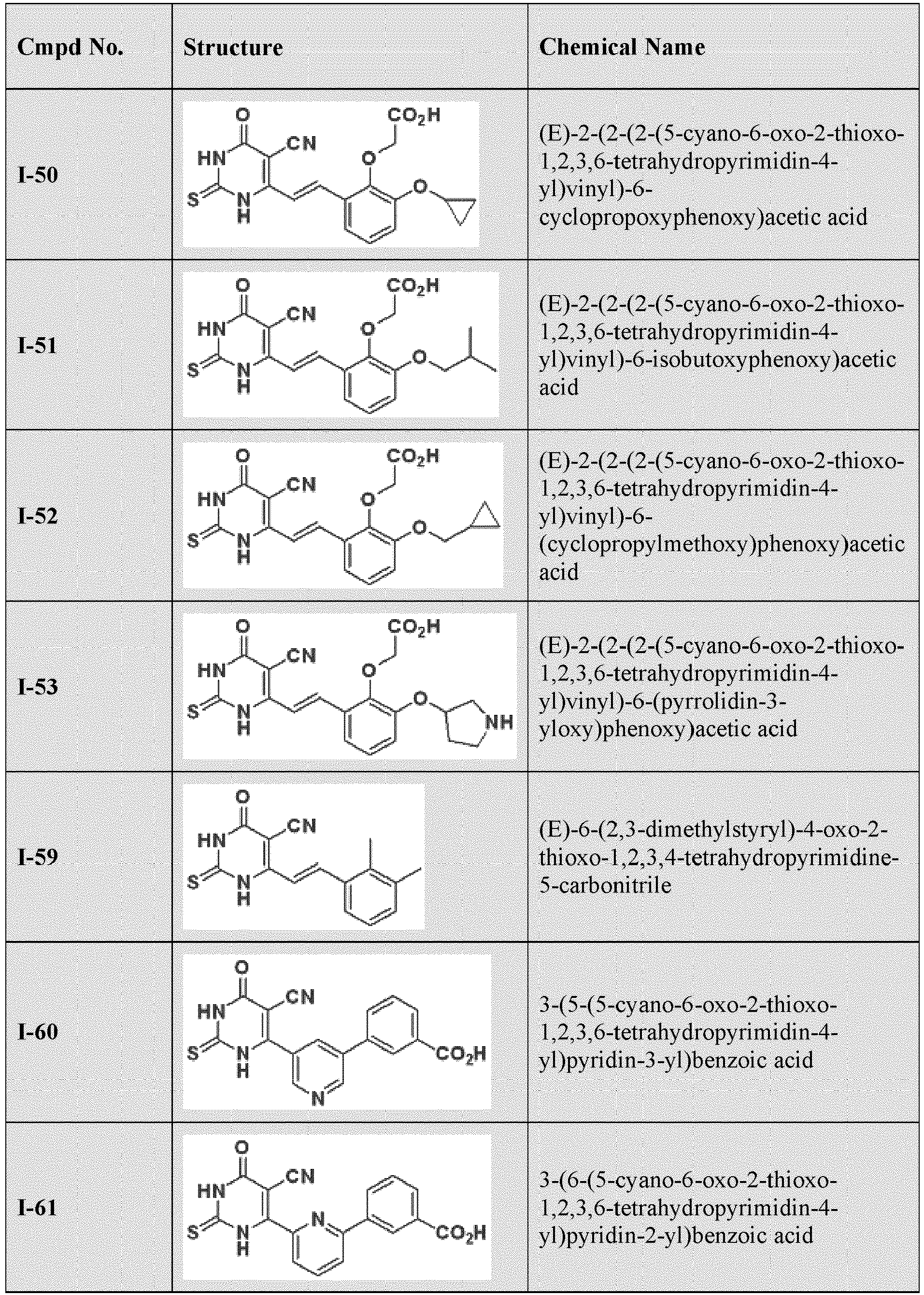

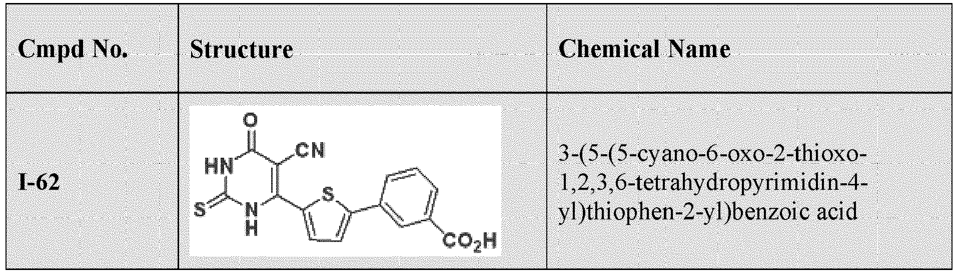

15. A pharmaceutical composition comprising a compound of any one of claims 1 to 14, or a pharmaceutically acceptable salt thereof, and at least one of a pharmaceutically acceptable carrier, diluent, or excipient,
optionally wherein the composition comprises one or more further therapeutic agents.

16. A compound of any one of claims 1-14 or a pharmaceutical composition of claim 15, for use in a method of treating, preventing, or reducing the risk of a disease or disorder associated with α-amino-β-carboxymuconate-ε-semialdehyde decarboxylase (ACMSD) dysfunction, the method comprising administering to the subject suffering from or susceptible to developing a disease or disorder associated with ACMSD dysfunction a therapeutically effective amount of the compound,
wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

17. A compound of any one of claims 1-14 or a pharmaceutical composition of claim 15, for use in a method of treating, preventing, or reducing the risk of a disease or disorder associated with reduced nicotinamide adenine dinucleotide (NAD⁺) levels, the method comprising administering to the subject suffering from or susceptible to developing a disease or disorder associated with reduced NAD⁺ levels a therapeutically effective amount of the compound,
wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

18. A compound of any one of claims 1-14 or a pharmaceutical composition of claim 15, for use in a method of treating, preventing, or reducing the risk of a disorder associated with mitochondrial dysfunction, the method comprising administering to the subject suffering from or susceptible to developing a metabolic disorder a therapeutically effective amount of the compound,
wherein said disorder associated with mitochondrial dysfunction is an inherited mitochondrial disease, a common metabolic disorder, a neurodegenerative disease, an aging related disorder, a kidney disorder, or a chronic inflammatory disease; optionally.
the common metabolic disorder is obesity or type II diabetes.

19. The compound for use according to claim 18, wherein the metabolic disorder is type II diabetes, obesity, hyperglycemia, glucose intolerance, insulin resistance, hypercholesterolemia, hypertension, hyperlipoproteinemia, hyperlipidemia, hypertriglylceridemia, cardiovascular disease, atherosclerosis, peripheral vascular disease, kidney disease, ketoacidosis, thrombotic disorders, nephropathy, diabetic neuropathy, diabetic retinopathy, sexual dysfunction, dermatopathy, dyspepsia, hypoglycaemia, cancer, or edema;
and/or wherein the neurodegenerative disease is photoreceptor degeneration, dementia, Alzheimer's disease, Parkinson's disease, or Huntington's disease;
and/or wherein the chronic inflammatory disease is celiac disease, vasculitis, lupus, chronic obstructive pulmonary disease (COPD), irritable bowel disease, atherosclerosis, arthritis, or psoriasis;
and/or wherein the kidney disease is acute kidney injury (AKI) or chronic kidney disease (CKD);
and/or wherein the disease associated with aging is cancer, dementia, cardiovascular disease, hypertension, diabetes mellitus (type I or type II), arthritis, cataracts, Alzheimer's disease, macular degeneration, or osteoporosis.

20. A compound for use in a method of treating, preventing, or reducing the risk of a disease or disorder associated with α-amino-β-carboxymuconate-ε-semialdehyde decarboxylase (ACMSD) dysfunction, the method comprising administering to the subject suffering from or susceptible to developing a disease or disorder associated with ACMSD dysfunction a therapeutically effective amount of the compound, wherein the compound is represented by Formula (II): 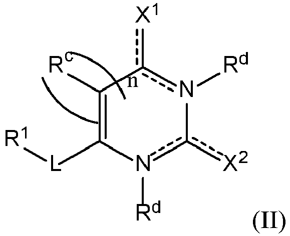 or a pharmaceutically acceptable salt or tautomer thereof, wherein:
X¹ is H, O, S, OR², or SH;
X² is O, S, OR², or SR²;
L is -(CH₂)ₘCH=CH(CH₂)ₚ-, -(CH₂)*ₒ*-, 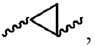 -(CH₂)ₘC=(O)NR³(CH₂)ₚ-,-(CH₂)ₘNR³C=(O)(CH₂)ₚ-, pyridinyl, or thiophenyl;
Y² is O, NH or S;
R¹ is C₆-C₁₀ aryl or heteroaryl, wherein the heteroaryl comprises one or two 5-to 7-membered rings and 1-4 heteroatoms selected from N, O and S, and wherein the aryl and heteroaryl are substituted with R^{a} and R^{b}, and optionally substituted with one to two R^{e};
R² is H or C₁-C₄ alkyl;
R³ is H or C₁-C₄ alkyl;
R^{a} is H, C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl, -(C(R^{f})₂)ᵣheteroaryl, -O(C(R^{f})₂)ᵣheteroaryl, -O(C(R^{f})₂)ᵣheterocycloalkyl, -O(C(R^{f})₂)ᵣ(C₃-C₇)cycloalkyl, -(C(R^{f})₂)ᵣP(O)(OH)OR^{x}, -O(C(R_{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, - O(C(R^{f})₂)ᵣOH, -OR^{y}, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are optionally substituted with one to three substituents each independently selected from halogen and OH, and wherein the heterocycloalkyl is substituted with one to two =O or =S;
R^{b} is C₁-C₄ alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣC₆-C₁₀ aryl, -(C(R^{f})₂)ᵣS-C₆-C₁₀ aryl,-(C(R^{f})₂)ᵣheteroaryl, -O(C(R^{f})₂)ᵣheteroaryl, -O(C(R^{f})₂)ᵣheterocycloalkyl,-(C(R^{f})₂)ᵣP(O)(OH)OR^{x}, -O(C(Rf)₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH,- O(C(R^{f})₂)ᵣOH, -(C(R^{f})₂)ᵣC(O)NHCN, -CH=CHCO₂R^{x}, or -(C(R^{f})₂)ᵣC(O)NHS(O)₂alkyl, wherein the aryl and heteroaryl are substituted with one to three substituents selected from halogen and OH, and wherein the heterocycloalkyl is substituted with one to two =O or =S; or
R^{a} and R^{b} when on adjacent atoms together with the atoms to which they are attached form a C₆-C₁₀ aryl ring optionally substituted with one or more -CO₂H; R^{a} and R^{b} when on adjacent atoms together with the atoms to which they are attached form a 5- to 6-membered heteroaryl ring optionally substituted with one or more - CO₂H;
R^{c} is -CN or -NO₂;
each R^{d} is independently at each occurrence absent or H;
each R^{e} is independently at each occurrence C₁-C₆ alkyl, C₂-C₆ alkenyl, C₂-C₆ alkynyl, halogen, C₁-C₆ haloalkyl, -NHR^{z}, -OH, or -CN;
each R^{f} is independently H or C₁-C₆ alkyl;
R^{g} is H, C₁-C₆ alkyl, OH, -S(O)₂(C₁-C₆ alkyl), or -S(O)₂N(C₁-C₆ alkyl)₂;
R^{x} is H or C₁-C₆ alkyl;
each R^{y} and R^{z} is independently H, C₁-C₆ alkyl, or C₁-C₆ haloalkyl;
each m, p, and r is independently 0, 1 or 2;
n is 1;
o is 1, 2, 3, or 4; and
the dotted line is an optional double bond;
wherein said disease is chronic liver disease selected from primary biliary cirrhosis (PBC), cerebrotendinous xanthomatosis (CTX), primary sclerosing cholangitis (PSC), drug induced cholestasis, intrahepatic cholestasis of pregnancy, parenteral nutrition associated cholestasis (PNAC), bacterial overgrowth or sepsis associated cholestasis, autoimmune hepatitis, chronic viral hepatitis, alcoholic liver disease, nonalcoholic fatty liver disease (NAFLD), nonalcoholic steatohepatitis (NASH), liver transplant associated graft versus host disease, living donor transplant liver regeneration, congenital hepatic fibrosis, choledocholithiasis, granulomatous liver disease, intra- or extrahepatic malignancy, Sjogren's syndrome, Sarcoidosis, Wilson's disease, Gaucher's disease, hemochromatosis, and alpha 1-antitrypsin deficiency.

21. The compound for use of claim 20, having any one of the following formula:

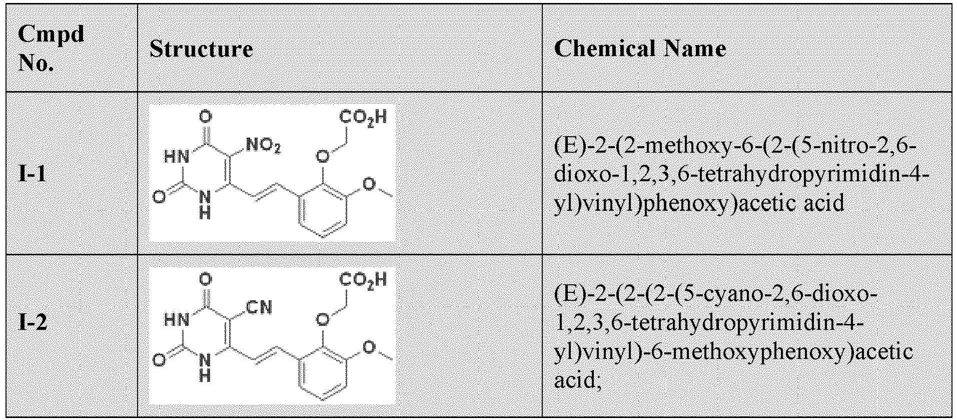

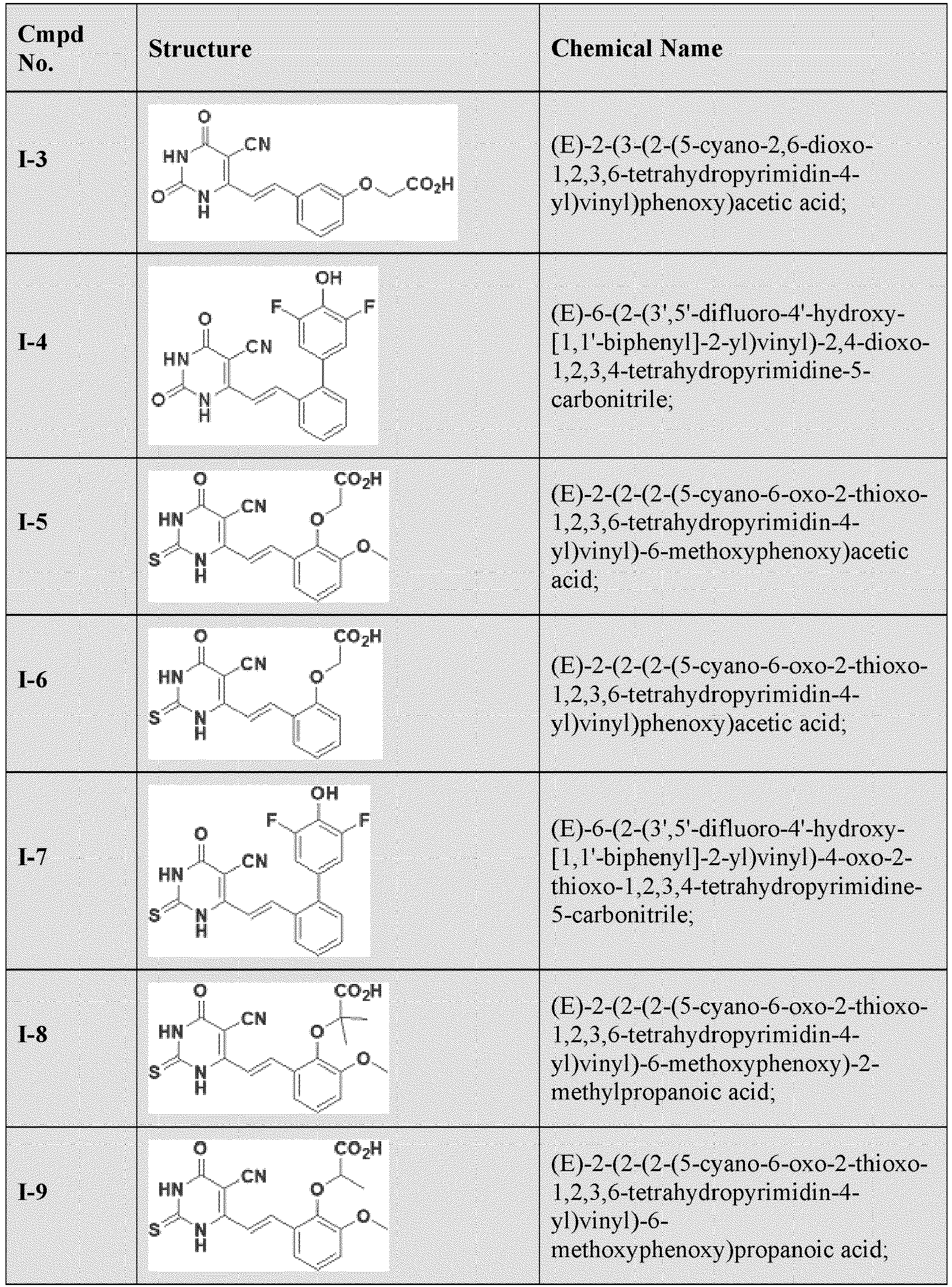

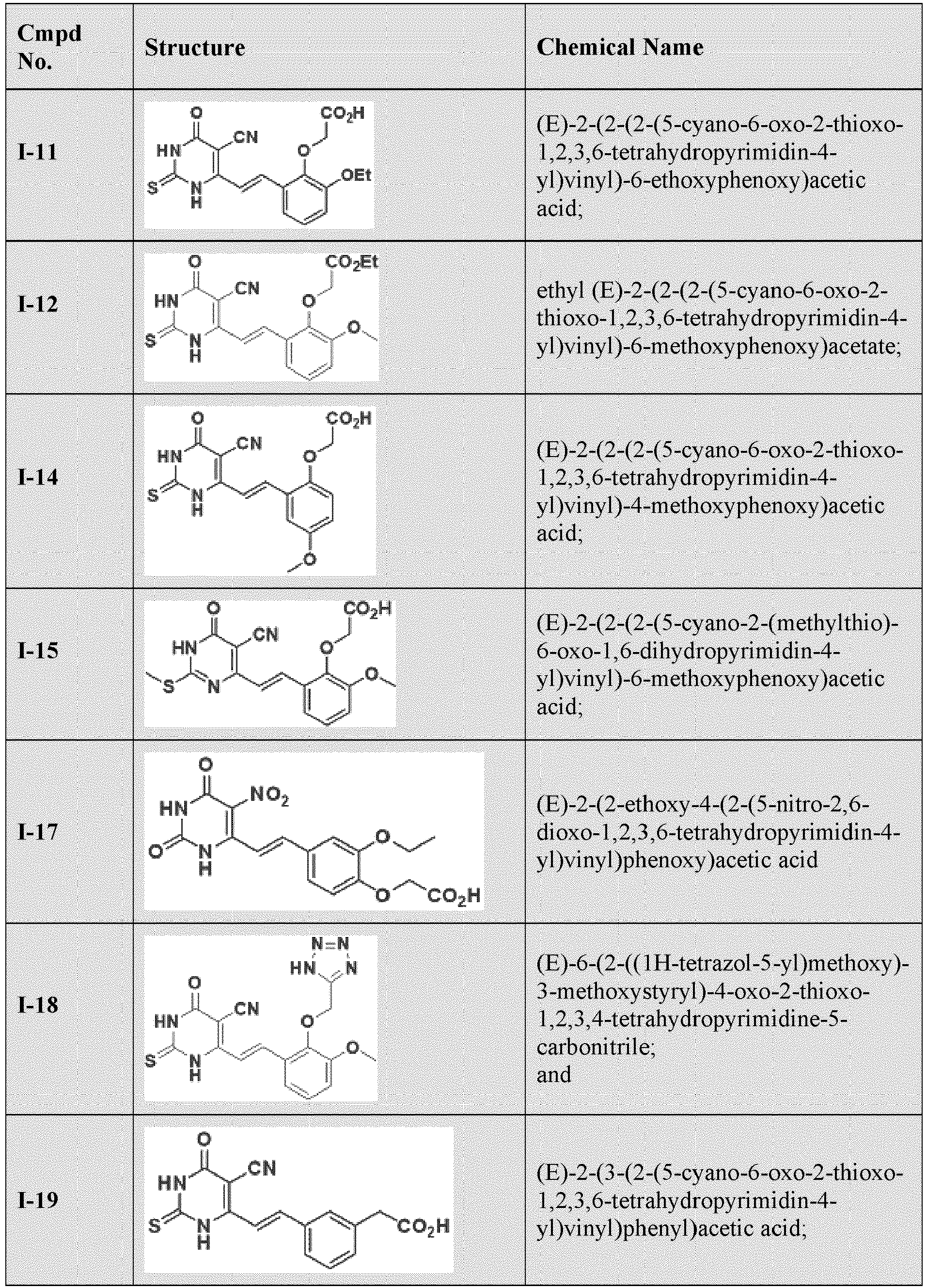

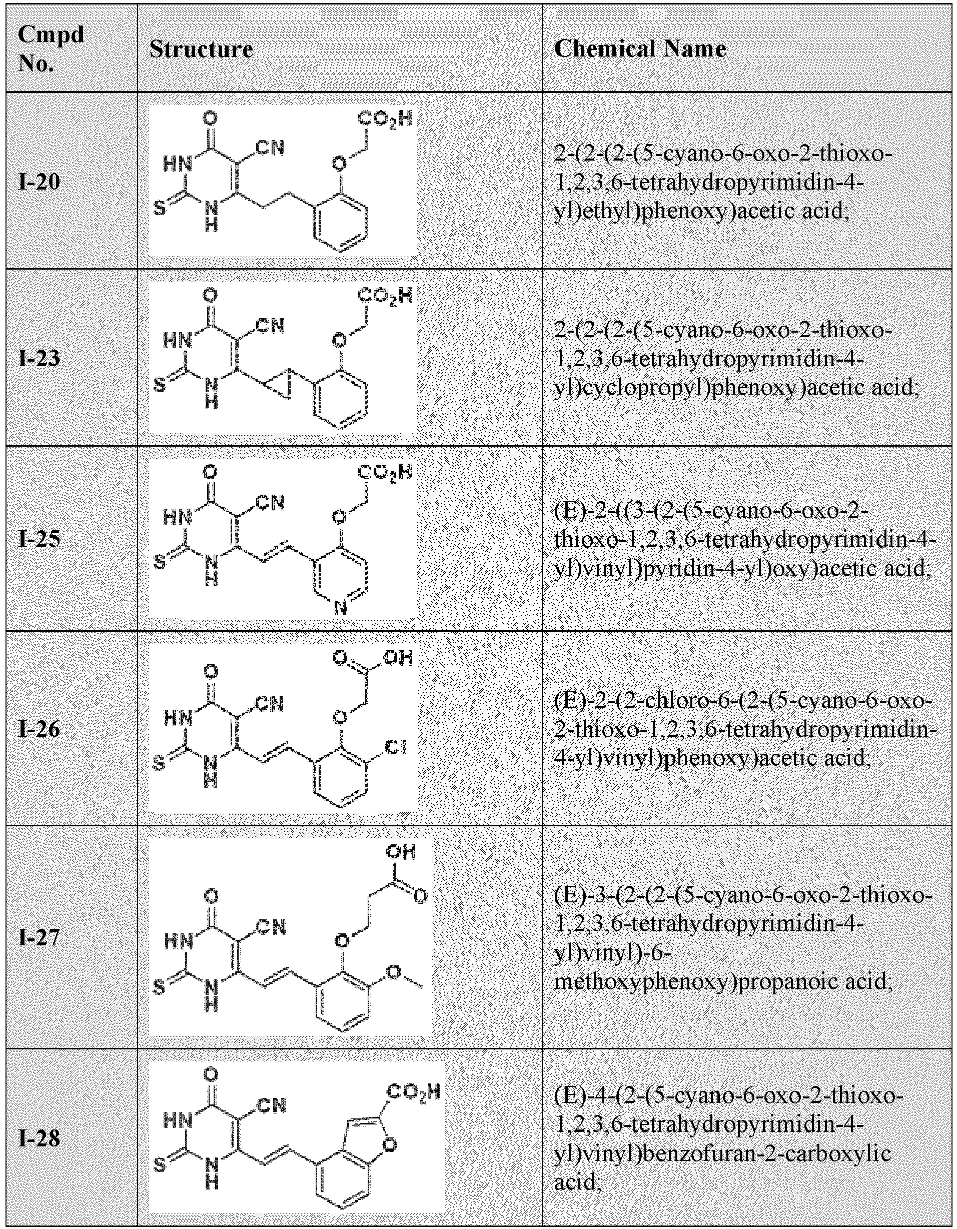

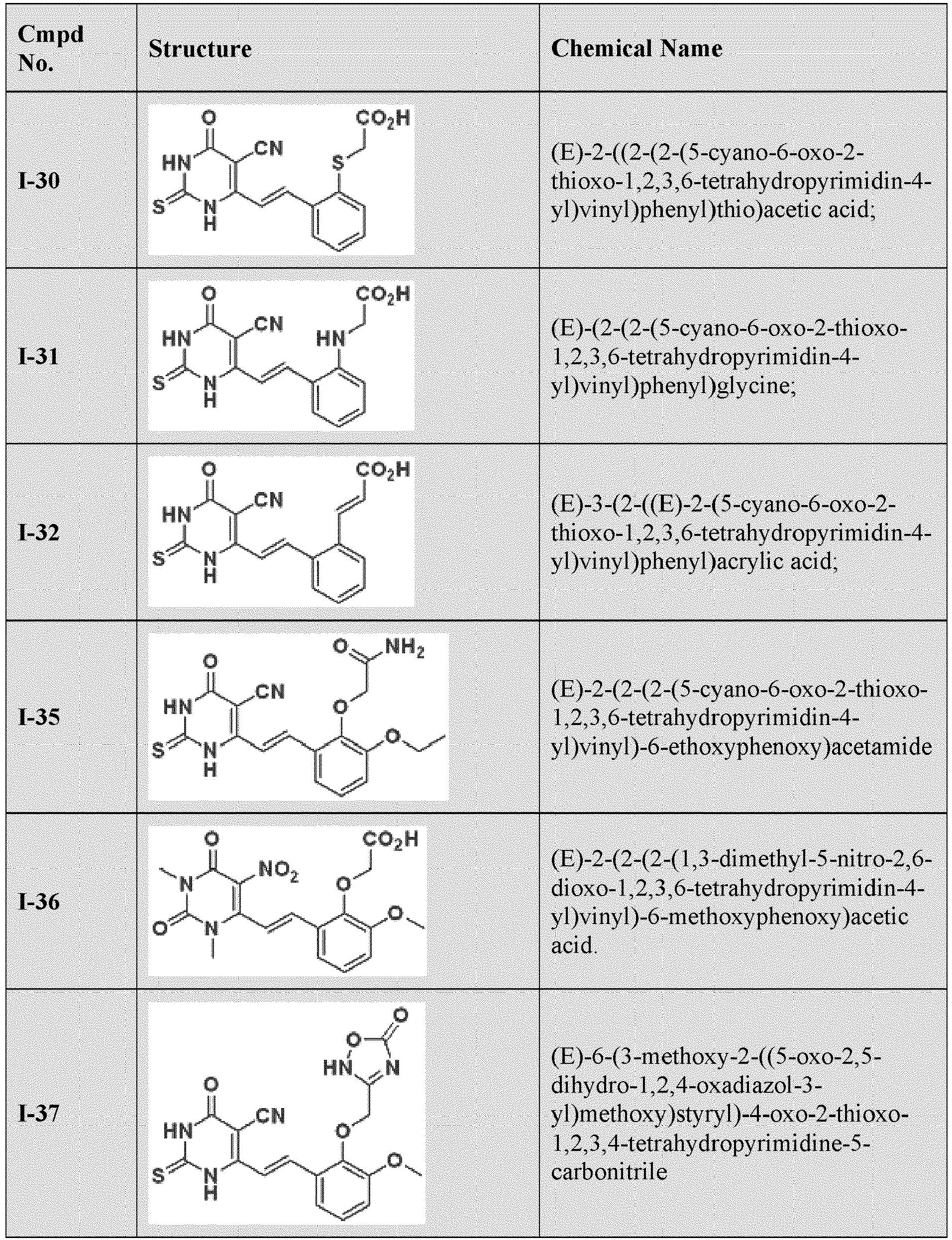

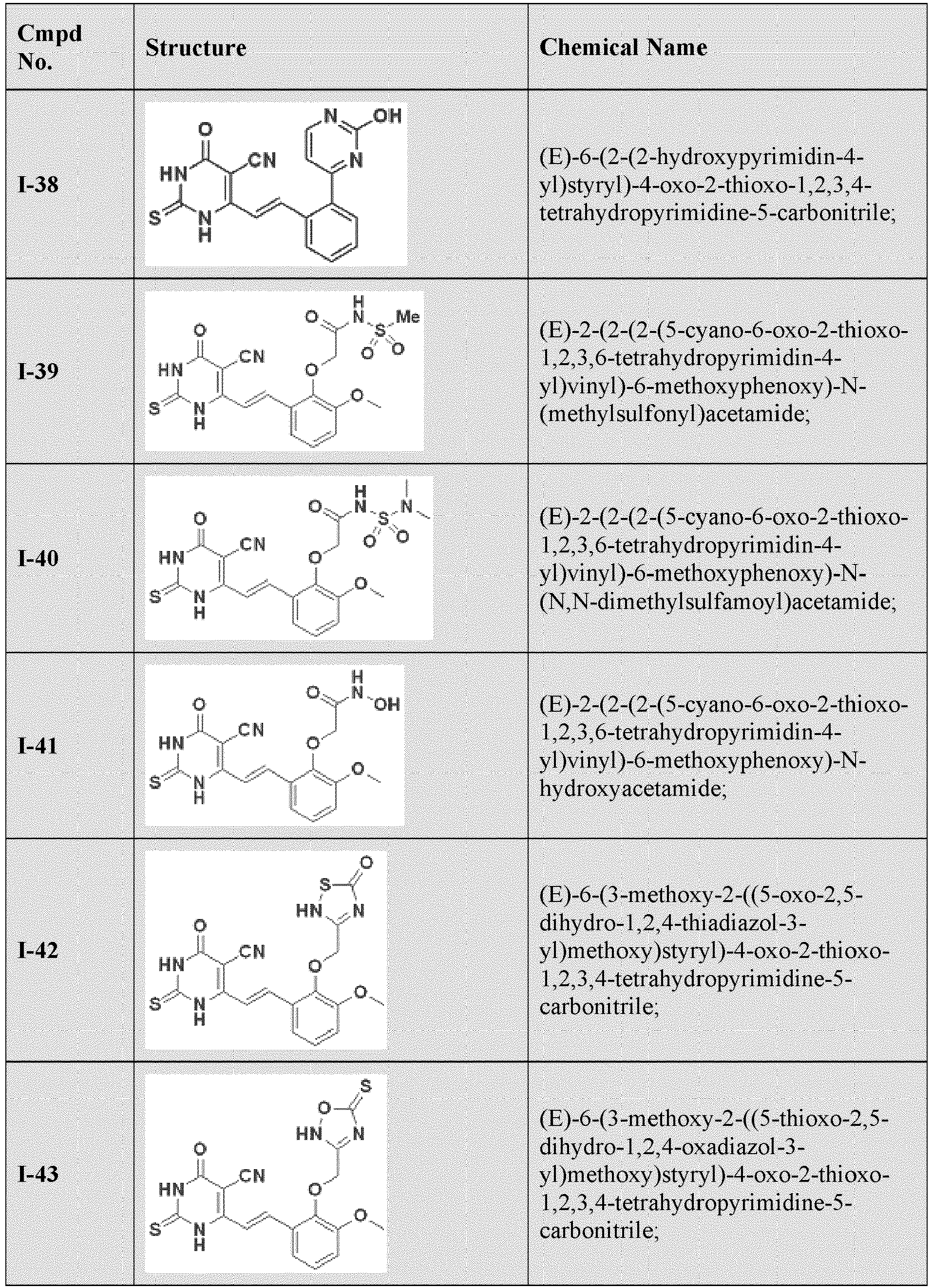

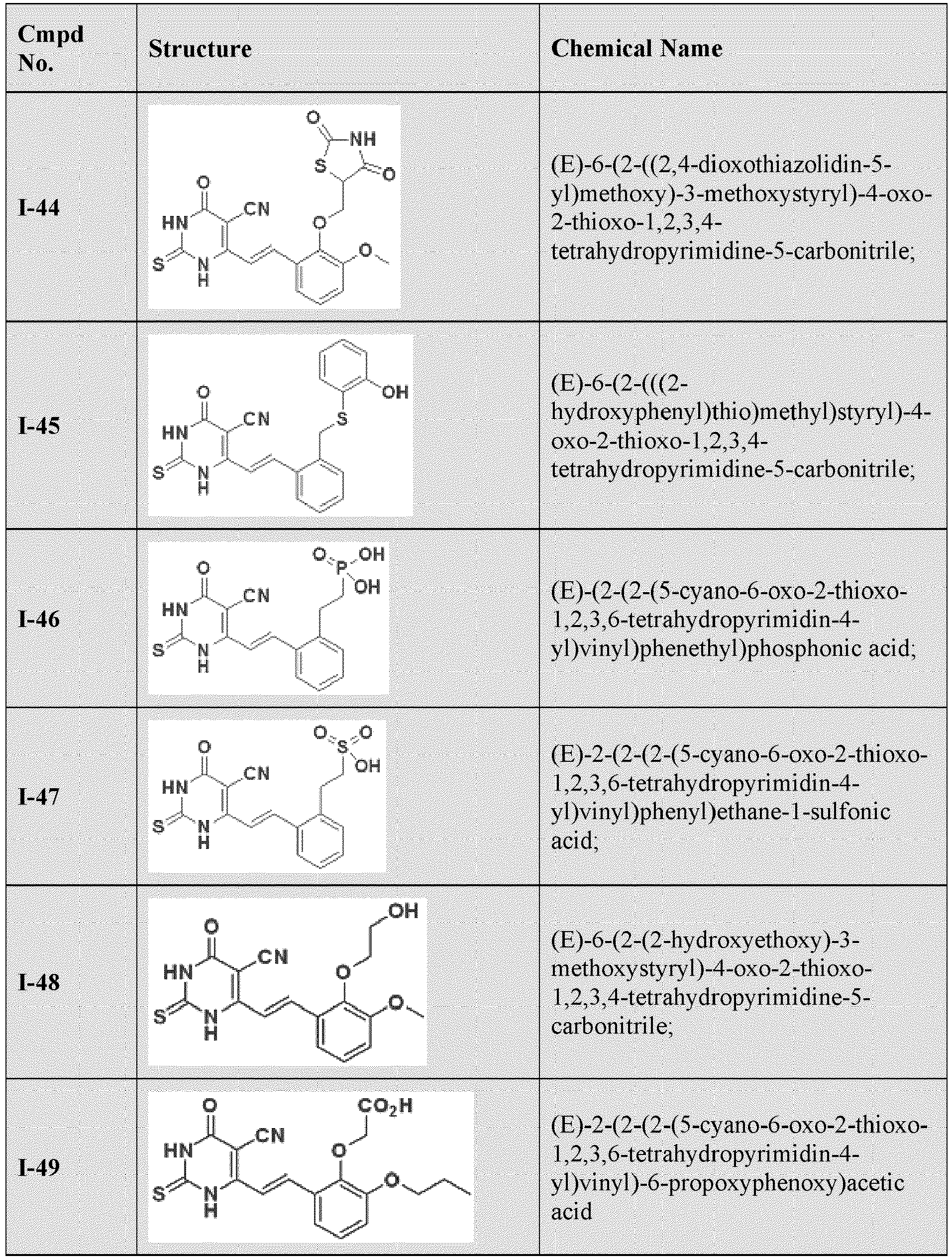

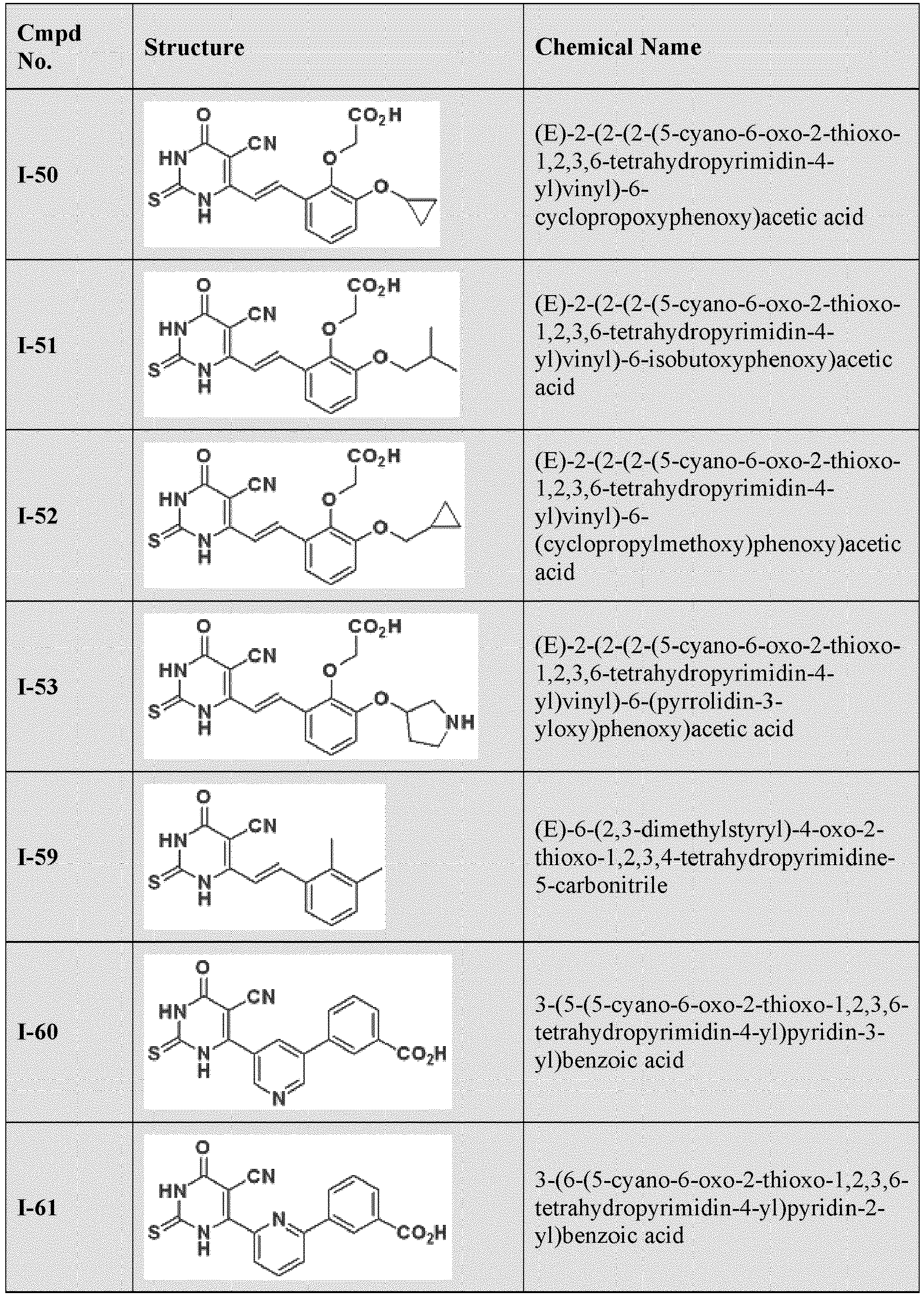

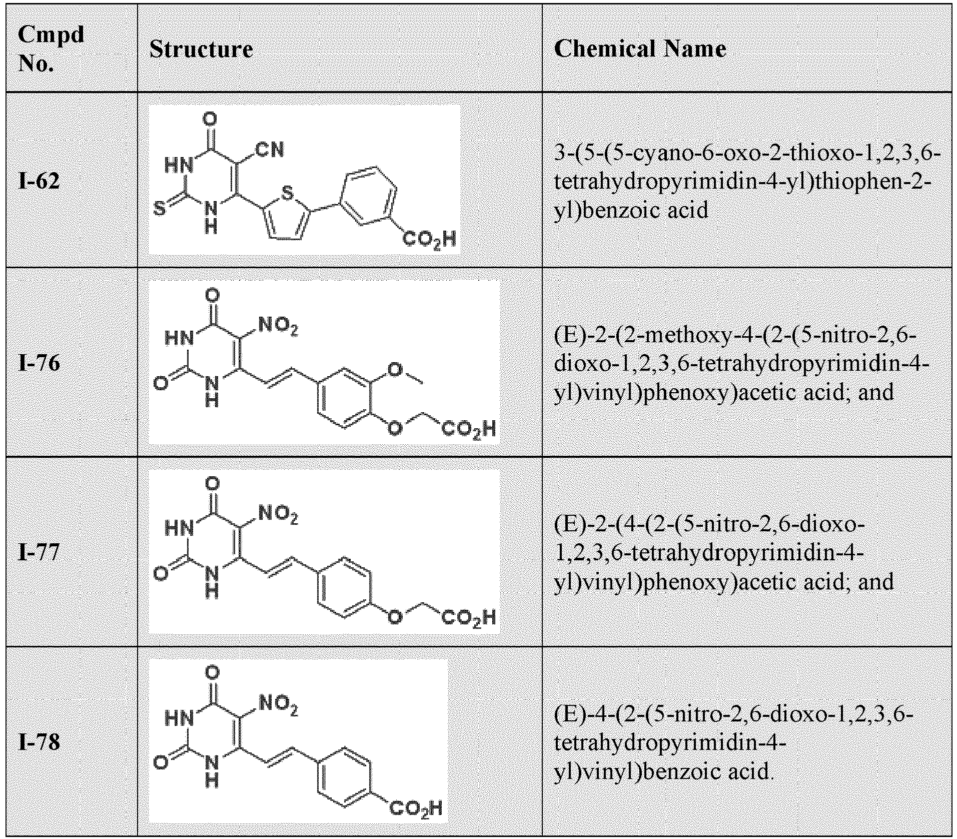

## Patentansprüche

1. Verbindung, repräsentiert durch Formel (I): 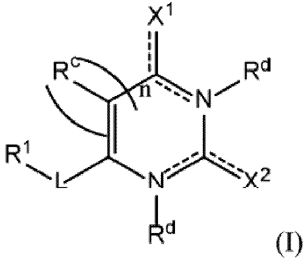 oder ein pharmazeutisch annehmbares Salz oder Tautomer davon,
wobei:
X¹ O, S, OR² oder SH ist;
X² O, S, OR² oder SR² ist;
L -(CH₂)ₘCH=CH(CH₂)ₚ-, -(CH₂)*ₒ*-, 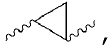 -(CH₂)ₘC=(O)NR³(CH₂)ₚ-, -(CH₂)ₘNR³C=(O)(CH₂)ₚ-, Pyridinyl oder Thiophenyl ist;
Y² O, NH oder S ist;
R¹ C₆-C₁₀-Aryl oder -Heteroaryl ist, wobei das Heteroaryl ein oder zwei 5- bis 7-gliedrige Ringe und 1-4 Heteroatome, ausgewählt aus N, O und S, umfasst, und wobei das Aryl und Heteroaryl mit R^{a} und R^{b} substituiert sind, und optional mit einem bis zwei R^{e} substituiert sind;
R² H oder C₁-C₄-Alkyl ist;
R³ H oder C₁-C₄-Alkyl ist;
R^{a} H, C₁-C₄-Alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣ-C₆-C₁₀-Aryl, -(C(R^{f})₂)ᵣS-C₆-C₁₀-Aryl, -(C(R^{f})₂)ᵣ-Heteroaryl, -O(C(R^{f})₂)ᵣ-Heteroaryl, -O(C(R^{f})₂)ᵣ-Heterocycloalkyl, -O(C(R^{f})₂)ᵣ(C₃-C₇)-Cycloalkyl, -(C(R^{f})₂)ᵣP(O)(OH)OR^{x}, -O(C(R^{f})₂)ᵣP(O)(OH)OR^{x}, - (C-(R^{f})₂)ᵣS(O)₂OH, -O(C(R^{f})₂)ᵣS(O)₂OH, -(C(R^{f})₂)ᵣP(O)₂OH, -O-(C(R^{f})₂)ᵣP(O)₂OH, -O(C(R^{f})₂)ᵣOH, -OR^{y}, -(C(R^{f})₂)ᵣC(O)NHCN, -CH=CHCO₂R^{x} oder -(C(R^{f})₂)ᵣC(O)NHS(O)₂-Alkyl ist, wobei das Aryl und das Heteroaryl optional mit einem bis drei Substituenten substituiert sind, die jeweils unabhängig voneinander ausgewählt sind aus Halogen und OH, und wobei das Heterocycloalkyl mit einem bis zwei =O oder =S substituiert ist;
R^{b}C₁-C₄-Alkyl, -(C(R^{f})₂)ᵣCO₂R^{x}, -Y²(C(R^{f})₂)ᵣCO₂R^{x}, -O-(C(R^{f})₂)ᵣC(O)NHR^{g}, -(C(R^{f})₂)ᵣ-C₆-C₁₀-Aryl, -(C(R^{f})₂)ᵣS-C₆-C₁₀-Aryl, -(C(R^{f})₂)ᵣ-Heteroaryl, -O(C(R^{f})₂)ᵣ-Heteroaryl, -O(C(R^{f})₂)ᵣ-Heterocycloalkyl, -(C(R^{f})₂)ᵣP(O)(OH)OR^{x}, -O(C-(R^{f})₂)ᵣP(O)(OH)OR^{x}, -(C(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -(C-(R^{f})₂)ᵣC(O)NHCN, -CH=CHCO₂R^{x} oder -(C(R^{f})₂)ᵣC(O)NHS(O)₂-Alkyl ist, wobei das Aryl und das Heteroaryl mit einem bis drei Substituenten, ausgewählt aus Halogen und OH, substituiert sind, und wobei das Heterocycloalkyl mit einem bis zwei =O oder =S substituiert ist; oder
R^{a} und R^{b}, wenn sie auf benachbarten Atomen befindlich sind, zusammen mit den Atomen, an die sie gebunden sind, einen C₆-C₁₀-Arylring bilden, der optional mit einem oder mehreren -CO₂H substituiert ist; R^{a} und R^{b}, wenn sie auf benachbarten Atomen befindlich sind, zusammen mit den Atomen, an die sie gebunden sind, einen 5- bis 6-gliedrigen Heteroarylring, der optional mit einem oder mehreren -CO₂H substituiert ist, bilden;
R^{c} -CN ist;
jedes R^{d} unabhängig bei jedem Vorkommen abwesend oder H ist;
jedes R^{e} unabhängig bei jedem Vorkommen C₁-C₆-Alkyl, C₂-C₆-Alkenyl, C₂-C₆-Alkinyl, Halogen, C₁-C₆-Haloalkyl, -NHR^{z}, -OH oder -CN ist;
jedes R^{f} unabhängig H oder C₁-C₆-Alkyl ist;
R^{g} H, C₁-C₆-Alkyl, OH, -S(O)₂(C₁-C₆-Alkyl) oder -S(O)₂N(C₁-C₆-Alkyl)₂ ist;
R^{x} H oder C₁-C₆-Alkyl ist;
jedes R^{y} und R^{z} unabhängig H, C₁-C₆-Alkyl oder C₁-C₆-Haloalkyl ist;
jedes m, p und r unabhängig 0, 1 oder 2 ist;
n 1 ist;
∘ 1, 2, 3 oder 4 ist; und
die gepunktete Linie eine optionale Doppelbindung ist.

2. Verbindung nach Anspruch 1, wobei:
X¹ O oder OR² ist;
X² S oder OR² ist;
L -(CH₂)ₘCH=CH(CH₂)ₚ-, -(CH₂)*ₒ*-, 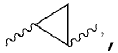 -(CH₂)ₘC=(O)NR³(CH₂)ₚ- oder -(CH₂)ₘNR³C=(O)(CH₂)ₚ- ist;
Y² O, NH oder S ist;
R¹ C₆-C₁₀-Aryl oder -Heteroaryl ist, wobei das Heteroaryl ein oder zwei 5- bis 7-gliedrige Ringe und 1-4 Heteroatome ausgewählt aus N, O und S umfasst, und wobei das Aryl und Heteroaryl mit R^{a} und R^{b} substituiert sind, und optional mit einem bis zwei R^{e} substituiert sind;
R² H oder C₁-C₄-Alkyl ist;
R^{a} H, -(C(R^{f})₂)ᵣCO₂R^{x}, -Y²(C(R^{f})₂)ᵣCO₂R^{x}, -O(C(R^{f})₂)ᵣ-C(O)NHR^{g}, -(C(R^{f})₂)ᵣS-C₆-C₁₀-Aryl, -(C(R^{f})₂)ᵣ-Heteroaryl, - O(C(R^{f})₂)ᵣ-Heteroaryl, -O(C(R^{f})₂)ᵣ-Heterocycloalkyl, -(C-(R^{f})₂)ᵣP(O)₂OH, -(C(R^{f})₂)ᵣS(O)₂OH, -O(C(R^{f})₂)ᵣOH, -OR^{y} oder -CH=CHCO₂R^{x} ist, wobei das Aryl und das Heteroaryl optional mit einem bis drei Substituenten substituiert sind, die jeweils unabhängig voneinander ausgewählt sind aus Halogen und OH, und wobei das Heterocycloalkyl mit einem bis zwei =O oder =S substituiert ist;
R^{b} -(C(R^{f})₂)ᵣCO₂R^{x}, -Y²(C(R^{f})₂)ᵣCO₂R^{x}, -O(C(R^{f})₂)ᵣC(O)-NHR^{g}, -(C(R^{f})₂)ᵣS-C₆-C₁₀-Aryl, -(C(R^{f})₂)ᵣ-Heteroaryl, -O(C(R^{f})₂)ᵣ-Heteroaryl, -O(C(R^{f})₂)ᵣ-Heterocycloalkyl, -(C(R^{f})₂)ᵣP(O)₂OH, -(C(R^{f})₂)ᵣS(O)₂OH, -O(C(R^{f})₂)ᵣOH oder -CH=CHCO₂R^{x} ist, wobei das Aryl und Heteroaryl mit einem bis drei Substituenten, ausgewählt aus Halogen und OH, substituiert sind, und wobei das Heterocycloalkyl mit einem bis zwei =O oder =S substituiert ist; oder
R^{a} und R^{b}, wenn sie auf benachbarten Atomen befindlich sind, zusammen mit den Atomen, an die sie gebunden sind, einen C₆-C₁₀-Arylring bilden, der optional mit einem oder mehreren -CO₂H substituiert ist; R^{a} und R^{b}, wenn sie auf benachbarten Atomen befindlich sind, zusammen mit den Atomen, an die sie gebunden sind, einen 5- bis 6-gliedrigen Heteroarylring, der optional mit einem oder mehreren -CO₂H substituiert ist, bilden;
R^{c} -CN ist;
jedes R^{d} unabhängig bei jedem Vorkommen abwesend oder H ist;
jedes R^{e} unabhängig bei jedem Vorkommen C₁-C₆-Alkyl, C₂-C₆-Alkenyl, C₂-C₆-Alkinyl, Halogen, C₁-C₆-Haloalkyl, -NHR^{z}, -OH oder -CN ist;
jedes R^{f} unabhängig H oder C₁-C₆-Alkyl ist;
R^{g} H, C₁-C₆-Alkyl, OH, -S(O)₂(C₁-C₆-Alkyl) oder -S(O)₂N(C₁-C₆-Alkyl)₂ ist;
R^{x} H oder C₁-C₆-Alkyl ist;
jedes R^{y} und R^{z} unabhängig H, C₁-C₆-Alkyl oder C₁-C₆-Haloalkyl ist;
jedes m, p und r unabhängig 0, 1 oder 2 ist;
n 1 ist;
∘ 1, 2, 3 oder 4 ist; und
die gepunktete Linie eine optionale Doppelbindung ist.

3. Verbindung nach Anspruch 1, wobei:
X¹ O ist;
X² O, S oder SR² ist;
L -(CH₂)ₘCH=CH(CH₂)ₚ- ist;
Y² O, NH oder S ist;
R¹ C₆-C₁₀-Aryl, substituiert mit R^{a} und R^{b}, und optional substituiert mit einem bis zwei R^{e}, ist;
R² H oder C₁-C₄-Alkyl ist;
R^{a} H, -(C(R^{f})₂)ᵣCO₂R^{x}, -O(C(R^{f})₂)ᵣCO₂R^{x}, -(C(R^{f})₂)ᵣC₆-C₁₀-Aryl oder -OR^{y} ist, wobei das Aryl mit einem bis drei Substituenten, ausgewählt aus Halogen und OH, substituiert ist;
R^{b} -(C(R^{f})₂)ᵣCO₂R^{x}, -O(C(R^{f})₂)ᵣCO₂R^{x} oder -(C(R^{f})₂)ᵣC₆-C₁₀-Aryl ist, wobei das Aryl mit einem bis drei Substituenten, ausgewählt aus Halogen und OH, substituiert ist;
R^{c} -CN ist;
jedes R^{d} unabhängig bei jedem Vorkommen abwesend oder H ist;
jedes R^{e} unabhängig bei jedem Vorkommen C₁-C₆-Alkyl, C₂-C₆-Alkenyl, C₂-C₆-Alkinyl, Halogen, C₁-C₆-Haloalkyl, -NHR^{z}, -OH oder -CN ist;
jedes R^{f} unabhängig H oder C₁-C₆-Alkyl ist;
R^{x} H oder C₁-C₆-Alkyl ist;
jedes R^{y} und R^{z} unabhängig H, C₁-C₆-Alkyl oder C₁-C₆-Haloalkyl ist;
jedes m, p und r unabhängig 0, 1 oder 2 ist;
n 1 ist;
∘ 1, 2, 3 oder 4 ist; und
die gepunktete Linie eine optionale Doppelbindung ist.

4. Verbindung nach Anspruch 1, wobei:
X¹ O ist;
X² O, S oder SR² ist;
L -(CH₂)ₘCH=CH(CH₂)ₚ- ist;
Y² O, NH oder S ist;
R¹ C₆-C₁₀-Aryl, substituiert mit R^{a} und R^{b}, und optional substituiert mit einem bis zwei R^{e}, ist;
R² H oder C₁-C₄-Alkyl ist;
R^{a} H, -(C(R^{f})₂)ᵣCO₂R^{x}, -O(C(R^{f})₂)ᵣCO₂R^{x}, -(C(R^{f})₂)ᵣC₆-C₁₀-Aryl, -O(C(R^{f})₂)ᵣ-Heteroaryl oder -OR^{y} ist, wobei das Aryl mit einem bis drei Substituenten, ausgewählt aus Halogen und OH, substituiert ist;
R^{b} -(C(R^{f})₂)ᵣCO₂R^{x}, -O(C(R^{f})₂)ᵣCO₂R^{x}, -(C(R^{f})₂)ᵣC₆-C₁₀-Aryl oder -O(C(R^{f})₂)ᵣ-Heteroaryl ist, wobei das Aryl mit einem bis drei Substituenten, ausgewählt aus Halogen und OH, substituiert ist;
R^{c} -CN ist;
jedes R^{d} unabhängig bei jedem Vorkommen abwesend oder H ist;
jedes R^{e} unabhängig bei jedem Vorkommen C₁-C₆-Alkyl, C₂-C₆-Alkenyl, C₂-C₆-Alkinyl, Halogen, C₁-C₆-Haloalkyl, -NHR^{z}, -OH oder -CN ist;
jedes R^{f} unabhängig H oder C₁-C₆-Alkyl ist;
R^{x} H oder C₁-C₆-Alkyl ist;
jedes R^{y} und R^{z} unabhängig H, C₁-C₆-Alkyl oder C₁-C₆-Haloalkyl ist;
jedes m, p und r unabhängig 0, 1 oder 2 ist;
n 1 ist;
∘ 1, 2, 3 oder 4 ist; und
die gepunktete Linie eine optionale Doppelbindung ist.

5. Verbindung nach Anspruch 1, wobei die Verbindung repräsentiert wird durch die Formel (Ia), (Ib), (Ic), (Id), (Ie), (If), (Ig), (Ih), (Ii) oder (Ij): 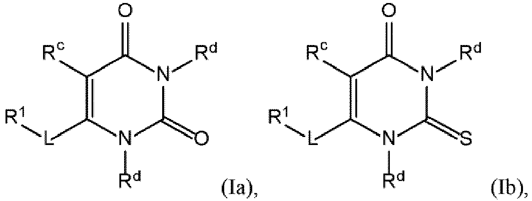 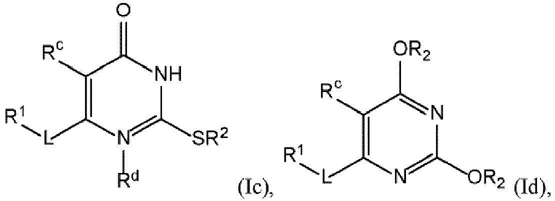 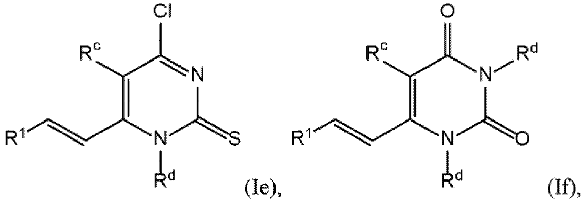 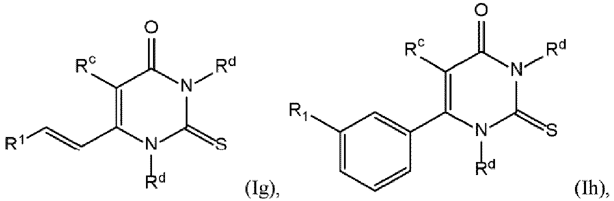 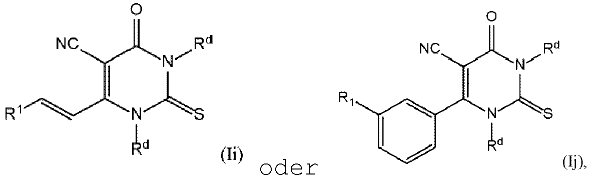 oder ein pharmazeutisch annehmbares Salz davon oder ein Tautomer davon.

6. Verbindung nach einem der Ansprüche 1 bis 5, wobei R¹ C₆-C₁₀-Aryl, substituiert mit R^{a} und R^{b}, und optional substituiert mit einem bis zwei R^{e}, ist.

7. Verbindung nach einem der Ansprüche 1 bis 5, wobei R¹ Phenyl, substituiert mit R^{a} und R^{b}, und optional substituiert mit einem bis zwei R^{e}, ist.

8. Verbindung nach einem der Ansprüche 1 bis 5, wobei R¹ Heteroaryl ist, das einen 5- bis 7-gliedrigen Ring und 1-4 Heteroatome, ausgewählt aus N, O und S, umfasst und mit R^{a} und R^{b} substituiert ist, und optional mit einem bis zwei R^{e} substituiert ist.

9. Verbindung nach einem der Ansprüche 1 bis 5, wobei, wobei R¹ Pyridinyl, substituiert mit R^{a} und R^{b}, und optional substituiert mit einem bis zwei R^{e} ist.

10. Verbindung nach einem der Ansprüche 1 bis 9, wobei R^{a} H ist und R^{b} -(C(R^{f})₂)ᵣCO₂R^{x}, -O(C(R^{f})₂)ᵣCO₂R^{x} oder - (C(R^{f})₂)ᵣ-C₆-C₁₀-Aryl ist.

11. Verbindung nach einem der Ansprüche 1 bis 9, wobei R^{a} H ist und R^{b} -CO₂H, -CH₂CO₂H, -OCH₂CO₂R^{x}, -OCH(CH₃)CO₂R^{x}, -OC(CH₃)₂CO₂R^{x} oder 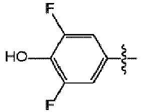 ist.

12. Verbindung nach einem der Ansprüche 1 bis 9, wobei R^{a}OR^{y} ist und R^{b} -O(C(R^{f})₂)ᵣCO₂R^{x} oder -O(C(R^{f})₂)ᵣ-Heteroaryl ist.

13. Verbindung nach einem der Ansprüche 1 bis 9, wobei R^{a} H ist und R^{b} -(C(R^{f})₂)ᵣCO₂R^{x}ᵣ, -Y²(C(R^{f})₂)ᵣCO₂R^{x}, -(C-(R^{f})₂)ᵣS-C₆-C₁₀-Aryl, -(C(R^{f})₂)ᵣ-Heteroaryl, -(C(R^{f})₂)ᵣ-P(O)₂OH, -(C(R^{f})₂)ᵣS(O)₂OH, oder -CH=CHCO₂R^{x} ist, wobei das Aryl und Heteroaryl mit einem bis drei Substituenten, ausgewählt aus Halogen und OH, substituiert sind; oder R^{a} und R^{b}, wenn sie auf benachbarten Atomen befindlich sind, zusammen mit den Atomen, an die sie gebunden sind, einen C₆-C₁₀-Arylring, optional substituiert mit einem oder mehreren -CO₂H, bilden; R^{a} und R^{b}, wenn sie auf benachbarten Atomen befindlich sind, zusammen mit den Atomen, an die sie gebunden sind, einen 5- bis 6-gliedrigen Heteroarylring, optional substituiert mit einem oder mehreren -CO₂H, bilden.

14. Verbindung nach einem der Ansprüche 1-13, aufweisend eine der folgenden Formeln:

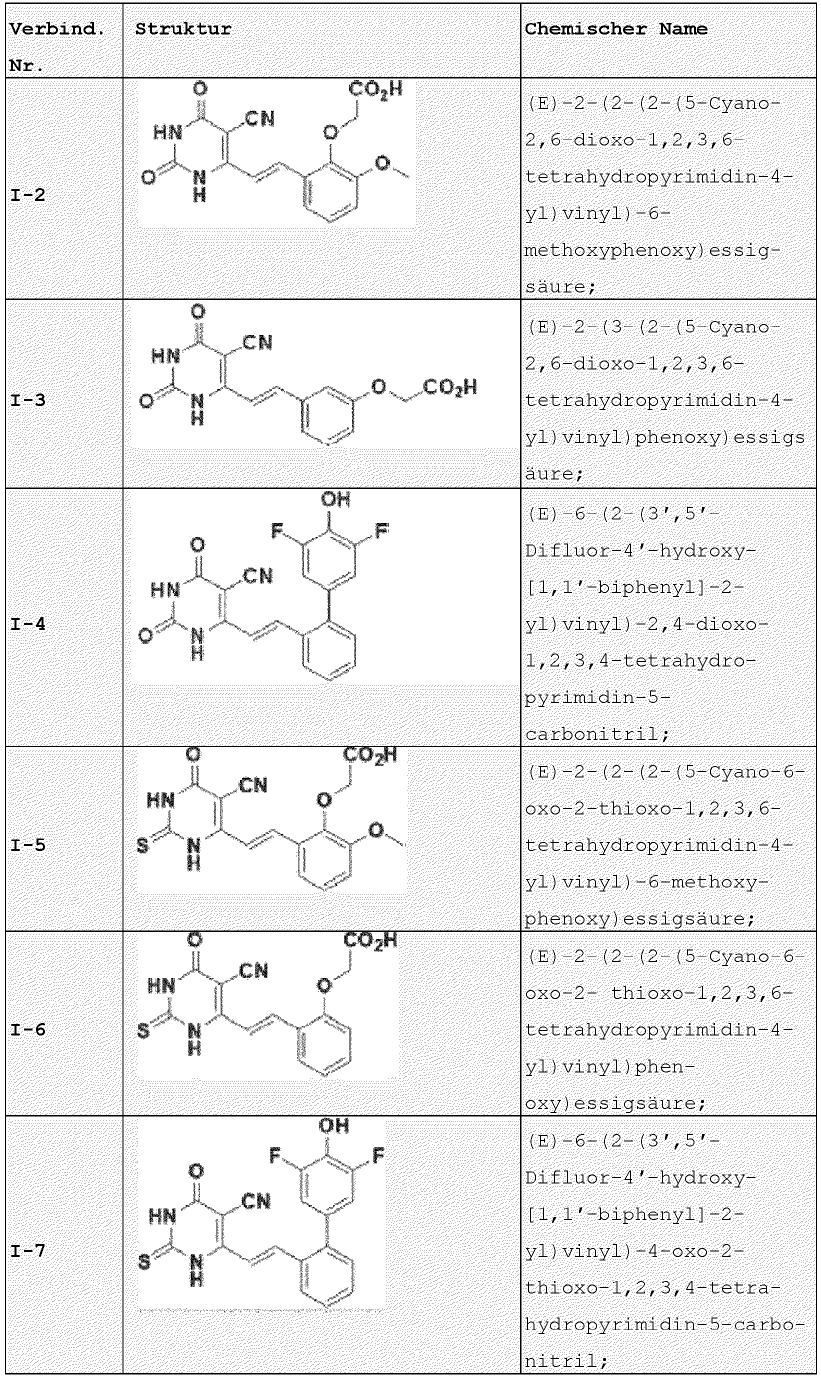

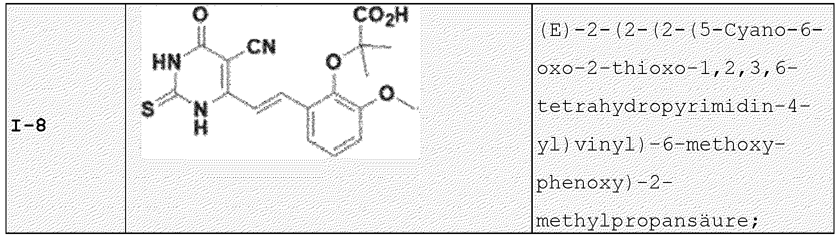

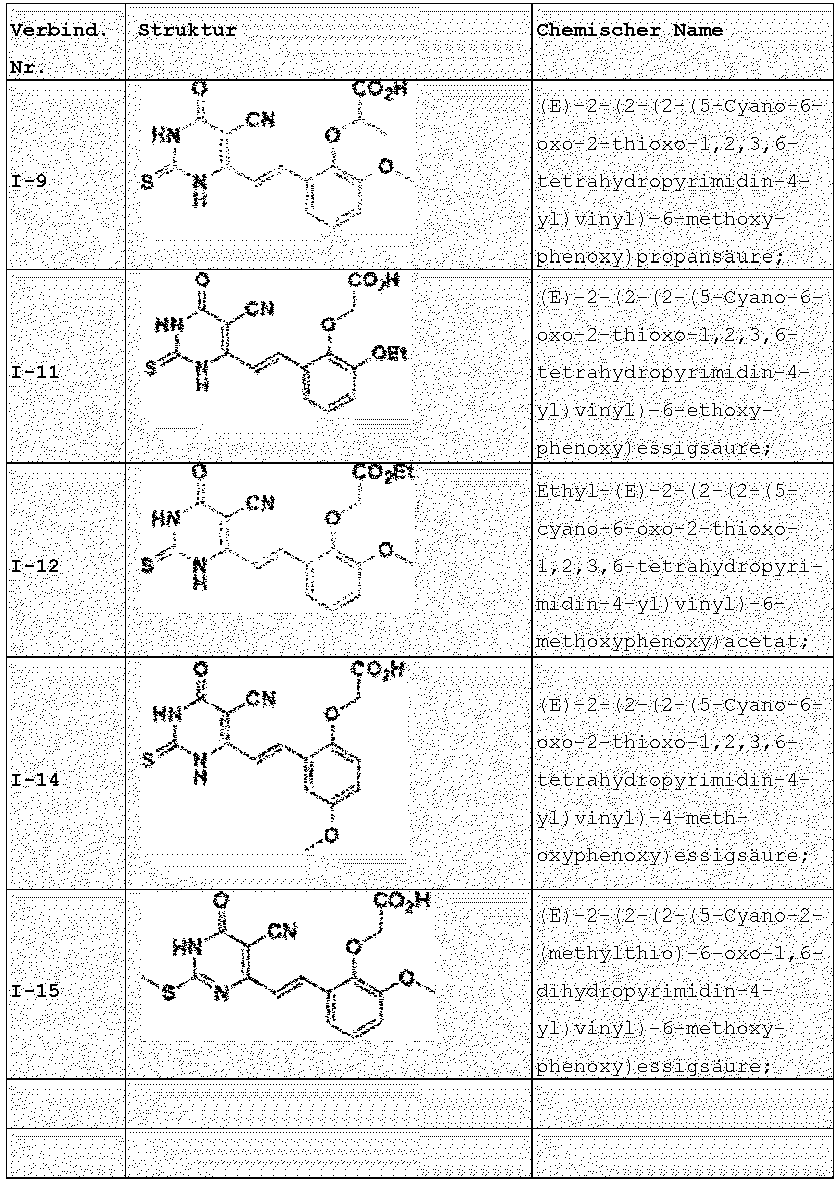

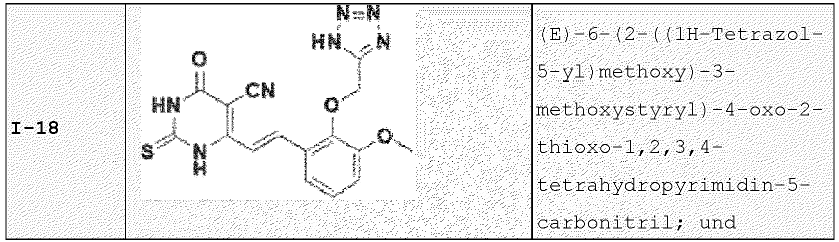

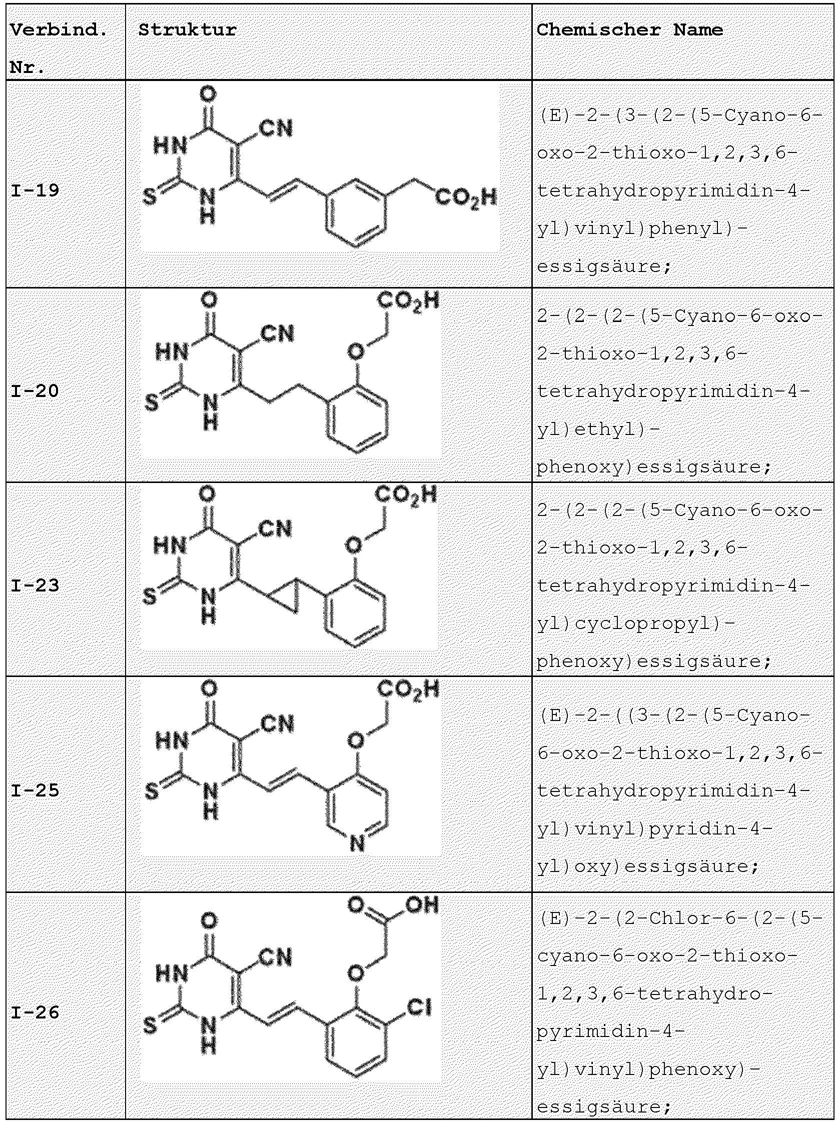

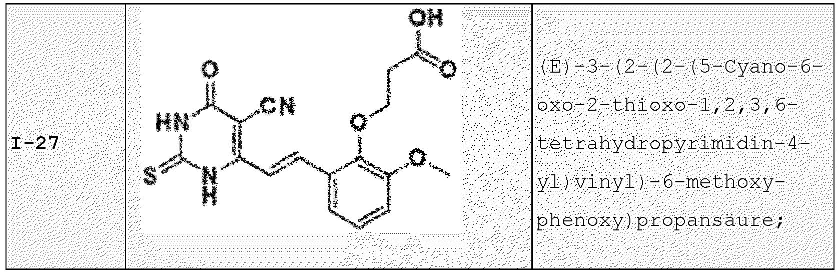

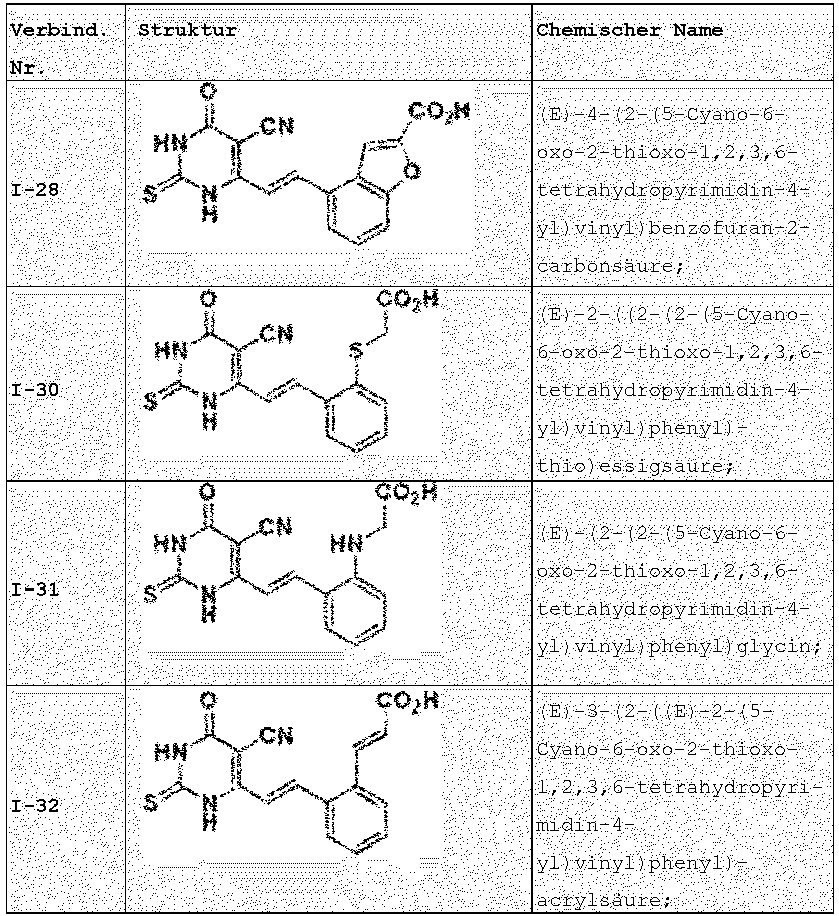

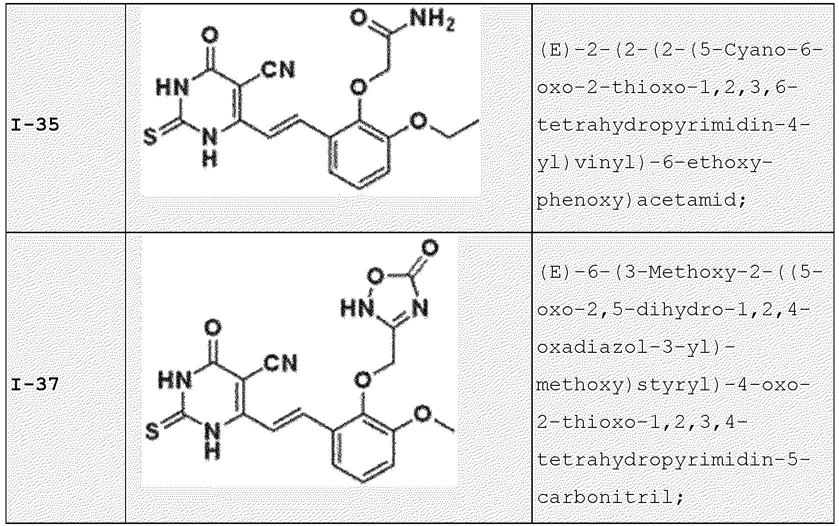

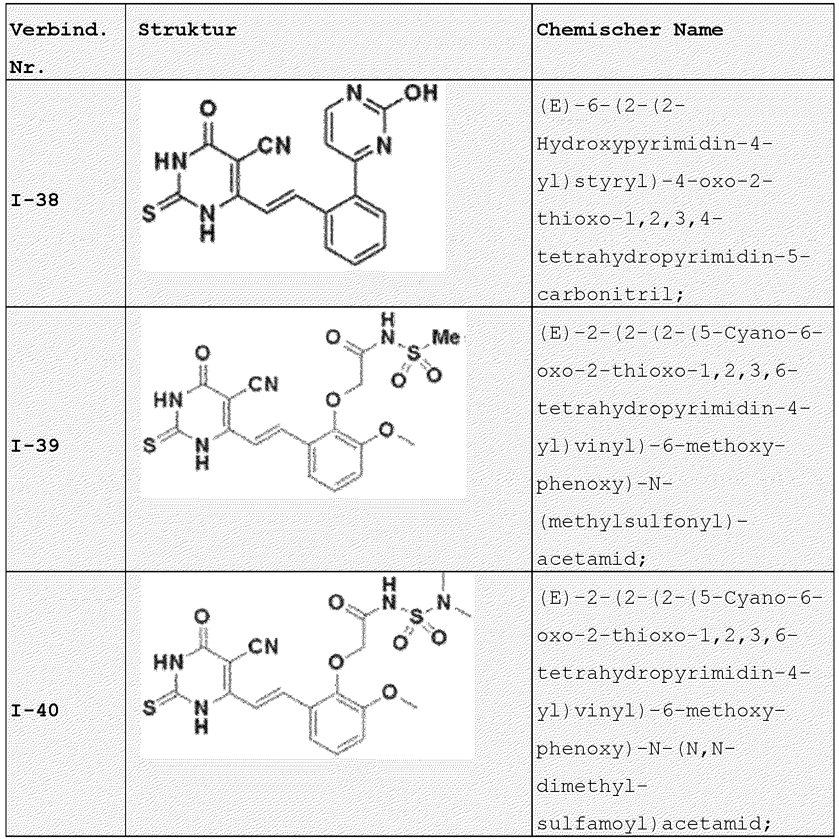

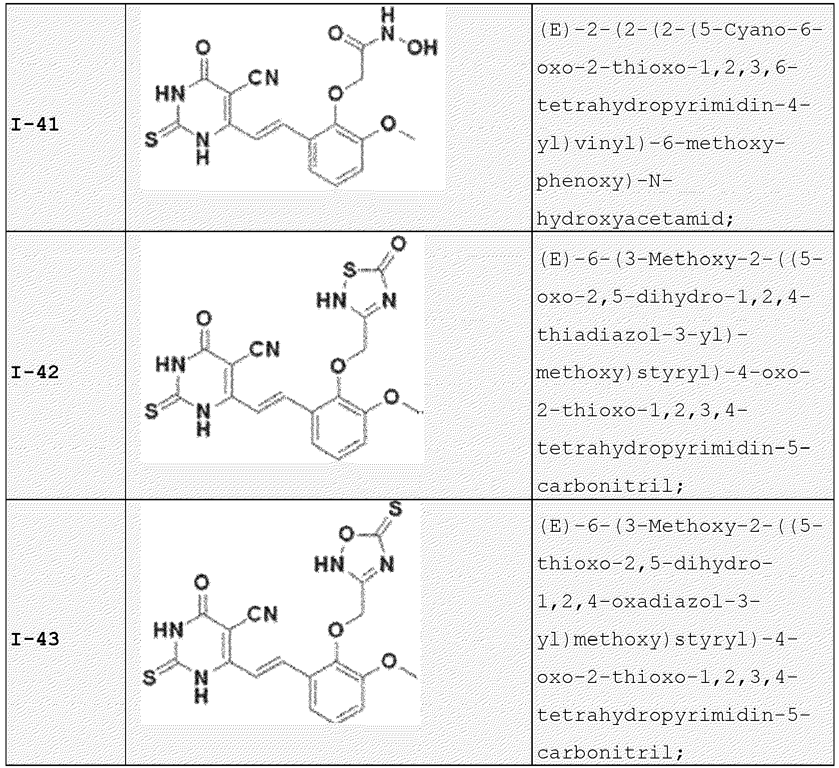

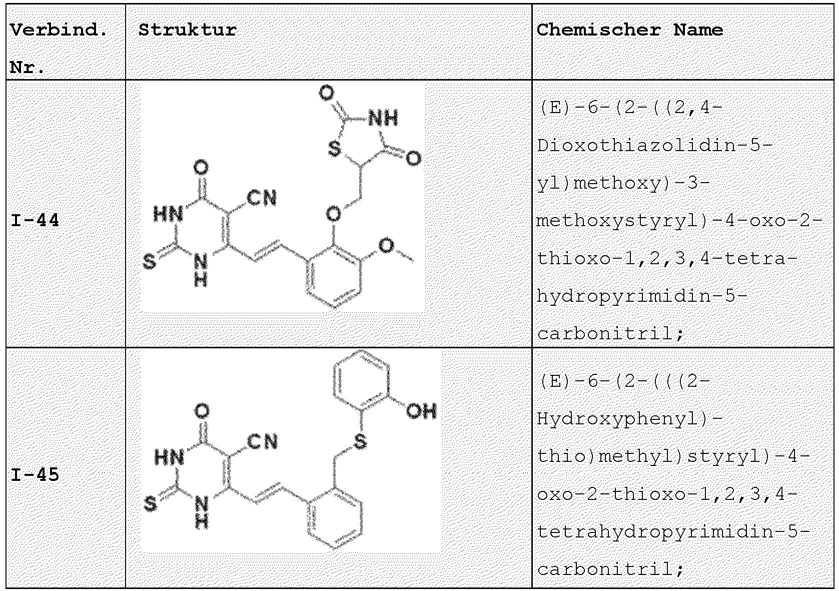

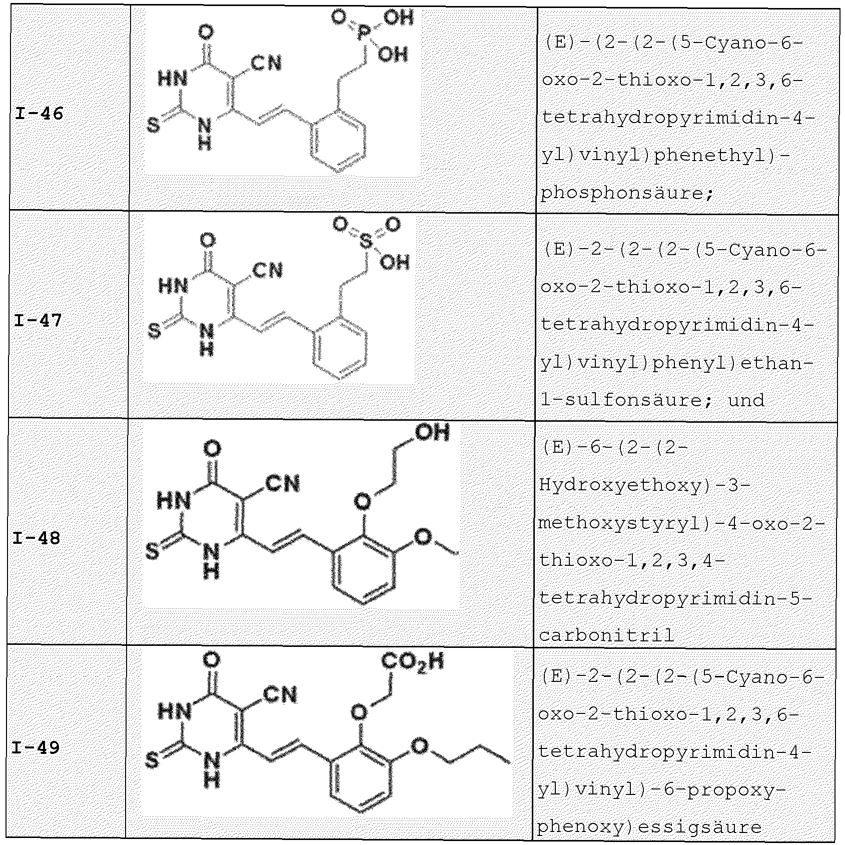

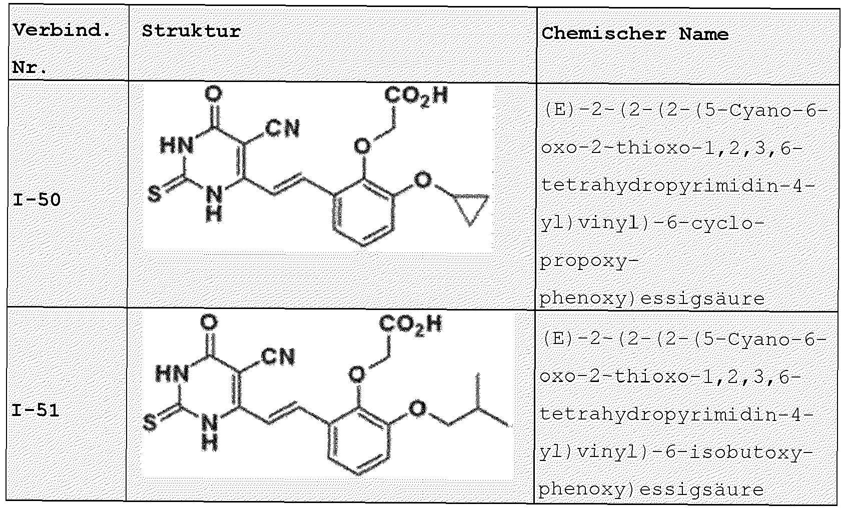

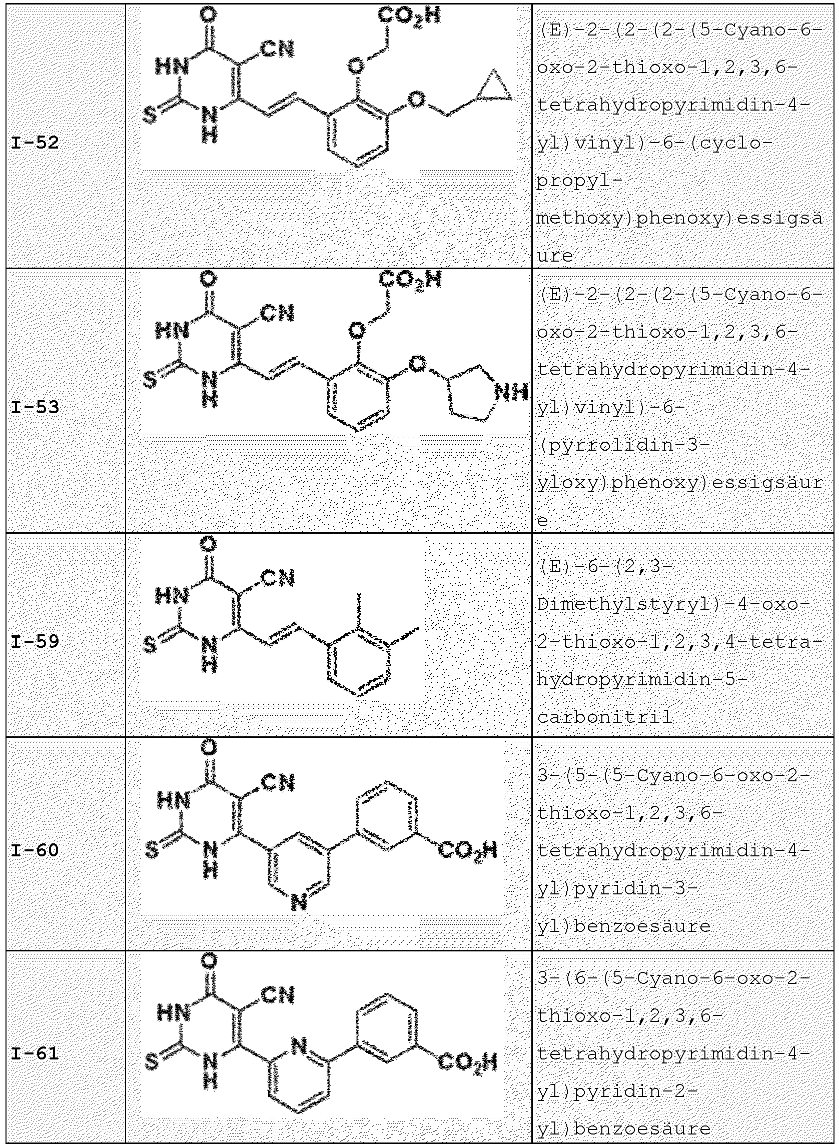

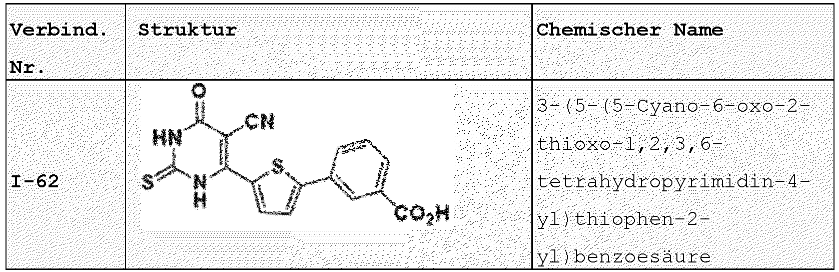

15. Pharmazeutische Zusammensetzung, umfassend eine Verbindung nach einem der Ansprüche 1 bis 14 oder ein pharmazeutisch annehmbares Salz davon und mindestens eines von einem pharmazeutisch annehmbaren Träger, Verdünnungsmittel oder Exzipienten,
wobei optional die Zusammensetzung ein oder mehrere weitere therapeutische Mittel umfasst.

16. Verbindung nach einem der Ansprüche 1-14 oder pharmazeutische Zusammensetzung nach Anspruch 15 zur Verwendung in einem Verfahren zum Behandeln, Verhindern oder Reduzieren des Risikos einer Erkrankung oder Störung, die mit α-Amino-β-carboxymuconat-ε-semialdehyd-Decarboxylase (ACMSD)-Dysfunktion assoziiert ist, wobei das Verfahren das Verabreichen einer therapeutisch wirksamen Menge der Verbindung an das Subjekt, das an einer mit ACMSD-Dysfunktion assoziierten Erkrankung oder Störung leidet oder für eine Entwicklung derselben anfällig ist, umfasst,
wobei die Erkrankung eine chronische Lebererkrankung ist, ausgewählt aus primärer biliärer Zirrhose (PBC), zerebrotendinöser Xanthomatose (CTX), primärer sklerosierender Cholangitis (PSC), arzneimittelinduzierter Cholestase, intrahepatischer Schwangerschaftscholestase, parenteraler ernährungsassoziierter Cholestase (PNAC), mit bakterieller Überwucherung oder Sepsis assoziierter Cholestase, Autoimmunhepatitis, chronischer Virushepatitis, alkoholischer Lebererkrankung, nichtalkoholischer Fettlebererkrankung (NAFLD), nichtalkoholischer Steatohepatitis (NASH), Lebertransplantat-assoziierter Graft-versus-Host-Erkrankung, Lebendspendetransplantat-Leberregeneration, angeborener Leberfibrose, Choledocholithiasis, granulomatöser Lebererkrankung, intra- oder extrahepatischer Malignität, Sjögren-Syndrom, Sarkoidose, Morbus Wilson, Morbus Gaucher, Hämochromatose und Alpha-1-Antitrypsin-Mangel.

17. Verbindung nach einem der Ansprüche 1-14 oder pharmazeutische Zusammensetzung nach Anspruch 15 zur Verwendung in einem Verfahren zum Behandeln, Verhindern oder Reduzieren des Risikos einer Erkrankung oder Störung, die mit verringerten Nicotinamidadenindinukleotid (NAD⁺)-Spiegeln assoziiert ist, wobei das Verfahren das Verabreichen einer therapeutisch wirksamen Menge der Verbindung an das Subjekt, das an einer mit verringerten NAD⁺-Spiegeln assoziierten Erkrankung oder Störung leidet oder für eine Entwicklung derselben anfällig ist, umfasst,
wobei die Krankheit eine chronische Lebererkrankung ist, ausgewählt aus primärer biliärer Zirrhose (PBC), zerebrotendinöser Xanthomatose (CTX), primärer sklerosierender Cholangitis (PSC), arzneimittelinduzierter Cholestase, intrahepatischer Schwangerschaftscholestase, parenteraler ernährungsassoziierter Cholestase (PNAC), mit bakterieller Überwucherung oder Sepsis assoziierter Cholestase, Autoimmunhepatitis, chronischer Virushepatitis, alkoholischer Lebererkrankung, nichtalkoholischer Fettlebererkrankung (NAFLD), nichtalkoholischer Steatohepatitis (NASH), Lebertransplantat-assoziierter Graft-versus-Host-Erkrankung, Lebendspendetransplantat-Leberregeneration, angeborener Leberfibrose, Choledocholithiasis, granulomatöser Lebererkrankung, intra- oder extrahepatischer Malignität, Sjögren-Syndrom, Sarkoidose, Morbus Wilson, Morbus Gaucher, Hämochromatose und Alpha-1-Antitrypsin-Mangel.

18. Verbindung nach einem der Ansprüche 1-14 oder pharmazeutische Zusammensetzung nach Anspruch 15 zur Verwendung in einem Verfahren zum Behandeln, Verhindern oder Reduzieren des Risikos einer Störung, die mit mitochondrialer Dysfunktion assoziiert ist, wobei das Verfahren das Verabreichen einer therapeutisch wirksamen Menge der Verbindung an das Subjekt, das an einer Stoffwechselstörung leidet oder für eine Entwicklung derselben anfällig ist, umfasst,
wobei die mit mitochondrialer Dysfunktion assoziierte Störung eine vererbte mitochondriale Erkrankung, eine allgemeine Stoffwechselstörung, eine neurodegenerative Erkrankung, eine altersbedingte Störung, eine Nierenkrankheit oder eine chronische entzündliche Erkrankung ist; wobei optional die allgemeine Stoffwechselstörung Fettleibigkeit oder Typ-II-Diabetes ist.

19. Verbindung nach Anspruch 18, wobei es sich bei der Stoffwechselstörung um Typ-II-Diabetes, Fettleibigkeit, Hyperglykämie, Glucose-Intoleranz, Insulinresistenz, Hypercholesterinämie, Bluthochdruck, Hyperlipoproteinämie, Hyperlipidämie, Hypertriglyceridämie, kardiovaskuläre Erkrankung, Atherosklerose, periphere Gefäßerkrankung, Nierenkrankheit, Ketoazidose, thrombotische Störungen, Nephropathie, diabetische Neuropathie, diabetische Retinopathie, sexuelle Dysfunktion, Dermatopathie, Dyspepsie, Hypoglykämie, Krebs oder Ödem handelt;
und/oder wobei es sich bei der neurodegenerativen Erkrankung um Photorezeptordegeneration, Demenz, Alzheimer-Krankheit, Parkinson-Krankheit oder Huntington-Krankheit handelt;
und/oder wobei es sich bei der chronisch entzündlichen Erkrankung um Zöliakie, Vaskulitis, Lupus, chronisch obstruktive Lungenerkrankung (COPD), Reizdarmerkrankung, Atherosklerose, Arthritis oder Psoriasis handelt;
und/oder wobei es sich bei der Nierenkrankheit um eine akute Nierenschädigung (AKI) oder eine chronische Nierenerkrankung (CKD) handelt;
und/oder wobei es sich bei der mit dem Altern verbundenen Krankheit um Krebs, Demenz, kardiovaskuläre Erkrankung, Bluthochdruck, Diabetes mellitus (Typ I oder Typ II), Arthritis, Katarakte, Alzheimer-Krankheit, Makuladegeneration oder Osteoporose handelt.

20. Verbindung für die Verwendung in einem Verfahren zum Behandeln, Verhindern oder Reduzieren des Risikos einer Erkrankung oder Störung, die mit α-Amino-β-carboxymuconat-ε-semialdehyd-Decarboxylase (ACMSD)-Dysfunktion assoziiert ist, wobei das Verfahren das Verabreichen einer therapeutisch wirksamen Menge der Verbindung an das Subjekt, das an einer mit ACMSD-Dysfunktion assoziierten Erkrankung oder Störung leidet oder für eine Entwicklung derselben anfällig ist, umfasst, wobei die Verbindung repräsentiert wird durch Formel (II): 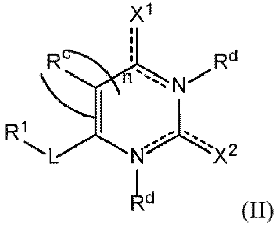 oder ein pharmazeutisch annehmbares Salz oder Tautomer davon,
wobei:
X¹ H, O, S, OR² oder SH ist;
X² O, S, OR² oder SR² ist;
L -(CH₂)ₘCH=CH(CH₂)ₚ-, -(CH₂)*ₒ*-, 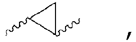 -(CH₂)ₘC=(O)NR³(CH₂)ₚ-, -(CH₂)ₘNR³C=(O)(CH₂)ₚ-, Pyridinyl oder Thiophenyl ist;
Y² O, NH oder S ist;
R¹ C₆-C₁₀-Aryl oder -Heteroaryl ist, wobei das Heteroaryl ein oder zwei 5- bis 7-gliedrige Ringe und 1-4 Heteroatome, ausgewählt aus N, O und S, umfasst, und wobei das Aryl und Heteroaryl mit R^{a} und R^{b} substituiert sind, und optional mit einem bis zwei R^{e} substituiert sind;
R² H oder C₁-C₄-Alkyl ist;
R³ H oder C₁-C₄-Alkyl ist;
R^{a} H, C₁-C₄-Alkyl, -(C(R^{f})₂)ᵣCO₂R^{x}, -Y²(C(R^{f})₂)ᵣ-CO₂R^{x}, -O(C(R^{f})₂)ᵣC(O)NHR^{g}, -(C(R^{f})₂)ᵣ-C₆-C₁₀-Aryl,-(C(R^{f})₂)ᵣS-C₆-C₁₀-Aryl, -(C(R^{f})₂)ᵣ-Heteroaryl, -O(C-(R^{f})₂)ᵣ-Heteroaryl, -O(C(R^{f})₂)ᵣ-Heterocycloalkyl,-O(C(R^{f})₂)ᵣ(C₃-C₇)-Cycloalkyl, -(C(R^{f})₂)ᵣP(O)(OH)OR^{x}, -O(C(R^{f})₂)ᵣP(O)(OH)OR^{x}, -(C(R^{f})₂)ᵣS(O)₂OH, -O(C-(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -OR^{y}, -(C(R^{f})₂)ᵣC(O)NHCN, -CH=CHCO₂R^{x} oder -(C(R^{f})₂)ᵣC(O)NHS(O)₂-Alkyl ist, wobei das Aryl und das Heteroaryl optional mit einem bis drei Substituenten substituiert sind, die jeweils unabhängig voneinander ausgewählt sind aus Halogen und OH, und wobei das Heterocycloalkyl mit einem bis zwei =O oder =S substituiert ist;
R^{b} C₁-C₄-Alkyl, -(C(R^{f})₂)ᵣCO₂R^{x}, -Y²(C(R^{f})₂)ᵣCO₂R^{x}, -O(C(R^{f})₂)ᵣC(O)NHR^{g}, -(C(R^{f})₂)ᵣ-C₆-C₁₀-Aryl, -(C-(R^{f})₂)ᵣS-C₆-C₁₀-Aryl, -(C(R^{f})₂)ᵣ-Heteroaryl, -O(C-(R^{f})₂)ᵣ-Heteroaryl, -O(C(R^{f})₂)ᵣ-Heterocycloalkyl,-(C(R^{f})₂)ᵣP(O)(OH)OR^{x}, -O(C(R^{f})₂)ᵣP(O)(OH)OR^{x}, -(C-(R^{f})₂)ᵣS(O)₂OH, -O(C(R^{f})₂)ᵣS(O)₂OH, -(C(R^{f})₂)ᵣP(O)₂OH, -O(C(R^{f})₂)ᵣP(O)₂OH, -O(C(R^{f})₂)ᵣOH, -(C(R^{f})₂)ᵣC(O)NHCN, -CH=CHCO₂R^{x} oder -(C(R^{f})₂)ᵣC(O)NHS(O)₂-Alkyl ist, wobei das Aryl und das Heteroaryl mit einem bis drei Substituenten, ausgewählt aus Halogen und OH, substituiert sind, und wobei das Heterocycloalkyl mit einem bis zwei =O oder =S substituiert ist; oder
R^{a} und R^{b}, wenn sie auf benachbarten Atomen befindlich sind, zusammen mit den Atomen, an die sie gebunden sind, einen C₆-C₁₀-Arylring bilden, der optional mit einem oder mehreren -CO₂H substituiert ist; R^{a} und R^{b}, wenn sie auf benachbarten Atomen befindlich sind, zusammen mit den Atomen, an die sie gebunden sind, einen 5- bis 6-gliedrigen Heteroarylring bilden, der optional mit einem oder mehreren -CO₂H substituiert ist; R^{c} -CN oder -NO₂ ist;
jedes R^{d} unabhängig bei jedem Vorkommen abwesend oder H ist;
jedes R^{e} unabhängig bei jedem Vorkommen C₁-C₆-Alkyl, C₂-C₆-Alkenyl, C₂-C₆-Alkinyl, Halogen, C₁-C₆-Haloalkyl, -NHR^{z}, -OH oder -CN ist;
jedes R^{f} unabhängig H oder C₁-C₆-Alkyl ist;
R^{g} H, C₁-C₆-Alkyl, OH, -S(O)₂(C₁-C₆-Alkyl) oder-S(O)₂N(C₁-C₆-Alkyl)₂ ist;
R^{x} H oder C₁-C₆-Alkyl ist;
jedes R^{y} und R^{z} unabhängig H, C₁-C₆-Alkyl oder C₁-C₆-Haloalkyl ist;
jedes m, p und r unabhängig 0, 1 oder 2 ist;
n 1 ist;
o 1, 2, 3 oder 4 ist; und
die gepunktete Linie eine optionale Doppelbindung ist,
wobei die Erkrankung eine chronische Lebererkrankung ist, ausgewählt aus primärer biliärer Zirrhose (PBC), zerebrotendinöser Xanthomatose (CTX), primärer sklerosierender Cholangitis (PSC), arzneimittelinduzierter Cholestase, intrahepatischer Schwangerschaftscholestase, parenteraler ernährungsassoziierter Cholestase (PNAC), mit bakterieller Überwucherung oder Sepsis assoziierter Cholestase, Autoimmunhepatitis, chronischer Virushepatitis, alkoholischer Lebererkrankung, nichtalkoholischer Fettlebererkrankung (NAFLD), nichtalkoholischer Steatohepatitis (NASH), Lebertransplantat-assoziierter Graft-versus-Host-Erkrankung, Lebendspendetransplantat-Leberregeneration, angeborener Leberfibrose, Choledocholithiasis, granulomatöser Lebererkrankung, intra- oder extrahepatischer Malignität, Sjögren-Syndrom, Sarkoidose, Morbus Wilson, Morbus Gaucher, Hämochromatose und Alpha-1-Antitrypsin-Mangel.

21. Verbindung für die Verwendung nach Anspruch 20, aufweisend eine der folgenden Formeln:

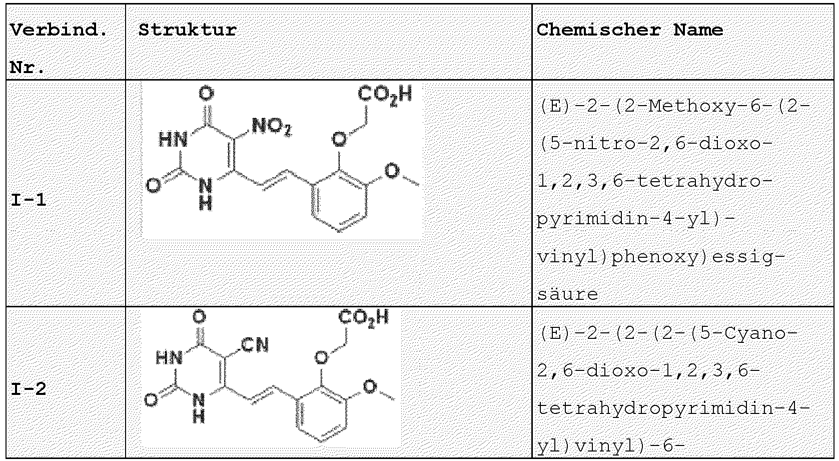

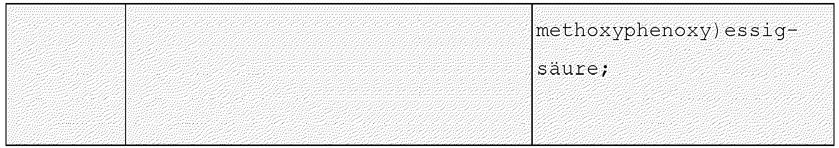

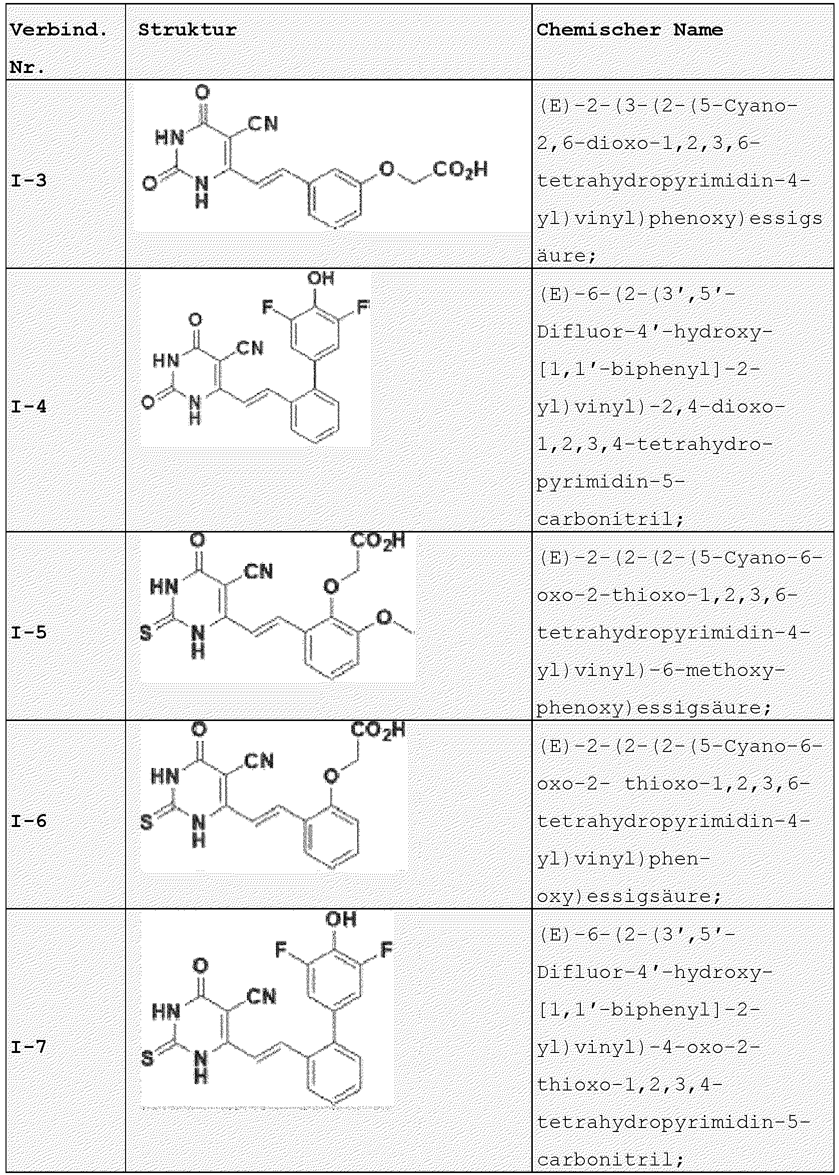

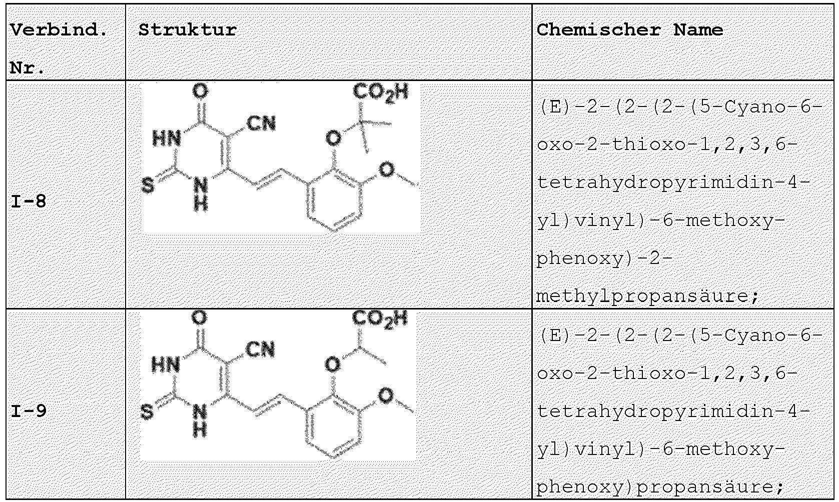

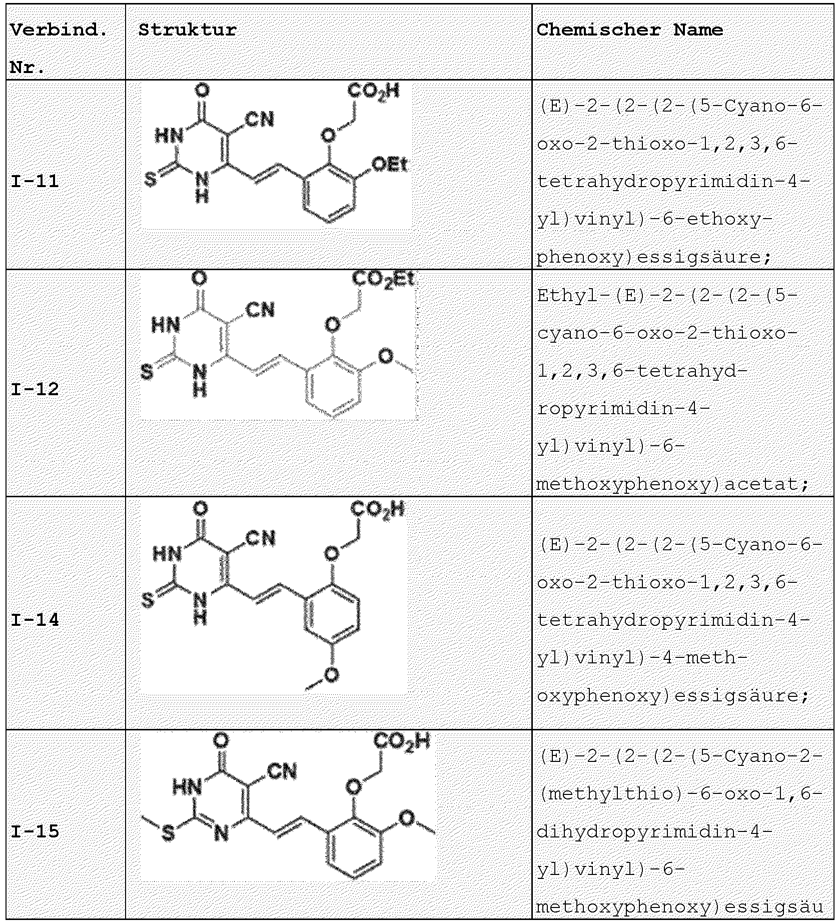

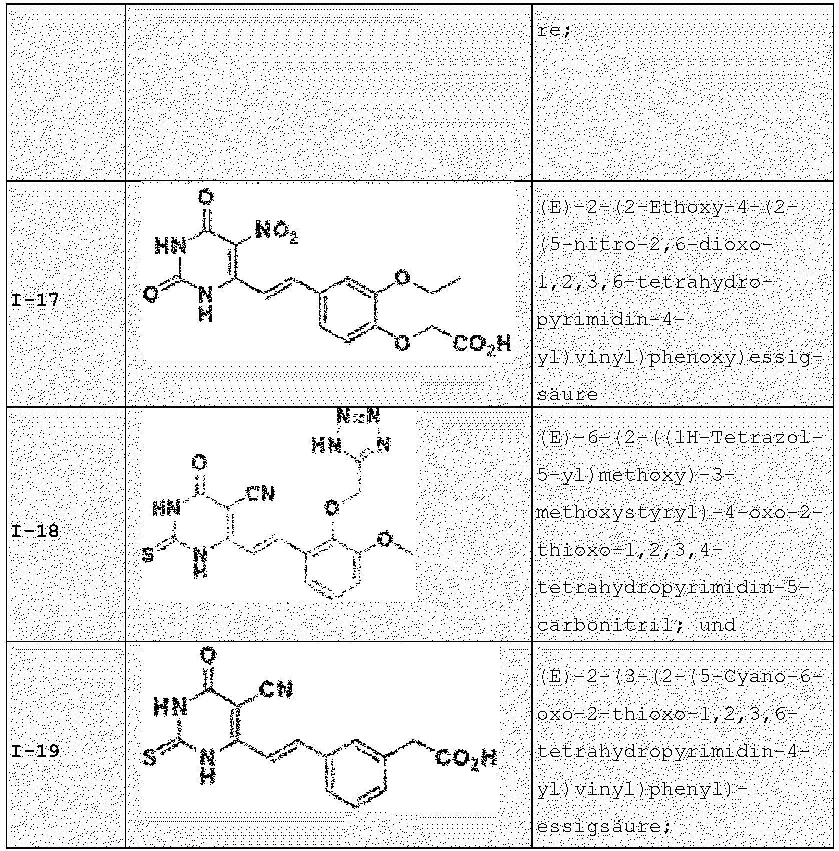

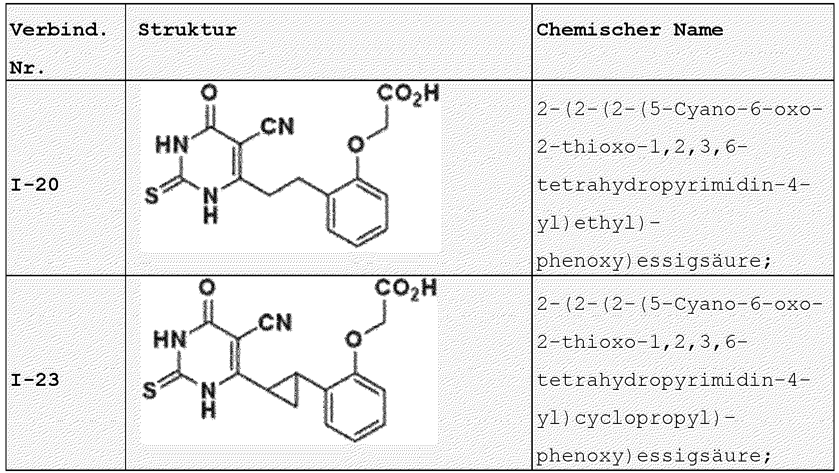

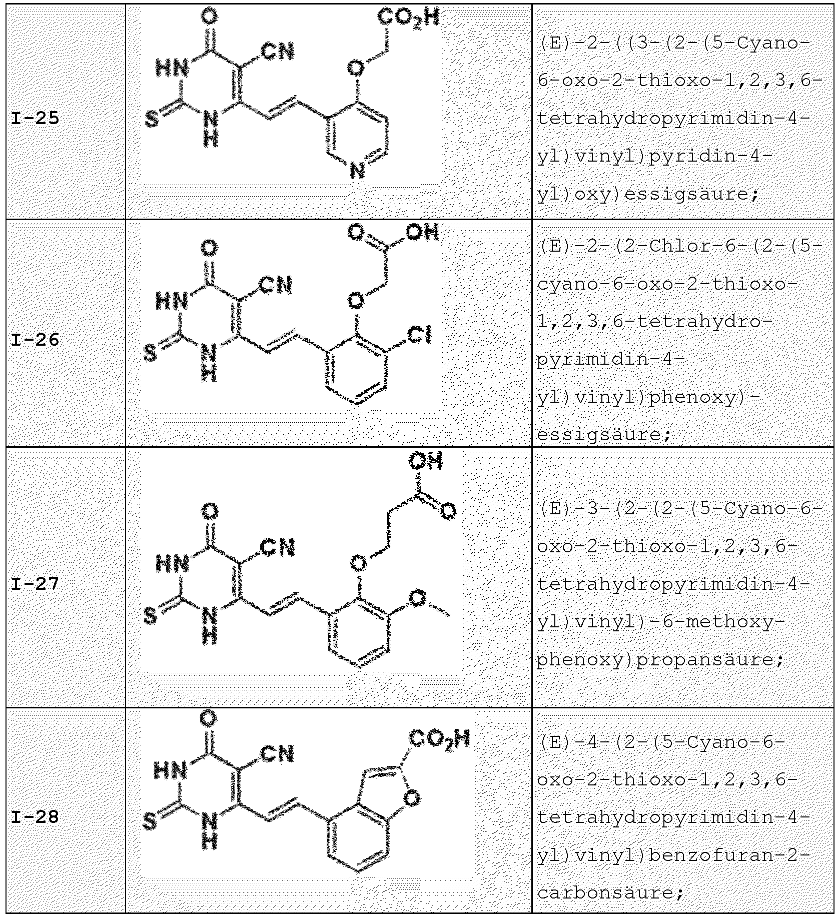

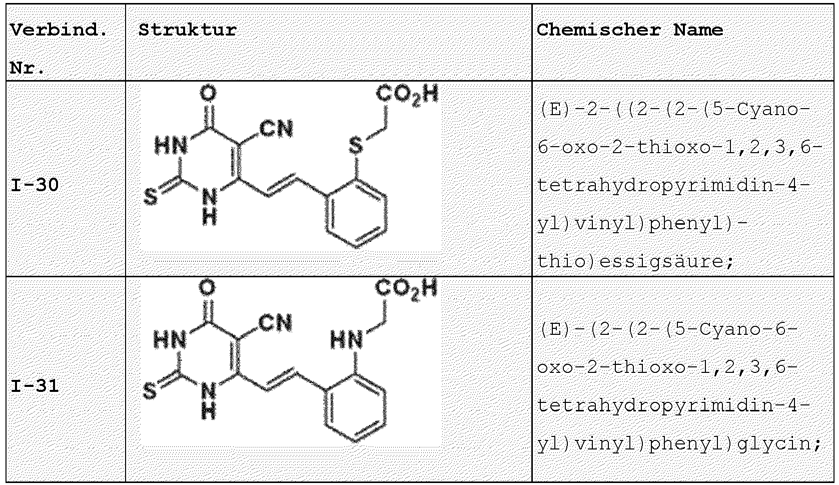

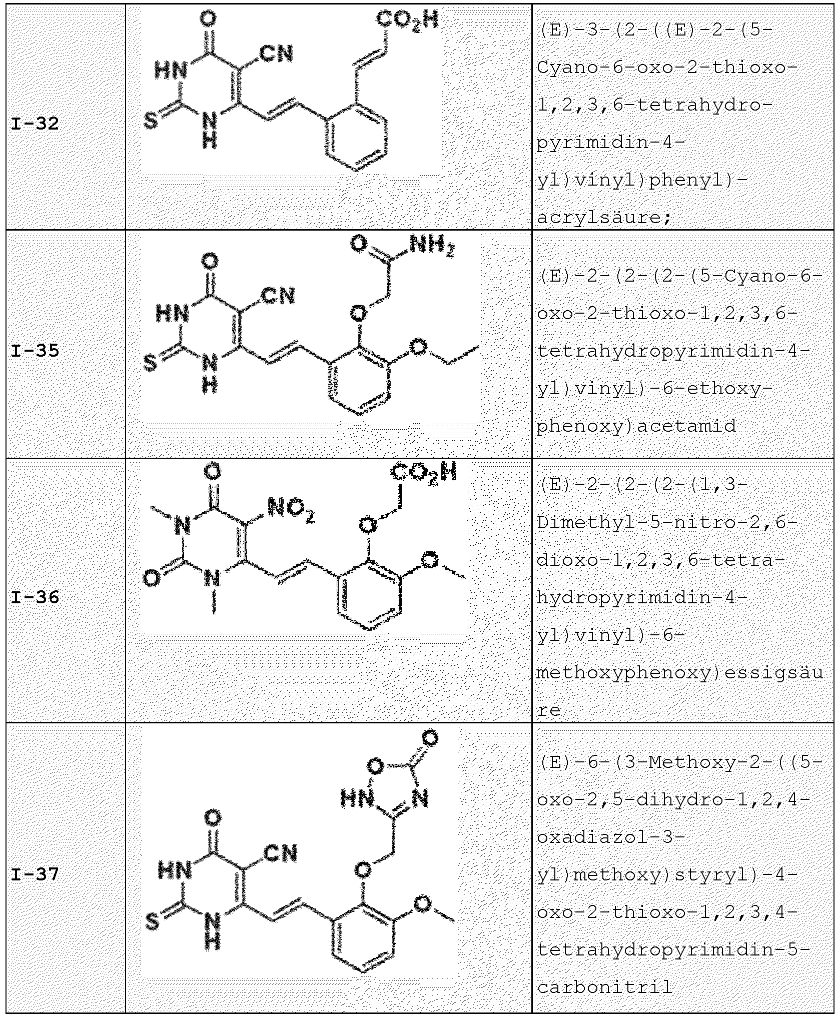

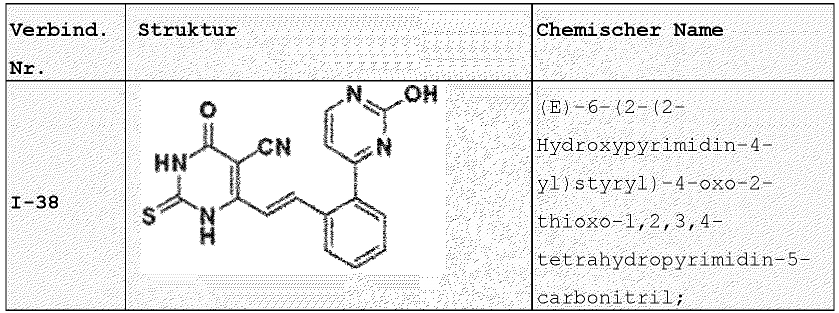

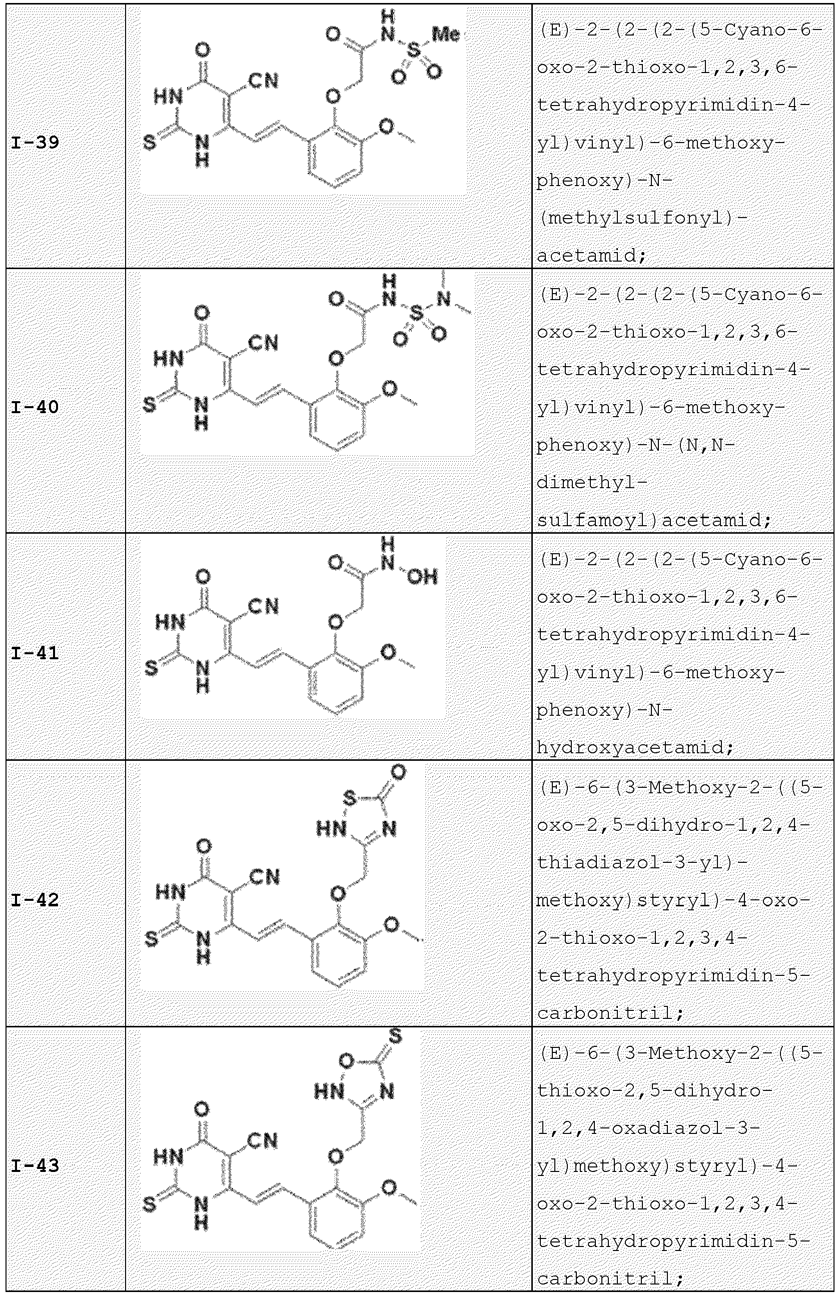

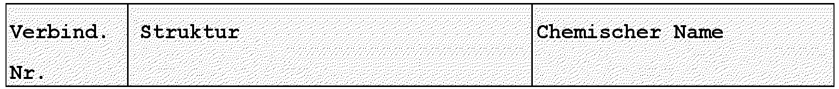

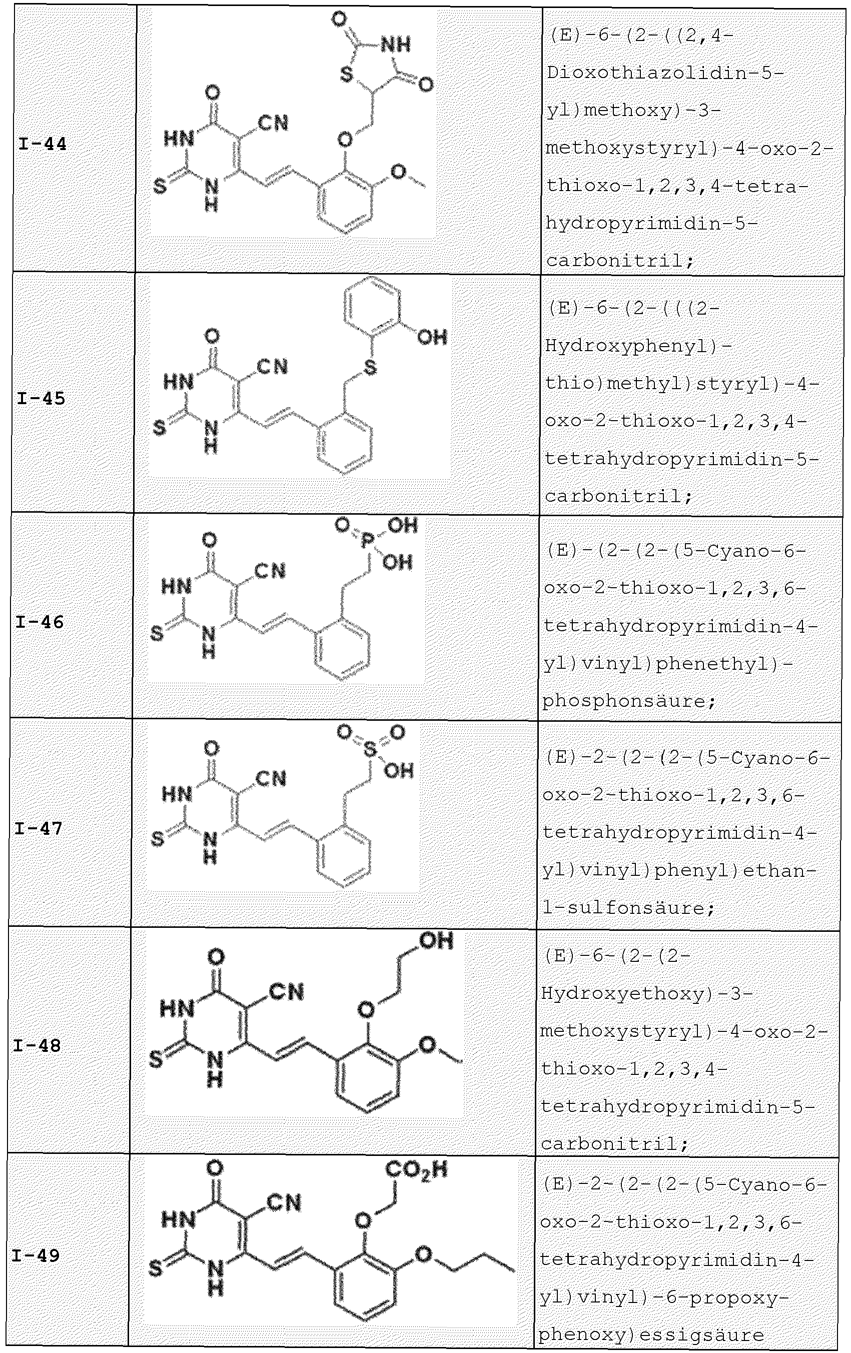

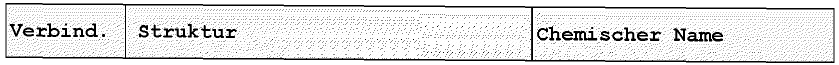

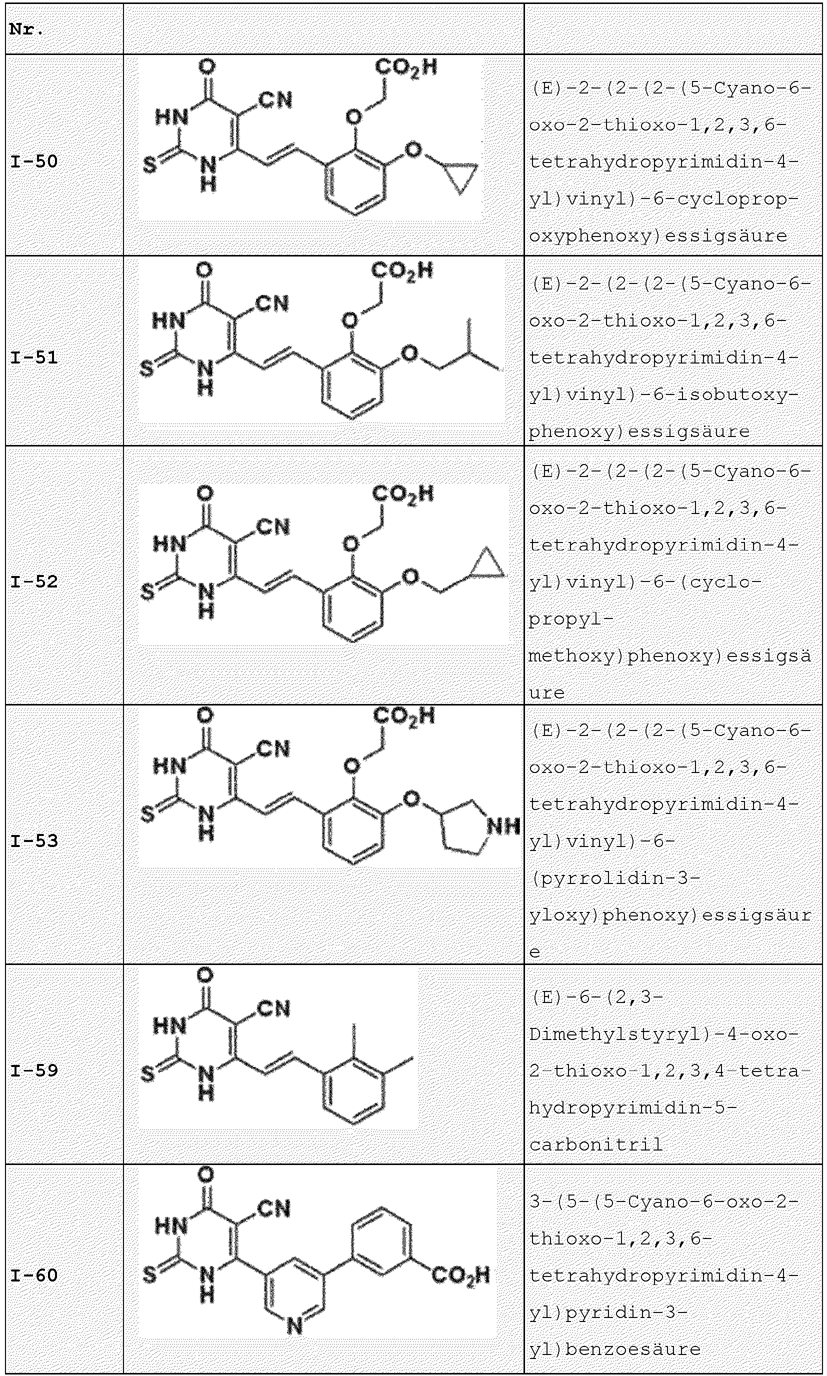

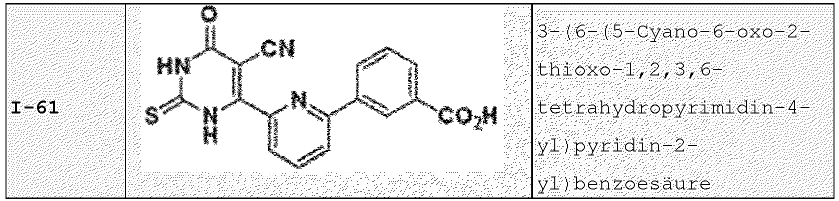

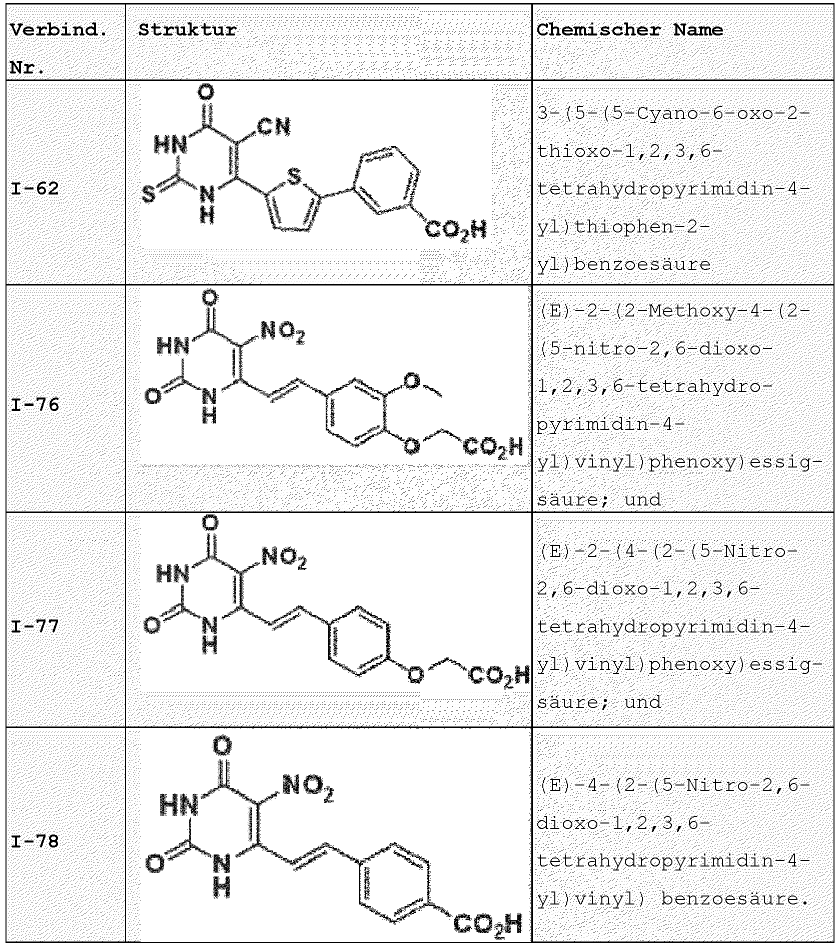

## Revendications

1. Composé représenté par la Formule (I) : 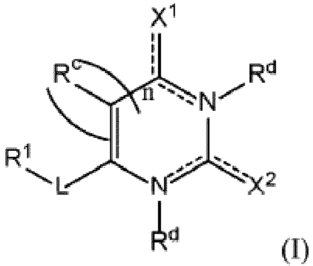 ou sel ou forme tautomère pharmaceutiquement acceptable correspondant(e),
X¹ étant O, S, OR², ou SH ;
X² étant O, S, OR², ou SR² ;
L étant - (CH₂)ₘCH=CH(CH₂)ₚ-, -(CH₂)*ₒ*-, 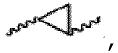 (CH₂)ₘC=(O)NR³(CH₂)ₚ-, -(CH₂)ₘNR³C=(O)(CH₂)ₚ-, pyridinyle, ou thiophényle ;
Y² étant O, NH ou S ;
R¹ étant C₆-C₁₀ aryle ou hétéroaryle, l'hétéroaryle comprenant un ou deux cycles à 5 à 7 chaînons et 1 à 4 hétéroatomes choisis parmi N, O et S, et l'aryle et l'hétéroaryle étant substitués par R^{a} et R^{b}, et éventuellement substitués par un à deux R^{e} ;
R² étant H ou C₁-C₄ alkyle ;
R³ étant H ou C₁-C₄ alkyle ;
R^{a} étant H, C₁-C₄ alkyle, -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryle, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -O(C(R^{f})₂)ᵣ(C₃-C₇) cycloalkyle, -(C(R^{f})₂)ᵣP(O)(OH)OR^{x},-O(C(R^{f})₂)ᵣP(O)(OH)OR^{x}, -(C(R^{f})₂)ᵣS(O)₂OH,-O(C(R^{f})₂)ᵣS(O)₂OH, -(C(R^{f})₂)ᵣP(O)₂OH,-O(C(R^{f})₂)ᵣP(O)₂OH, -O(C(R^{f})₂)ᵣOH, -OR^{y},-(C(R^{f})₂)ᵣC(O)NHCN, -CH=CHCO₂R^{x}, ou-(C(R^{f})₂)ᵣC(O)NHS(O)₂alkyle, l'aryle et l'hétéroaryle étant éventuellement substitués par un à trois substituants chacun indépendamment choisi parmi halogène et OH, et l'hétérocycloalkyle étant substitué par un à deux =O ou =S ;
R^{b} étant C₁-C₄ alkyle, -(C(R^{f})₂)RCO₂R^{X},-Y²(C(R^{f})₂)_{R}CO₂R^{x}, -O(C(R^{f})₂)ᵣC(O)NHR^{g}, -(C(R^{f})₂)ᵣC₆-C₁₀ aryle, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -(C(R^{f})₂)ᵣP(O)(OH)OR^{x},-O(C(R^{f})₂)ᵣP(O)(OH)OR^{x}, -(C(R^{f})₂)ᵣS(O)₂OH,-O(C(R^{f})₂)ᵣS(O)₂OH, -(C(R^{f})₂)ᵣP(O)₂OH,-O(C(R^{f})₂)ᵣP(O)₂OH, -O(C(R^{f})₂)ᵣOH, -(C(R^{f})₂)ᵣC(O)NHCN,-CH=CHCO₂R^{x}, ou -(C(R^{f})₂)ᵣC(O)NHS(O)₂alkyle, l'aryle et l'hétéroaryle étant substitués par un à trois substituants choisis parmi halogène et OH, et l'hétérocycloalkyle étant substitué par un à deux =O ou =S ; ou
R^{a} et R^{b}, lorsqu'ils sont sur des atomes adjacents, formant, conjointement avec les atomes auxquels ils sont fixés, un cycle C₆-C₁₀ aryle éventuellement substitué par un ou plusieurs-CO₂H; R^{a} et R^{b}, lorsqu'ils sont sur des atomes adjacents, formant, conjointement avec les atomes auxquels ils sont fixés, un cycle hétéroaryle à 5 à 6 chaînons éventuellement substitué par un ou plusieurs -CO₂H ;
R^{c} étant -CN ;
chaque R^{d} étant indépendamment en chaque occurrence absent ou H ;
chaque R^{e} étant indépendamment en chaque occurrence C₁-C₆ alkyle, C₂-C₆ alcényle, C₂-C₆ alcynyle, halogène, C₁-C₆ halogénoalkyle, -NHR^{z},-OH, ou -CN ;
chaque R^{f} étant indépendamment H ou C₁-C₆ alkyle ;
R^{g} étant H, C₁-C₆ alkyle, OH, -S(O)₂(C₁-C₆ alkyle), ou -S(O)₂N(C₁-C₆ alkyle)₂ ;
R^{x} étant H ou C₁-C₆ alkyle ;
chaque R^{y} et R^{z} étant indépendamment H, C₁-C₆ alkyle, ou C₁-C₆ halogénoalkyle ;
chaque m, p, et r étant indépendamment 0, 1 ou 2 ;
n étant 1 ;
o étant 1, 2, 3, ou 4 ; et
la ligne en pointillés étant une double liaison éventuelle.

2. Composé selon la revendication 1,
X¹ étant O ou OR² ;
X² étant S ou OR² ;
L étant -(CH₂)ₘCH=CH(CH₂)ₚ-, -(CH₂)*ₒ*-, 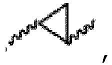 (CH₂)ₘC=(O)NR³(CH₂)ₚ-, ou -(CH₂)ₘNR³C=(O)(CH₂)ₚ- ; Y² étant O, NH ou S ;
R¹ étant C₆-C₁₀ aryle ou hétéroaryle, l'hétéroaryle comprenant un ou deux cycles à 5 à 7 chaînons et 1 à 4 hétéroatomes choisis parmi N, O et S, et l'aryle et l'hétéroaryle étant substitués par R^{a} et R^{b}, et éventuellement substitués par un à deux R^{e} ;
R² étant H ou C₁-C₄ alkyle ;
R^{a} étant H, -(C(R^{f})₂)ᵣCO₂R^{x}, -Y²(C(R^{f})₂)ᵣCO₂R^{x},-O(C(R^{f})₂)ᵣC(O)NHR^{g}, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -(C(R^{f})₂)ᵣP(O)₂OH,-(C(R^{f})₂)ᵣS(O)₂OH, -O(C(R^{f})₂)ᵣOH, -OR^{y}, ou -CH=CHCO₂R^{x}, l'aryle et l'hétéroaryle étant éventuellement substitués par un à trois substituants chacun indépendamment choisi parmi halogène et OH, et l'hétérocycloalkyle étant substitué par un à deux =O ou =S ;
R^{b} étant -(C(R^{f})₂)ᵣCO₂R^{x}, -Y²(C(R^{f})₂)ᵣCO₂R^{x},-O(C(R^{f})₂)ᵣC(O)NHR^{g}, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -(C(R^{f})₂)ᵣP(O)₂OH,-(C(R^{f})₂)ᵣS(O)₂OH, -O(C(R^{f})₂)ᵣOH, ou -CH=CHCO₂R^{x}, l'aryle et l'hétéroaryle étant substitués par un à trois substituants choisis parmi halogène et OH, et l'hétérocycloalkyle étant substitué par un à deux =O ou =S ; ou
R^{a} et R^{b}, lorsqu'ils sont sur des atomes adjacents, formant, conjointement avec les atomes auxquels ils sont fixés, un cycle C₆-C₁₀ aryle éventuellement substitué par un ou plusieurs-CO₂H; R^{a} et R^{b}, lorsqu'ils sont sur des atomes adjacents, formant, conjointement avec les atomes auxquels ils sont fixés, un cycle hétéroaryle à 5 à 6 chaînons éventuellement substitué par un ou plusieurs - CO₂H ;
R^{c} étant -CN ;
chaque R^{d} étant indépendamment en chaque occurrence absent ou H ;
chaque R^{e} étant indépendamment en chaque occurrence C₁-C₆ alkyle, C₂-C₆ alcényle, C₂-C₆ alcynyle, halogène, C₁-C₆ halogénoalkyle, -NHR^{z},-OH, ou -CN ;
chaque R^{f} étant indépendamment H ou C₁-C₆ alkyle ;
R^{g} étant H, C₁-C₆ alkyle, OH, -S(O)₂(C₁-C₆ alkyle), ou -S(O)₂N(C₁-C₆ alkyle)₂ ;
R^{x} étant H ou C₁-C₆ alkyle ;
chaque R^{y} et R^{z} étant indépendamment H, C₁-C₆ alkyle, ou C₁-C₆ halogénoalkyle ;
chaque m, p, et r étant indépendamment 0, 1 ou 2 ;
n étant 1 ;
o étant 1, 2, 3, ou 4 ; et
la ligne en pointillés étant une double liaison éventuelle.

3. Composé selon la revendication 1,
X¹ étant O ;
X² étant O, S, ou SR² ;
L étant - (CH₂)ₘCH=CH(CH₂)ₚ- ;
Y² étant O, NH ou S ;
R¹ étant C₆-C₁₀ aryle substitué par R^{a} et R^{b}, et éventuellement substitué par un à deux R^{e} ;
R² étant H ou C₁-C₄ alkyle ;
R^{a} étant H, -(C(R^{f})₂)ᵣCO₂R^{x}, -O(C(R^{f})₂)ᵣCO₂R^{x},-(C(R^{f})₂)ᵣC₆-C₁₀ aryle, ou -OR^{y}, l'aryle étant substitué par un à trois substituants choisis parmi halogène et OH ;
R^{b} étant - (C (R^{f})₂)ᵣCO₂R^{x}, -O(C (R^{f})₂)ᵣCO₂R^{x}, ou-(C(R^{f})₂)ᵣC₆-C₁₀ aryle, l'aryle étant substitué par un à trois substituants choisis parmi halogène et OH ;
R^{c} étant -CN ;
chaque R^{d} étant indépendamment en chaque occurrence absent ou H ;
chaque R^{e} étant indépendamment en chaque occurrence C₁-C₆ alkyle, C₂-C₆ alcényle, C₂-C₆ alcynyle, halogène, C₁-C₆ halogénoalkyle, -NHR^{z},-OH, ou -CN ;
chaque R^{f} étant indépendamment H ou C₁-C₆ alkyle ;
R^{x} étant H ou C₁-C₆ alkyle ;
chaque R^{y} et R^{z} étant indépendamment H, C₁-C₆ alkyle, ou C₁-C₆ halogénoalkyle ;
chaque m, p, et r étant indépendamment 0, 1 ou 2 ;
n étant 1 ;
o étant 1, 2, 3, ou 4 ; et
la ligne en pointillés étant une double liaison éventuelle.

4. Composé selon la revendication 1,
X¹ étant O ;
X² étant O, S, ou SR² ;
L étant -(CH₂)ₘCH=CH(CH₂)ₚ- ;
Y² étant O, NH ou S ;
R¹ étant C₆-C₁₀ aryle substitué par R^{a} et R^{b}, et éventuellement substitué par un à deux R^{e} ;
R² étant H ou C₁-C₄ alkyle ;
R^{a} étant H, -(C(R^{f})₂)ᵣCO₂R^{x}, -O(C(R^{f})₂)ᵣCO₂R^{x},-(C(R^{f})₂)ᵣC₆-C₁₀ aryle, -O(C(R^{f})₂)ᵣhétéroaryle, ou-OR^{y}, l'aryle étant substitué par un à trois substituants choisis parmi halogène et OH ;
R^{b} étant -(C(R^{f})₂)ᵣCO₂R^{x}, -O(C(R^{f})₂)ᵣCO₂R^{x},-(C(R^{f})₂)ᵣC₆-C₁₀ aryle, ou -O(C(R^{f})₂)ᵣhétéroaryle, l'aryle étant substitué par un à trois substituants choisis parmi halogène et OH ;
R^{c} étant -CN ;
chaque R^{d} étant indépendamment en chaque occurrence absent ou H ;
chaque R^{e} étant indépendamment en chaque occurrence C₁-C₆ alkyle, C₂-C₆ alcényle, C₂-C₆ alcynyle, halogène, C₁-C₆ halogénoalkyle, -NHR^{z},-OH, ou -CN ;
chaque R^{f} étant indépendamment H ou C₁-C₆ alkyle ;
R^{x} étant H ou C₁-C₆ alkyle ;
chaque R^{y} et R^{z} étant indépendamment H, C₁-C₆ alkyle, ou C₁-C₆ halogénoalkyle ;
chaque m, p, et r étant indépendamment 0, 1 ou 2 ;
n étant 1 ;
o étant 1, 2, 3, ou 4 ; et
la ligne en pointillés étant une double liaison éventuelle.

5. Composé selon la revendication 1, le composé étant représenté par la Formule (Ia), (Ib), (Ic), (Id), (Ie), (If), (Ig), (Ih), (Ii), ou (Ij) : 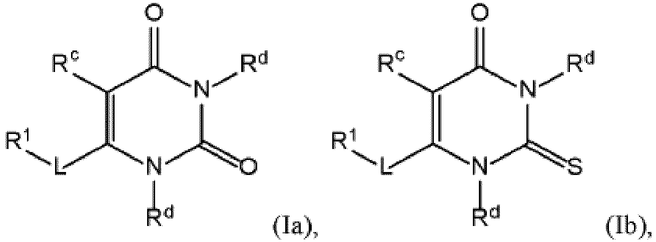 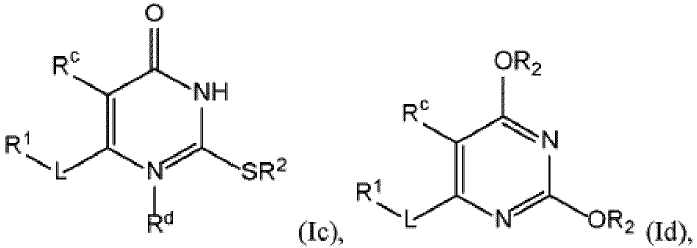 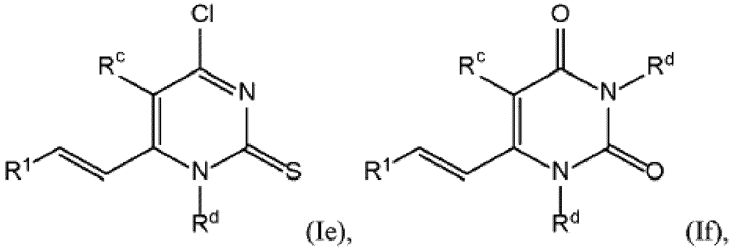 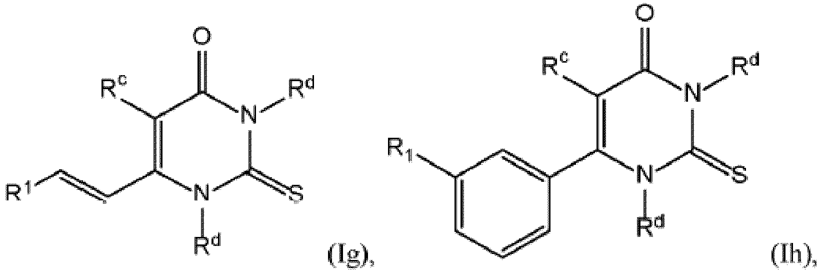 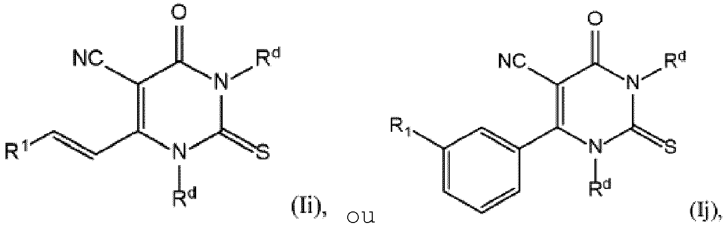 ou sel pharmaceutiquement acceptable correspondant, ou forme tautomère correspondante.

6. Composé selon l'une quelconque des revendications 1 à 5, R¹ étant C₆-C₁₀ aryle substitué par R^{a} et R^{b}, et éventuellement substitué par un à deux R^{e}.

7. Composé selon l'une quelconque des revendications 1 à 5, R¹ étant phényle substitué par R^{a} et R^{b}, et éventuellement substitué par un à deux R^{e}.

8. Composé selon l'une quelconque des revendications 1 à 5, R¹ étant hétéroaryle comprenant un cycle à 5 à 7 chaînons et 1 à 4 hétéroatomes choisis parmi N, O et S, et substitué par R^{a} et R^{b}, et éventuellement substitués par un à deux R^{e}.

9. Composé selon l'une quelconque des revendications 1 à 5, R¹ étant pyridinyle substitué par R^{a} et R^{b}, et éventuellement substitué par un à deux R^{e}.

10. Composé selon l'une quelconque des revendications 1 à 9, R^{a} étant H et R^{b} étant - (C (R^{f})₂)ᵣCO₂R^{x},-O(C (R^{f})₂)ᵣCO₂R^{x}, ou -(C(R^{f})₂)ᵣC₆-C₁₀ aryle.

11. Composé selon l'une quelconque des revendications 1 à 9, R^{a} étant H et R^{b} étant -CO₂H, -CH₂CO₂H,-OCH₂CO₂R^{X}, -OCH (CH₃) CO₂R^{x}, -OC (CH₃)₂CO₂R^{x}, ou

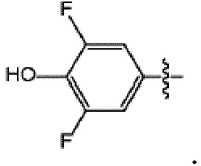

12. Composé selon l'une quelconque des revendications 1 à 9, R^{a} étant OR^{y} et R^{b} étant -O(C(R^{f})₂)ᵣCO₂R^{x}, ou -O(C(R^{f})₂)ᵣhétéroaryle.

13. Composé selon l'une quelconque des revendications 1 à 9, R^{a} étant H et R^{b} étant -(C(R^{f})₂)ᵣCO₂R^{x},-Y²(C(R^{f})₂)ᵣCO₂R^{x}, -(C(R^{f})₂)ᵣS-C₆-C₁₀ aryle,-(C(R^{f})₂)ᵣhétéroaryle, -(C(R^{f})₂)ᵣP(O)₂OH,-(C(R^{f})₂)ᵣS(O)₂OH, ou -CH=CHCO₂R^{x}, l'aryle et l'hétéroaryle étant substitués par un à trois substituants choisis parmi halogène et OH ; ou R^{a} et R^{b}, lorsqu'ils sont sur des atomes adjacents, formant, conjointement avec les atomes auxquels ils sont fixés, un cycle C₆-C₁₀ aryle éventuellement substitué par un ou plusieurs -CO₂H ; R^{a} et R^{b}, lorsqu'ils sont sur des atomes adjacents, formant, conjointement avec les atomes auxquels ils sont fixés, un cycle hétéroaryle à 5 à 6 chaînons éventuellement substitué par un ou plusieurs -CO₂H.

14. Composé selon l'une quelconque des revendications 1 à 13, possédant l'une quelconque des formules suivantes :

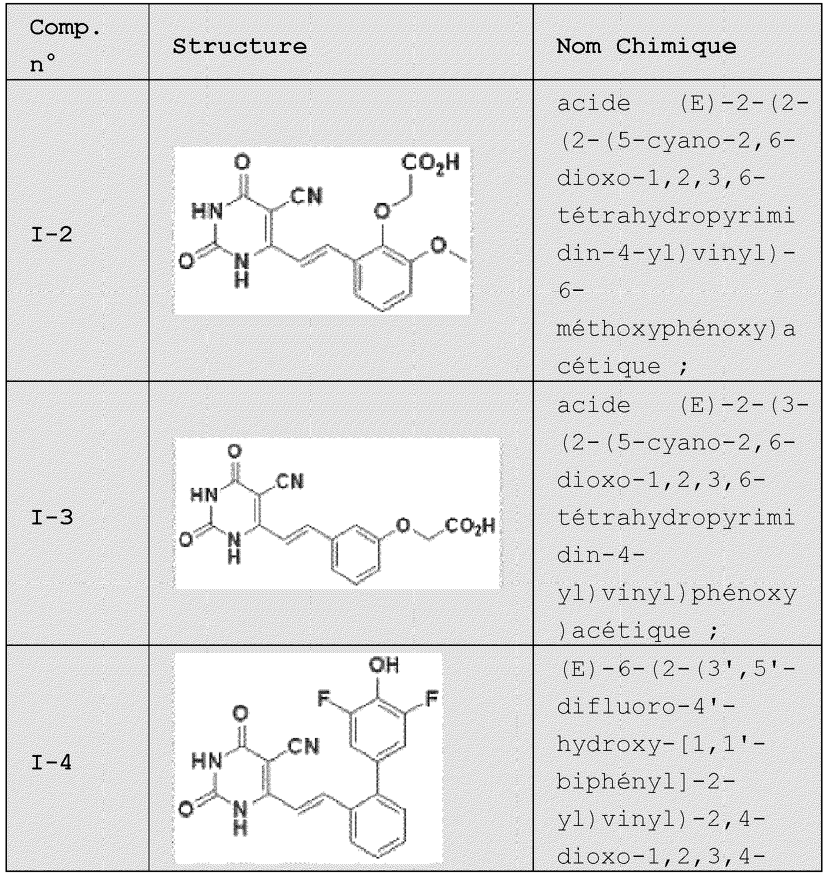

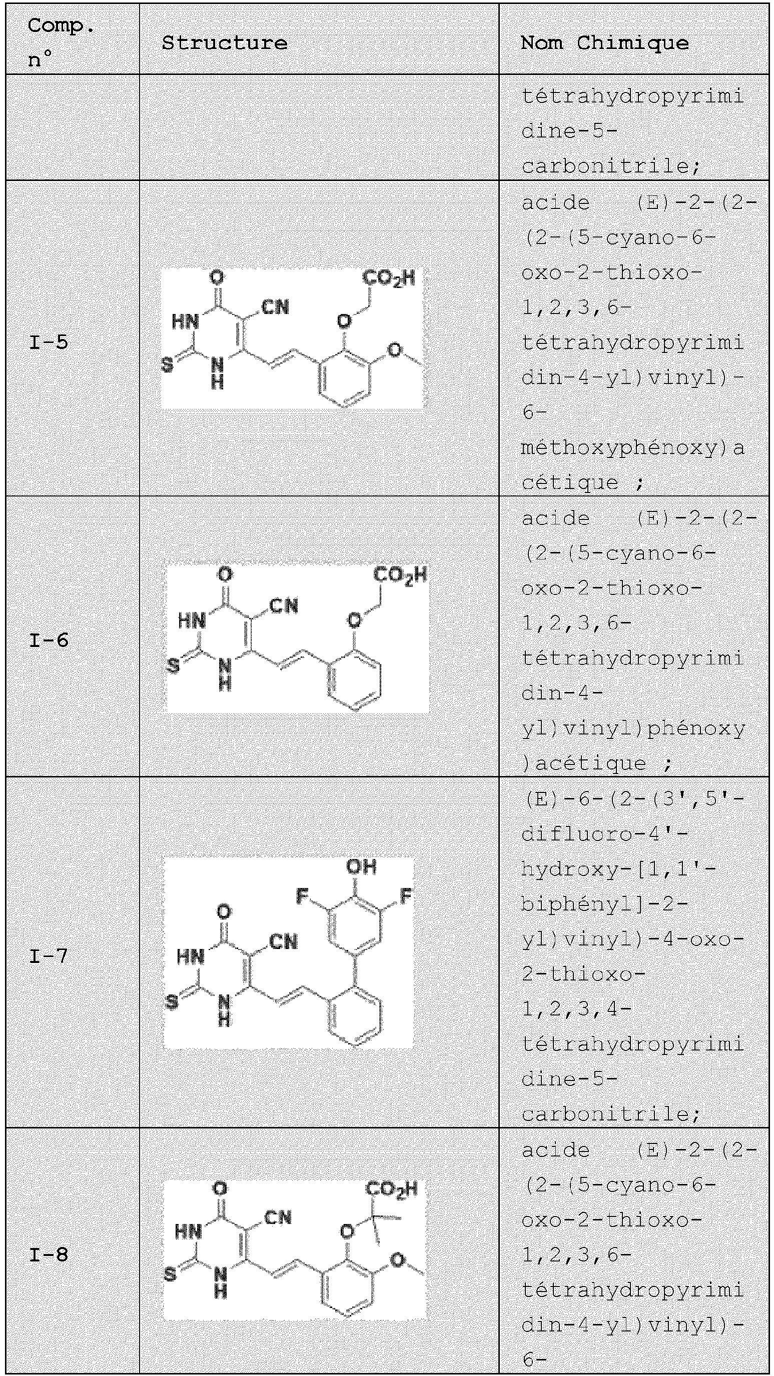

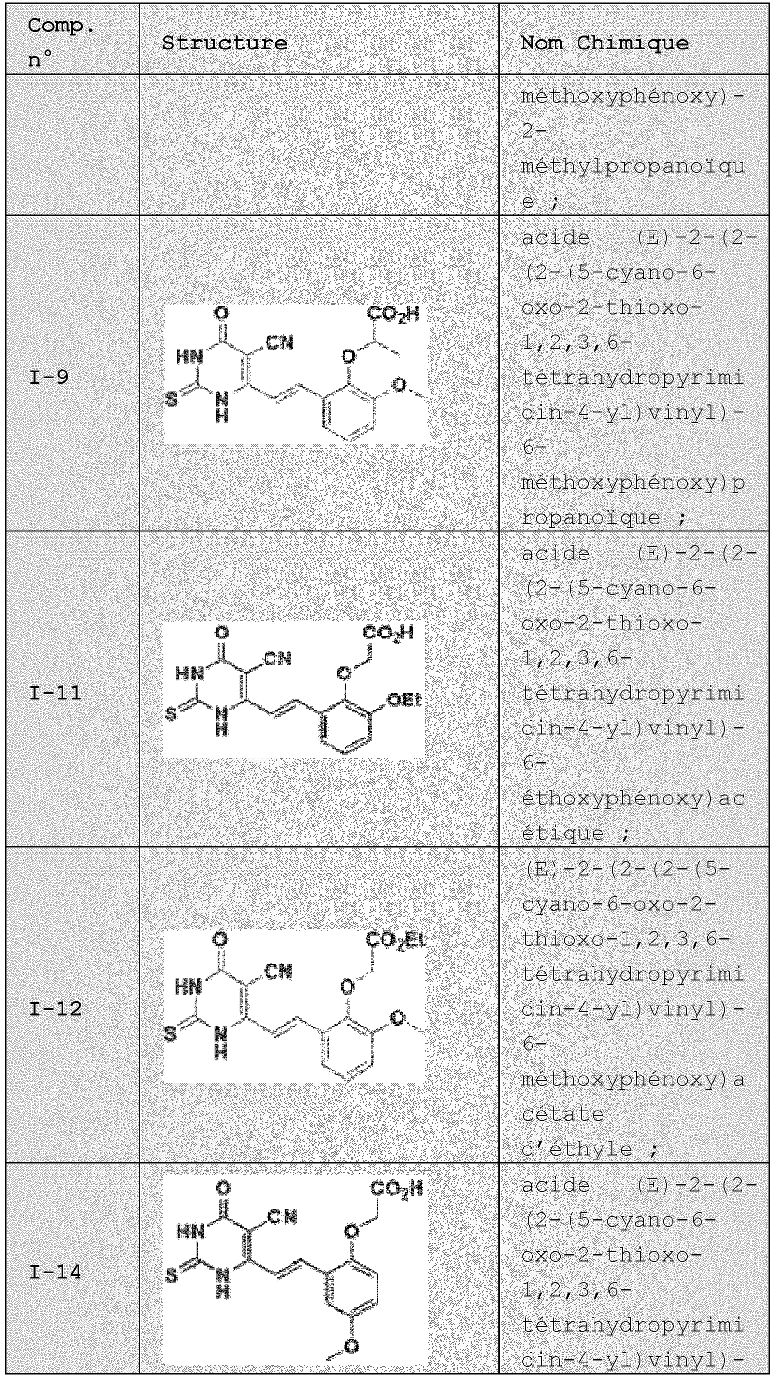

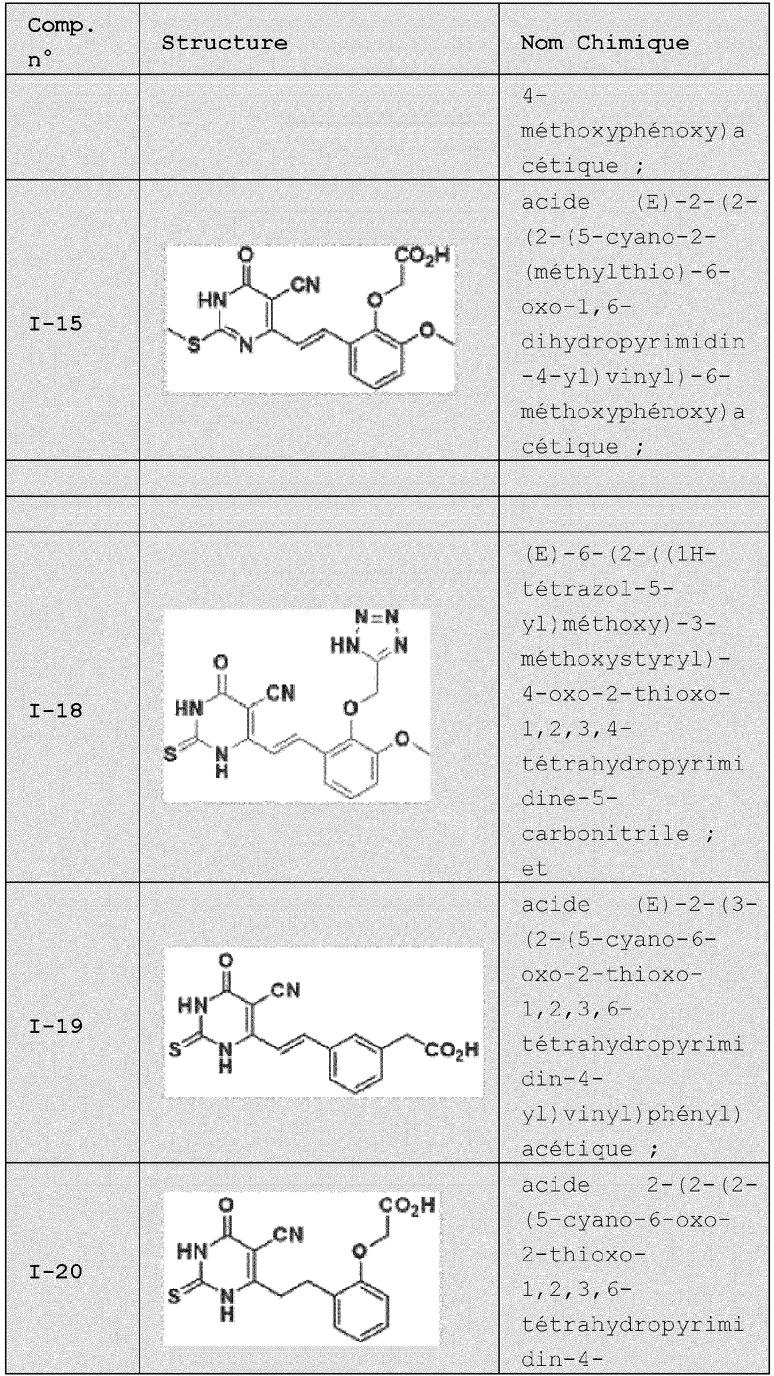

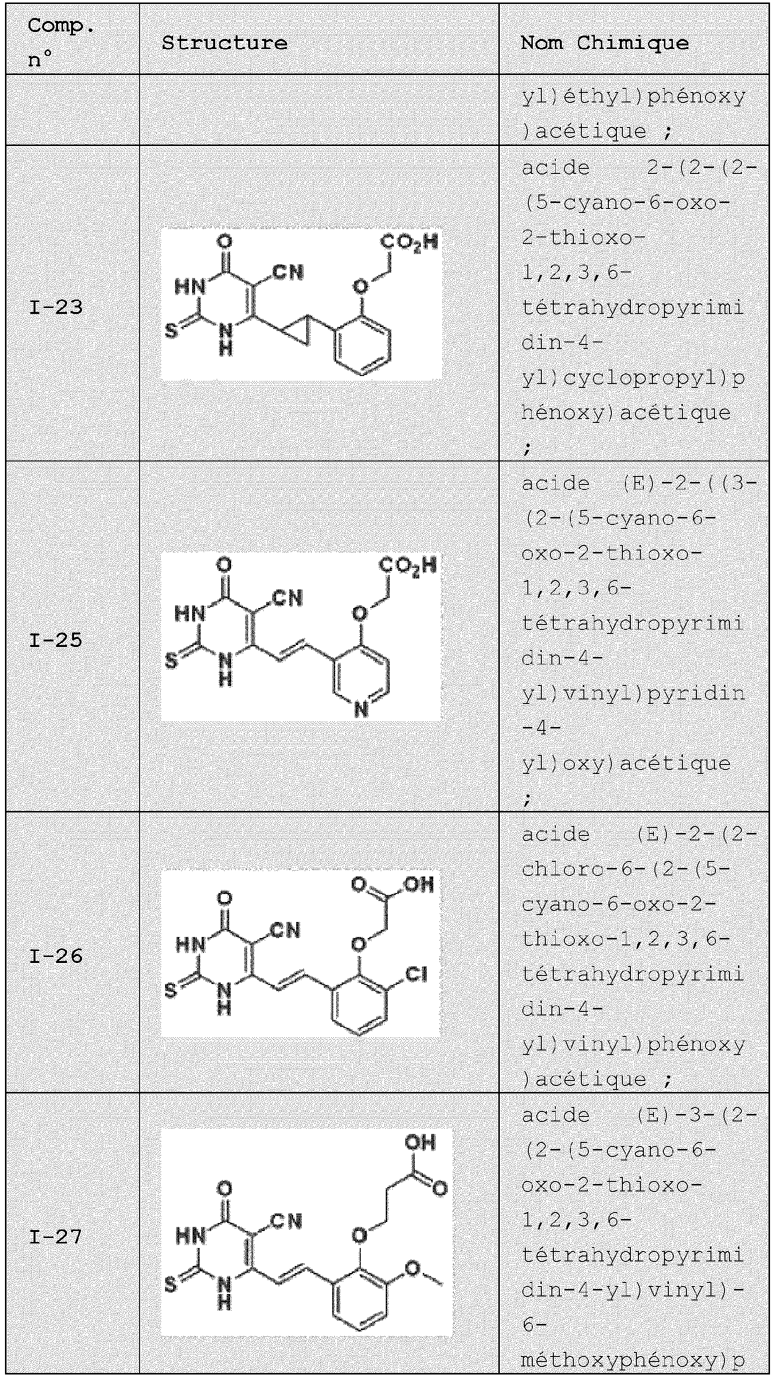

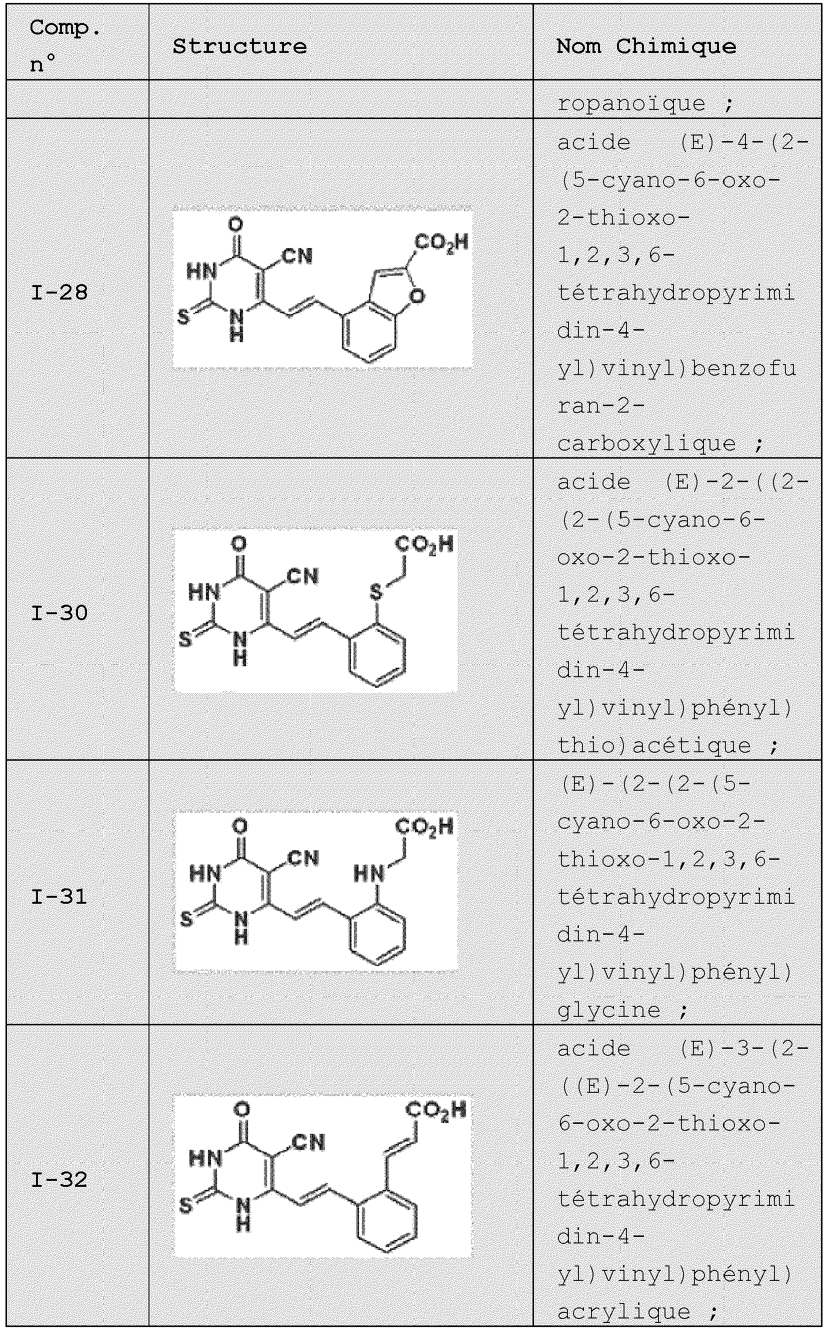

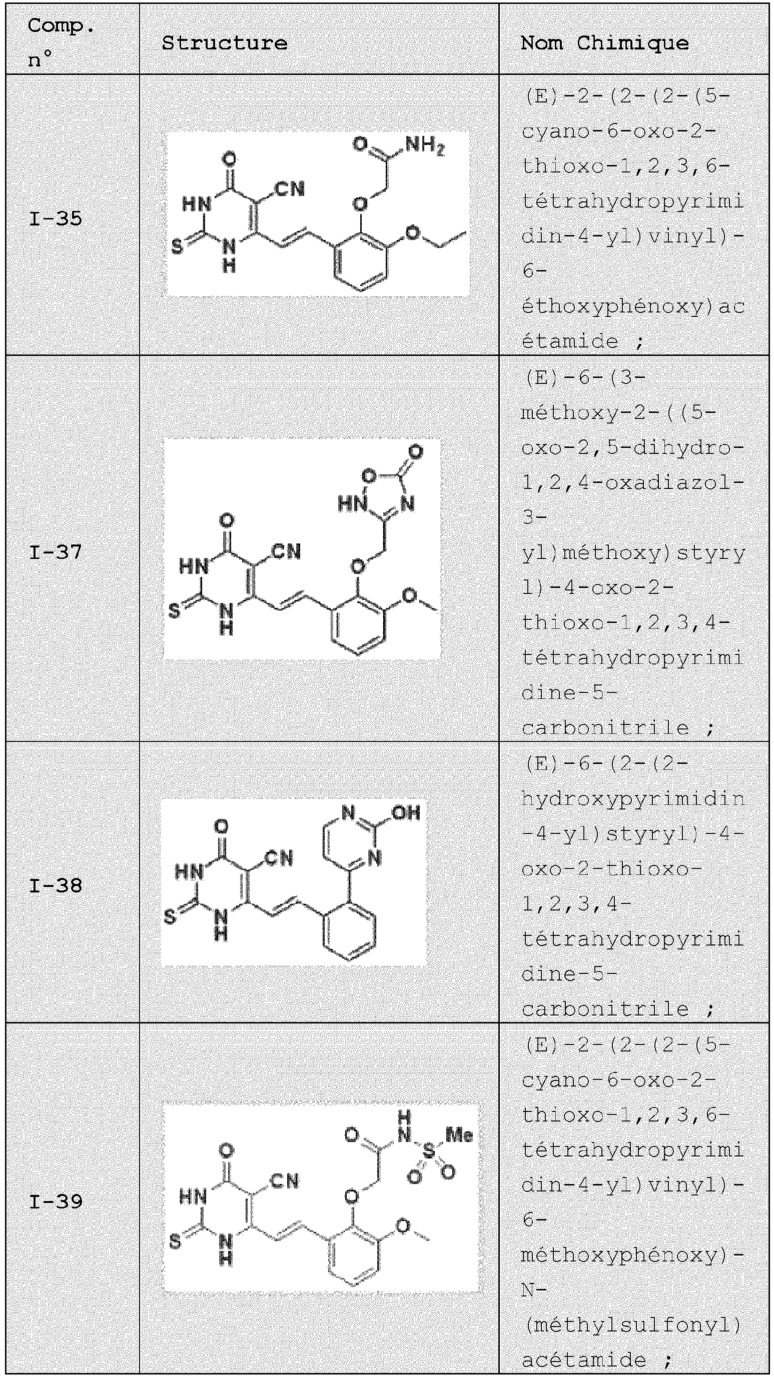

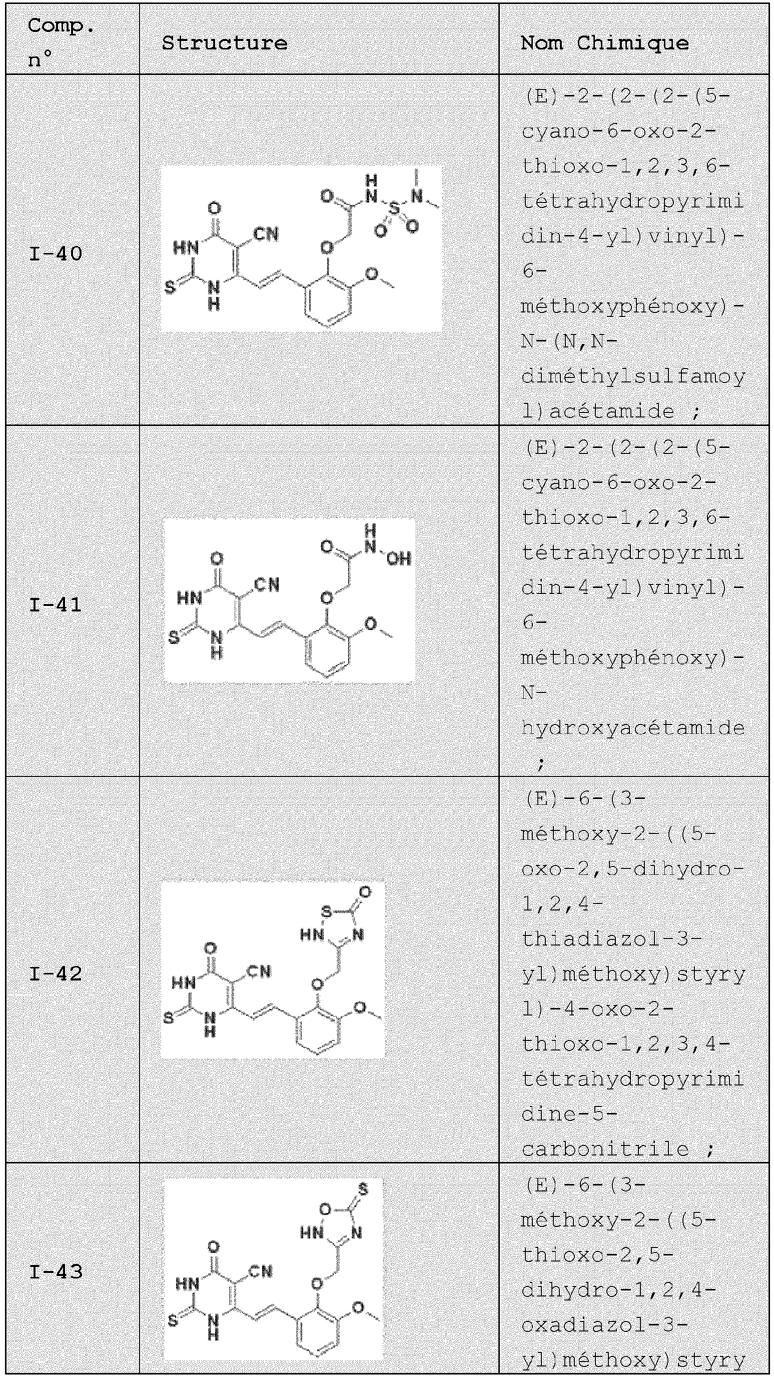

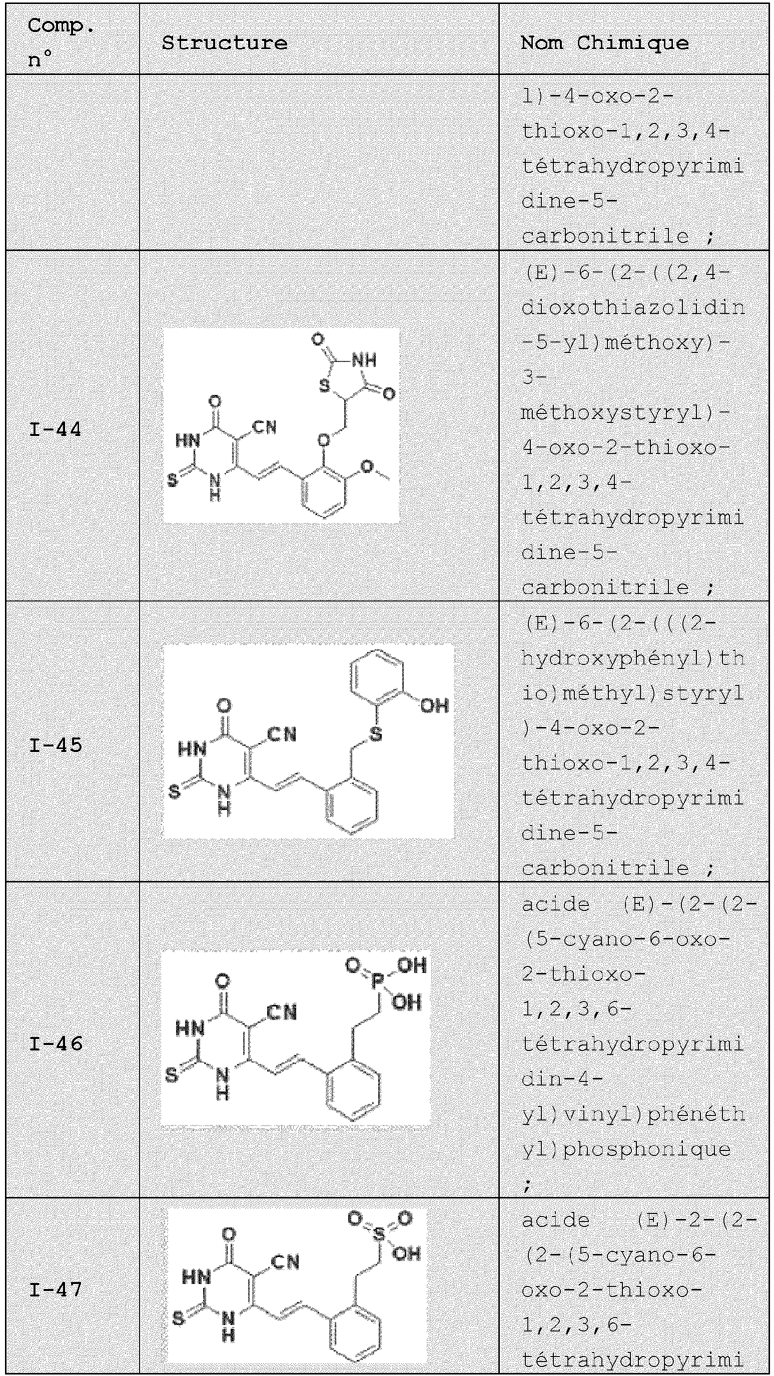

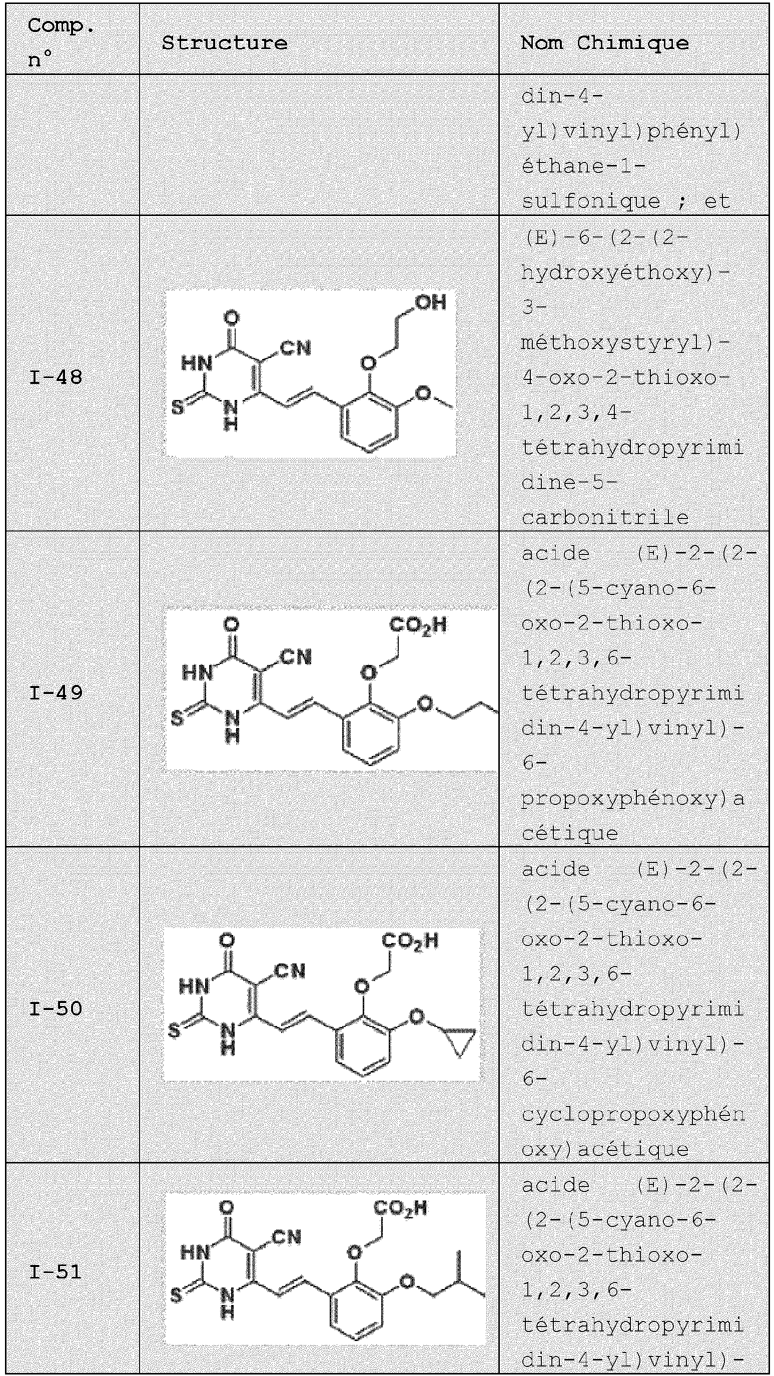

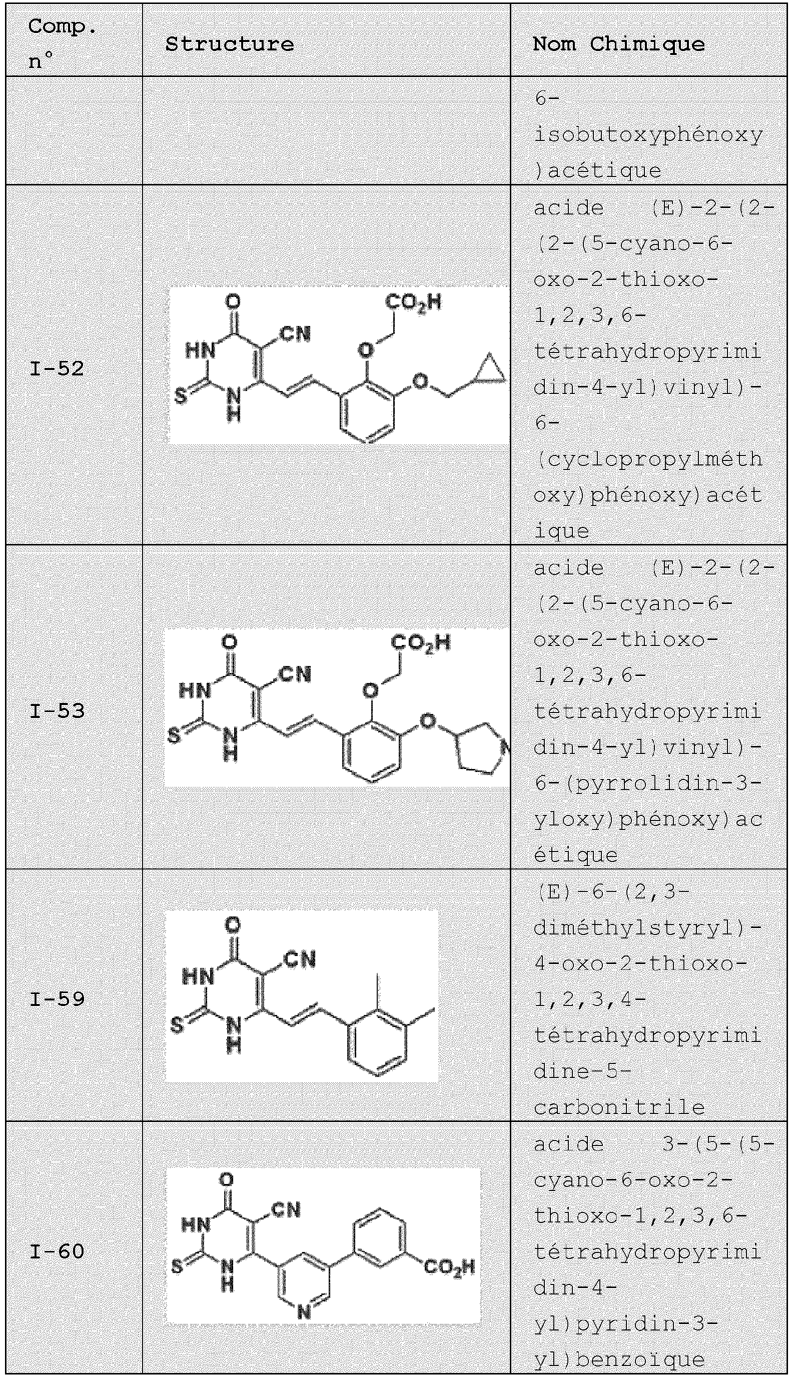

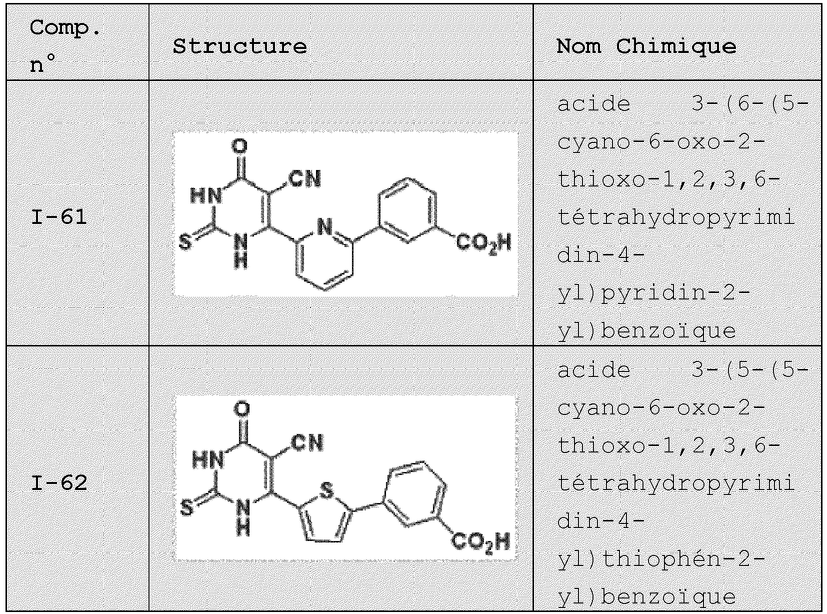

15. Composition pharmaceutique comprenant un composé selon l'une quelconque des revendications 1 à 14, ou sel pharmaceutiquement acceptable correspondant, et au moins l'un parmi un support, diluant ou excipient pharmaceutiquement acceptable,
éventuellement, la composition comprenant un ou plusieurs agents thérapeutiques supplémentaires.

16. Composé selon l'une quelconque des revendications 1 à 14 ou composition pharmaceutique selon la revendication 15, pour une utilisation dans un procédé de traitement, de prévention ou de réduction du risque d'une maladie ou d'un trouble associé (e) à un dysfonctionnement de l'α-amino-β-carboxymuconate-ε-semialdéhyde décarboxylase (ACMSD), le procédé comprenant l'administration au sujet souffrant de, ou susceptible de développer, une maladie ou un trouble associé (e) à un dysfonctionnement de l'ACMSD, d'une quantité thérapeutiquement efficace du composé,
ladite maladie étant une maladie chronique du foie choisie parmi une cirrhose biliaire primaire, (PBC), une xanthomatose cérébrotendineuse (CTX), une cholangite sclérosante primaire (PSC), une cholestase induite par un médicament, une cholestase intrahépatique de grossesse, une cholestase associée à la nutrition parentérale (PNAC), une cholestase associée à une surcroissance bactérienne ou une septicémie, une hépatite auto-immune, une hépatite virale chronique, une maladie du foie alcoolique, une maladie du foie gras non alcoolique (NAFLD), une stéatohépatite non alcoolique (NASH), une maladie de greffon contre l'hôte associée à une greffe de foie, une régénération du foie d'une greffe de foie d'un donneur vivant, une fibrose hépatique congénitale, une cholédocholithiase, une maladie du foie granulomateuse, une malignité intrahépatique ou extrahépatique, le syndrome de Sjogren, une sarcoïdose, la maladie de Wilson, la maladie de Gaucher, une hémochromatose, et une carence en alpha 1-antitrypsine.

17. Composé selon l'une quelconque des revendications 1 à 14 ou composition pharmaceutique selon la revendication 15, pour une utilisation dans un procédé de traitement, de prévention ou de réduction du risque d'une maladie ou d'un trouble associé(e) à des taux réduits de nicotinamide adénine dinucléotide (NAD⁺), le procédé comprenant l'administration au sujet souffrant de, ou susceptible de développer, une maladie ou un trouble associé(e) à des taux réduits de NAD⁺, d'une quantité thérapeutiquement efficace du composé,
ladite maladie étant une maladie chronique du foie choisie parmi une cirrhose biliaire primaire, (PBC), une xanthomatose cérébrotendineuse (CTX), une cholangite sclérosante primaire (PSC), une cholestase induite par un médicament, une cholestase intrahépatique de grossesse, une cholestase associée à la nutrition parentérale (PNAC), une cholestase associée à une surcroissance bactérienne ou une septicémie, une hépatite auto-immune, une hépatite virale chronique, une maladie du foie alcoolique, une maladie du foie gras non alcoolique (NAFLD), une stéatohépatite non alcoolique (NASH), une maladie de greffon contre l'hôte associée à une greffe de foie, une régénération du foie d'une greffe de foie d'un donneur vivant, une fibrose hépatique congénitale, une cholédocholithiase, une maladie du foie granulomateuse, une malignité intrahépatique ou extrahépatique, le syndrome de Sjogren, une sarcoïdose, la maladie de Wilson, la maladie de Gaucher, une hémochromatose, et une carence en alpha 1-antitrypsine.

18. Composé selon l'une quelconque des revendications 1 à 14 ou composition pharmaceutique selon la revendication 15, pour une utilisation dans un procédé de traitement, de prévention ou de réduction du risque d'une maladie ou d'un trouble associé(e) à un dysfonctionnement mitochondrial, le procédé comprenant l'administration au sujet souffrant de, ou susceptible de développer, un trouble métabolique, d'une quantité thérapeutiquement efficace du composé,
ledit trouble associé à un dysfonctionnement mitochondrial étant une maladie mitochondriale héritée, un trouble métabolique commun, une maladie neurodégénérative, un trouble lié au vieillissement, un trouble des reins, ou une maladie inflammatoire chronique ; éventuellement, le trouble métabolique commun étant l'obésité ou le diabète de type II.

19. Composé pour une utilisation selon la revendication 18, le trouble métabolique étant le diabète de type II, l'obésité, une hyperglycémie, une intolérance au glucose, une résistance à l'insuline, une hypercholestérolémie, l'hypertension, une hyperlipoprotéinémie, une hyperlipidémie, une hypertriglycéridémie, une maladie cardiovasculaire, l'athérosclérose, une maladie vasculaire périphérique, une maladie des reins, une acidocétose, des troubles thrombotiques, une néphropathie, une neuropathie diabétique, une rétinopathie diabétique, un dysfonctionnement sexuel, une dermatopathie, une dyspepsie, une hypoglycémie, un cancer ou un œdème ;
et/ou la maladie neurodégénérative étant une dégénérescence de photorécepteurs, une démence, la maladie d'Alzheimer, la maladie de Parkinson ou la maladie de Huntington ;
et/ou, la maladie inflammatoire chronique étant la maladie cœliaque, une vascularite, un lupus, une maladie pulmonaire obstructive chronique (COPD), une maladie de l'intestin irritable, l'athérosclérose, l'arthrite ou le psoriasis ;
et/ou, la maladie des reins étant une lésion rénale aiguë (AKI) ou une maladie chronique des reins (CKD) ;
et/ou, la maladie associée au vieillissement étant un cancer, une démence, une maladie cardiovasculaire, l'hypertension, un diabète sucré (type I ou type II), l'arthrite, une cataracte, la maladie d'Alzheimer, une dégénérescence maculaire ou l'ostéoporose.

20. Composé pour une utilisation dans un procédé de traitement, de prévention ou de réduction du risque d'une maladie ou d'un trouble associé(e) à un dysfonctionnement de l'α-amino-β-carboxymuconate-ε-semialdéhyde décarboxylase (ACMSD), le procédé comprenant l'administration au sujet souffrant de, ou susceptible de développer, une maladie ou un trouble associé (e) à un dysfonctionnement de l'ACMSD, d'une quantité thérapeutiquement efficace du composé, le composé étant représenté par la Formule (II) : 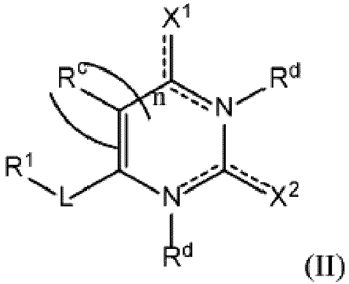 ou un sel ou une forme tautomère pharmaceutiquement acceptable correspondant(e),
X¹ étant H, O, S, OR², ou SH ;
X² étant O, S, OR², ou SR² ;
L étant -(CH₂)ₘCH=CH(CH₂)ₚ-, -(CH₂)*ₒ*-, 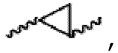 (CH₂) ₘC=(O)NR³(CH₂)ₚ-, -(CH₂)ₘNR³C=(O)(CH₂)ₚ-, pyridinyle, ou thiophényle ;
Y² étant O, NH ou S ;
R¹ étant C₆-C₁₀ aryle ou hétéroaryle, l'hétéroaryle comprenant un ou deux cycles à 5 à 7 chaînons et 1 à 4 hétéroatomes choisis parmi N, O et S, et l'aryle et l'hétéroaryle étant substitués par R^{a} et R^{b}, et éventuellement substitués par un à deux R^{e} ;
R² étant H ou C₁-C₄ alkyle ;
R³ étant H ou C₁-C₄ alkyle ;
R^{a} étant H, C₁-C₄ alkyle, -(C(R^{f})₂)ᵣCO₂R^{x}, - Y²(C(R^{f})₂)ᵣC₂R^{x}, -O(C(R^{f})₂)ᵣC(O)NHR^{g}, -(C(R^{f})₂)ᵣC₆-C₁₀ aryle, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -O(C(R^{f})₂)ᵣ(C₃-C₇) cycloalkyle, -(C(R^{f})₂)ᵣP(O)(OH)OR^{x},-O(C(R^{f})₂)ᵣP(O)(OH)OR^{x}, -(C(R^{f})₂)ᵣS(O)₂OH,-O(C(R^{f})₂)ᵣS(O)₂OH, -(C(R^{f})₂)ᵣP(O)₂OH,-O(C(R^{f})₂)ᵣP(O)₂OH, -O(C(R^{f})₂)ᵣOH, -OR^{y},-(C(R^{f})₂)ᵣC(O)NHCN, -CH=CHCO₂R^{x}, ou-(C(R^{f})₂)ᵣC(O)NHS(O)₂alkyle, l'aryle et l'hétéroaryle étant éventuellement substitués par un à trois substituants chacun indépendamment choisi parmi halogène et OH, et l'hétérocycloalkyle étant substitué par un à deux =O ou =S ;
R^{b} étant C₁-C₄ alkyle, -(C(R^{f})₂)ᵣCO₂R^{x},-Y²(C(R^{f})₂)ᵣCO₂R^{x}, -O(C(R^{f})₂)ᵣC(O)NHR^{g}, -(C(R^{f})₂)ᵣC₆-C₁₀ aryle, -(C(R^{f})₂)ᵣS-C₆-C₁₀ aryle,-(C(R^{f})₂)ᵣhétéroaryle, -O(C(R^{f})₂)ᵣhétéroaryle,-O(C(R^{f})₂)ᵣhétérocycloalkyle, -(C(R^{f})₂)ᵣP(O)(OH)OR^{x},-O(C(R^{f})₂)ᵣP(O)(OH)OR^{x}, -(C(R^{f})₂)ᵣS(O)₂OH,-O(C(R^{f})₂)ᵣS(O)₂OH, -(C(R^{f})₂)ᵣP(O)₂OH,-O(C(R^{f})₂)ᵣP(O)₂OH,- O(C(R^{f})₂)ᵣOH, -(C(R^{f})₂)ᵣC(O)NHCN, -CH=CHCO₂R^{×}, ou -(C(R^{f})₂)ᵣC(O)NHS(O)₂alkyle, l'aryle et l'hétéroaryle étant substitués par un à trois substituants choisis parmi halogène et OH, et l'hétérocycloalkyle étant substitué par un à deux =O ou =S ; ou
R^{a} et R^{b}, lorsqu'ils sont sur des atomes adjacents, formant, conjointement avec les atomes auxquels ils sont fixés, un cycle C₆-C₁₀ aryle éventuellement substitué par un ou plusieurs - CO₂H; R^{a} et R^{b}, lorsqu'ils sont sur des atomes adjacents, formant, conjointement avec les atomes auxquels ils sont fixés, un cycle hétéroaryle à 5 à 6 chaînons éventuellement substitué par un ou plusieurs -CO₂H ;
R^{c} étant -CN ou -NO₂ ;
chaque R^{d} étant indépendamment en chaque occurrence absent ou H ;
chaque R^{e} étant indépendamment en chaque occurrence C₁-C₆ alkyle, C₂-C₆ alcényle, C₂-C₆ alcynyle, halogène, C₁-C₆ halogénoalkyle, -NHR^{z},-OH, ou -CN ;
chaque R^{f} étant indépendamment H ou C₁-C₆ alkyle ;
R^{g} étant H, C₁-C₆ alkyle, OH, -S(O)₂(C₁-C₆ alkyle), ou -S(O)₂N(C₁-C₆ alkyle)₂ ;
R^{x} étant H ou C₁-C₆ alkyle ;
chaque R^{y} et R^{z} étant indépendamment H, C₁-C₆ alkyle, ou C₁-C₆ halogénoalkyle ;
chaque m, p, et r étant indépendamment 0, 1 ou 2 ;
n étant 1 ;
o étant 1, 2, 3, ou 4 ; et
la ligne en pointillés étant une double liaison éventuelle ;
ladite maladie étant une maladie chronique du foie choisie parmi une cirrhose biliaire primaire, (PBC), une xanthomatose cérébrotendineuse (CTX), une cholangite sclérosante primaire (PSC), une cholestase induite par un médicament, une cholestase intrahépatique de grossesse, une cholestase associée à la nutrition parentérale (PNAC), une cholestase associée à une surcroissance bactérienne ou une septicémie, une hépatite auto-immune, une hépatite virale chronique, une maladie du foie alcoolique, une maladie du foie gras non alcoolique (NAFLD), une stéatohépatite non alcoolique (NASH), une maladie de greffon contre l'hôte associée à une greffe de foie, une régénération du foie d'une greffe de foie d'un donneur vivant, une fibrose hépatique congénitale, une cholédocholithiase, une maladie du foie granulomateuse, une malignité intrahépatique ou extrahépatique, le syndrome de Sjogren, une sarcoïdose, la maladie de Wilson, la maladie de Gaucher, une hémochromatose, et une carence en alpha 1-antitrypsine.

21. Composé pour une utilisation selon la revendication 20, possédant l'une quelconque des formules suivantes :
